# EUROPEAN PATENT APPLICATION

(11) **EP 4 623 914 A1**
(43) Date of publication of application: **01.10.2025**
(21) Application number: 23893774.2
(22) Date of filing: 20.11.2023
(51) Int. Cl.: A61K 31/47, A61K 31/496, C07D 215/28, C07D 401/04, A61P 31/20

(54) **USE OF 8-HYDROXYQUINOLINE DERIVATIVE**

(30) Priority: 22.11.2022 CN 202211468308
(71) Applicant: Jiangsu Yahong Meditech Co., Ltd., Taizhou, Jiangsu 225316 (CN); Asieris Pharmaceuticals (Shanghai) Co., Ltd., Pudong, Shanghai 201203 (CN)
(72) Inventor: DENG, Yijun, Shanghai 201203 (CN)
(74) Representative: Regimbeau
(86) International application number: PCT/CN2023/132589
(87) International publication number: WO 2024/109684

(57) **Abstract**

The present invention relates to use of a 8-hydroxyquinoline derivative, particularly, to use of a 8-hydroxyquinoline derivative represented by general formula (I), a pharmaceutically acceptable salt thereof, a crystal form thereof, a solvate thereof, an isotope derivative thereof, or a prodrug thereof in the preparation of an antiviral drug, and particularly, to use thereof in the preparation of an anti-human papillomavirus drug. The definition of each group in the general formula (I) is the same as that in the description.

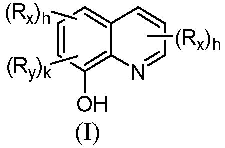

## Description

### FIELD OF THE INVENTION

The present invention relates to the use of an 8-hydroxyquinoline derivative. Particularly, the present invention relates to the use of an 8-hydroxyquinoline derivative of formula (I), a pharmaceutically acceptable salt thereof, a crystal form thereof, a solvate thereof, an isotope derivative thereof, or a prodrug thereof in the preparation of an antiviral drug, and particularly in the preparation of an anti-human papillomavirus drug.

### BACKGROUND OF THE INVENTION

Human papillomavirus (*HPV*) is a DNA virus belonging to the family *Papillomaviridae* and is a very common virus worldwide, and the members thereof have mucosal epithelial and skin epithelial tropism. In addition to a variety of reproductive tract diseases such as genital warts, they can cause a variety of malignant tumors, including cervical cancer, penile cancer, anal cancer, oral cancer, pharyngeal and laryngeal cancer, tonsil cancer, esophageal cancer, etc. There are more than 100 types of human papillomavirus, among which at least a dozen types can cause cancer, making it the most important kind of human tumor virus. The virus has high infection rate and strong pathogenicity, is very harmful to the population, especially women, and has attracted extensive attention from domestic and international medical communities. At present, clinically, there have been prophylactic multivalent vaccines (*HPV6*, *HPV11*, *HPV16, HPV18,* etc.) that can prevent infection with multiple types of virus, but the use of prophylactic vaccines for infected patients has no significant therapeutic effect. The development of effective drugs that target multiple types of *HPV* can provide new methods for the clinical treatment and prevention of diseases related to human papillomavirus, including malignant tumors.

### SUMMARY OF THE INVENTION

The present invention relates to a use of a compound of formula (I), a pharmaceutically acceptable salt thereof, a crystal form thereof, a solvate thereof, an isotope derivative thereof, or a prodrug thereof in the preparation of an antiviral drug, and especially in the preparation of an anti-human papillomavirus (*HPV*) drug.

The present invention solves the above technical problem through the following technical solutions:
Provided is a use of a compound of formula (I), a pharmaceutically acceptable salt thereof, a crystal form thereof, a solvate thereof, an isotope derivative thereof, or a prodrug thereof in the preparation of an antiviral drug, 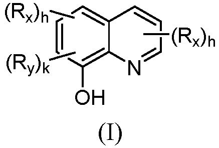 wherein:
   Rₓ is each independently optional, and h is each independently 1, 2 or 3;
   R_{y} is selected from the group consisting of nitro and cyano, and k is 1 or 2;
   provided that not all Rₓ are H atoms simultaneously.

In one embodiment, provided is a use of the compound of formula (I), the pharmaceutically acceptable salt thereof, the crystal form thereof, the solvate thereof, the isotope derivative thereof, or the prodrug thereof which is a compound of formula (IA), a pharmaceutically acceptable salt thereof, a crystal form thereof, a solvate thereof, an isotope derivative thereof, or a prodrug thereof, in the preparation of an antiviral drug, 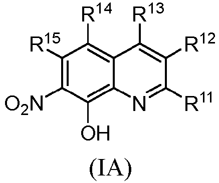 wherein:
R¹¹ is selected from the group consisting of H atom, halogen, D atom and C₁₋₆ alkyl;
R¹² is selected from the group consisting of H atom, C₁₋₆ alkyl, C₃₋₈ cycloalkyl, C₆₋₁₄ aryl, -C(O)-OR¹⁶, -CH₂-NR¹⁷R¹⁸ and -CH₂-NR¹⁹R¹¹⁰;
R¹³ is selected from the group consisting of H atom, D atom, C₁₋₆ alkyl, halogen, C₁₋₆ alkoxy, hydroxy, haloC₁₋₆ alkyl, haloC₁₋₆ alkoxy, cyano, C₃₋₈ cycloalkyl, -(CH₂)ₙ-3 to 8 membered heterocyclyl, C₆₋₁₄ aryl, 5 to 14 membered heteroaryl, -NR¹⁷R¹⁸, -O-(CH₂)ₙ-NR¹⁷R¹⁸, -O-(CH₂)ₙ-R¹¹¹, -(CH=CH)ₘC(O)-NR¹⁷R¹⁸ and -(CH=CH)ₘC(O)-NR¹⁹R¹¹⁰, wherein the 3 to 8 membered heterocyclyl is optionally further substituted by one or more substituents selected from the group consisting of C₁₋₆ alkyl, C₁₋₆ alkoxy, halogen, haloC₁₋₆ alkyl, oxo, -C(O)-NR¹⁷R¹⁸, -C(O)-OR¹⁶, -C(O)-R¹⁶, -S(O)ₓR¹⁶, -O-(CH₂)ₙ-R¹¹¹ and C₆₋₁₄ aryl;
R¹⁴ is selected from the group consisting of H atom, halogen, cyano, C₁₋₆ alkyl, C₃₋₈ cycloalkyl, haloC₁₋₆ alkyl, hydroxyC₁₋₆ alkyl, -CH₂-NR¹⁷R¹⁸ and -CH₂-NR¹⁹R¹¹⁰;
R¹⁵ is selected from the group consisting of H atom, D atom, halogen, C₁₋₆ alkyl and -NR¹⁷R¹⁸;
R¹⁶ is C₁₋₆ alkyl;
R¹⁷ and R¹⁸ are each independently selected from the group consisting of H atom, C₁₋₆ alkyl and 3 to 8 membered heterocyclyl;
R¹⁹ and R¹¹⁰ together with the nitrogen atom to which they are attached form a 3 to 8 membered nitrogen-containing heterocyclyl, the 3 to 8 membered nitrogen-containing heterocyclyl optionally comprises one or more heteroatoms selected from the group consisting of N, O and S in addition to N, and the 3 to 8 membered nitrogen-containing heterocyclyl is optionally further substituted by one or more oxo groups;
R¹¹¹ is selected from the group consisting of halogen, C₃₋₈ cycloalkyl, C₆₋₁₄ aryl, haloC₁₋₆ alkyl, C₁₋₆ alkyl and C₁₋₆ alkoxy;
m is 1;
n is 0, 1, 2 or 3; and
x is 2.

In one embodiment, in the compound of formula (IA), the pharmaceutically acceptable salt thereof, the crystal form thereof, the solvate thereof, the isotope derivative thereof, or the prodrug thereof:
R¹¹ is selected from the group consisting of H atom, F atom, -CH₃ and D atom;
and/or, R¹² is selected from the group consisting of H atom, -CH₃, 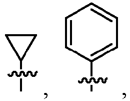 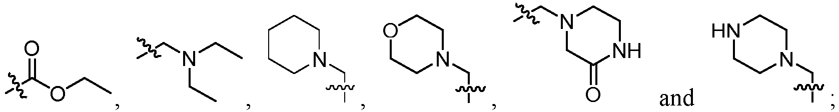
and/or, R¹³ is selected from the group consisting of H atom, -CH₃, 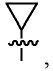 Cl atom, 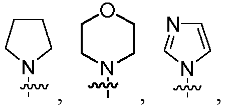 Br atom, 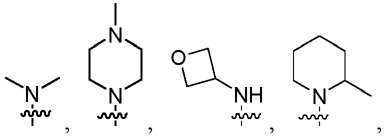 -OCH₃, 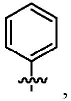 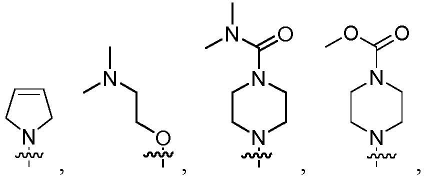 F atom, 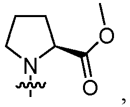 -OH, 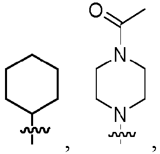 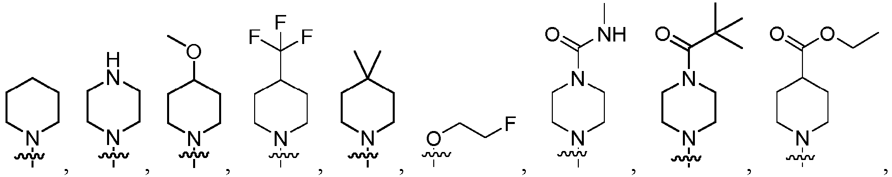 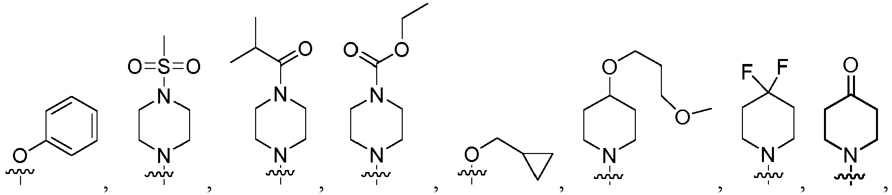 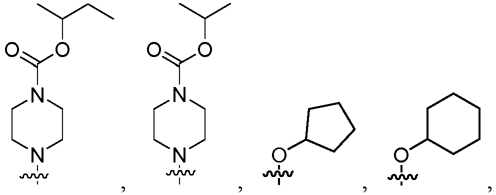 -OCH₂CH₃, -CF₃, 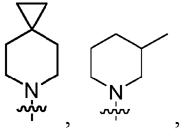 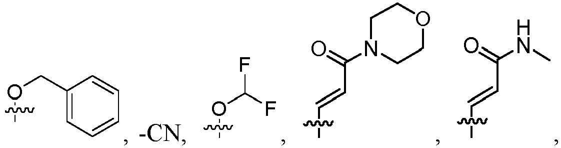 -CHF₂, 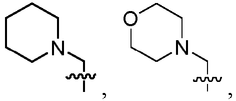 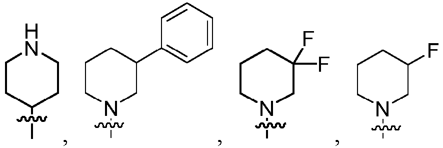 and D atom;
and/or, R¹⁴ is selected from the group consisting of H atom, I atom, -CH₃, Cl atom, -CN, F atom, 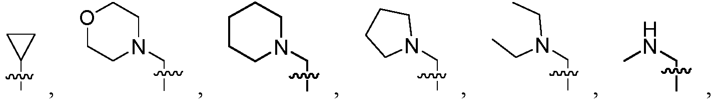 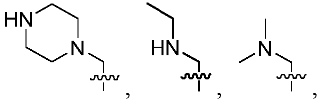 -CHF₂, -CH₂OH and Br atom;
and/or, R¹⁵ is selected from the group consisting of H atom, -CH₃, F atom, Br atom, Cl atom, D atom and -NHCH₃.

In one embodiment, in the compound of formula (IA), the pharmaceutically acceptable salt thereof, the crystal form thereof, the solvate thereof, the isotope derivative thereof, or the prodrug thereof, the compound of formula (IA) is selected from the group consisting of:

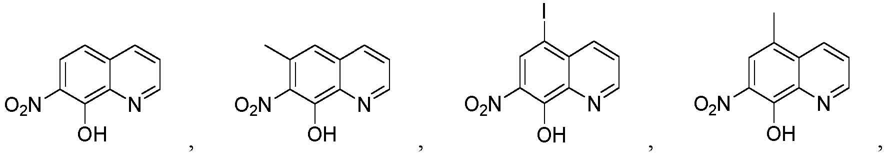

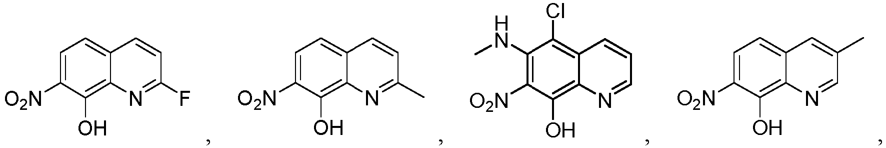

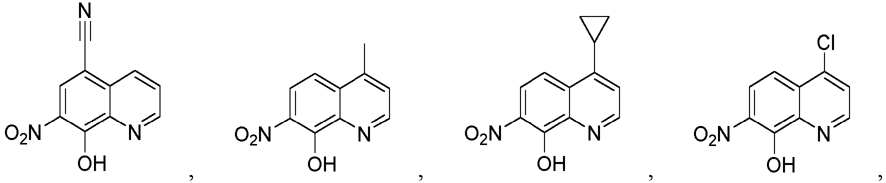

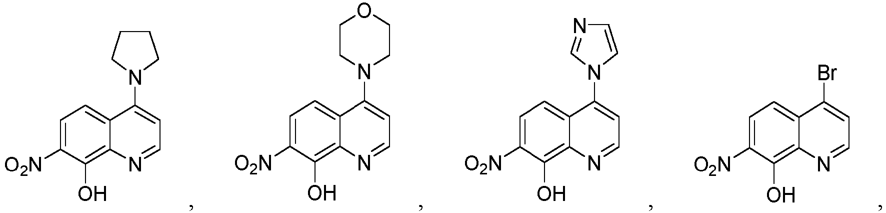

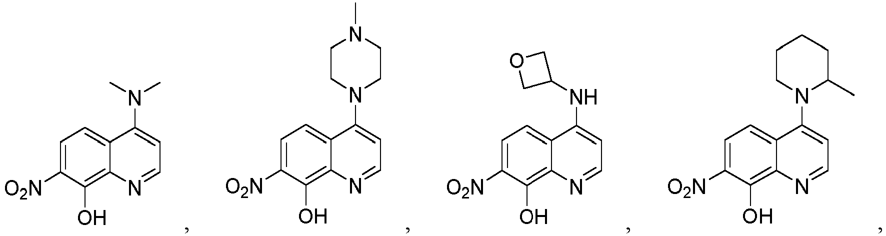

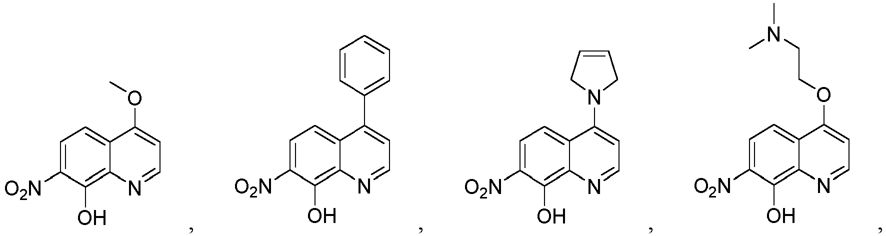

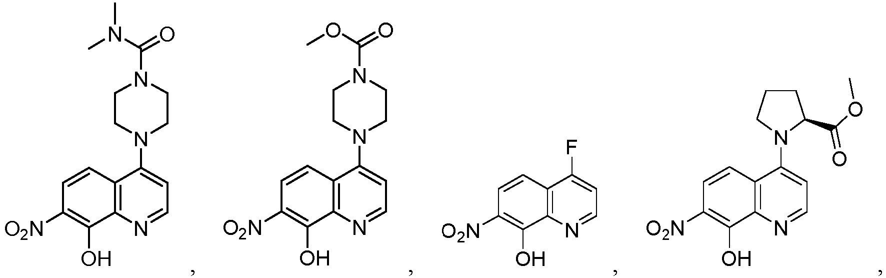

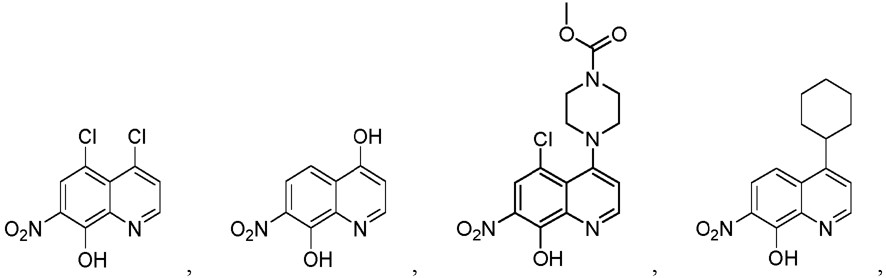

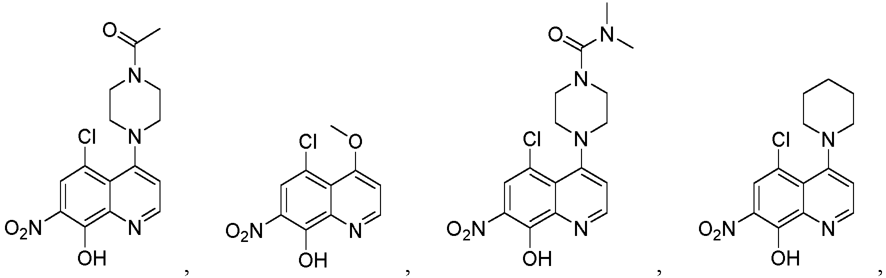

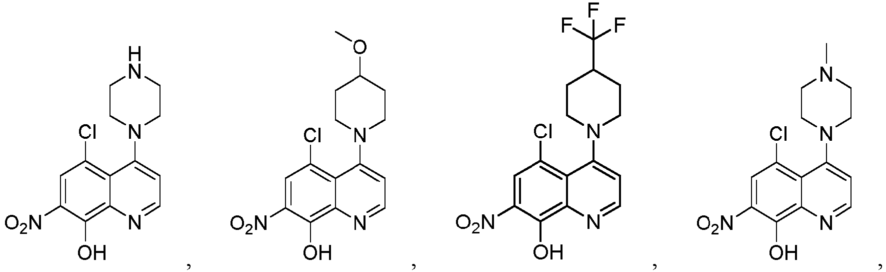

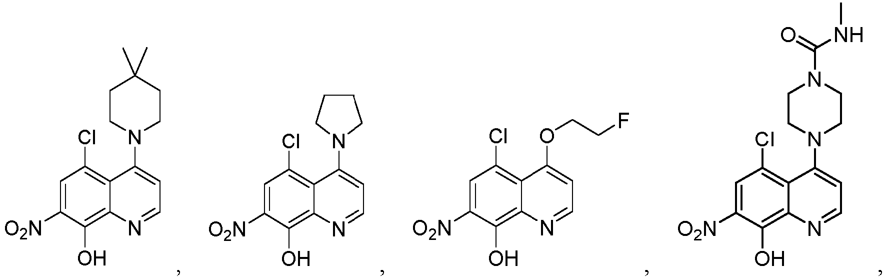

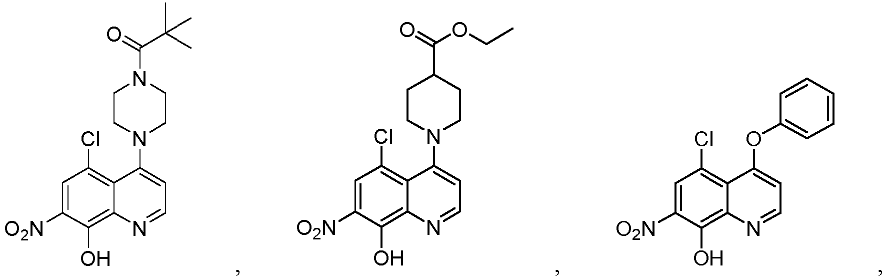

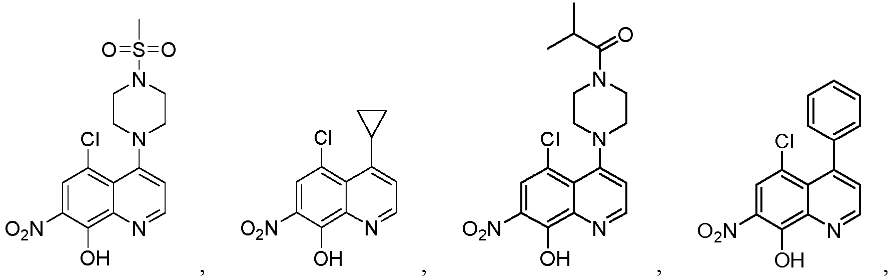

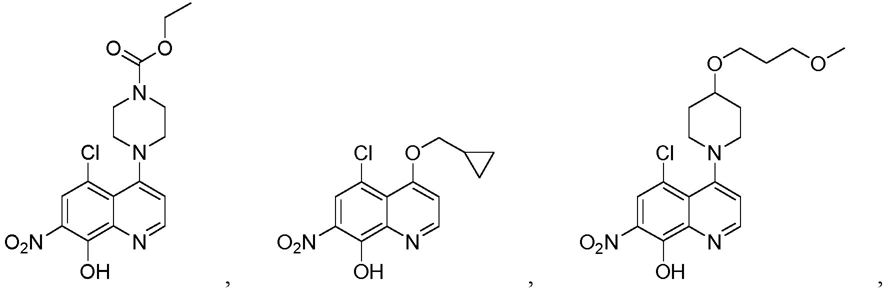

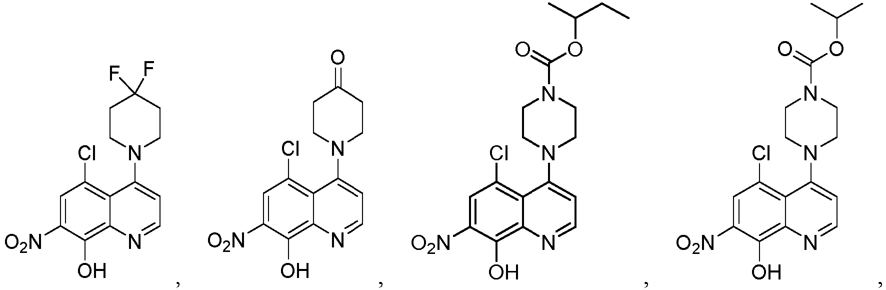

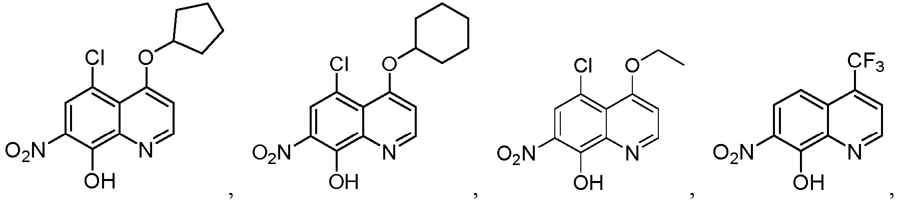

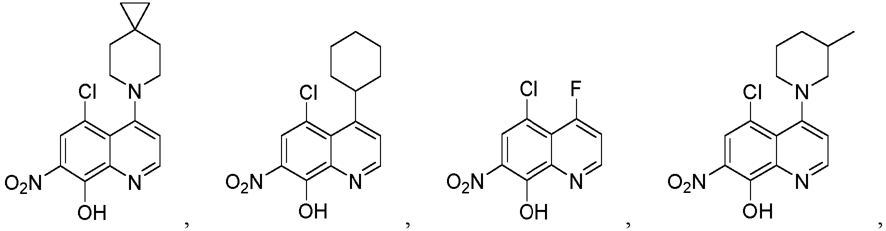

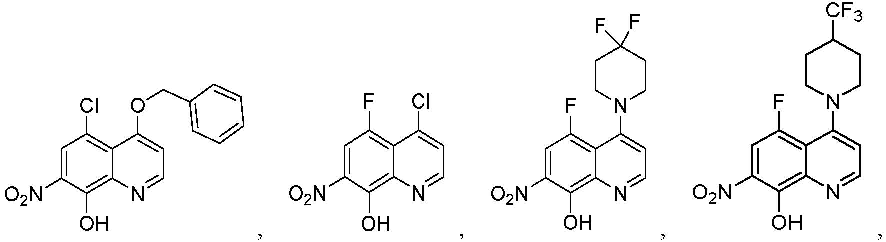

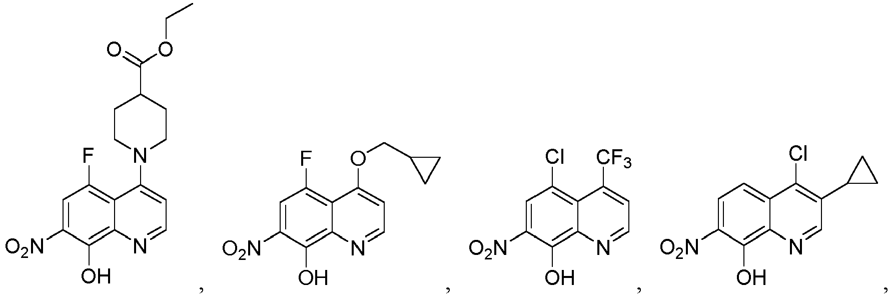

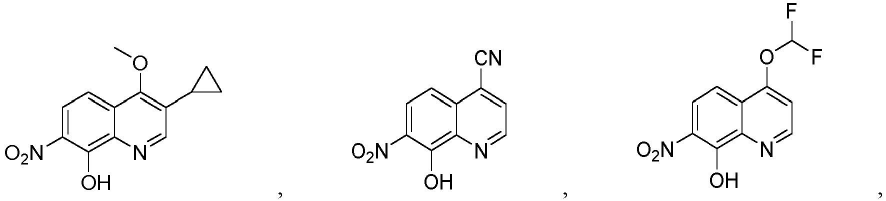

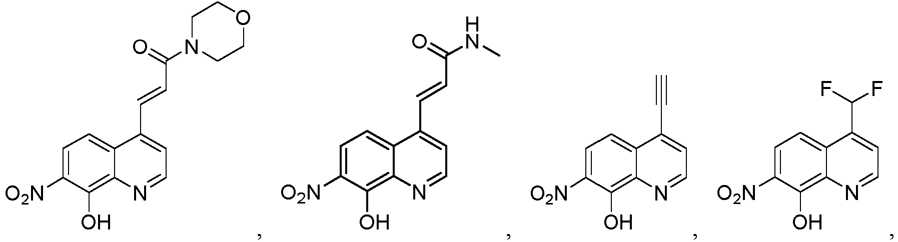

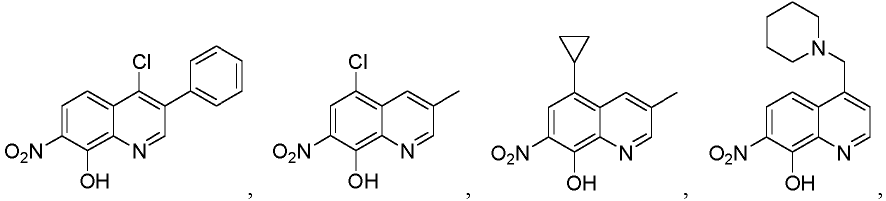

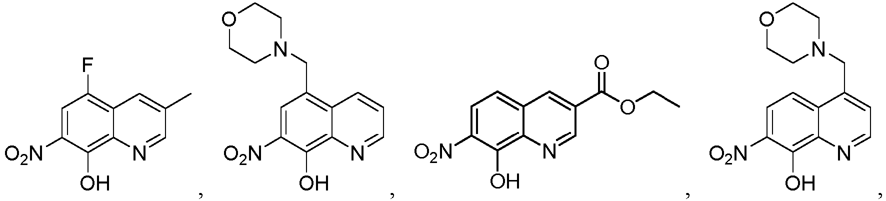

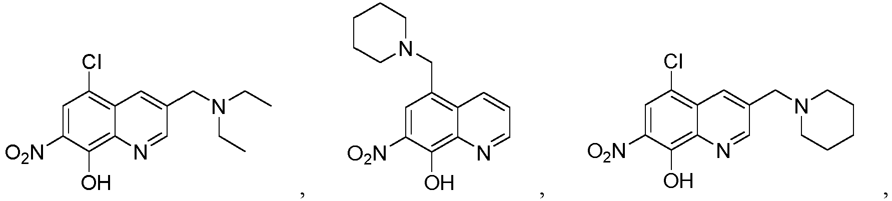

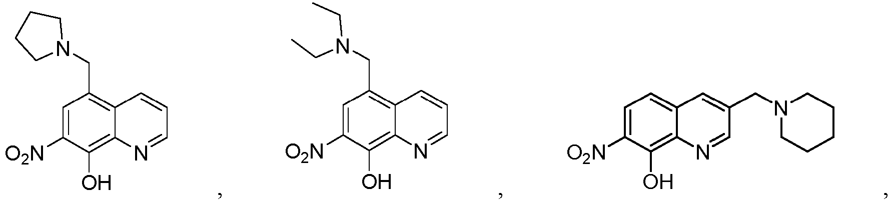

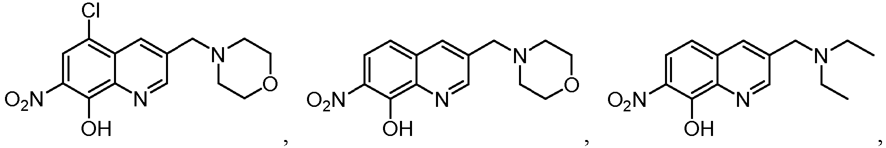

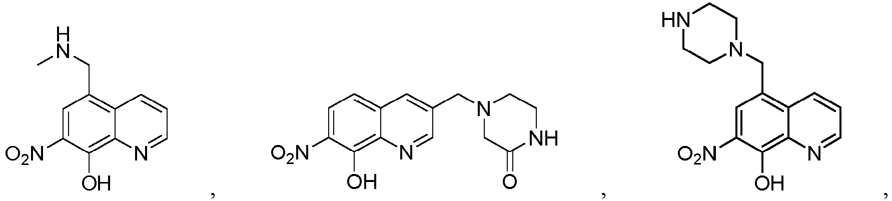

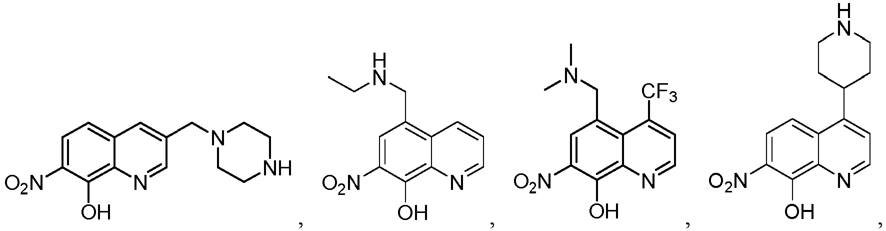

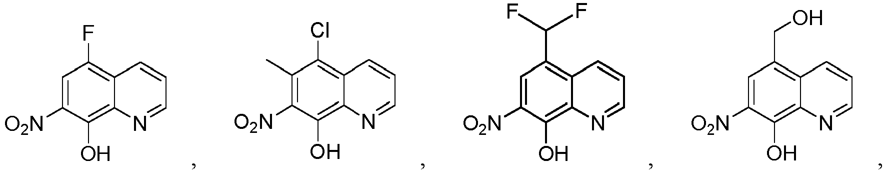

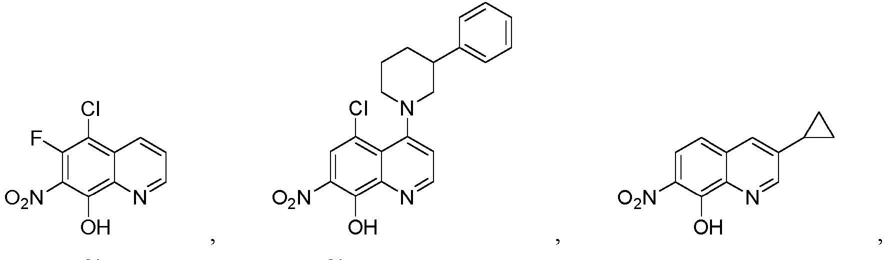

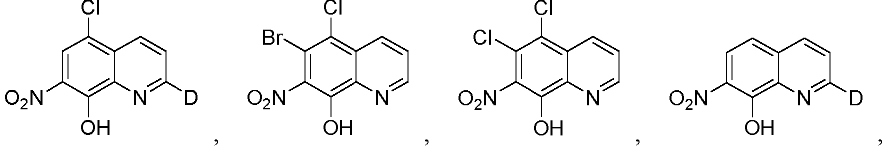

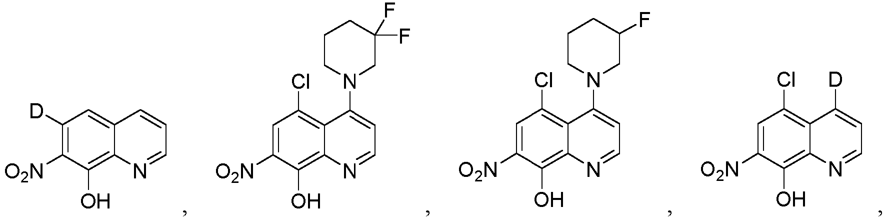

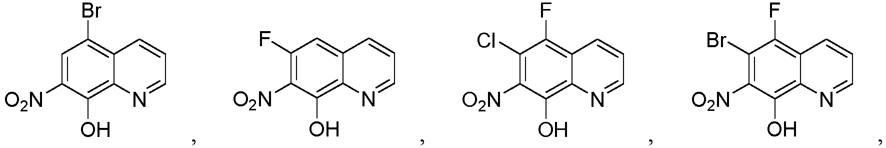

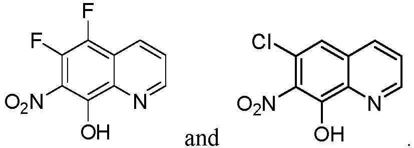

In one embodiment, in the compound of formula (IA), the pharmaceutically acceptable salt thereof, the crystal form thereof, the solvate thereof, the isotope derivative thereof, or the prodrug thereof:
R¹¹ is selected from the group consisting of H atom, halogen, D atom and C₁-C₆ alkyl;
R¹² is H atom, C₁-C₆ alkyl or C₃₋₈ cycloalkyl;
R¹³ is selected from the group consisting of H atom, D atom, C₁-C₆ alkyl, halogen, C₁₋₆ alkoxy, hydroxy, cyano, haloC₁₋₆ alkyl and C₃₋₈ cycloalkyl;
R¹⁴ is selected from the group consisting of H atom, halogen, cyano, haloC₁₋₆ alkyl and C₁-C₆ alkyl;
R¹⁵ is selected from the group consisting of H atom, D atom, halogen, C₁-C₆ alkyl and -NR¹⁷R¹⁸;
R¹⁷ and R¹⁸ are each independently H atom or C₁₋₆ alkyl.

In one embodiment, in the compound of formula (IA), the pharmaceutically acceptable salt thereof, the crystal form thereof, the solvate thereof, the isotope derivative thereof, or the prodrug thereof, the compound of formula (IA) is selected from the group consisting of: 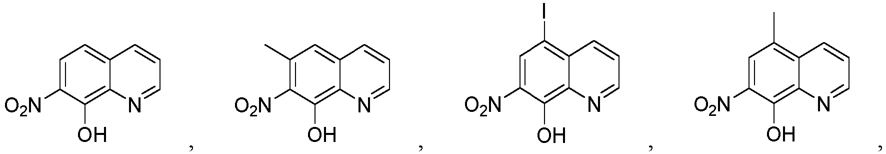 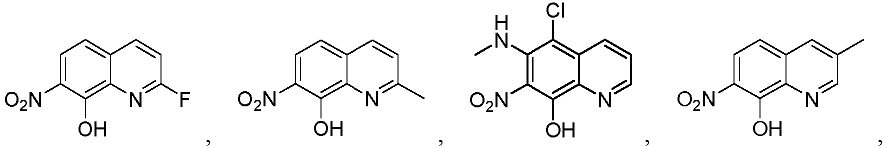 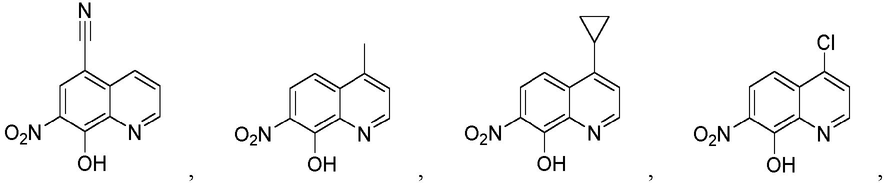 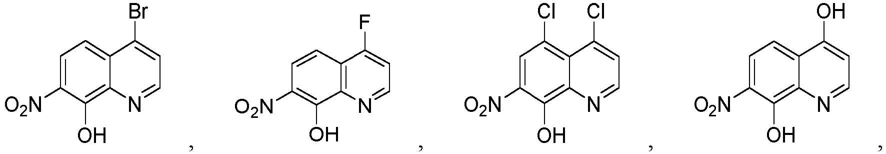 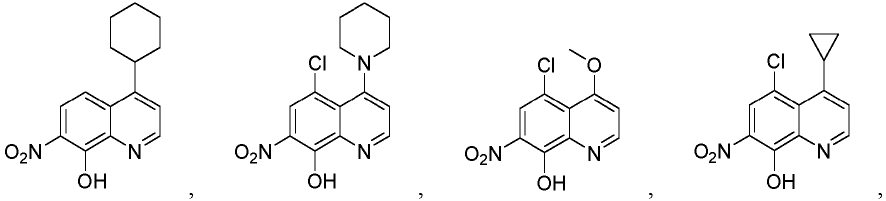 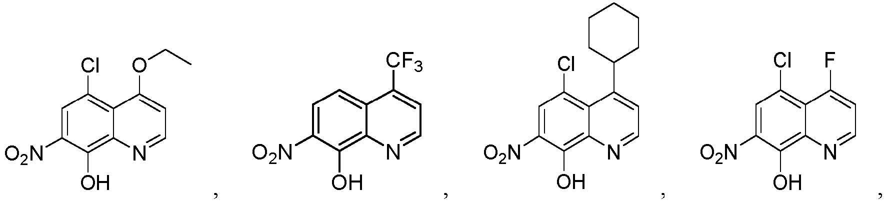 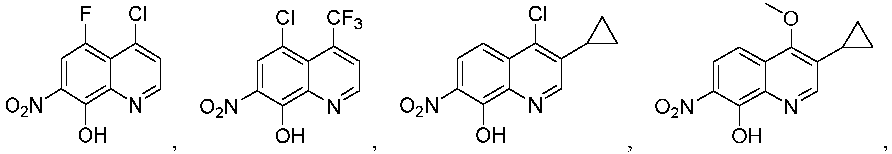 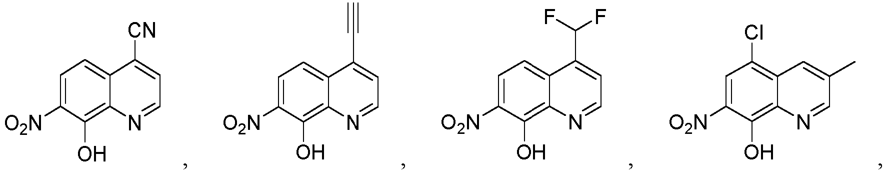 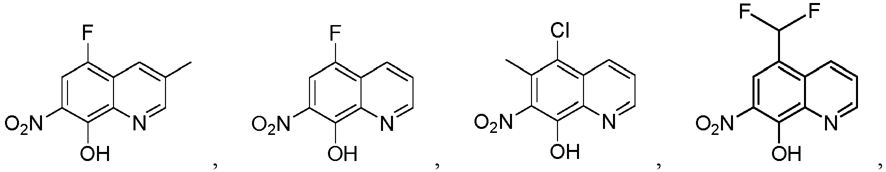 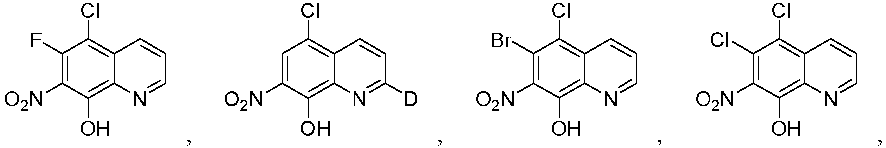 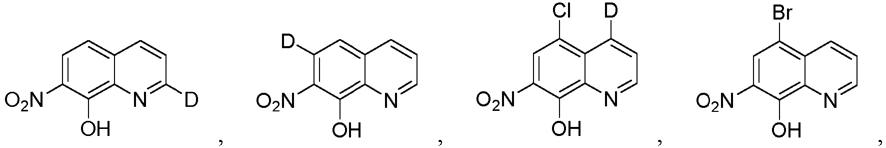 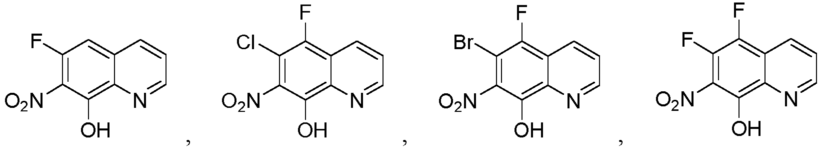 and

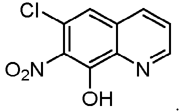

In one embodiment, in the compound of formula (IA), the pharmaceutically acceptable salt thereof, the crystal form thereof, the solvate thereof, the isotope derivative thereof, or the prodrug thereof:
R¹¹ is H atom;
R¹² is H atom or C₃₋₈ cycloalkyl;
R¹³ is selected from the group consisting of H atom, haloC₁₋₆ alkyl and C₃₋₈ cycloalkyl;
R¹⁴ is H atom or halogen;
R¹⁵ is selected from the group consisting of H atom, halogen and -NR¹⁷R¹⁸;
R¹⁷ and R¹⁸ are each independently H atom or C₁₋₆ alkyl.

In one embodiment, in the compound of formula (IA), the pharmaceutically acceptable salt thereof, the crystal form thereof, the solvate thereof, the isotope derivative thereof, or the prodrug thereof, the compound of formula (IA) is selected from the group consisting of: 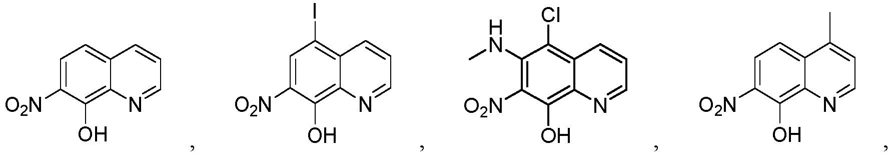 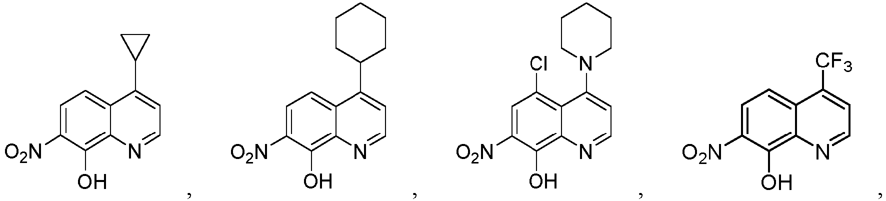 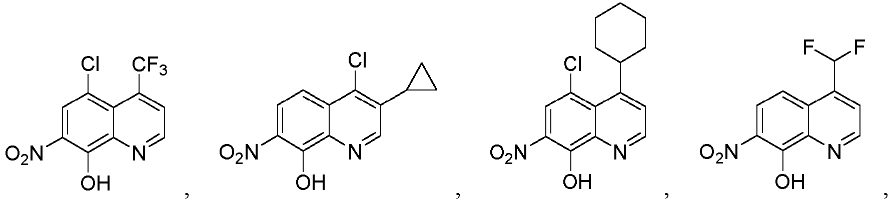 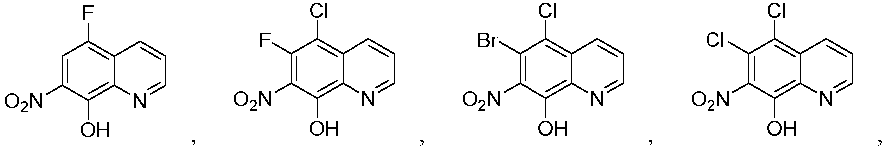 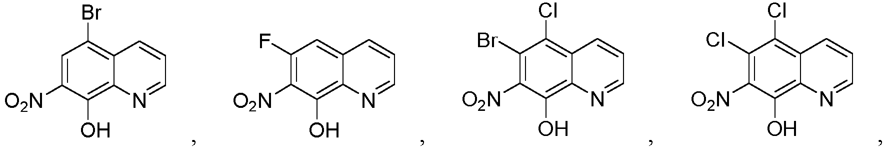 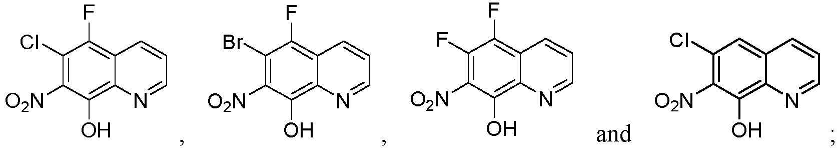 and preferably selected from the group consisting of 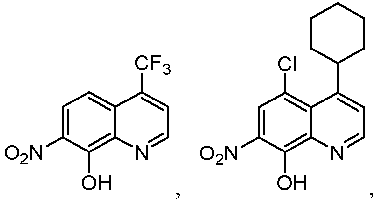 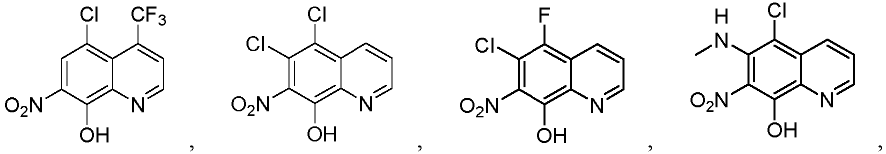 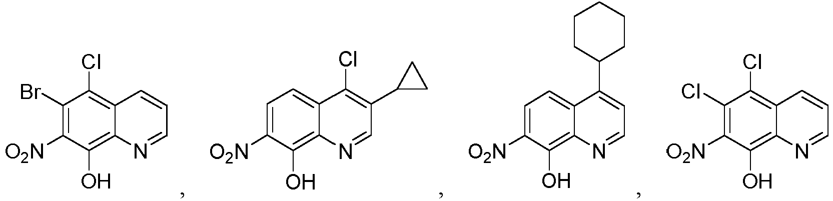 and

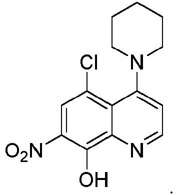

In another embodiment, provided is a use of the compound of formula (I), the pharmaceutically acceptable salt thereof, the crystal form thereof, the solvate thereof, the isotope derivative thereof, or the prodrug thereof which is a compound of formula (IB), a pharmaceutically acceptable salt thereof, a crystal form thereof, a solvate thereof, an isotope derivative thereof, or a prodrug thereof, in the preparation of an antiviral drug, 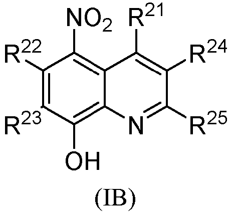 wherein:
R²¹ is selected from the group consisting of hydrogen atom, halogen, cyano, alkyl, alkenyl, cycloalkyl, heterocyclyl, aryl, heteroaryl, -C(O)OR^{a}, -NR^{a}R^{b}, -OR^{a} and -S(O)ₚR^{a}; wherein the alkyl, alkenyl, cycloalkyl, heterocyclyl, aryl and heteroaryl are each optionally further substituted by one or more groups selected from Q;
R²² is selected from the group consisting of hydrogen atom, halogen, alkyl and cycloalkyl;
R²³ is selected from the group consisting of hydrogen atom, halogen and alkyl;
R²⁴ is selected from the group consisting of hydrogen atom, halogen, alkyl, - C(O)OR^{a} and -C(O)NR^{a}R^{b}, wherein R^{a} and R^{b} are each independently selected from the group consisting of hydrogen atom, C₁-C₆ alkyl, C₃-C₆ cycloalkyl, C₃-C₆ cycloalkyl-C₁-C₆ alkyl;
R²⁵ is selected from the group consisting of hydrogen atom, halogen and alkyl;
R^{a} and R^{b} are each independently selected from the group consisting of hydrogen atom, alkyl and cycloalkyl;
Q is halogen, alkyl, oxo, -C(O)R^{c} , -C(O)OR^{c}, -C(O)NR^{c}R^{d} or -C(O)N(R^{c})(CH₂)_{q}R^{d};
R^{c} and R^{d} are each independently selected from the group consisting of hydrogen atom, alkyl, aryl and heteroaryl, wherein the alkyl, aryl and heteroaryl are each optionally further substituted by alkoxy;
or, R^{c} and R^{d} together with the nitrogen atom to which they are attached form a nitrogen-containing heterocyclyl, and the nitrogen-containing heterocyclyl optionally comprises one or more heteroatoms selected from the group consisting of N and O in addition to N;
p is 1 or 2;
q is an integer from 0 to 6;
provided that R²¹, R²², R²³, R²⁴ and R²⁵ are not hydrogen atoms simultaneously.

In another embodiment, in the compound of formula (IB), the pharmaceutically acceptable salt thereof, the crystal form thereof, the solvate thereof, the isotope derivative thereof, or the prodrug thereof:
R²¹ is as defined above, and the halogen is chlorine or fluorine;
and/or, R²¹ is as defined above, and the alkyl is C₁-C₆ alkyl; the C₁-C₆ alkyl is preferably C₁-C₆ alkyl substituted by halogen, more preferably -CF₃, -CHF₂ or -CH₂F, and further more preferably -CF₃;
and/or, R²¹ is as defined above, and the alkenyl is C₂-C₆ alkenyl; preferably, the C₂-C₆ alkenyl is alkenyl substituted by one or more groups selected from Q, Q is -C(O)R^{c}, - C(O)OR^{c}, -C(O)NR^{c}R^{d} or -C(O)N(R^{c})(CH₂)_{q}R^{d}, R^{c} and R^{d} are as defined above; and more preferably, the C₂-C₆ alkenyl is -CH=CH-COOH, 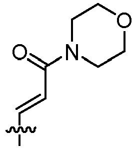 or 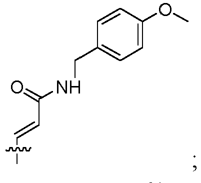
and/or, R²¹ is as defined above, and the cycloalkyl is C₃-C₁₀ cycloalkyl, preferably C₃-C₆ cycloalkyl, more preferably unsubstituted C₃-C₆ cycloalkyl, and further more preferably unsubstituted cyclopropyl, unsubstituted cyclopentyl or unsubstituted cyclohexyl;
and/or, R²¹ is as defined above, and the heterocyclyl is C₄-C₇ heterocyclyl; preferably, the C₄-C₇ heterocyclyl is nitrogen-containing C₄-C₇ heterocyclyl; more preferably, the C₄-C₇ heterocyclyl is -NR^{e}R^{f}, R^{e} and R^{f} together with the nitrogen atom to which they are attached form a nitrogen-containing heterocyclyl, the nitrogen-containing heterocyclyl optionally comprises one or more heteroatoms selected from the group consisting of N and O in addition to N, the nitrogen-containing heterocyclyl is optionally further substituted by one or more groups selected from Q, and Q is C₁-C₆ alkyl, oxo or C₁-C₆ alkoxycarbonyl, wherein the C₁-C₆ alkyl is preferably methyl, the C₁-C₆ alkoxycarbonyl is preferably -C(O)OCH₂CH₃; and further more preferably, the C₄-C₇ heterocyclyl is 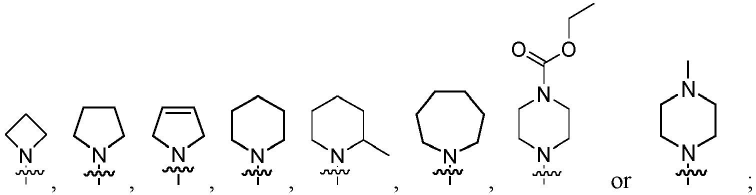
and/or, R²¹ is as defined above, and the aryl is C₆-C₁₀ aryl, and preferably unsubstituted phenyl;
and/or, R²¹ is as defined above, and the heteroaryl is 5 to 10 membered heteroaryl, preferably pyridyl, pyrazolyl or imidazolyl, unsubstituted or substituted by C₁-C₆ alkyl, and more preferably 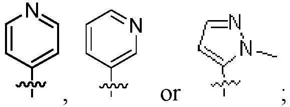
and/or, R²¹ is as defined above, and in the -C(O)OR^{c}, R^{g} is C₁-C₆ alkyl; preferably-C(O)OR^{c} is -C(O)OCH₂CH₃;
and/or, R²¹ is as defined above, and the -NR^{a}R^{b} is -NH₂ or -N(CH₃)₂;
and/or, R²¹ is as defined above, and the -OR^{a} is -OCH₃;
and/or, R²¹ is as defined above, and the -S(O)ₚR^{a} is -S(O)₂CH₃;
and/or, R²² is as defined above, and the halogen is fluorine or chlorine;
and/or, R²² is as defined above, and the alkyl is C₁-C₆ alkyl, and preferably methyl;
and/or, R²² is as defined above, and the cycloalkyl is C₁-C₆ cycloalkyl, and preferably cyclopropyl;
and/or, R²³ is as defined above, and the halogen is fluorine or chlorine;
and/or, R²³ is as defined above, and the alkyl is C₁-C₆ alkyl, and preferably methyl;
and/or, R²⁴ is as defined above, and the halogen is fluorine or chlorine;
and/or, R²⁴ is as defined above, and the alkyl is C₁-C₆ alkyl, and preferably methyl;
and/or, R²⁴ is as defined above, and R^{a} in the -C(O)OR^{a} is alkyl or cycloalkyl, the alkyl is C₁-C₆ alkyl, and preferably ethyl; and the cycloalkyl is C₁-C₆ cycloalkyl, and preferably methylcyclopropyl or cyclohexyl;
and/or, R²⁴ is as defined above, and R^{a} and R^{b} in the -C(O)NR^{a}R^{b} are each independently alkyl, and the alkyl is C₁-C₆ alkyl, and preferably ethyl, methyl or isopropyl;
and/or, R²⁵ is as defined above, and the halogen is fluorine or chlorine;
and/or, R²⁵ is as defined above, and the alkyl is C₁-C₆ alkyl, and preferably methyl;
provided that R²¹, R²², R²³, R²⁴ and R²⁵ are not hydrogen atoms simultaneously.

In another embodiment, in the compound of formula (IB), the pharmaceutically acceptable salt thereof, the crystal form thereof, the solvate thereof, the isotope derivative thereof, or the prodrug thereof, the compound of formula (IB) is selected from the group consisting of:

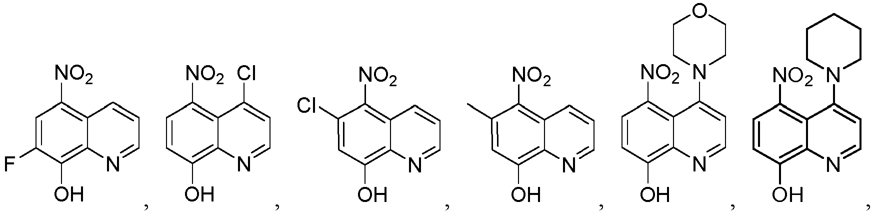

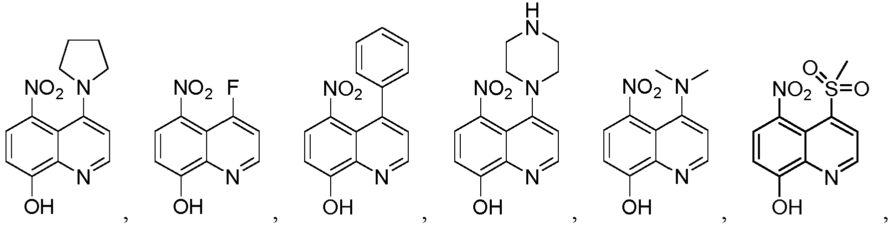

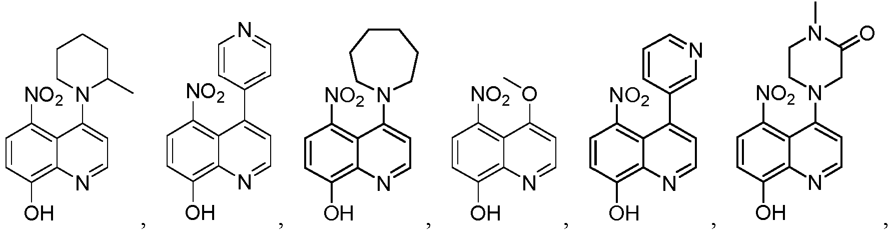

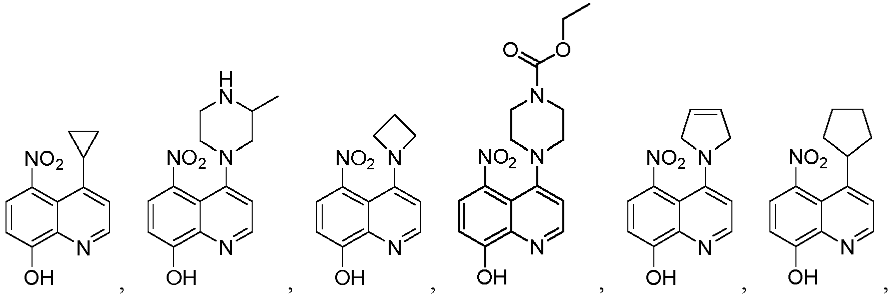

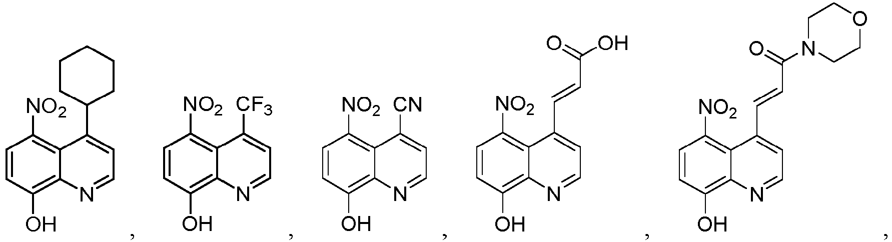

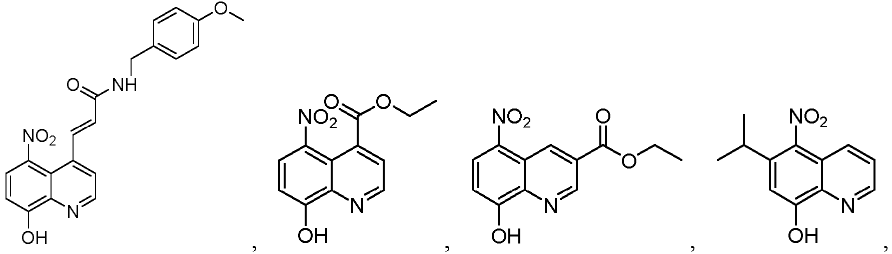

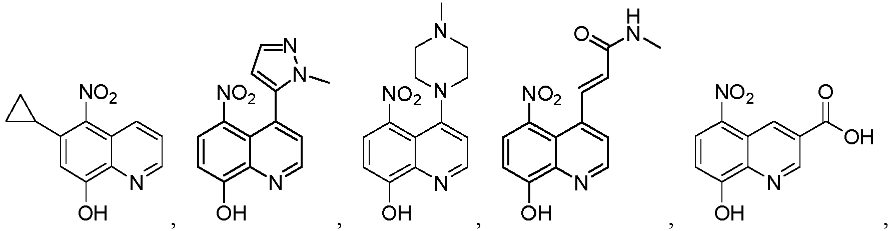

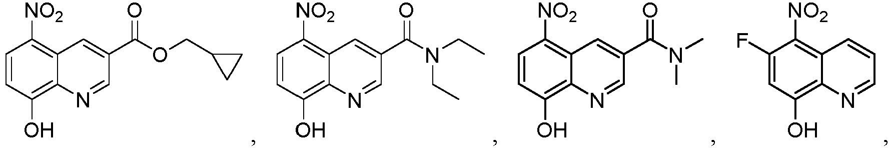

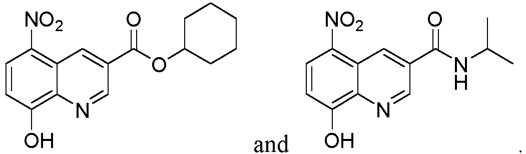

In another embodiment, in the compound of formula (IB), the pharmaceutically acceptable salt thereof, the crystal form thereof, the solvate thereof, the isotope derivative thereof, or the prodrug thereof:
R²¹ is selected from the group consisting of hydrogen atom, halogen, cyano, C₁-C₆ alkyl, 4 to 8 membered heterocyclyl, haloC₁-C₆ alkyl, -C(O)OR^{a} and C₃-C₆ cycloalkyl;
R²² is selected from the group consisting of hydrogen atom, halogen and C₁-C₆ alkyl;
R²³ is selected from the group consisting of hydrogen atom, halogen and C₁-C₆ alkyl;
R²⁴ is selected from the group consisting of hydrogen atom, halogen, C₁-C₆ alkyl and -C(O)OR^{a}, wherein R^{a} is selected from the group consisting of hydrogen atom, C₁-C₆ alkyl and C₃-C₆ cycloalkyl; and
R²⁵ is selected from the group consisting of hydrogen atom, halogen and C₁-C₆ alkyl;
provided that R²¹, R²², R²³, R²⁴ and R²⁵ are not hydrogen atoms simultaneously.

In another embodiment, in the compound of formula (IB), the pharmaceutically acceptable salt thereof, the crystal form thereof, the solvate thereof, the isotope derivative thereof, or the prodrug thereof, the compound of formula (IB) is selected from the group consisting of:

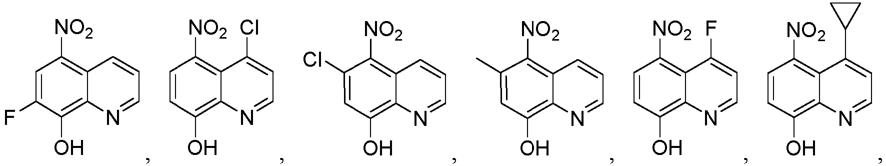

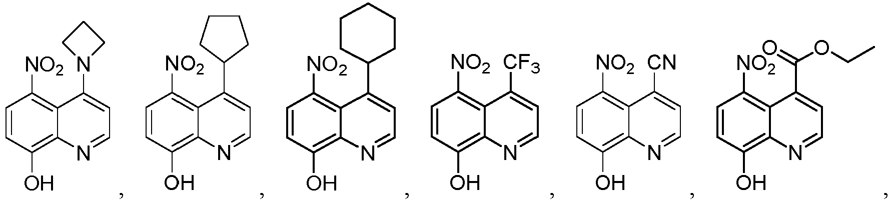

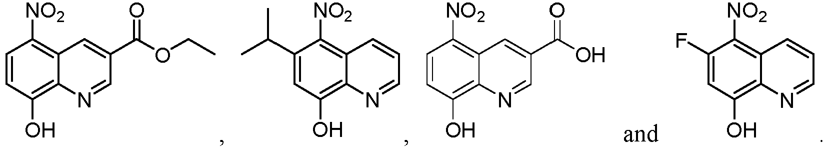

In another embodiment, in the compound of formula (IB), the pharmaceutically acceptable salt thereof, the crystal form thereof, the solvate thereof, the isotope derivative thereof, or the prodrug thereof:
R²¹ is selected from the group consisting of hydrogen atom, 4 to 8 membered heterocyclyl, haloC₁-C₆ alkyl, -C(O)OR^{a} and C₃-C₆ cycloalkyl;
R²² is hydrogen atom;
R²³ is hydrogen atom;
R²⁴ is hydrogen atom or -C(O)OR^{a};
R²⁵ is hydrogen atom;
wherein R^{a} is hydrogen atom or C₁-C₆ alkyl.

In another embodiment, in the compound of formula (IB), the pharmaceutically acceptable salt thereof, the crystal form thereof, the solvate thereof, the isotope derivative thereof, or the prodrug thereof, the compound of formula (IB) is selected from the group consisting of

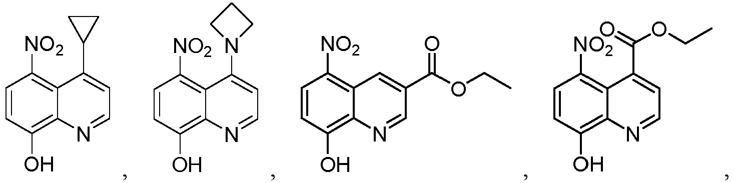

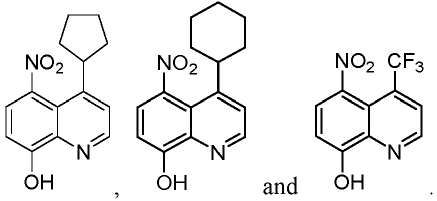

In another embodiment, the compound of formula (I), the pharmaceutically acceptable salt thereof, the crystal form thereof, the solvate thereof, the isotope derivative thereof, or the prodrug thereof is a compound of formula (IC), a stereoisomer thereof or a pharmaceutically acceptable salt thereof: 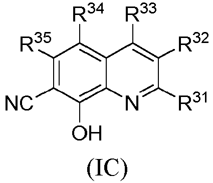 wherein:
R³¹ is selected from the group consisting of hydrogen atom, halogen, alkyl, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
R³² is selected from the group consisting of hydrogen atom, halogen, alkyl, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
R³³ is selected from the group consisting of hydrogen atom, alkyl, halogen, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
R³⁴ is selected from the group consisting of hydrogen atom, halogen, alkyl, haloalkyl, carboxy, hydroxy, hydroxyalkyl, alkoxy, haloalkoxy, amino, thiol, alkenyl, alkynyl, cycloalkyl, heterocyclyl, aryl and heteroaryl;
R³⁵ is selected from the group consisting of hydrogen atom, halogen, alkyl, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
provided that R³¹, R³², R³³, R³⁴ and R³⁵ are not hydrogen atoms simultaneously.

In another embodiment, in the compound of formula (IC), the stereoisomer thereof or the pharmaceutically acceptable salt thereof, R³⁴ is selected from the group consisting of hydrogen atom, halogen, C₁₋₆ alkyl, haloC₁₋₆ alkyl, carboxy and hydroxy, preferably selected from the group consisting of hydrogen atom, halogen, C₁₋₆ alkyl, haloC₁₋₆ alkyl and carboxy, and more preferably selected from the group consisting of hydrogen atom, bromine atom, chlorine atom, methyl, trifluoromethyl and carboxy.

In another embodiment, in the compound of formula (IC), the stereoisomer thereof or the pharmaceutically acceptable salt thereof, R³³ is selected from the group consisting of hydrogen atom, C₁₋₆ alkyl, halogen and hydroxy, preferably hydrogen atom or C₁₋₆ alkyl, and more preferably hydrogen atom or methyl.

In another embodiment, in the compound of formula (IC), the stereoisomer thereof or the pharmaceutically acceptable salt thereof, the compound of formula (IC) is selected from the group consisting of: 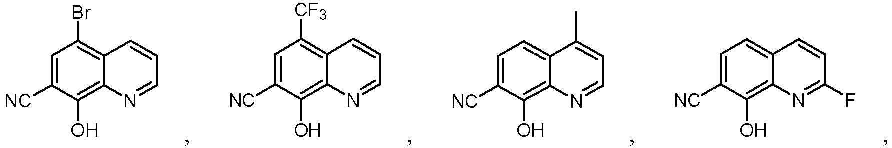 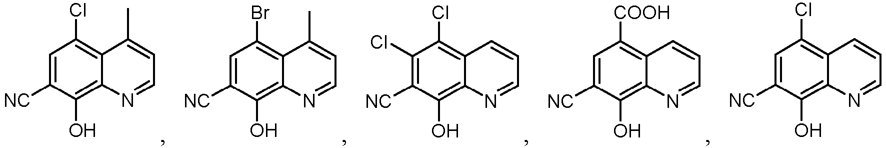 and 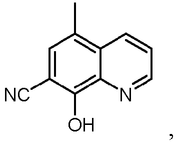 and preferably selected from the group consisting of

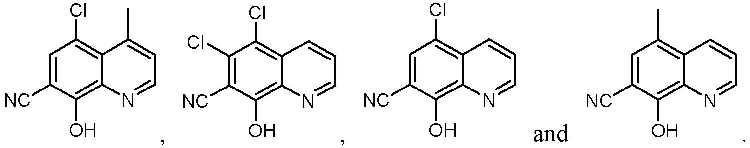

In another embodiment, provided is a use of the compound of formula (I), the pharmaceutically acceptable salt thereof, the crystal form thereof, the solvate thereof, the isotope derivative thereof, or the prodrug thereof in the preparation of an antiviral drug, wherein the antiviral drug is used in a mammal, preferably in a human.

In another embodiment, provided is a use of the compound of formula (I), the pharmaceutically acceptable salt thereof, the crystal form thereof, the solvate thereof, the isotope derivative thereof, or the prodrug thereof in the preparation of an antiviral drug, wherein the virus is human papillomavirus; preferably one or more of *Human Papillomavirus 6 (HPV6), Human Papillomavirus 11* (*HPV11*), *Human Papillomavirus 16* (*HPV16*) and *Human Papillomavirus 18* (*HPV18*); and more preferably *Human Papillomavirus 6, Human Papillomavirus 11, Human Papillomavirus 16* or *Human Papillomavirus 18.*

In another embodiment, provided is a use of the compound of formula (I), the pharmaceutically acceptable salt thereof, the crystal form thereof, the solvate thereof, the isotope derivative thereof, or the prodrug thereof in the preparation of an antiviral drug, wherein the daily dose of the compound of formula (I) is 0.01 mg to 1000 mg per kilogram of body weight, and preferably 0.1 mg to 500 mg per kilogram of body weight.

In another embodiment, provided is a use of the compound of formula (I), the pharmaceutically acceptable salt thereof, the crystal form thereof, the solvate thereof, the isotope derivative thereof, or the prodrug thereof in the preparation of an antiviral drug, wherein the drug further comprises another antiviral agent, and preferably, the antiviral agent is selected from the group consisting of acyclovir, valacyclovir, zidovudine, ganciclovir, penciclovir, famciclovir, foscarnet, ribavirin, lamivudine, amantadine, IFNα, cidofovir and rimantadine.

In another embodiment, provided is a use of the compound of formula (I), the pharmaceutically acceptable salt thereof, the crystal form thereof, the solvate thereof, the isotope derivative thereof, or the prodrug thereof in the preparation of an antiviral drug, wherein the drug is in a dosage form as follows: oral liquid, suspension, powder, granule, tablet, capsule, pill, emulsion, syrup, aerosol or injection.

In another embodiment, provided is a use of the compound of formula (I), the pharmaceutically acceptable salt thereof, the crystal form thereof, the solvate thereof, the isotope derivative thereof, or the prodrug thereof in the preparation of an antiviral drug, wherein the drug is administrated through an oral, intramuscular, intraperitoneal, intravenous, subcutaneous, intranasal, epidural, sublingual, intranasal, intracerebral, inraventricular, intrathecal, intravaginal, rectal, inhalable or topical route.

In another embodiment, the present invention further provides a method for preventing or treating viral infection, comprising a step of administering a therapeutically effective amount of the 8-hydroxyquinoline derivative, the pharmaceutically acceptable salt thereof, the crystal form thereof, the solvate thereof, the isotope derivative thereof, or the prodrug thereof as defined in the preceding use, to a subject.

In another embodiment, provided is a method for preventing or treating viral infection in a mammal, comprising a step of administering a therapeutically effective amount of the 8-hydroxyquinoline derivative, the pharmaceutically acceptable salt thereof, the crystal form thereof, the solvate thereof, the isotope derivative thereof, or the prodrug thereof as defined in the preceding use, to a subject, wherein the mammal is preferably a human.

In another embodiment, the present invention further provides a method for *in vitro* disinfection, comprising the following step of:
contacting an environment or object to be treated with an effective amount of the 8-hydroxyquinoline derivative, the pharmaceutically acceptable salt thereof, the crystal form thereof, the solvate thereof, or the isotope derivative thereof as defined in the preceding use.

In another embodiment, in the method for preventing or treating viral infection in a mammal or the method for *in vitro* disinfection, the virus is human papillomavirus; preferably one or more of *Human Papillomavirus 6 (HPV6), Human Papillomavirus 11* (*HPV11*), *Human Papillomavirus 16* (*HPV16*) and *Human Papillomavirus 18* (*HPV18*); and more preferably *Human Papillomavirus 6 (HPV6), Human Papillomavirus 11 (HPV11*), *Human Papillomavirus 16* (*HP V16*) or *Human Papillomavirus 18* (*HPV18*).

In another embodiment, the present invention further provides a pharmaceutical composition for treating viral infection, comprising the 8-hydroxyquinoline derivative, the pharmaceutically acceptable salt thereof, the crystal form thereof, the solvate thereof, the isotope derivative thereof, or the prodrug thereof as defined in the preceding use, and pharmaceutically acceptable excipients.

In another embodiment, provided is a pharmaceutical composition for treating viral infection in a mammal, comprising the 8-hydroxyquinoline derivative, the pharmaceutically acceptable salt thereof, the crystal form thereof, the solvate thereof, the isotope derivative thereof, or the prodrug thereof as defined in the preceding use, and pharmaceutically acceptable excipients.

In another embodiment, in the pharmaceutical composition for treating viral infection in a mammal, the virus is human papillomavirus; preferably one or more of *Human Papillomavirus 6 (HPV6), Human Papillomavirus 11* (*HPV11*), *Human Papillomavirus 16* (*HPV16*) and *Human Papillomavirus 18* (*HPV18*); and more preferably *Human Papillomavirus 6 (HPV6), Human Papillomavirus 11* (*HPV11*), *Human Papillomavirus 16 (HPVl6)* or *Human Papillomavirus 18* (*HPV18*).

The pharmaceutical composition of the present invention can be various conventional dosage forms, for example, tablet, aqueous suspension, oily suspension, dispersible powder, dispersible granule, emulsion, hard capsule, soft capsule, sterile aqueous solution for injection, sterile oil-in-water microemulsion for injection, sterile powder for injection, cream, ointment, gel, film, patch, emplastrum, spray, lotion, or suppository. Each of the above dosage forms can be prepared by conventional preparation methods.

It is well known to those skilled in the art that the dosage of a drug depends on a variety of factors including, but not limited to the following factors: activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound or the type of pharmaceutically acceptable salt thereof can be verified according to the traditional therapeutic regimens.

### Definition of terms

Regarding the terms not defined herein, they have the meanings commonly understood by those skilled in the art. Regarding the terms defined herein, they have the meanings defined in the description.

The term "substitution" or "substituent" refers to one or more hydrogen atoms being replaced by a group indicated. When the substitution position is not indicated, the substitution can occur at any position, provided that the formation of a stable or chemically feasible chemical is allowed.

The term "optional" or "optionally" means that the event or circumstance described subsequently can, but need not, occur, and such a description includes the situation in which the event or circumstance occurs or the situation in which the event or circumstance does not occur.

Where any variable (such as R) appears more than once in the structure of a compound, its definition in each case is independent. For example, if a group is substituted by 0 to 2 R, the group can optionally be substituted by up to two R, and R in each case has independent options.

The term "alkyl" refers to a saturated linear or branched monovalent hydrocarbon group having 1 to 20 (for example, 1, 2, 3, 4, 5, 6, 7, 8, 9, 10, 11, 12, 13, 14, 15, 16, 17, 18, 19 and 20) carbon atoms. The alkyl is preferably a C₁₋₁₀ alkyl, and more preferably C₁₋₆ alkyl. Examples of alkyl include, but are not limited to, methyl, ethyl, n-propyl, isopropyl, n-butyl, isobutyl, *tert*-butyl, *sec*-butyl, *n*-pentyl, 2,2-dimethylpropyl, 2-methylbutyl, *n*-hexyl, 2,2-dimethylbutyl, 2-methylpentyl, 3-methylpentyl, 2,3-dimethylbutyl, *n*-heptyl, 2-methylhexyl, 3-methylhexyl, 2,3-dimethylpentyl, 2,4-dimethylpentyl, 2,2-dimethylpentyl, 3,3-dimethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 3-ethylhexyl, 4-ethylhexyl, 2-methyl-3-ethylpentyl, *n*-nonyl, 2-methyl-3-ethylhexyl, *n*-decyl and 3,3-diethylhexyl.

The term "alkenyl" refers to a linear or branched monovalent hydrocarbon group having 2 to 6 (for example 2, 3, 4, 5 and 6) carbon atoms and at least one carbon-carbon double bond, wherein the carbon-carbon double bond may be located anywhere within the alkenyl. The alkenyl is preferably a C₂₋₅ alkenyl. Examples of alkenyl include, but are not limited to, -CH=CH₂, -CH=CH-CH₃, -CH₂-CH=CH₂, -CH=CH-CH₂-CH₃, -CH₂-CH=CH-CH₃, -CH=CH-CH=CH₂, -CH=C(CH₃)-CH₃ and -CH₂-C(CH₃)=CH₂.

The term "alkynyl" refers to a linear or branched monovalent hydrocarbon group having 2 to 6 (for example 2, 3, 4, 5 and 6) carbon atoms and at least one carbon-carbon triple bond, wherein the carbon-carbon triple bond may be located anywhere within the alkynyl. The alkynyl is preferably a C₂₋₅ alkynyl. Examples of alkynyl include, but are not limited to, -C≡CH, -C≡C-CH₃, -CH₂-C≡CH, -C≡C-CH₂-CH₃, -CH₂-CH₂-C≡CH,-CH(CH₃)C≡CH and -CH₂-C≡C-CH₃.

The term "cycloalkyl" includes two categories, one is conventional cycloalkyl, and the other is heterostructured cycloalkyl.

The conventional cycloalkyl refers to an aliphatic saturated or partially unsaturated monovalent cyclic hydrocarbon group having 3 to 20 (for example, 3, 4, 5, 6, 7, 8, 9, 10, 11, 12, 13, 14, 15, 16, 17, 18, 19 and 20) carbon atoms. The conventional cycloalkyl is preferably a C₃₋₁₂ conventional cycloalkyl, more preferably a C₃₋₁₀ conventional cycloalkyl, further preferably a C₃₋₈ conventional cycloalkyl, and most preferably a C₃₋₆ conventional cycloalkyl. The conventional cycloalkyl optionally comprises one or more double or triple bonds.

The conventional cycloalkyl can be a monocyclic cycloalkyl. Examples of monocyclic cycloalkyl include, but are not limited to, cyclopropyl, cyclobutyl, cyclopentyl, cyclopentenyl, cyclohexyl, cyclohexenyl, cyclohexadienyl, cycloheptyl, cycloheptatrienyl and cyclooctyl. The conventional cycloalkyl can also be a polycyclic cycloalkyl (for example, bicycloalkyl and tricycloalkyl). Polycyclic cycloalkyl includes spiro cycloalkyl, fused cycloalkyl and bridged cycloalkyl.

The term "spiro cycloalkyl" refers to a 5 to 20 membered (for example, 5, 6, 7, 8, 9, 10, 11, 12, 13, 14, 15, 16, 17, 18, 19 and 20 membered) spiro cycloalkyl. The spiro cycloalkyl is preferably a 6 to 14 membered spiro cycloalkyl, and more preferably a 7 to 10 membered spiro cycloalkyl. The spiro cycloalkyl can be a mono-spiro cycloalkyl, a di-spiro cycloalkyl, or a poly-spiro cycloalkyl, and the spiro cycloalkyl is preferably a mono-spiro cycloalkyl, and more preferably a 4-membered/4-membered, 4-membered/5-membered, 4-membered/6-membered, 5-membered/5-membered, or 5-membered/6-membered mono-spiro cycloalkyl. Examples of spiro cycloalkyl include, but are not limited to:

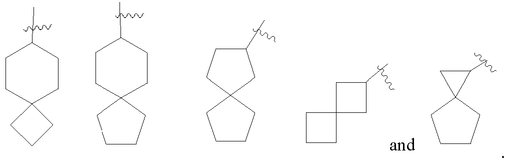

The term "fused cycloalkyl" refers to a 5 to 20 membered (for example, 5, 6, 7, 8, 9, 10, 11, 12, 13, 14, 15, 16, 17, 18, 19 and 20 membered) fused cycloalkyl. The fused cycloalkyl is preferably a 6 to 14 membered fused cycloalkyl, and more preferably a 7 to 10 membered fused cycloalkyl. The fused cycloalkyl can be a bicyclic, tricyclic, tetracyclic, pentacyclic or more fused cycloalkyl, and the fused cycloalkyl is preferably a bicyclic or tricyclic fused cycloalkyl, and more preferably a 5-membered/5-membered, or 5-membered/6-membered fused cycloalkyl. Examples of fused cycloalkyl include, but are not limited to:

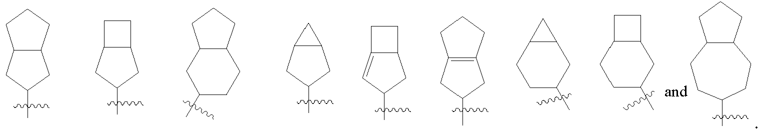

The term "bridged cycloalkyl" refers to a 5 to 20 membered (for example, 5, 6, 7, 8, 9, 10, 11, 12, 13, 14, 15, 16, 17, 18, 19 and 20 membered) bridged cycloalkyl. The bridged cycloalkyl is preferably a 6 to 14 membered bridged cycloalkyl, and more preferably a 7 to 10 membered bridged cycloalkyl. The bridged cycloalkyl can be a bicyclic, tricyclic, tetracyclic, pentacyclic or more bridged cycloalkyl, and the bridged cycloalkyl is preferably a bicyclic, tricyclic or tetracyclic bridged cycloalkyl, and more preferably a bicyclic or tricyclic bridged cycloalkyl. Examples of bridged cycloalkyl include, but are not limited to:

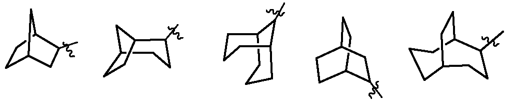

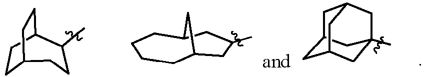

The term "heterostructured cycloalkyl" includes monocyclic cycloalkyl, spiro cycloalkyl, fused cycloalkyl and bridged cycloalkyl fused with any one selected from the group consisting of conventional aryl, conventional heteroaryl and conventional heterocyclyl, and the attachment point is located on the corresponding conventional cycloalkyl (referring to monocyclic cycloalkyl, spiro cycloalkyl, fused cycloalkyl or bridged cycloalkyl). Examples of heterostructured cycloalkyl include, but are not limited to:

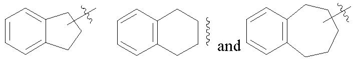

The term "heterocyclyl" includes two categories, one is conventional heterocyclyl, and the other is heterostructured heterocyclyl.

The conventional heterocyclyl refers to an aliphatic saturated or partially unsaturated monovalent cyclic hydrocarbon group having 3 to 20 (for example, 3, 4, 5, 6, 7, 8, 9, 10, 11, 12, 13, 14, 15, 16, 17, 18, 19 and 20) ring atoms, wherein one or more ring atoms are replaced by one or more elements selected from the group consisting of N, O, S, S(O) and S(O)₂, and the replacement does not form -O-O-, -O-S- or -S-S-. The conventional heterocyclyl is preferably a C₃₋₁₂ conventional heterocyclyl, wherein 1 to 4 (for example 1, 2, 3 and 4) atoms are heteroatoms; more preferably a C₃₋₈ conventional heterocyclyl, wherein 1 to 3 (for example 1, 2 and 3) atoms are heteroatoms; and most preferably a C₅₋₇ conventional heterocyclyl, wherein 1 to 2 or 1 to 3 atoms are heteroatoms.

The conventional heterocyclyl can be a monocyclic heterocyclyl. Examples of monocyclic heterocyclyl include, but are not limited to oxetanyl, 3-pyrrolinyl, pyrrolidinyl, imidazolidinyl, tetrahydrofuranyl, tetrahydrothienyl, dihydroimidazolyl, dihydrofuryl, dihydropyrazolyl, dihydropyrrolyl, piperidinyl, piperazinyl, morpholinyl, thiomorpholinyl, homopiperazinyl and pyranyl, and preferably 1,2,5-oxadiazolyl, pyranyl or morpholinyl. The conventional heterocyclyl can also be a polycyclic heterocyclyl. Polycyclic heterocyclyl includes spiro heterocyclyl, fused heterocyclyl and bridged heterocyclyl.

The term "spiro heterocyclyl" refers to a 5 to 20 membered (for example, 5, 6, 7, 8, 9, 10, 11, 12, 13, 14, 15, 16, 17, 18, 19 and 20 membered) spiro heterocyclyl. The spiro heterocyclyl is preferably a 6 to 14 membered spiro heterocyclyl, and more preferably a 7 to 10 membered spiro heterocyclyl. The spiro heterocyclyl can be a mono-spiro heterocyclyl, a di-spiro heterocyclyl, or a poly-spiro heterocyclyl, and the spiro heterocyclyl is preferably a mono-spiro heterocyclyl or a di-spiro heterocyclyl, and more preferably a 3-membered/6-membered, 4-membered/4-membered, 4-membered/5-membered, 4-membered/6-membered, 5-membered/5-membered, or 5-membered/6-membered mono-spiro heterocyclyl. Examples of spiro heterocyclyl include, but are not limited to:

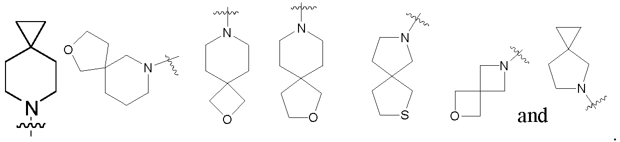

The term "fused heterocyclyl" refers to a 5 to 20 membered (for example, 5, 6, 7, 8, 9, 10, 11, 12, 13, 14, 15, 16, 17, 18, 19 and 20 membered) fused heterocyclyl. The fused heterocyclyl is preferably a 6 to 14 membered fused heterocyclyl, and more preferably a 7 to 10 membered fused heterocyclyl. The fused heterocyclyl can be a bicyclic, tricyclic, tetracyclic, pentacyclic or more fused heterocyclyl, and the fused heterocyclyl is preferably a bicyclic or tricyclic fused heterocyclyl, and more preferably a 5-membered/5-membered, or 5-membered/6-membered bicyclic fused heterocyclyl. Examples of fused heterocyclyl include, but are not limited to:

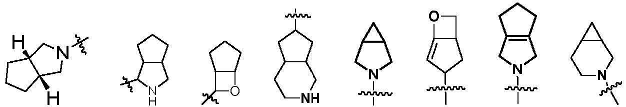

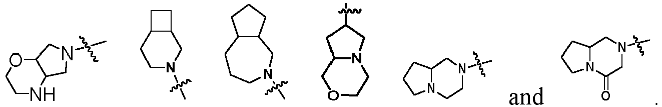

The term "bridged heterocyclyl" refers to a 5 to 14 membered (for example, 5, 6, 7, 8, 9, 10, 11, 12, 13 and 14 membered) bridged heterocyclyl. The bridged heterocyclyl is preferably a 6 to 14 membered bridged heterocyclyl, and more preferably a 7 to 10 membered bridged heterocyclyl. The bridged heterocyclyl can be a bicyclic, tricyclic, tetracyclic, pentacyclic or more bridged heterocyclyl, and the bridged heterocyclyl is preferably a bicyclic, tricyclic or tetracyclic bridged heterocyclyl, and more preferably a bicyclic or tricyclic bridged heterocyclyl. Examples of bridged heterocyclyl include, but are not limited to:

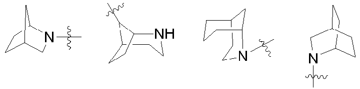

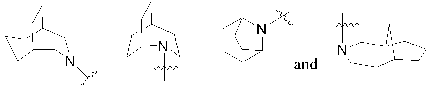

The term "heterostructured heterocyclyl" includes monocyclic heterocyclyl, spiro heterocyclyl, fused heterocyclyl and bridged heterocyclyl fused with any one selected from the group consisting of conventional aryl, conventional heteroaryl and conventional cycloalkyl, and the attachment point is located on the corresponding conventional heterocyclyl (referring to monocyclic heterocyclyl, spiro heterocyclyl, fused heterocyclyl or bridged heterocyclyl). Examples of heterostructured heterocyclyl include, but are not limited to:

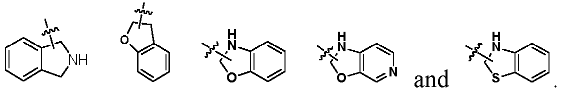

The term "aryl" includes two categories, one is conventional aryl, and the other is heterostructured aryl.

The conventional aryl refers to a 6 to 14 membered (for example, 6, 7, 8, 9, 10, 11, 12, 13 and 14 membered) aromatic hydrocarbon group. The conventional aryl is preferably a C₆₋₁₀ conventional aryl, and more preferably phenyl, naphthyl, phenanthryl or anthracenyl.

The term "heterostructured aryl" includes conventional aryl fused with any one selected from the group consisting of conventional heteroaryl, conventional heterocyclyl and conventional cycloalkyl, and the attachment point is located on the conventional aryl. Examples of heterostructured aryl include, but are not limited to:

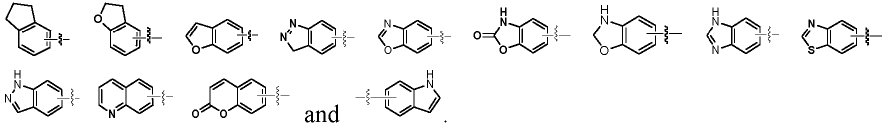

The term "heteroaryl" includes two categories, one is conventional heteroaryl, and the other is heterostructured heteroaryl.

The conventional heteroaryl refers to a 5 to 14 membered (for example, 5, 6, 7, 8, 9, 10, 11, 12, 13 and 14 membered) aromatic hydrocarbon group, wherein 1 to 4 (for example, 1, 2, 3 and 4) carbon atoms are replaced by heteroatoms, the heteroatom is selected from the group consisting of O, S and N. Preferably, the number of ring atoms is 5 to 10, including 1 to 3 (for example, 1, 2 and 3) heteroatoms. More preferably, the number of ring atoms is 5 or 6, including 1 to 2 heteroatoms. Examples of conventional heteroaryl include, but are not limited to imidazolyl, furyl, thienyl, thiazolyl, pyrazolyl, oxazolyl, pyrrolyl, tetrazolyl, pyridyl, pyrimidinyl, thiadiazolyl and pyrazinyl, preferably imidazolyl, thiazolyl, pyrazolyl, pyrimidinyl or thiazolyl, and more preferably pyrazolyl or thiazolyl.

The term "heterostructured heteroaryl" includes conventional heteroaryl fused with any one selected from the group consisting of conventional aryl, conventional cycloalkyl and conventional heterocyclyl, and the attachment point is located on the conventional heteroaryl. Examples of heterostructured heteroaryl include, but are not limited to:

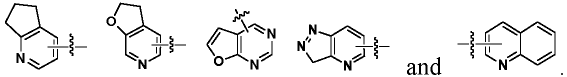

The term "alkoxy" includes -O-alkyl and -O-cycloalkyl, wherein the "alkyl" and "cycloalkyl" are as defined above. Examples of alkoxy include, but are not limited to: methoxy, ethoxy, propoxy, butoxy, cyclopropyloxy, cyclobutyloxy, cyclopentyloxy and cyclohexyloxy.

The term "haloalkyl" refers to an alkyl substituted by one or more halogen(s), wherein the alkyl is as defined above.

The term "haloalkoxy" refers to an alkoxy substituted by one or more halogen(s), wherein the alkoxy is as defined above.

The term "hydroxy" refers to an -OH group.

The term "halogen" refers to -F, -Cl, -Br or -I group.

The term "amino" refers to a -NH₂ group.

The term "cyano" refers to a -CN group.

The term "nitro" refers to a -NO₂ group.

The term "oxo" refers to an =O group.

The term "carboxy" refers to a -C(=O)OH group.

The term "thiol" refers to a -SH group.

The term "alkoxycarbonyl" refers to a -C(=O)O-alkyl or a -C(=O)O-cycloalkyl, wherein the alkyl and cycloalkyl are as defined above.

The term "acyl" refers to a -C(=O)R group, where R is selected from the group consisting of alkyl, cycloalkyl, heterocyclyl, aryl and heteroaryl.

The symbol " 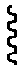 " refers to the attachment point.

The term "pharmaceutically acceptable" refers to the use in the preparation of a pharmaceutical composition that is generally safe, non-toxic, biologically satisfactory and can be accepted as a medicament to a mammal, such as a human.

The term "pharmaceutically acceptable salt" should be understood as referring to the following salts, which are pharmaceutically acceptable salts and which possess the expected pharmacological activity of the parent compound (referring to the compound of the formula). Such salts include:
(1) Acid addition salts formed with inorganic acids, or acid addition salts formed with organic acids; wherein, the inorganic acids can be one or more of hydrochloric acid, hydrobromic acid, sulfuric acid, nitric acid and phosphoric acid; the organic acids can be one or more of formic acid, oxalic acid, succinic acid, acetic acid, benzenesulfonic acid, benzoic acid, camphorsulfonic acid, citric acid, ethanesulfonic acid, fumaric acid, glucoheptonic acid, gluconic acid, glutamic acid, glycollic acid, hydroxynaphthoic acid, 2-hydroxyethanesulfonic acid, lactic acid, maleic acid, malic acid, mandelic acid, methanesulfonic acid, muconic acid, 2-naphthalenesulfonic acid, propionic acid, salicylic acid, succinic acid, dibenzoyl-L-tartaric acid, tartaric acid, p-toluenesulfonic acid, trimethylacetic acid and trifluoroacetic acid; and
(2) Salts formed when acid protons present in the parent compound are substituted by metal ions, for example, alkali metal ions (e.g., Na⁺, K⁺ or Li⁺), alkaline earth metal ions (such as Ca²⁺ or Mg²⁺) or aluminum ions; or, when coordinated with organic or inorganic bases; wherein, the organic bases can be one or more of pyridines, imidazoles, pyrazines, indoles, purines, tertiary amines and anilines, preferably one or more of pyridine, methylpyridine, 4-dimethylaminopyridine, 2-methyl-5-ethylpyridine, triethylamine, N,N-diisopropylethanamine, N,N-dimethylaniline, diethanolamine, ethanolamine, N-methylglucosamine, triethanolamine and tromethamine; the inorganic bases can be one or more of aluminum hydroxide, calcium hydroxide, potassium hydroxide, sodium carbonate and sodium hydroxide.

The term "isotope derivative" refers to a compound that differs from the parent compound described herein only in the presence of one or more isotopically enriched atoms. For example, a isotope derivative has the structure shown by the formula, wherein hydrogen is replaced by "deuterium" or "tritium", and/or fluorine is replaced by ¹⁸F, and/or carbon is replaced by ¹¹C, ¹³C or ¹⁴C, while the rest is left unchanged. Isotope derivatives may be used as analytical tools or probes in biological assays, or as tracers for *in vivo* diagnostic imaging of disease, or as tracers for pharmacodynamic, pharmacokinetic, or receptor studies. Deuterated compounds often retain activity comparable to that of non-deuterated compounds, and when deuterated at specific sites they can achieve better metabolic stability, resulting in certain therapeutic advantages (e.g. increased half-life *in vivo* or decreased dosage requirements). Therefore, the isotope derivative is preferably a deuterated compound.

The term "solvate" refers to a substance formed from the parent compound described herein and a suitable solvent. The solvent is preferably water or an organic solvent.

The term "prodrug" refers to a derivative of the parent compound described herein comprising a biologically reactive functional group, such that the biologically reactive functional group may cleave from the derivative or otherwise react under biological conditions (*in vitro* or *in vivo*) to provide the parent compound herein. Usually, the prodrug is inactive, or at least less active than the parent compound itself, so that the parent compound herein can not exert the activity thereof until it is separated from the biologically reactive functional group. The biologically reactive functional group may be hydrolyzed or oxidized under biological conditions to provide the parent compound herein. For example, the prodrug may comprise a biologically hydrolyzable functional group; and examples of the biologically hydrolyzable group include, but are not limited to, biologically hydrolyzable phosphates, biologically hydrolyzable esters, biologically hydrolyzable amides, biologically hydrolyzable carbonates, biologically hydrolyzable carbamates, and biologically hydrolyzable ureides.

The term "pharmaceutical composition" refers to a mixture consisting of a pharmaceutical compound (referring to one or more of the compound of the formula, a pharmaceutically acceptable salt thereof, a tautomer thereof, a stereoisomer thereof, a enantiomer thereof, a diastereoisomer thereof, a isotope derivative thereof, a crystal form thereof, a solvate thereof, a prodrug thereof, a metabolite thereof and a racemate containing the same described herein) and pharmaceutically acceptable excipients.

The term "pharmaceutically acceptable excipient" refers to a pharmaceutically acceptable excipient used to deliver a pharmaceutical compound described herein to a subject. Depending on the administration method, the pharmaceutical composition may comprise 0.1% to 99% by weight of the pharmaceutical compound.

The term "subject" refers to a mammal, including, for example, camel, donkey, zebra, cattle, pig, horse, goat, sheep, cat, dog, rat, rabbit, guinea pig, mouse, primate. In some particular embodiments, the subject is a human. In some particular embodiments, the subject is a human who is susceptible to, suspected of having, or has suffered from a cancer or bacterial infection.

The term "treatment" refers to eliminating the disease, preventing disease progression, slowing disease progression, reducing the duration of one or more symptoms associated with the disease, improving or reversing at least one measurable parameter associated with the disease, or increasing the survival of subjects with the disease.

The term "effective amount" refers to an amount of the active ingredient of the drug (referring to the pharmaceutical compound) that elicits the desired effect in a subject. In particular embodiments, those skilled in the art can determine the selection of an effective amount based on consideration of a variety of factors (e.g., through clinical trials), and the factors include the disease to be treated, the symptoms involved, the route of administration, the severity of the disease, the patient's body weight, the patient's immune status, and other factors known to those skilled in the art. The effective amount can be obtained from the dose-response curve derived from an animal model testing system, and can be determined according to the opinion of a physician and the situation of each patient. The correlation between animal and human doses is described in Freireich et al., 1966, Cancer Chemother Rep 50:219, and the body surface area of human can be approximately determined by the height and body weight of the patient. The effective amount of the pharmaceutical compounds of the present invention can be 0.5 mg/kg to 500 mg/kg, preferably 1 mg/kg to 200 mg/kg, more preferably 10 mg/kg to 100 mg/kg.

Herein, the same pharmaceutical active ingredient (which refers to a single pharmaceutical compound) or different pharmaceutical active ingredients (which refer to two or more pharmaceutical compounds) can be administered at one time, or can be divided into multiple smaller doses, and administered at a certain time interval. It should be understood that the exact dose, duration, and interval of treatment are functions of the disease being treated, and can be determined by inference using animal or clinical trial data. The administration can include a single administration, or two or more administrations at an appropriate time interval. Wherein, the time interval between two adjacent administrations can be 30 minutes, 40 minutes, 50 minutes, 60 minutes, 2 hours, 3 hours, 4 hours, 5 hours, 6 hours, 7 hours, 8 hours, 9 hours, 10 hours, 12 hours, 14 hours, 16 hours, 18 hours, 20 hours, 22 hours, 24 hours, one and a half days, 2 days, 3 days, 4 days, 5 days, 6 days, 7 days, 8 days, 9 days, 10 days, 1 week, 2 weeks, 3 weeks, 4 weeks, 5 weeks, 6 weeks, 1 month, 2 months, 3 months , 4 months, 5 months, 6 months, 7 months, 8 months, 9 months, 10 months, 11 months or 12 months.

Each pharmaceutical active ingredient (each pharmaceutical compound) mentioned herein can be used as the only active compound, or can be administered in combination with other active compounds (which refer to compounds other than the pharmaceutical compounds described herein), as long as they do not produce other adverse effects, such as allergic responses. Combined administration includes simultaneous or sequential administration of each active compound.

The term "combined administration" refers to a method of providing two or more active compounds simultaneously or sequentially to a subject for therapeutic purposes. When "combined administration" is involved, the time interval between each administration is sufficient to achieve synergy between each active compound administered.

When the term "about" is applied to a parameter such as weight, volume, pH, concentration, temperature, etc., it is intended that the parameter may vary by ±10%, and is sometimes more preferably within ±5%. As those skilled in the art will appreciate, when a parameter is not critical, numbers are generally given for purposes of illustration only and not limitation.

### DETAILED DESCRIPTION OF THE INVENTION

The present invention is further illustrated through examples below, but it is not intended to limit the present invention to the scope of the examples. The experimental methods without specifying specific conditions in the following examples shall be selected according to conventional methods and conditions, or according to the product instruction.

The compound of the present invention is prepared by using convenient starting materials and general preparation procedures. The present invention provides typical or preferential reaction conditions, such as reaction temperature, time, solvent, pressure and molar ratio of reactants. However, unless otherwise specified, other reaction conditions can also be adopted. Optimal conditions may vary with the use of specific reactants or solvents, but under normal circumstances, the steps and conditions optimized for reaction can be determined.

In addition, certain protecting groups may be used in the present invention to protect certain functional groups from unnecessary reactions. The protecting groups suitable for various functional groups and their protection or deprotection conditions are well known to those skilled in the art. For example, "Protective Groups in Organic Synthesis" by T. W. Greene and G. M. Wuts (3rd edition, Wiley, New York, 1999) and the citations in the book describe the protection or deprotection of a large number of protecting groups in detail.

The isolation and purification of compounds and intermediates are carried out by appropriate methods and steps according to specific needs, such as filtration, extraction, distillation, crystallization, column chromatography, preparative thin-layer chromatography, preparative high performance liquid chromatography or a combination of the above methods. The specific method used may be referred to the examples described in the present invention. Of course, other similar isolation and purification methods can also be used. They can be characterized using conventional methods (including physical constants and spectral data).

The purity analysis method is as follows: a Kinetex EVO C18 (50×4.6 mm, 5 µm, 100Å) chromatographic column is used, acetonitrile-water is used as the mobile phase for gradient elution, the flow rate is 1.5 mL/min, and the detection wavelength is 220 nm.

MS is determined by a LC (Agilent 1260 Infinity II)/MS (G6125B single quadrupole) mass spectrograph (manufacturer: Agilent) (Photodiode Array Detector).

The structures of the compounds are identified by hydrogen nuclear magnetic resonance, and the equipment model is WNMR-I-400MHz.

Preparative liquid chromatography is conducted on an Agilent 1260 Infinity II high performance liquid chromatograph (manufacturer: Agilent). The chromatographic column is Daisogel C18 10 µm 100A (30 mm×250 mm), and the mobile phase is acetonitrile/water.

GF254 silica gel plates from Qingdao Haiyang Chemical are used for thin-layer chromatography (TLC). For the silica gel plates used, the specification is 0.20 mm to 0.25 mm for the thin-layer chromatography for monitoring of reactions, and 0.5 mm for the thin-layer chromatography for separation and purification.

Silica gel of 100 to 200 mesh, 200 to 300 mesh or 300 to 400 mesh of Qingdao Haiyang Chemical is used as a carrier for silica gel column chromatography.

The known starting materials of the present invention can be prepared by the known methods in the art, or can be purchased from Wanghua Mall, Beijing Ouhe Technology, Sigma, J&K Scientific, Yishiming, Shanghai Shuya Chemical, Shanghai Innochem Science & Technology, Energy Chemical, Shanghai Bide Pharmatech and the like.

Unless otherwise stated, the reactions are carried out under a nitrogen atmosphere.

Nitrogen atmosphere means that a reaction flask is equipped with a nitrogen balloon (about 1 L).

The reaction solvent, organic solvent or inert solvent is each expressed as the solvent used that does not participate in the reaction under the described reaction conditions, which includes for example, benzene, toluene, acetonitrile, tetrahydrofuran (THF), dimethylformamide (DMF), chloroform, dichloromethane, ether, methanol, N-methylpyrrolidone (NMP).

Unless otherwise specified in the examples, a solution means an aqueous solution.

Unless otherwise defined, all professional and scientific terms used herein have the same meaning as those familiar to those skilled in the art. In addition, any methods and materials similar or equivalent to the content described herein can be applied to the method of the present invention.

Unless otherwise specified, the mixing ratios of different solvents are volume ratios.

### Example 1

### Synthesis of 7-nitroquinolin-8-ol (1)

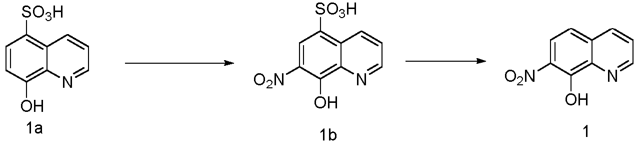

At 0°C, nitric acid (65 weight%) (8.0 mL, 116.4 mmol) was slowly added dropwise to a suspension of 8-hydroxyquinoline-5-sulfonic acid (1a) (10.0 g, 44.4 mmol) in sulfuric acid (98 weight%) (40.0 mL). The resulting mixture was stirred at 0°C for 10 minutes, poured into ice water (50.0 mL) and continued to stir for 15 minutes. The pH of the aqueous phase was adjusted to about 5 to 6 with saturated aqueous potassium carbonate solution. The resulting mixture was filtered under reduced pressure, and the filter cake was washed with water (10.0 mL x 3), and dried under vacuum to obtain 8-hydroxy-7-nitroquinoline-5-sulfonic acid (1b) (9.0 g, yield 76%).

At room temperature, sulfuric acid (98 weight%) (2.8 mL) was slowly added to a suspension of 8-hydroxy-7-nitroquinoline-5-sulfonic acid (1b) (9.0 g, 33.3 mmol) in acetic acid (28.0 mL). The resulting mixture was stirred at 120°C for 6 hours, cooled to room temperature, poured into ice water (100.0 mL) and continued to stir for 15 minutes. The pH of the aqueous phase was adjusted to about 6 to 7 with ammonia (25 to 28 weight%, NH₃ content). The resulting mixture was filtered under reduced pressure, and the filter cake was washed successively with water (10.0 mL x 2) and methanol (5.0 mL x 2), and dried under vacuum to obtain 7-nitroquinolin-8-ol (1) (5.0 g, yield 78%).

¹H NMR (400 MHz, DMSO-*d₆*) δ: 9.02 (dd, *J =* 4.0, 1.6 Hz, 1H), 8.52 (dd, *J =* 8.4, 1.6 Hz, 1H), 8.04 (d, *J* = 9.2 Hz, 1H), 7.81 (dd, *J =* 8.4, 4.4Hz, 1H), 7.48 (d, *J* = 9.2 Hz, 1H).

MS calculated: 190.04, MS found: 191.2 [M+H]⁺.

### Example 2

### Synthesis of 6-methyl-7-nitroquinolin-8-ol (2)

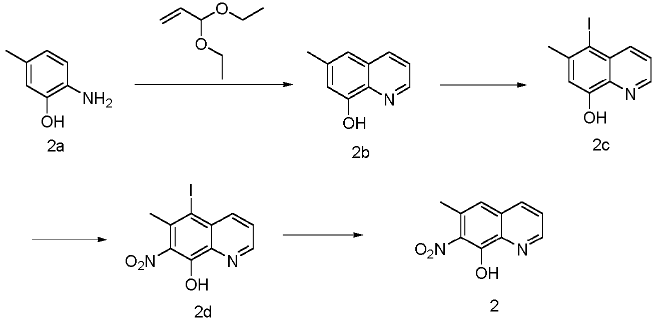

At room temperature, acrolein diethyl acetal (1.5 mL, 20.25 mmol) was added to a solution of 2-amino-5-methylphenol (2a) (1.0 g, 8.1 mmol) in hydrochloric acid (1.0 M) (40.0 mL). The reaction mixture was stirred at 110°C for 24 hours, and cooled to room temperature. The pH of the aqueous phase was adjusted to about 7 with solid sodium carbonate. The resulting mixture was extracted with dichloromethane (30.0 mL x 3), the combined organic phase was dried over anhydrous sodium sulfate and filtered, and the filtrate was concentrated under reduced pressure. The resulting residue was purified by silica gel column chromatography (eluent: dichloromethane/methanol = 10/1) to obtain 6-methylquinolin-8-ol (2b) (0.81 g, yield 63%).

At room temperature, 6-methylquinolin-8-ol (2b) (500.0 mg, 3.12 mmol) and N-iodosuccinimide (858.0 mg, 3.0 mmol) were added to chloroform (40.0 mL). The resulting mixture was stirred at 40°C for 15 minutes, cooled to room temperature, and diluted with aqueous sodium thiosulfate solution (10 weight%) (30.0 mL). The organic phase was separated, and the aqueous phase was extracted with dichloromethane (20.0 mL x 2). The combined organic phase was washed with saturated brine (20.0 mL x 2), dried over anhydrous sodium sulfate and filtered. The filtrate was concentrated to dryness under reduced pressure to obtain 5-iodo-6-methylquinolin-8-ol (2c) (232.0 mg, yield 92%).

At 0°C, a solution of sodium nitrate (59.4 mg, 0.70 mol) and lanthanum nitrate hexahydrate (30.2 mg, 0.070 mmol) in hydrochloric acid (6.0 M) (14.4 mL) was added dropwise to a solution of 5-iodo-6-methylquinolin-8-ol (2c) (200.0 mg, 0.70 mmol) in diethyl ether (20.0 mL). The reaction mixture was warmed up to room temperature, stirred for 1 hour, and extracted with ethyl acetate (50.0 mL x 3). The combined organic phase was washed with saturated brine (20.0 mL), dried over anhydrous sodium sulfate and filtered, and the filtrate was concentrated under reduced pressure. The resulting residue was purified by silica gel column chromatography (eluent: petroleum ether/ethyl acetate = 3/1) to obtain 5-iodo-6-methyl-7-nitroquinolin-8-ol (2d) (50.0 mg, yield 22%).

At room temperature, 5-iodo-6-methyl-7-nitroquinolin-8-ol (2d) (50.0 mg, 0.15 mmol) and tetrakis(triphenylphosphine)palladium (7.27 mg, 0.015 mmol) were added to N,N-dimethylformamide (3.0 mL). The reaction solution was stirred in a microwave reactor at 140 °C for 30 minutes, cooled to room temperature, and diluted with ammonia (2.0 M) (20.0 mL). The resulting mixture was extracted with ethyl acetate (20.0 mL x 2). The combined organic phase was washed with saturated brine (20.0 mL), dried over anhydrous sodium sulfate and filtered, and the filtrate was concentrated under reduced pressure. The resulting residue was purified by silica gel column chromatography (eluent: dichloromethane/methanol = 10/1) to obtain 6-methyl-7-nitroquinolin-8-ol (2) (16.0 mg, yield 52%).

¹H NMR (400 MHz, DMSO-*d₆*) δ: 8.95-8.93 (m, 1H), 8.29-8.27 (m, 1H), 7.93-7.90 (m, 1H), 7.10 (s, 1H), 2.51 (s, 3H).

MS calculated: 204.05; MS found: 205.0 [M+H]⁺.

### Example 3

### Synthesis of 5-iodo-7-nitroquinolin-8-ol (3)

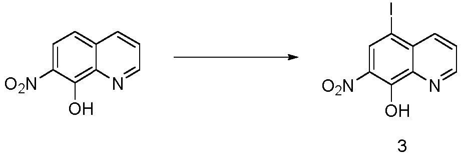

At room temperature, 7-nitroquinolin-8-ol (250.0 mg, 1.31 mmol) and N-iodosuccinimide (429.0 mg, 1.5 mmol) were added to chloroform (15.0 mL). The resulting mixture was stirred at 40°C for 15 minutes, cooled to room temperature, and diluted with aqueous sodium thiosulfate solution (10 weight%) (20.0 mL). The organic phase was separated, and the aqueous phase was extracted with dichloromethane (20.0 mL x 2). The combined organic phase was washed with saturated brine (20.0 mL x 2), dried over anhydrous sodium sulfate and filtered. The filtrate was concentrated to dryness under reduced pressure to obtain 5-iodo-7-nitroquinolin-8-ol (3) (367.0 mg, yield 88%).

¹H NMR (400 MHz, DMSO-*d₆*) δ: 9.02 (d, *J =* 2.8 Hz, 1H), 8.55 (d, *J =* 3.6 Hz, 1H), 8.45 - 8.42 (m, 1H), 7.95 - 7.92 (m, 1H).

MS calculated: 315.93; MS found: 316.9 [M+H]⁺.

### Example 4

### Synthesis of 5-methyl-7-nitroquinolin-8-ol (4)

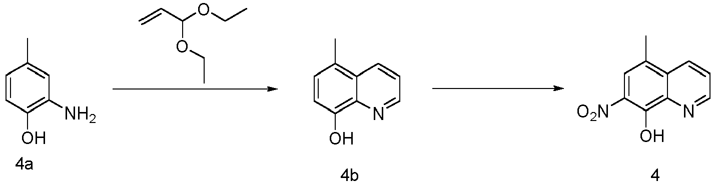

At room temperature, acrolein diethyl acetal (1.5 mL, 20.25 mmol) was added to a solution of 2-amino-4-methylphenol (4a) (1.0 g, 8.1 mmol) in hydrochloric acid (1.0 M) (40.0 mL). The reaction mixture was stirred at 110°C for 24 hours, and cooled to room temperature. The pH of the aqueous phase was adjusted to about 7 to 8 with solid sodium carbonate. The resulting mixture was extracted with dichloromethane (30.0 mL x 3), the combined organic phase was dried over anhydrous sodium sulfate and filtered, and the filtrate was concentrated under reduced pressure. The resulting residue was purified by silica gel column chromatography (eluent: petroleum ether/ethyl acetate = 1/1) to obtain 5-methylquinolin-8-ol (4b) (0.50 g, yield 46%).

At 0°C, nitric acid (65 weight%) (0.8 mL, 11.6 mmol) was slowly added dropwise to a suspension of 5-methylquinolin-8-ol (200.0 mg, 1.26 mmol) in sulfuric acid (98 weight%) (4.0 mL). The resulting mixture was stirred at 0°C for 10 minutes, poured into ice water (15.0 mL) and continued to stir for 15 minutes. The pH of the aqueous phase was adjusted to about 6 to 7 with saturated aqueous potassium carbonate solution. The resulting mixture was filtered under reduced pressure, the filter cake was washed with water (2.5 mL x 3), and dried under vacuum to obtain 5-methyl-7-nitroquinolin-8-ol (4) (180.0 mg, yield 70%).

¹H NMR (400 MHz, DMSO-*d₆*) δ: 9.04 - 9.03 (m, 1H), 8.55 - 8.52 (m, 1H), 7.89 (s, 1H), 7.85 - 7.82 (m, 1H), 2.59 (s, 3H).

MS calculated:204.05; MS found: 205.1 [M+H]⁺.

### Example 5

### Synthesis of 2-fluoro-7-nitroquinolin-8-ol (5)

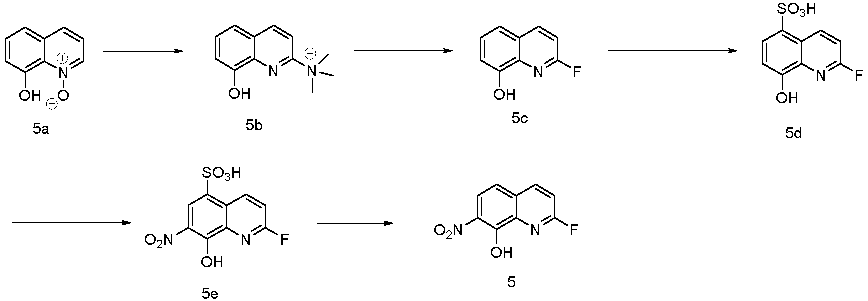

At 0°C, trifluoroacetic anhydride (2.5 mL, 37.0 mmol) was added to a solution of 8-hydroxyquinoline-N-oxide (5a) (2.0 g, 12.5 mmol) and trimethylamine (6.0 mL, 125.0 mmol) in dichloromethane (15.0 mL). The resulting mixture was warmed up to room temperature, stirred for 2 hours, and concentrated under reduced pressure to remove the organic solvent. Diethyl ether (10.0 mL) was added to the resulting residue, stirred for 10 minutes, and filtered under reduced pressure. The filter cake was washed with diethyl ether (10.0 mL), and then purified by silica gel column chromatography (eluent: dichloromethane/methanol = 10/1) to obtain 8-hydroxy-N,N,N-trimethylquinolin-2-aminium (5b) (1.7 g, yield 73%).

At room temperature, a solution (1.0 M) (3.0 mL) of tetra-n-butylammonium fluoride in tetrahydrofuran was added to a solution of 8-hydroxy-N,N,N-trimethylquinolin-2-aminium (5b) (203.0 mg, 1.0 mmol) in N,N-dimethylformamide (5.0 mL). The reaction solution was stirred at 90°C for 1 hour, cooled to room temperature, diluted with ethyl acetate (20.0 mL), and washed with water (20.0 mL) and saturated brine (20.0 mL) successively. The organic phase was separated, dried over anhydrous sodium sulfate and filtered, and the filtrate was concentrated under reduced pressure. The resulting residue was purified by silica gel column chromatography (eluent: petroleum ether/ethyl acetate = 5/1) to obtain 2-fluoroquinolin-8-ol (5c) (106.0 mg, yield 65%).

At room temperature, fuming sulfuric acid (18 to 24 weight% SO₃ content) (0.2 mL) was added to a solution of 2-fluoroquinolin-8-ol (5c) (100.0 mg, 0.61 mmol) in sulfuric acid (98 weight%) (0.4 mL). The reaction solution was stirred at 65°C for 2 hours, cooled to room temperature, poured into ice water (5.0 mL), and filtered under reduced pressure. The filter cake was washed with acetone (2.0 mL x 3), and dried under vacuum to obtain 2-fluoro-8-hydroxyquinoline-5-sulfonic acid (5d) (130.0 mg, yield 94%).

At 0°C, nitric acid (65 weight%) (0.8 mL, 11.6 mmol) was added dropwise to a solution of 2-fluoro-8-hydroxyquinoline-5-sulfonic acid (5d) (100.0 mg, 0.41 mmol) in sulfuric acid (98 weight%) (4.0 mL). The resulting mixture was stirred at 0°C for 10 minutes, poured into ice water (15.0 mL) and continued to stir for 15 minutes. The pH of the aqueous phase was adjusted to about 5 to 6 with saturated aqueous potassium carbonate solution. The resulting mixture was filtered under reduced pressure, the filter cake was washed with water (1.0 mL x 3), and dried under vacuum to obtain 2-fluoro-8-hydroxy-7-nitroquinoline-5-sulfonic acid (5e) (90.0 mg, yield 76%).

At room temperature, sulfuric acid (98 weight%) (0.8 mL) was slowly added to a suspension of 2-fluoro-8-hydroxy-7-nitroquinoline-5-sulfonic acid (5e) (90.0 mg, 0.32 mmol) in acetic acid (2.8 mL). The resulting mixture was stirred at 120°C for 6 hours, cooled to room temperature, poured into ice water (15.0 mL) and continued to stir for 15 minutes. The pH of the aqueous phase was adjusted to about 6 to 7 with ammonia (25 to 28 weight%, NH₃ content). The resulting mixture was filtered under reduced pressure, the filter cake was washed with water (1.0 mL x 2), and dried under vacuum to obtain 2-fluoro-7-nitroquinolin-8-ol (5) (52.0 mg, yield 78%).

¹H NMR (400 MHz, CDCl₃) δ: 9.50-9.46 (m, 1H), 8.56 (d, *J =* 8.8 Hz, 1H), 8.22 (s, 1H), 7.42-7.39 (m, 1H), 7.28 (s, 1H).

MS calculated:208.03; MS found: 207.1 [M-H]⁻.

### Example 6

### Synthesis of 2-methyl-7-nitroquinolin-8-ol (6)

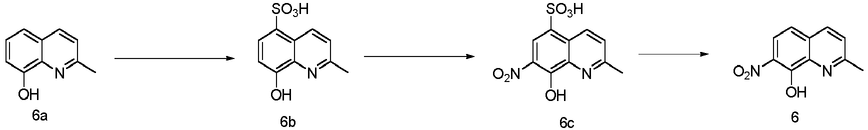

At room temperature, fuming sulfuric acid (18 to 24 weight% SO₃ content) (2.0 mL) was added to a solution of 2-methyl-8-hydroxyquinoline (6a) (1.0 g, 6.28 mmol) in sulfuric acid (98 weight%) (4.0 mL). The reaction solution was stirred at 65°C for 2 hours, cooled to room temperature, and poured into ice water (20.0 mL). The resulting suspension was diluted with acetone (60.0 mL), stirred for 10 minutes, and filtered under reduced pressure. The filter cake was washed with acetone (15.0 mL x 2), and dried under vacuum to obtain 8-hydroxy-2-methylquinoline-5-sulfonic acid (6b) (1.4 g, yield 94%).

At 0°C, nitric acid (65 weight%) (0.8 mL, 11.6 mmol) was added dropwise to a solution of 8-hydroxy-2-methylquinoline-5-sulfonic acid (6b) (100.0 mg, 0.42 mmol) in sulfuric acid (98 weight%) (4.0 mL). The resulting mixture was stirred at 0°C for 10 minutes, poured into ice water (15.0 mL) and continued to stir for 15 minutes. The pH of the aqueous phase was adjusted to about 5 to 6 with saturated aqueous potassium carbonate solution. The resulting mixture was filtered under reduced pressure, the filter cake was washed with water (1.0 mL x 3), and dried under vacuum to obtain 8-hydroxy-2-methyl-7-nitroquinoline-5-sulfonic acid (6c) (94.3 mg, yield 79%).

At room temperature, sulfuric acid (98 weight%) (0.8 mL) was slowly added to a suspension of 8-hydroxy-2-methyl-7-nitroquinoline-5-sulfonic acid (6c) (90.0 mg, 0.32 mmol) in acetic acid (2.8 mL). The resulting mixture was stirred at 120°C for 6 hours, cooled to room temperature, poured into ice water (15.0 mL) and continued to stir for 15 minutes. The pH of the aqueous phase was adjusted to about 6 to 7 with ammonia (25 to 28 weight%, NH₃ content). The resulting mixture was filtered under reduced pressure, the filter cake was washed with water (1.0 mL x 2), and dried under vacuum to obtain 2-methyl-7-nitroquinolin-8-ol (6) (51.0 mg, yield 78%).

¹H NMR (400 MHz, DMSO-*d₆*) δ: 8.38 (d, *J =* 8.4 Hz, 1H), 7.97 (d, *J =* 9.2 Hz, 1H), 7.68 (d, *J =* 8.4 Hz, 1H), 7.41 (d, *J =* 9.2 Hz, 1H), 2.77 (s, 3H).

MS calculated: 204.05; MS found: 205.1 [M+H]⁺.

### Example 7

### Synthesis of 5-chloro-6-(methylamino)-7-nitroquinolin-8-ol (7)

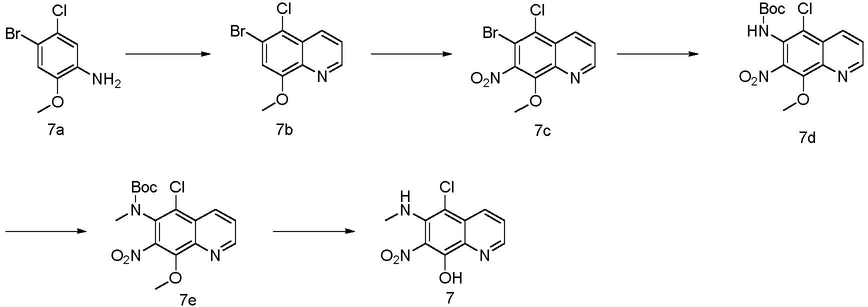

At room temperature, 4-bromo-5-chloro-2-methoxyaniline (7a) (3.80 g, 16.1 mmol), sodium 3-nitrobenzene sulfonate (4.34 g, 19.3 mmol) and glycerol (2.96 mL, 40.2 mmol) were successively added to sulfuric acid (70 wt.%) (40.4 mL). The resulting mixture was stirred at 140°C for 4 hours, and cooled to room temperature. The pH of the aqueous phase was adjusted to about 8 to 9 with aqueous sodium hydroxide solution (1.0 M). The resulting mixture was extracted with dichloromethane (50.0 mL x 3). The organic phases were combined, dried over anhydrous sodium sulfate, filtered, and concentrated to dryness under reduced pressure to obtain crude 6-bromo-5-chloro-8-methoxyquinoline (7b) (3.80 g, crude yield 87%).

At 0°C, nitric acid (65 wt.%) (8.4 mL, 198.0 mmol) was slowly added dropwise to a solution of crude 6-bromo-5-chloro-8-methoxyquinoline (7b) (1.80 g, 6.6 mmol) in acetic anhydride (30.0 mL), and stirred for 30 minutes. Sulfuric acid (98 wt.%) (1.20 mL, 22.6 mmol) was slowly added dropwise. The reaction mixture was stirred at 0°C for 30 minutes, then warmed up to room temperature and stirred for 48 hours, and then cooled to 0°C. The pH of the aqueous phase was adjusted to about 8 to 9 with aqueous sodium hydroxide solution (1.0 M). The resulting mixture was extracted with ethyl acetate (50.0 mL x 3). The organic phases were combined, washed with saturated brine (50.0 mL), dried over anhydrous sodium sulfate, filtered, and concentrated under reduced pressure. The resulting residue was purified by silica gel column chromatography (gradient elution, eluent: petroleum ether/ethyl acetate = 9/1 -2/1) to obtain 6-bromo-5-chloro-8-methoxy-7-nitroquinoline (7c) (0.551 g, yield 24%).

At room temperature, 6-bromo-5-chloro-8-methoxy-7-nitroquinoline (7c) (300.0 mg, 0.94 mmol), tert-butyl carbamate (165.0 mg, 1.41 mmol), palladium acetate (21.0 mg, 0.094 mmol), 4,5-bis(diphenylphosphino)-9,9-dimethylxanthene (108.0 mg, 0.187 mmol) and cesium carbonate (606.0 mg, 1.86 mmol) were added successively to 1,4-dioxane (6.0 mL). The resulting mixture was stirred at 95°C for 4 hours, cooled to room temperature, and filtered through diatomite, and the filter cake was washed with ethyl acetate (10.0 mL x 3). The filtrates were combined and concentrated to dryness under reduced pressure. The resulting residue was purified by silica gel column chromatography (gradient elution, eluent: petroleum ether/ethyl acetate = 9/1 -2/1) to obtain *tert*-butyl (5-chloro-8-methoxy-7-nitroquinolin-6-yl)carbamate (7d) (130.0 mg, yield 56%).

At room temperature, *tert*-butyl (5-chloro-8-methoxy-7-nitroquinolin-6-yl)carbamate (7d) (120.0 mg, 0.34 mmol), cesium carbonate (332.0 mg, 1.02 mmol), and methyl iodide (62.8 mg, 0.44 mmol) were added successively to N,N-dimethylformamide (3.0 mL). The resulting mixture was stirred at room temperature for 2 hours, and concentrated to dryness under reduced pressure. The resulting residue was purified by silica gel column chromatography (eluent: petroleum ether/ethyl acetate = 1.5/1) to obtain *tert*-butyl (5-chloro-8-methoxy-7-nitroquinolin-6-yl)(methyl)carbamate (7e) (122.0 mg, yield 96%).

At room temperature, *tert*-butyl (5-chloro-8-methoxy-7-nitroquinolin-6-yl)(methyl)carbamate (7e) (122.0 mg, 0.33 mmol) and lithium chloride (278.6 mg, 6.6 mmol) were added to N,N-dimethylformamide (4.0 mL). The resulting mixture was stirred at 120°C for 1.5 hours, cooled to room temperature, and concentrated to dryness under reduced pressure. A solution of hydrogen chloride in 1,4-dioxane (4.0 M) (1.0 mL) was added to the resulting residue, stirred at room temperature for 2 hours, and concentrated to dryness under reduced pressure. The pH of the aqueous phase was adjusted to about 9 to 10 by the addition of ammonia (25 to 28 wt.%, NH₃ content) to the resulting residue. The resulting mixture was filtered under reduced pressure, the filter cake was washed with water (0.5 mL x 2), and dried under vacuum to obtain 5-chloro-6-(methylamino)-7-nitroquinolin-8-ol (7) (31.0 mg, yield 37%).

¹H NMR (400 MHz, DMSO-*d6*) δ 8.39 (s, 1H), 8.12 (d, *J =* 8.4 Hz, 1H), 7.50 (dd, *J* = 8.4, 4.0 Hz, 1H), 5.62 (br s, 1H), 2.80 (s, 3H).

MS calculated: 253.03; MS found: 254.1, 256.1 [M+H]⁺.

### Example 8

### Synthesis of 3-methyl-7-nitroquinolin-8-ol (8)

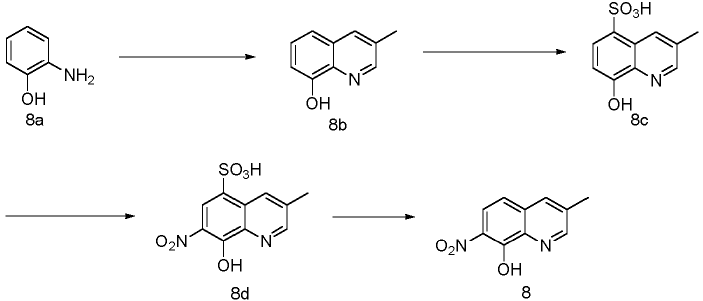

At 110°C, 2-methacrolein (1.0 mL, 4.0 mmol) was slowly added dropwise to a solution of 2-aminophenol (8a) (200.0 mg, 1.83 mmol) in hydrochloric acid (6.0 M) (10.0 mL). The reaction mixture was stirred at 110°C for 2 hours, and cooled to room temperature. The pH of the aqueous phase was adjusted to about 7 to 8 with aqueous sodium hydroxide solution (6.0 M). The resulting mixture was extracted with ethyl acetate (15.0 mL x 3). The combined organic phase was washed with saturated brine (20.0 mL), dried over anhydrous magnesium sulfate and filtered, and the filtrate was concentrated under reduced pressure. The resulting residue was purified by silica gel column chromatography (gradient elution, eluent: petroleum ether/ethyl acetate=5/1-3/1) to obtain 3-methylquinolin-8-ol (8b) (172.0 mg, yield 52%).

At room temperature, fuming sulfuric acid (18 to 24 weight% SO₃ content) (2.0 mL) was added to a solution of 3-methylquinolin-8-ol (8b) (100.0 mg, 0.63 mmol) in sulfuric acid (98 weight%) (4.0 mL). The reaction solution was stirred at 65°C for 2 hours, cooled to room temperature, and poured into ice water (20.0 mL). The resulting suspension was diluted with acetone (60.0 mL), stirred for 10 minutes, and filtered under reduced pressure. The filter cake was washed with acetone (15.0 mL x 2), and dried under vacuum to obtain 8-hydroxy-3-methylquinoline-5-sulfonic acid (8c) (139.0 mg, yield 77%).

At 0°C, nitric acid (65 weight%) (0.8 mL, 11.6 mmol) was added dropwise to a solution of 8-hydroxy-3-methylquinoline-5-sulfonic acid (8c) (139.0 mg, 0.58 mmol) in sulfuric acid (98 weight%) (4.0 mL). The resulting mixture was stirred at 0°C for 10 minutes, poured into ice water (15.0 mL) and continued to stir for 15 minutes. The pH of the aqueous phase was adjusted to about 5 to 6 with saturated aqueous potassium carbonate solution. The resulting mixture was filtered under reduced pressure, the filter cake was washed with water (1.0 mL x 3), and dried under vacuum to obtain 8-hydroxy-3-methyl-7-nitroquinoline-5-sulfonic acid (8d) (128.6 mg, yield 78%).

At room temperature, sulfuric acid (98 weight%) (0.8 mL) was slowly added to a suspension of 8-hydroxy-3-methyl-7-nitroquinoline-5-sulfonic acid (8d) (100.0 mg, 0.35 mmol) in acetic acid (2.8 mL). The resulting mixture was stirred at 120°C for 6 hours, cooled to room temperature, poured into ice water (15.0 mL) and continued to stir for 15 minutes. The pH of the aqueous phase was adjusted to about 6 to 7 with ammonia (25 to 28 weight%, NH₃ content). The resulting mixture was filtered under reduced pressure, the filter cake was washed with water (1.0 mL x 2), and dried under vacuum to obtain 3-methyl-7-nitroquinolin-8-ol (8) (49.3 mg, yield 69%).

¹H NMR (400 MHz, DMSO-*d₆*) δ: 8.87 (s, 1H), 8.25 (s, 1H), 8.01 (d, *J =* 9.2 Hz, 1H), 7.32 (d, *J =* 8.0 Hz, 1H), 2.54(s, 3H).

MS calculated: 204.05; MS found: 205.0 [M+H]⁺.

### Example 9

### Synthesis of 8-hydroxy-7-nitroquinoline-5-carbonitrile (9)

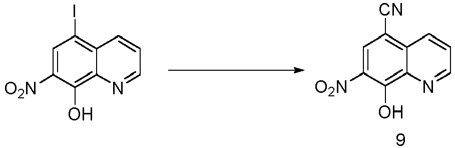

At room temperature, 5-iodo-7-nitroquinolin-8-ol (200.0 mg, 0.63 mmol), zinc cyanide (147.0 mg, 1.26 mmol) and tetrakis(triphenylphosphine)palladium (72.7 mg, 0.063 mmol) were added successively to N,N-dimethylformamide (3.0 mL). The reaction mixture was stirred in a microwave reactor at 140°C for 30 minutes, cooled to room temperature, and diluted with ammonia (2.0 M) (20.0 mL). The resulting mixture was extracted with ethyl acetate (20.0 mL x 2). The combined organic phase was washed with saturated brine (20.0 mL x 2), dried over anhydrous sodium sulfate and filtered, and the filtrate was concentrated under reduced pressure. The resulting residue was purified by silica gel column chromatography (eluent: dichloromethane/methanol = 10/1) to obtain 8-hydroxy-7-nitroquinoline-5-carbonitrile (9) (36.0 mg, yield 27%).

¹H NMR (400 MHz, DMSO-*d₆*) δ: 8.64 (s, 1H), 8.58 (s, 1H), 8.46 (s, *J =* 8.0 Hz, 1H), 7.93 - 7.90 (m, 1H), 7.06 (s, 1H).

MS calculated: 215.03; MS found: 216.0 [M+H]⁺.

### Example 10

### Synthesis of 4-methyl-7-nitroquinolin-8-ol (10)

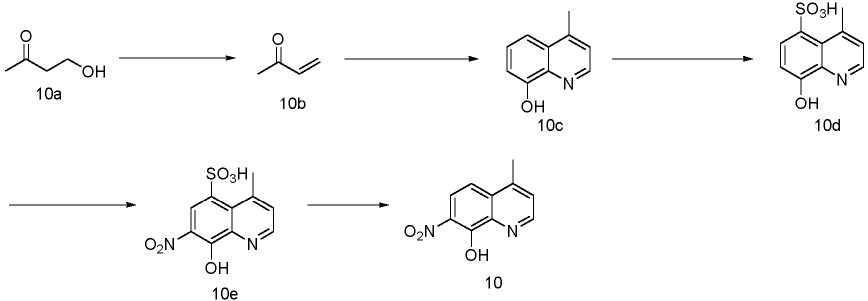

At room temperature, 4-hydroxy-2-butanone (10a) (12.3 g, 139.6 mmol) was added to a mixture of methanol (10.0 mL) and water (2.0 mL), followed by the addition of phosphoric acid (85 weight%) (0.4 mL). The resulting mixture was stirred at room temperature for 30 minutes, and distilled under reduced pressure (pressure range: 150.0 to 200.0 mmHg), and the fraction with a boiling point of 80°C was collected. Saturated brine (10.0 mL) was added to the collected fraction, and stirred at 4°C for 1 hour. The organic phase was separated, dried over anhydrous sodium sulfate, and filtered to obtain but-3-en-2-one (10b) (0.86 g, yield 9%).

At 110°C, but-3-en-2-one (10b) (0.86 g, 12.3 mmol) was slowly added dropwise to a solution of 2-aminophenol (200.0 mg, 1.83 mmol) in hydrochloric acid (6.0 M) (10.0 mL). The reaction mixture was stirred at 110°C for 2 hours, and cooled to room temperature. The pH of the aqueous phase was adjusted to about 7 to 8 with aqueous sodium hydroxide solution (6.0 M). The resulting mixture was extracted with ethyl acetate (20.0 mL x 3). The combined organic phase was washed with saturated brine (20.0 mL), dried over anhydrous magnesium sulfate and filtered, and the filtrate was concentrated under reduced pressure. The resulting residue was purified by silica gel column chromatography (gradient elution, eluent: petroleum ether/ethyl acetate=5/1-3/1) to obtain 4-methylquinolin-8-ol (10c) (171.9 mg, yield 63%).

At room temperature, fuming sulfuric acid (18 to 24 weight% SO₃ content) (2.0 mL) was added to a solution of 4-methylquinolin-8-ol (10c) (171.9 mg, 1.08 mmol) in sulfuric acid (98 weight%) (4.0 mL). The reaction solution was stirred at 65°C for 2 hours, cooled to room temperature, and poured into ice water (20.0 mL). The resulting suspension was diluted with acetone (60.0 mL), stirred for 10 minutes, and filtered under reduced pressure. The filter cake was washed with acetone (15.0 mL x 2), and dried under vacuum to obtain 8-hydroxy-4-methylquinoline-5-sulfonic acid (10d) (193.8 mg, yield 75%).

At 0°C, nitric acid (65 weight%) (0.8 mL, 11.6 mmol) was added dropwise to a solution of 8-hydroxy-4-methylquinoline-5-sulfonic acid (10d) (193.8 mg, 0.81 mmol) in sulfuric acid (98 weight%) (4.0 mL). The resulting mixture was stirred at 0°C for 10 minutes, poured into ice water (15.0 mL) and continued to stir for 15 minutes. The pH of the aqueous phase was adjusted to about 5 to 6 with saturated aqueous potassium carbonate solution. The resulting mixture was filtered under reduced pressure, the filter cake was washed with water (1.0 mL x 3), and dried under vacuum to obtain 8-hydroxy-4-methyl-7-nitroquinoline-5-sulfonic acid (10e) (179.6 mg, yield 78%).

At room temperature, sulfuric acid (98 weight%) (0.8 mL) was slowly added to a suspension of 8-hydroxy-4-methyl-7-nitroquinoline-5-sulfonic acid (10e) (90.0 mg, 0.32 mmol) in acetic acid (2.8 mL). The resulting mixture was stirred at 120°C for 6 hours, cooled to room temperature, poured into ice water (15.0 mL) and continued to stir for 15 minutes. The pH of the aqueous phase was adjusted to about 6 to 7 with ammonia (25 to 28 weight%, NH₃ content). The resulting mixture was filtered under reduced pressure, the filter cake was washed with water (1.0 mL x 2), and dried under vacuum to obtain 4-methyl-7-nitroquinolin-8-ol (10) (51.0 mg, yield 78%).

¹H NMR (400 MHz, DMSO-*d₆*) δ: 8.87 (s, 1H), 8.25 (s, 1H), 8.01 (d, *J =* 9.2 Hz, 1H), 7.32 (d, *J =* 8.0 Hz, 1H), 2.54(s, 3H).

MS calculated: 204.05; MS found: 205.0 [M+H]⁺.

### Example 11

### Synthesis of 4-cyclopropyl-7-nitroquinolin-8-ol (11)

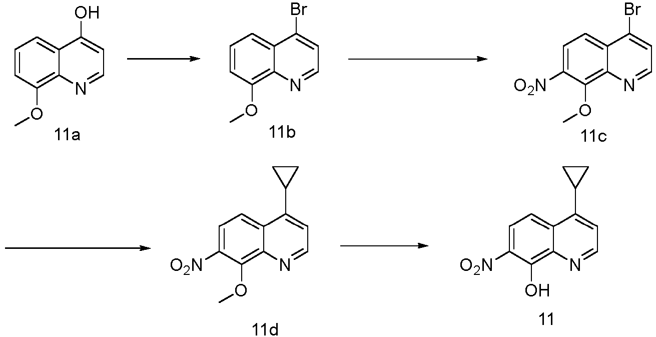

At room temperature, 4-hydroxy-8-methoxyquinoline (11a) (3.0 g, 17.1 mmol) and phosphorus tribromide (9.3 g, 34.2 mmol) were added successively to N,N-dimethylformamide (25.0 mL). The reaction mixture was stirred at 65°C for 6 hours, cooled to 0°C, and diluted with water (50.0 mL). The pH of the aqueous phase was adjusted to about 8 to 9 with ammonia (25 to 28 weight%, NH₃ content). The resulting mixture was extracted with ethyl acetate (100.0 mL x 3), the combined organic phase was dried over anhydrous sodium sulfate and filtered, and the filtrate was concentrated under reduced pressure. The resulting residue was purified by silica gel column chromatography (eluent: petroleum ether/ethyl acetate = 7/3) to obtain 4-bromo-8-methoxyquinoline (11b) (3.7 g, yield 91%).

At 0°C, 4-bromo-8-methoxyquinoline (11b) (3.0 g, 12.7 mmol), concentrated nitric acid (65 weight%) (10.0 mL) and concentrated sulfuric acid (98 weight%) (7.0 mL) were added successively to acetic anhydride (25.0 mL). The reaction mixture was warmed up to room temperature, stirred for 2 hours, and diluted with ice water (100.0 mL). The pH of the aqueous phase was adjusted to about 8 to 9 with ammonia (25 to 28 weight%, NH₃ content). The resulting mixture was extracted with dichloromethane (100.0 mL x 3), the combined organic phase was dried over anhydrous sodium sulfate and filtered, and the filtrate was concentrated under reduced pressure. The resulting residue was purified by silica gel column chromatography (eluent: petroleum ether/ethyl acetate = 4/1) to obtain 4-bromo-7-nitro-8-methoxyquinoline (11c) (0.30 g, yield 8%).

At room temperature, 4-bromo-7-nitro-8-methoxyquinoline (11c) (100.0 mg, 0.35 mmol), cyclopropylboronic acid (61.6 mg, 0.72 mmol), tetrakis(triphenylphosphine)palladium (82.0 mg, 0.07 mmol) and potassium carbonate (147.0 mg, 1.07 mmol) were added successively to a mixture of toluene (5.0 mL) and water (0.5 mL). The reaction solution was stirred at 100°C for 2 hours, cooled to room temperature, and diluted with water (20.0 mL). The resulting mixture was extracted with ethyl acetate (50.0 mL x 3), the combined organic phase was dried over anhydrous sodium sulfate and filtered, and the filtrate was concentrated under reduced pressure. The resulting residue was purified by silica gel column chromatography (eluent: petroleum ether/ethyl acetate = 3/1) to obtain 4-cyclopropyl-7-nitro-8-methoxyquinoline (11d) (60.0 mg, yield 70%).

At room temperature, 4-cyclopropyl-7-nitro-8-methoxyquinoline (11d) (60.0 mg, 0.25 mmol) and lithium chloride (105.0 mg, 2.5 mmol) were added to N,N-dimethylformamide (2.0 mL). The reaction solution was stirred at 180°C for 1 hour, and cooled to room temperature. The reaction solution was concentrated under reduced pressure to remove the organic solvent. Water (3.0 mL) was added to the resulting residue, stirred for 3 minutes, and filtered under reduced pressure. The filter cake was washed successively with water (0.5 mL x 2) and ethanol (0.5 mL x 2), and dried under vacuum to obtain 4-cyclopropyl-7-nitroquinolin-8-ol (11) (16.0 mg, yield 28%).

¹H-NMR (400 Hz, DMSO-*d₆*) δ: 8.50 - 8.40 (m, 1H), 8.00 - 7.88 (m, 1H), 7.15 - 7.06 (m, 1H), 6.95 - 6.86 (m, 1H), 2.30 - 2.45 (m, 1H), 1.20 - 1.05 (m, 2H), 0.90 - 0.70 (m, 2H).

MS calculated: 230.07; MS found: 231.1 [M+H]⁺.

### Example 12

### Synthesis of 4-chloro-7-nitroquinolin-8-ol (12)

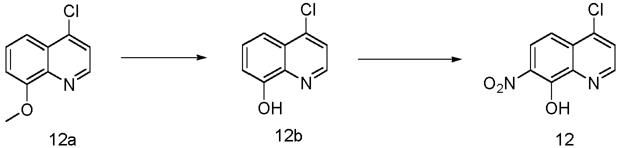

At 0°C, a solution of boron tribromide in dichloromethane (1.0 M) (50.0 mL, 50.0 mmol) was slowly added dropwise to a solution of 4-chloro-8-methoxyquinoline (12a) (2.0 g, 10.0 mmol) in dichloromethane (20.0 ml). The reaction mixture was warmed up to room temperature and stirred for 30 minutes. The pH of the aqueous phase was adjusted to about 8 to 9 with saturated aqueous sodium bicarbonate solution. The organic phase was separated, and the aqueous phase was extracted with dichloromethane (20.0 mL x 2). The combined organic phase was dried over anhydrous sodium sulfate, filtered, and concentrated under reduced pressure. The resulting residue was dried under vacuum to obtain crude 4-chloro-8-hydroxyquinoline (12b) (1.6 g, crude yield 89%).

At 0°C, sodium nitrite (2.0 g, 30.0 mmol) was added in batches to a suspension of 4-chloro-8-hydroxyquinoline (1.08 g, 6.0 mmol) (12b) in hydrochloric acid (2.0 M) (36.0 mL). The reaction mixture was stirred at 0°C for 1 hour, and then at room temperature for 4 hours, and filtered under reduced pressure. The filter cake was added to methanol (30.0 mL), stirred for 15 minutes, and filtered under reduced pressure. The resulting filter cake was washed with methanol (5.0 mL x 2), and dried under vacuum to obtain 4-chloro-7-nitroquinolin-8-ol (12) (0.80 g, yield 59%).

¹H-NMR (400 MHz, DMSO-d6): δ 7.68 (d, *J =* 9.6Hz, 1H), 8.04 (d, *J =* 4.8Hz, 1H), 8.18 (d, *J =* 9.6Hz,1H), 8.95 (d, *J =* 4.8Hz,1H).

MS calculated: 224.60; MS found: 225.0 [M+H]⁺.

### Example 13

### Synthesis of 7-nitro-4-(pyrrolidin-1-yl)quinolin-8-ol

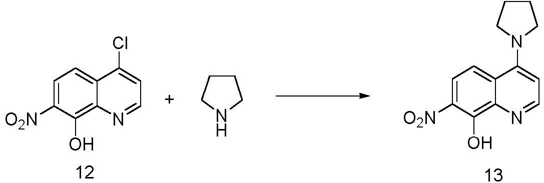

At room temperature, tetrahydropyrrole (142.0 mg, 4.45 mmol) was added to a solution of 4-chloro-7-nitroquinolin-8-ol (12) (100.0 mg, 0.45 mmol) in N,N-dimethylformamide (5.0 mL). The resulting mixture was stirred at 120°C for 2 hours, cooled to room temperature, and concentrated under reduced pressure to remove the organic solvent. Water (10.0 mL) was added to the resulting residue, stirred for 3 minutes, and filtered under reduced pressure. The filter cake was washed with water (1.0 mL x 3), and dried under vacuum to obtain 7-nitro-4-(pyrrolidin-1-yl)quinolin-8-ol (13) (92.0 mg, yield 79%).

¹H-NMR (400 MHz, DMSO-*d₆*) δ: 13.10 (br s, 1H), 8.08 (d, *J =* 5.6 Hz, 1H), 7.80 (d, *J* = 9.6 Hz, 1H), 6.92 (d, *J =* 10.0 Hz, 1H), 6.73 (d, *J =* 4.4 Hz, 1H), 3.84 (br s, 4H), 2.01 (br s, 4H).

MS calculated: 259.10; MS found: 260.1 [M+H]⁺.

### Example 14

### Synthesis of 4-morpholino-7-nitroquinolin-8-ol (14)

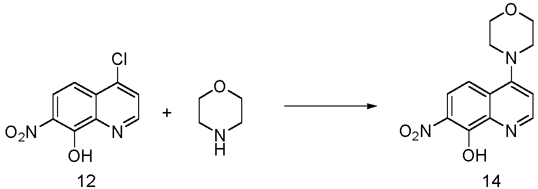

In accordance with the same synthesis method of Example 13, 4-morpholino-7-nitroquinolin-8-ol (14) (110.0 mg, yield 91%) was obtained from 4-chloro-7-nitroquinolin-8-ol (12) (100.0 mg, 0.44 mmol) and morpholine (192.0 mg, 2.2 mmol).

¹H NMR (400 MHz, DMSO-*d₆*) δ: 8.44 (d, *J =* 5.6 Hz, 1H), 7.94 (d, *J =* 9.6 Hz, 1H), 7.22 (d, *J =* 6.0 Hz, 1H), 6.66 (d, *J =* 9.6 Hz, 1H), 3.85 (br s, 4H), 3.54 (br s, 4H).

MS calculated: 275.09; MS found: 276.1 [M+H]⁺.

### Example 15

### Synthesis of 4-(1H-imidazol-1-yl)-7-nitroquinolin-8-ol (15)

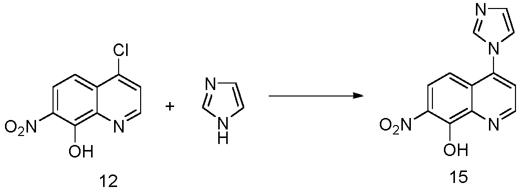

In accordance with the same synthesis method of Example 13, 4-(*1H*-imidazol-1-yl)-7-nitroquinolin-8-ol (15) (50.0 mg, yield 85%) was obtained from 4-chloro-7-nitroquinolin-8-ol (12) (50.0 mg, 0.23 mmol) and imidazole (61.5 mg, 0.9 mmol).

¹H-NMR (400 MHz, DMSO-*d₆*) δ: 8.15 (s, 1H), 8.05 - 8.08 (m, 2H), 7.78 (s,1H), 7.71 (m, 1H), 7.26(s, 1H),7.21(s, 1H).

MS calculated: 256.06; MS found: 257.1[M+H]⁺.

### Example 16

### Synthesis of 4-bromo-7-nitroquinolin-8-ol (16)

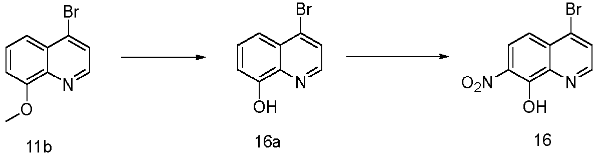

At 0°C, a solution of boron tribromide in dichloromethane (1.0 M) (36.4 mL, 36.4 mmol) was slowly added dropwise to a solution of 4-bromo-8-methoxyquinoline (11b) (1.9 g, 8.1 mmol) in dichloromethane (25.0 ml). The reaction mixture was warmed up to room temperature and stirred for 24 hours. The pH of the aqueous phase was adjusted to about 8 to 9 with saturated aqueous sodium bicarbonate solution. The organic phase was separated, and the aqueous phase was extracted with dichloromethane (50.0 mL x 3). The combined organic phase was dried over anhydrous sodium sulfate and filtered, and the filtrate was concentrated under reduced pressure. The resulting residue was purified by silica gel column chromatography (eluent: dichloromethane/methanol = 19/1) to obtain 4-bromoquinolin-8-ol (16a) (1.0 g, yield 55%).

At 0°C, sodium nitrite (1.4 g, 20.3 mmol) was added in batches to a suspension of 4-bromoquinolin-8-ol (16a) (460 mg, 2.05 mmol) in hydrochloric acid (2.0 M) (30.0 mL). The reaction mixture was warmed up to room temperature and stirred for 24 hours. The pH of the aqueous phase was adjusted to about 8 to 9 with ammonia (25 to 28 weight%, NH₃ content). The resulting mixture was filtered under reduced pressure, the filter cake was washed with ethanol (4.0 mL x 3), and dried under vacuum to obtain 4-bromo-7-nitroquinolin-8-ol (16) (500.0 mg, yield 91%).

¹H-NMR (400 Hz, DMSO-*d₆*) δ: 8.84 - 7.77 (m, 1 H), 8.20 - 8.12 (m, 2 H), 7.60 - 7.55 (m, 1 H).

MS calculated: 267.95; MS found: 269.0 [M+H]⁺.

### Example 17

### Synthesis of 4-(dimethylamino)-7-nitroquinolin-8-ol (17)

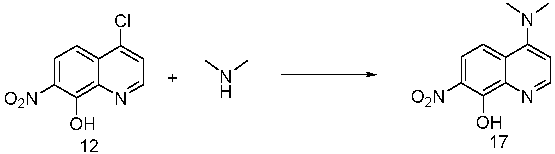

At room temperature, 4-chloro-7-nitroquinolin-8-ol (12) (100.0 mg, 0.44 mmol) was added to aqueous dimethylamine solution (40 weight%) (10.0 mL). The reaction mixture was stirred at 110°C for 2 hours, cooled to room temperature, and concentrated under reduced pressure to remove the solvent. Ethanol (5.0 mL) was added to the resulting residue, stirred for 3 minutes, and filtered under reduced pressure. The filter cake was washed with ethanol (1.0 mL x 2), and dried under vacuum to obtain 4-(dimethylamino)-7-nitroquinolin-8-ol (17) (36.0 mg, yield 35%).

¹H NMR (400 MHz, DMSO-*d₆*) δ: 8.18 (d, *J =* 6.8 Hz, 1H), 7.84 (d, *J =* 9.8 Hz, 1H), 6.95 (d, *J =* 6.8 Hz, 1H), 6.70 (d, *J =* 9.8 Hz, 1H), 3.32 (s, 6H).

MS calculated: 233.08; MS found: 234.1 [M+H]⁺.

### Example 18

### Synthesis of 4-(4-methylpiperazinyl)-7-nitroquinolin-8-ol (18)

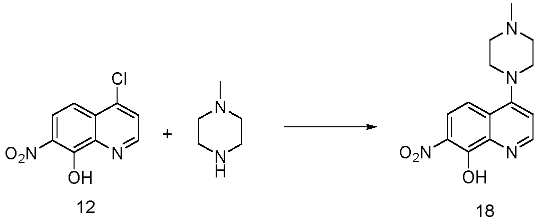

In accordance with the same synthesis method of Example 13, 4-(4-methylpiperazinyl)-7-nitroquinolin-8-ol (18) (40.0 mg, yield 63%) was obtained from 4-chloro-7-nitroquinolin-8-ol (12) (50.0 mg, 0.22 mmol) and 1-methylpiperazine (116.0 mg, 1.1 mmol).

¹H NMR (400 MHz, DMSO-*d₆*) δ: 8.57 (d, *J =* 6.0 Hz, 1H), 7.98 (d, *J =* 9.6 Hz, 1H), 7.34 (d, *J =* 6.0 Hz, 1H), 6.80 (d, *J =* 9.6 Hz, 1H), 3.55 - 3.21 (br s, 8H), 2.86 (s, 3H).

MS calculated: 288.12; MS found: 289.1 [M+H]⁺.

### Example 19

### Synthesis of 7-nitro-4-(3-oxetanylamino)quinolin-8-ol (19)

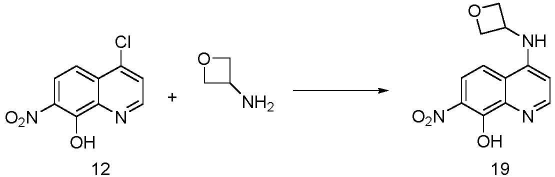

At room temperature, 3-oxetamine (78.9 mg, 1.08 mmol) was added to a solution of 4-chloro-7-nitroquinolin-8-ol (12) (80.0 mg, 0.36 mmol) in dimethyl sulfoxide (5.0 mL). The resulting mixture was stirred at 80°C for 4 hours, cooled to room temperature, and concentrated under reduced pressure to remove the organic solvent. Water (5.0 mL) was added to the resulting residue, stirred for 3 minutes, and filtered under reduced pressure. The resulting filter cake was mixed with methanol (10.0 mL), stirred for 3 minutes, and filtered under reduced pressure. The filter cake was washed with methanol (1.0 mL x 2), and dried under vacuum to obtain 7-nitro-4-(3-oxetanylamino)quinolin-8-ol (19) (65.0 mg, yield 69%).

¹H NMR (400 MHz, DMSO-*d₆*) δ: 8.89 (d, *J =* 4.4 Hz, 1H), 8.16 (d, *J =* 6.8 Hz, 1H), 7.96 (d, *J =* 9.6 Hz, 1H), 6.93 (d, *J =* 9.6 Hz, 1H), 6.57 (d, *J =* 6.8 Hz, 1H), 5.01 - 4.96 (m, 1H), 4.93 (t, *J =* 6.0 Hz, 2H), 4.74 (t, *J =* 6.0 Hz, 2H).

MS calculated: 261.07; MS found: 262.1 [M+H]⁺.

### Example 20

### Synthesis of 4-(2-methylpiperidinyl)-7-nitroquinolin-8-ol (20)

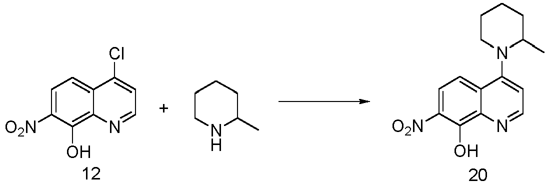

In accordance with the same synthesis method of Example 13, 4-(2-methylpiperidinyl)-7-nitroquinolin-8-ol (20) (19.0 mg, yield 15%) was obtained from 4-chloro-7-nitroquinolin-8-ol (12) (100.0 mg, 0.44 mmol) and 2-methylpiperidine (220.0 mg, 2.2 mmol).

¹H NMR (400 MHz, DMSO-*d₆*) δ: 8.02 - 7.86 (m, 2H), 7.20 - 7.10 (m, 1H), 6.76 - 6.62 (m, 1H), 3.14 - 2.83 (m, 3H), 1.85 - 1.55 (m, 6H), 1.10 - 0.95 (m, 3H).

MS calculated: 287.13; MS found: 288.1 [M+H]⁺.

### Example 21

### Synthesis of 4-methoxy-7-nitroquinolin-8-ol (21)

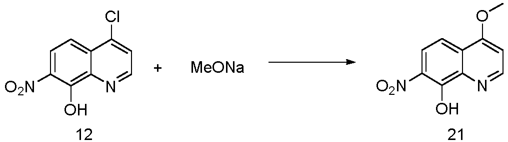

In accordance with the same synthesis method of Example 13, 4-methoxy-7-nitroquinolin-8-ol (21) (26.0 mg, yield 54%) was obtained from 4-chloro-7-nitroquinolin-8-ol (12) (50.0 mg, 0.22 mmol) and sodium methoxide (120.0 mg, 2.2 mmol).

¹H NMR (400 MHz, DMSO-*d₆*) δ: 8.56 - 8.42 (m, 1H), 7.91 - 7.84 (m, 1H), 7.10 - 7.03 (m, 1H), 6.65 - 6.56 (m, 1H), 4.01 (s, 3H).

MS calculated: 220.05; MS found: 221.0 [M+H]⁺.

### Example 22

### Synthesis of 4-phenyl-7-nitroquinolin-8-ol (22)

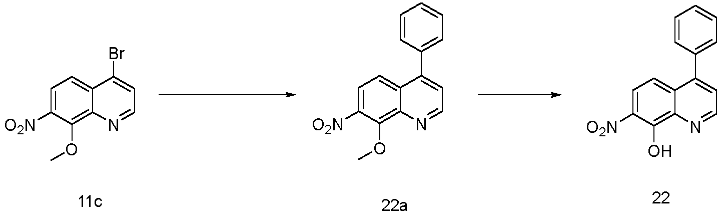

At room temperature, 4-bromo-7-nitro-8-methoxyquinoline (11c) (60.0 mg, 0.25 mmol), phenylboronic acid (62.0 mg, 0.50 mmol), tetrakis(triphenylphosphine)palladium (58.0 mg, 0.05 mmol) and potassium carbonate (100.0 mg, 0.75 mmol) were added successively to a mixed solvent of toluene (2.0 mL) and water (0.2 mL). The reaction mixture was stirred at 110°C for 2 hours, cooled to room temperature, and diluted with water (10.0 mL). The resulting mixture was extracted with ethyl acetate (10.0 mL x 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 7/3) to obtain 4-phenyl-7-nitro-8-methoxyquinoline (22a) (60.0 mg, yield 86%).

At room temperature, 4-phenyl-7-nitro-8-methoxyquinoline (22a) (60.0 mg, 0.21 mmol) and lithium chloride (90.0 mg, 2.1 mmol) were added to N,N-dimethylformamide (2.0 mL). The reaction mixture was stirred at 180°C for 1 hour, cooled to room temperature, and concentrated under reduced pressure to remove the organic solvent. Water (3.0 mL) was added to the resulting residue, stirred for 3 minutes, and filtered under reduced pressure. The filter cake was washed successively with water (0.5 mL x 2) and ethanol (0.5 mL x 2), and dried under vacuum to obtain 4-phenyl-7-nitroquinolin-8-ol (22) (25.0 mg, yield 45%).

¹H-NMR (400 MHz, DMSO-*d₆*) δ: 8.70 - 8.60 (m, 1H), 7.93 - 7.84 (m, 1H), 7.61 - 7.48 (m, 5H), 7.46 - 7.41 (m, 1H), 6.42 - 6.33 (m, 1H).

MS calculated: 266.07; MS found: 267.1 [M+H]⁺.

### Example 23

### Synthesis of 4-(2,5-dihydro-1H-pyrrolyl)-7-nitroquinolin-8-ol (23)

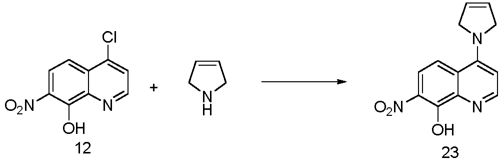

In accordance with the same synthesis method of Example 19, 4-chloro-7-nitroquinolin-8-ol (12) (60.0 mg, 0.27 mmol) and 3-pyrroline (91.0 mg, 1.32 mmol) were reacted at 100°C to obtain 4-(2,5-dihydro-1H-pyrrolyl)-7-nitroquinolin-8-ol (23) (34.6 mg, yield 50%).

¹H-NMR (400 MHz, DMSO-*d₆*) δ: 8.20 - 8.12 (m, 1 H), 7.87 - 7.80 (m, 1 H), 7.14 - 7.07 (m, 1 H), 6.75 - 6.70 (m, 1 H), 6.11 (s, 2 H), 4.75 (s, 4 H).

MS calculated: 257.08; MS found: 258.1 [M+H]⁺.

### Example 24

### Synthesis of 4-(2-(dimethylamino)ethoxy)-7-nitroquinolin-8-ol (24)

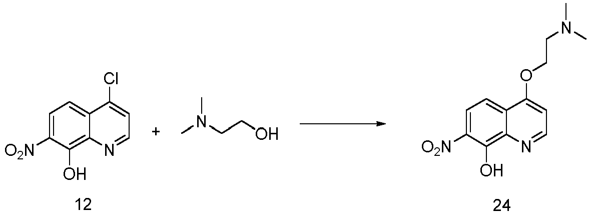

At room temperature, sodium hydride (60 weight%, a mixture in mineral oil) (130.0 mg, 3.24 mmol) was added to a solution of N,N-dimethylethanolamine (238.0 mg, 2.7 mmol) in dimethyl sulfoxide (2.0 mL). The resulting mixture was stirred at room temperature for 1 hour, followed by the addition of 4-chloro-7-nitroquinolin-8-ol (12) (60.0 mg, 0.27 mmol). The resulting mixture was warmed up to 95°C, stirred for 2 hours, cooled to room temperature, and concentrated under reduced pressure to remove the organic solvent. Water (3.0 mL) was added to the resulting residue, stirred for 3 minutes, and filtered under reduced pressure. The filter cake was washed successively with water (0.5 mL x 2) and ethanol (0.5 mL x 2), and dried under vacuum to obtain 4-(2-(dimethylamino)ethoxy)-7-nitroquinolin-8-ol (24) (25.7 mg, yield 34%).

¹H NMR (400 MHz, DMSO-*d₆*) δ: 8.90 - 8.70 (m, 1H), 8.20 - 8.10 (m, 1H), 7.60 - 7.35 (m, 2H), 4.71 - 4.50 (m, 2H), 3.80 - 3.60 (m, 2H), 3.10 - 2.80 (m, 4H).

MS calculated: 277.11; MS found: 278.1 [M+H]⁺.

### Example 25

### Synthesis of 4-(8-hydroxy-7-nitroquinolin-4-yl)-N,N-dimethylpiperazine-1-carboxamide (25)

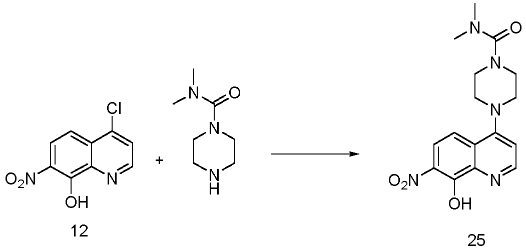

In accordance with the same synthesis method of Example 19, 4-chloro-7-nitroquinolin-8-ol (12) (60.0 mg, 0.27 mmol) and N,N-dimethylpiperazine-1-carboxamide (126.0 mg, 0.81 mmol) were reacted at 100°C to obtain 4-(8-hydroxy-7-nitroquinolin-4-yl)-N,N-dimethylpiperazine-1-carboxamide (25) (20.0 mg, yield 21%).

¹H NMR (400 MHz, DMSO-*d₆*) δ: 8.46 - 8.42 (m, 1H), 7.97 - 7.91 (m, 1H), 7.23 - 7.18 (m, 1H), 6.73 - 6.67 (m, 1H), 3.69 - 3.63 (m, 7H), 3.60 - 3.53 (m, 4H).

MS calculated: 345.14; MS found: 346.1 [M+H]⁺.

### Example 26

### Synthesis of methyl 4-(8-hydroxy-7-nitroquinolin-4-yl)-N,N-dimethylpiperazine-1-carboxylate (26)

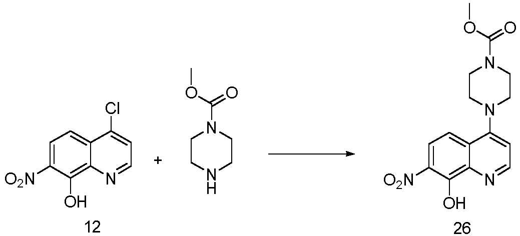

In accordance with the same synthesis method of Example 19, 4-chloro-7-nitroquinolin-8-ol (12) (60.0 mg, 0.27 mmol) and methyl piperazine-1-carboxylate (130.0 mg, 0.90 mmol) were reacted at 100°C to obtain methyl 4-(8-hydroxy-7-nitroquinolin-4-yl)-N,N-dimethylpiperazine-1-carboxylate (26) (21.7 mg, yield 24%).

¹H NMR (400 MHz, DMSO-*d₆*) δ: 8.46 - 8.42 (m, 1H), 7.97 - 7.91 (m, 1H), 7.23 - 7.18 (m, 1H), 6.73 - 6.67 (m, 1H), 3.69 - 3.63 (m, 7H), 3.60 - 3.53 (m, 4H).

MS calculated: 332.11; MS found: 333.1 [M+H]⁺.

### Example 27

### Synthesis of 4-fluoro-7-nitroquinolin-8-ol (27)

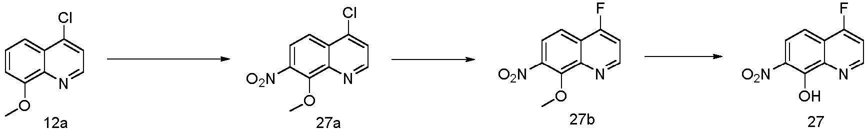

At 0°C, nitric acid (65 weight%) (3.0 mL, 43.6 mmol) was slowly added to a solution of 4-chloro-8-methoxyquinoline (12a) (5.0 g, 25.1 mmol) in acetic anhydride (40.0 mL), and stirred for 10 minutes. Sulfuric acid (98 weight%) (3.0 mL, 55.2 mmol) was slowly added dropwise. The reaction mixture was warmed up to room temperature, stirred for 1 hour, and diluted with ice water (50.0 mL). The pH of the aqueous phase was adjusted to about 8 to 9 with ammonia (25 to 28 weight%, NH₃ content). The resulting mixture was extracted with dichloromethane (10.0 mL x 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 2/1) to obtain 4-chloro-7-nitro-8-methoxyquinoline (27a) (1.19 g, yield 20%).

At room temperature, 4-chloro-7-nitro-8-methoxyquinoline (27a) (150.0 mg, 0.63 mmol) and cesium fluoride (480.0 mg, 3.1 mmol) were added to dimethyl sulfoxide (2.0 mL). The reaction mixture was stirred at 160°C for 30 minutes, cooled to room temperature, diluted with dichloromethane (10.0 mL), and washed with water (10.0 mL x 2). The organic phase was dried over anhydrous sodium sulfate and filtered, and the filtrate was concentrated under reduced pressure. The resulting residue was purified by silica gel column chromatography (eluent: petroleum ether/ethyl acetate = 4/1) to obtain 4-fluoro-7-nitro-8-methoxyquinoline (27b) (50.0 mg, yield 36%).

At room temperature, 4-fluoro-7-nitro-8-methoxyquinoline (27b) (50.0 mg, 0.22 mmol) and lithium chloride (100.0 mg, 2.2 mmol) were added to N,N-dimethylformamide (2.0 mL). The reaction mixture was stirred at 120°C for 1 hour, cooled to room temperature, and concentrated under reduced pressure to remove the organic solvent. Water (5.0 mL) was added to the resulting residue, stirred for 3 minutes, and filtered under reduced pressure. The filter cake was washed successively with water (0.5 mL x 2) and ethanol (0.5 mL x 2), and dried under vacuum to obtain 4-fluoro-7-nitroquinolin-8-ol (27) (46.0 mg, yield 98%).

¹H NMR (400 MHz, DMSO-*d₆*) δ 8.84 - 8.37 (m, 1H), 7.95 (d, *J =* 8.0 Hz, 1H), 7.50 - 7.22 (m, 1H), 6.44 (d, *J =* 8.0 Hz, 1H).

MS calculated: 208.03; MS found: 209.0 [M+H]⁺.

### Example 28

### Synthesis of methyl (8-hydroxy-7-nitroquinolin-4-yl)-L-prolinate (28)

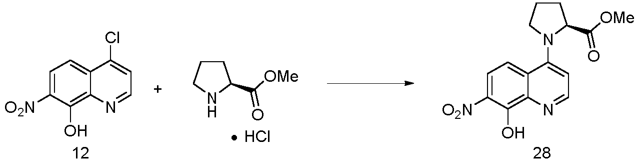

At room temperature, methyl *L*-prolinate hydrochloride (178.0 mg, 1.07 mmol) and N,N-diisopropylethylamine (139.0 mg, 1.08 mmol) were added to dimethyl sulfoxide (1.5 mL). The reaction solution was stirred at room temperature for 1 hour, followed by the addition of 4-chloro-7-nitroquinolin-8-ol (12) (80.0 mg, 0.36 mmol). The resulting mixture was stirred at 100°C for 2 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/acetonitrile=100%/0 - 70%/30%, gradient elution, 0.1% formic acid was added to the mobile phase by volume percentage, and the flow rate was 20.0 mL/min) to obtain the product methyl (8-hydroxy-7-nitroquinolin-4-yl)-L-prolinate (28) (115.0 mg, yield 99%).

¹H NMR (400 MHz, DMSO-*d₆*) δ: 8.22 - 8.11 (m, 1H), 7.89 - 7.72 (m, 1H), 6.85 - 6.77 (m, 1H), 6.74 - 6.66 (m, 1H), 5.16 - 5.06 (m, 1H), 4.10 - 3.95 (m, 2H), 3.67 (s, 3H), 2.16 - 1.89 (m, 4H).

MS calculated: 317.10; MS found: 318.1 [M+H]⁺.

### Example 29

### Synthesis of 4,5-dichloro-7-nitroquinolin-8-ol (29)

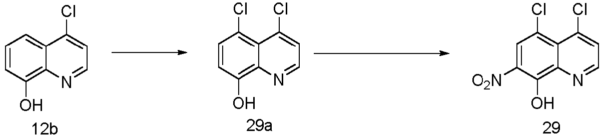

At 0°C, N-chlorosuccinimide (588 mg, 4.4 mmol) was added in three batches to a solution of 4-chloroquinolin-8-ol (12b) (790.0 mg, 4.4 mmol) in sulfuric acid (98 weight%) (5.0 mL). The reaction mixture was stirred at 0°C for 0.5 hours, and then at room temperature for 2 hours. The pH of the aqueous phase was adjusted to about 8 to 9 with ammonia (25 to 28 weight%, NH₃ content). The resulting mixture was extracted with dichloromethane (20.0 mL x 3). The combined organic phase was washed with saturated brine (20.0 mL), dried over anhydrous sodium sulfate, filtered, and concentrated under reduced pressure. The resulting residue was purified by silica gel column chromatography (eluent: petroleum ether/ethyl acetate = 1/1) to obtain 4,5-dichloroquinolin-8-ol (29a) (703.0 mg, yield 75%).

At 0°C, sodium nitrite (2.28 g, 33.0 mmol) was added in batches to a suspension of 4,5-dichloroquinolin-8-ol (29a) (0.703 g, 3.3 mmol) in hydrochloric acid (3.0 M) (20.0 mL). The reaction mixture was stirred at 0°C for 0.5 hours, and then at room temperature for 12 hours. The pH of the aqueous phase was adjusted to about 8 to 9 with ammonia (25 to 28 weight%, NH₃ content). The resulting mixture was filtered under reduced pressure, the filter cake was washed with water (3.0 mL x 2), and dried under vacuum to obtain 4,5-dichloro-7-nitroquinolin-8-ol (29) (0.641 g, yield 75%).

¹H NMR (400 MHz, DMSO-*d₆*) δ 8.93 (d, *J =* 4.8 Hz, 1H), 8.22 (s, 1H), 8.05 (d, *J* = 4.8 Hz, 1H).

MS calculated: 257.96; MS found: 259.0 [M+H]⁺.

### Example 30

### Synthesis of 7-nitroquinoline-4,8-diol (30)

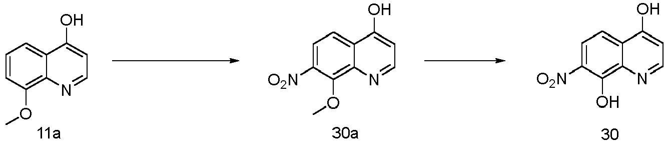

At 0°C, nitric acid (65 weight%) (10.0 mL, 145.5 mmol) was slowly added to a solution of 4-hydroxy-8-methoxyquinoline (11a) (3.0 g, 17.14 mmol) in acetic anhydride (25.0 mL), and stirred for 10 minutes. Sulfuric acid (98 weight%) (7.0 mL, 128.7 mmol) was slowly added dropwise. The reaction mixture was warmed up to room temperature, stirred for 2 hours, and diluted with ice water (100.0 mL). The pH of the aqueous phase was adjusted to about 8 to 9 with ammonia (25 to 28 weight%, NH₃ content). The resulting mixture was extracted with dichloromethane (100.0 mL x 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 1/4) to obtain 8-methoxy-7-nitroquinolin-4-ol (30a) (0.30 g, yield 8%).

At room temperature, 4-cyclopropyl-7-nitro-8-methoxyquinoline (70.0 mg, 0.32 mmol) and lithium chloride (200.0 mg, 4.78 mmol) were added to N,N-dimethylformamide (2.0 mL). The reaction mixture was stirred at 180°C for 1 hour, cooled to room temperature, and concentrated under reduced pressure to remove the organic solvent. Water (3.0 mL) was added to the resulting residue, stirred for 3 minutes, and filtered under reduced pressure. The filter cake was washed successively with water (0.5 mL x 2) and ethanol (0.5 mL x 2), and dried under vacuum to obtain 7-nitroquinoline-4,8-diol (30) (37.0 mg, yield 56%).

¹H NMR (400 MHz, DMSO-*d₆*) δ: 11.31 (br s, 1H), 7.71 - 6.65 (m, 1H), 7.62 - 7.56 (m, 1H), 6.65 - 6.55 (m, 1H), 6.10 - 6.00 (m, 1H).

MS calculated: 206.03; MS found: 207.1 [M+H]⁺.

### Example 31

### Synthesis of methyl 4-(5-chloro-8-hydroxy-7-nitroquinolin-4-yl)-piperazine-1-carboxylate (31)

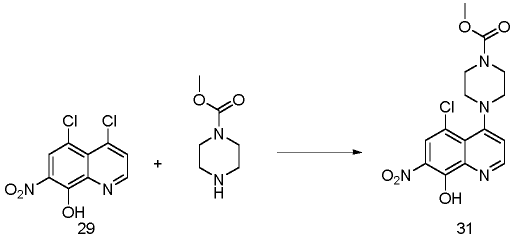

At room temperature, 4,5-dichloro-7-nitroquinolin-8-ol (29) (50.0 mg, 0.19 mmol) and methyl piperazine-1-carboxylate (82.2 mg, 0.57 mmol) were added to N,N-dimethylformamide (2.0 mL). The reaction solution was stirred at 100°C for 1 hour, cooled to room temperature, and concentrated under reduced pressure to remove the organic solvent. Water (3.0 mL) was added to the resulting residue, stirred for 3 minutes, and filtered under reduced pressure. The filter cake was washed successively with water (0.5 mL x 2) and ethanol (0.5 mL x 2), and dried under vacuum to obtain methyl 4-(5-chloro-8-hydroxy-7-nitroquinolin-4-yl)-piperazine-1-carboxylate (30.0 mg, yield 43%).

¹H NMR (400 MHz, DMSO-*d₆*) δ 8.49 - 8.29 (m, 1H), 8.00 - 7.85 (m, 1H), 7.46 - 7.28 (m, 1H), 3.63 (s, 3H), 3.37 (br s, 8H).

MS calculated: 366.07; MS found: 367.1 [M+H]⁺.

### Example 32

### Synthesis of 4-cyclohexyl-7-nitroquinolin-8-ol (32)

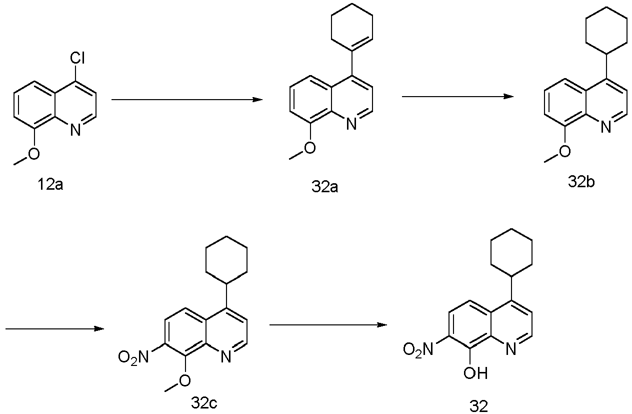

At room temperature, 4-chloro-8-methoxyquinoline (12a) (1.0 g, 5.2 mmol), cyclohexenylboronic acid (0.975 g, 7.7 mmol), tetrakis(triphenylphosphine)palladium (0.600 g, 0.50 mmol) and potassium carbonate (1.5 g, 11.0 mmol) were added successively to a mixed solvent of toluene (10.0 mL), ethanol (1.0 mL) and water (2.0 mL). The reaction mixture was stirred at 100°C for 12 hours, cooled to room temperature, and diluted with water (20.0 mL). The resulting mixture was extracted with ethyl acetate (50.0 mL x 2). The organic phases were combined, dried over anhydrous sodium sulfate and filtered, and the filtrate was concentrated under reduced pressure. The resulting residue was purified by silica gel column chromatography (eluent: petroleum ether/ethyl acetate = 1/1) to obtain 4-cyclohexenyl-8-methoxyquinoline (32a) (1.1 g, yield 89%).

At room temperature, 4-cyclohexenyl-8-methoxyquinoline (32a) (850.0 mg, 3.5 mmol) and palladium/carbon (palladium loading: 10 weight%) (85.0 mg, 0.080 mmol) were successively added to methanol (20.0 mL). The reaction mixture was stirred under a hydrogen atmosphere for 3 hours, and filtered under reduced pressure. The filter cake was washed with ethyl acetate (10.0 mL x 3), and the resulting filtrate was concentrated to dryness under reduced pressure to obtain 4-cyclohexyl-8-methoxyquinoline (32b) (850.0 mg, yield 99%).

At 0°C, nitric acid (65 weight%) (1.0 mL, 14.5 mmol) was slowly added to a solution of 4-cyclohexyl-8-methoxyquinoline (32b) (800.0 mg, 3.3 mmol) in acetic anhydride (5.0 mL), and stirred for 10 minutes. Concentrated sulfuric acid (0.5 mL, 9.2 mmol) was slowly added dropwise. The reaction mixture was stirred at 0°C for 1 hour, and diluted with water (20.0 mL). The pH of the aqueous phase was adjusted to about 9 to 10 with ammonia (25 to 28 weight%, NH₃ content). The resulting mixture was extracted with ethyl acetate (30.0 mL x 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 1/1) to obtain 4-cyclohexyl-7-nitro-8-methoxyquinoline (32c) (220.0 mg, yield 23%).

At room temperature, 4-cyclohexyl-7-nitro-8-methoxyquinoline (32c) (150.0 mg, 0.53 mmol) and lithium chloride (230.0 mg, 5.3 mmol) were added to N,N-dimethylformamide (5.0 mL). The reaction mixture was stirred at 160°C for 1 hour, cooled to room temperature, and concentrated under reduced pressure to remove the organic solvent. Water (2.0 mL) was added to the resulting residue, stirred for 3 minutes, and filtered under reduced pressure. The filter cake was washed with water (1.0 mL x 2), and dried under vacuum to obtain 4-cyclohexyl-7-nitroquinolin-8-ol (32) (124.9 mg, yield 87%).

¹H NMR (400 MHz, DMSO-*d₆*) δ 8.52 (d, *J =* 4.0 Hz, 1H), 7.93 (d, *J =* 8.0 Hz, 1H), 7.40 (d, *J =* 4.0 Hz, 1H), 6.71 (d, *J =* 8.0 Hz, 1H), 3.25 - 3.09 (m, 1H), 1.94 - 1.78 (m, 4H), 1.58 - 1.38 (m, 4H), 1.38 - 1.20 (m, 2H).

MS calculated: 272.12; MS found: 273.1 [M+H]⁺.

### Example 33

### Synthesis of 4-(5-chloro-8-hydroxy-7-nitroquinolin-4-yl)-1-acetylpiperazine (33)

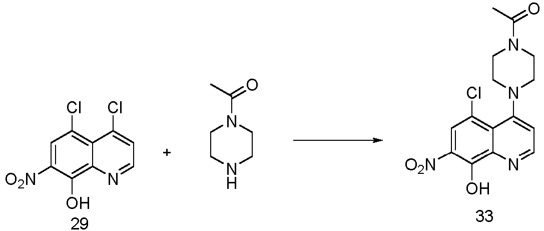

In accordance with the same synthesis method of Example 31, 4-(5-chloro-8-hydroxy-7-nitroquinolin-4-yl)-1-acetylpiperazine (33) (38.0 mg, yield 35%) was obtained from 4,5-dichloro-7-nitroquinolin-8-ol (29) (80.0 mg, 0.31 mmol) and 1-acetylpiperazine (119.0 mg, 0.93 mmol).

¹H NMR (400 MHz, DMSO-*d₆*) δ: 8.40 - 8.33 (m, 1H), 7.93 (s, 1H), 7.43 - 7.35 (m, 1H), 3.80 - 3.40 (m, 8H), 2.04 (s, 3H).

MS calculated: 350.08; MS found: 351.1 [M+H]⁺.

### Example 34

### Synthesis of 5-chloro-4-methoxy-7-nitroquinolin-8-ol (34)

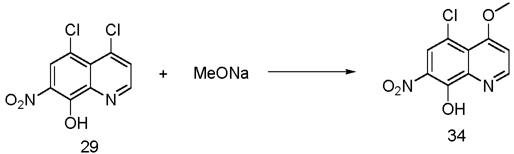

At room temperature, 4,5-dichloro-7-nitroquinolin-8-ol (29) (60.0 mg, 0.23 mmol) and sodium methoxide (62.1 mg, 1.15 mmol) were added to dimethyl sulfoxide (2.0 mL). The reaction mixture was stirred at 120°C for 2 hours, cooled to room temperature, and concentrated under reduced pressure to remove the organic solvent. Water (2.0 mL) was added to the resulting residue, stirred for 3 minutes, and filtered under reduced pressure. The filter cake was washed successively with water (0.5 mL x 2) and ethanol (0.5 mL x 2), and dried under vacuum to obtain 5-chloro-4-methoxy-7-nitroquinolin-8-ol (34) (51.0 mg, yield 87%).

¹H NMR (400 MHz, DMSO-*d₆*) δ: 8.50 - 8.44 (m, 1H), 7.83 (s, 1H), 7.15 - 7.09 (m, 1H), 3.93 (s, 3H).

MS calculated: 254.01; MS found: 255.0, 257.0 [M+H]⁺.

### Example 35

### Synthesis of 4-(5-chloro-8-hydroxy-7-nitroquinolin-4-yl)-N,N-dimethylpiperazine-1-carboxamide (35)

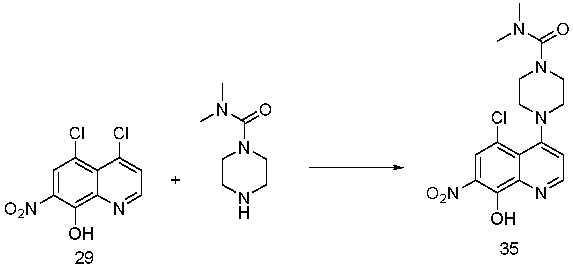

In accordance with the same synthesis method of Example 31, 4-(5-chloro-8-hydroxy-7-nitroquinolin-4-yl)-N,N-dimethylpiperazine-1-carboxamide (35) (30.0 mg, yield 42%) was obtained from 4,5-dichloro-7-nitroquinolin-8-ol (29) (50.0 mg, 0.19 mmol) and N,N-dimethylpiperazine-1-carboxamide (300.0 mg, 1.9 mmol).

¹H NMR (400 MHz, DMSO-*d*₆) δ 8.36 (d, *J =* 8.0 Hz, 1H), 7.92 (s, 1H), 7.39 (d, *J* = 8.0 Hz, 1H), 3.78 - 3.48 (m, 8H), 2.78 (s, 6H).

MS calculated: 379.10; MS found: 380.1 [M+H]⁺.

### Example 36

### Synthesis of 5-chloro-7-nitro-4-piperidinylquinolin-8-ol (36)

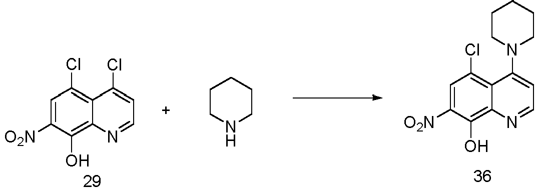

In accordance with the same synthesis method of Example 31, 5-chloro-7-nitro-4-piperidinylquinolin-8-ol (36) (50.0 mg, yield 71%) was obtained from 4,5-dichloro-7-nitroquinolin-8-ol (29) (60.0 mg, 0.23 mmol) and piperidine (170.0 mg, 2.3 mmol).

¹H NMR (400 MHz, DMSO-*d*₆) δ 8.32 (d, *J =* 8.0 Hz, 1H), 7.82 (s, 1H), 7.19 (d, *J* = 4.0 Hz, 1H), 3.12 - 3.01 (m, 4H), 1.79 - 1.61 (m, 6H).

MS calculated: 307.07; MS found: 308.1 [M+H]⁺.

### Example 37

### Synthesis of 5-chloro-7-nitro-4-(piperazin-1-yl)quinolin-8-ol (37)

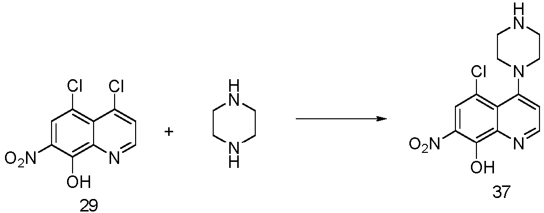

In accordance with the same synthesis method of Example 31, 5-chloro-7-nitro-4-(piperazin-1-yl)quinolin-8-ol (37) (200.0 mg, yield 65%) was obtained from 4,5-dichloro-7-nitroquinolin-8-ol (29) (259.0 mg, 1.0 mmol) and piperazine (258.6 mg, 3.0 mmol).

¹H NMR (400 MHz, CDCl₃) δ 8.89 - 8.70 (m, 1H), 8.25 - 8.06 (m, 1H), 7.88 - 7.73 (m, 1H), 3.05 - 2.97 (m, 4H), 2.29 - 1.98 (m, 4H).

MS calculated: 308.07; MS found: 309.1 [M+H]⁺.

### Example 38

### Synthesis of 5-chloro-4-(4-methoxypiperidin-1-yl)-7-nitroquinolin-8-ol (38)

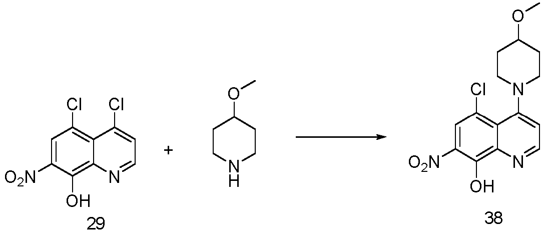

In accordance with the same synthesis method of Example 31, 5-chloro-4-(4-methoxypiperidin-1-yl)-7-nitroquinolin-8-ol (38) (40.0 mg, yield 51%) was obtained from 4,5-dichloro-7-nitroquinolin-8-ol (29) (60.0 mg, 0.23 mmol) and 4-methoxypiperidine (177.0 mg, 1.54 mmol).

¹H NMR (400 MHz, DMSO-*d₆*) δ 8.33 - 8.28 (m, 1H), 7.92 (s, 1H), 7.43 - 7.39 (m, 1H), 3.82 - 3.73 (m, 2H), 3.62 - 3.52 (m, 2H), 3.43 - 3.34 (m, 1H), 3.24 (s, 3H), 2.06 - 1.96 (m, 1H), 1.94 - 1.83 (m, 1H), 1.83 - 1.73 (m, 1H), 1.53 - 1.42 (m, 1H).

MS calculated: 337.08; MS found: 338.1 [M+H]⁺.

### Example 39

### Synthesis of 5-chloro-7-nitro-4-(4-(trifluoromethyl)piperidin-1-yl)quinolin-8-ol (39)

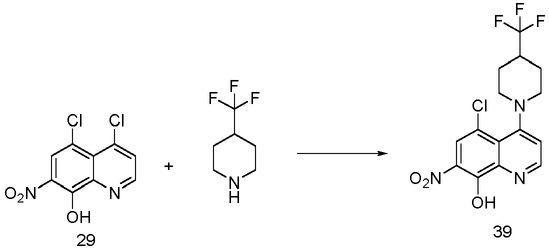

In accordance with the same synthesis method of Example 34, 5-chloro-7-nitro-4-(4-(trifluoromethyl)piperidin-1-yl)quinolin-8-ol (39) (80.0 mg, yield 93%) was obtained from 4,5-dichloro-7-nitroquinolin-8-ol (29) (60.0 mg, 0.23 mmol) and 4-(trifluoromethyl)piperidine (142.0 mg, 0.93 mmol).

¹H NMR (400 MHz, DMSO-*d*₆) δ: 8.50 - 8.40 (m, 1H), 7.87 (s, 1H), 7.25 - 7.14 (m, 1H), 3.70 - 3.50 (m, 1H), 3.00 - 2.60 (m, 4H), 2.00 - 1.55(m, 4H).

MS calculated: 375.06; MS found: 376.0 [M+H]⁺.

### Example 40

### Synthesis of 5-chloro-4-(4-methylpiperazin-1-yl)-7-nitroquinolin-8-ol (40)

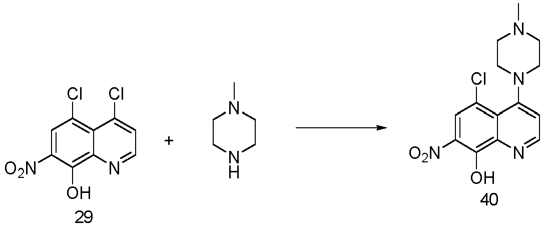

In accordance with the same synthesis method of Example 34, 5-chloro-4-(4-methylpiperazin-1-yl)-7-nitroquinolin-8-ol (40) (23.0 mg, yield 31%) was obtained from 4,5-dichloro-7-nitroquinolin-8-ol (29) (60.0 mg, 0.23 mmol) and 1-methylpiperazine (116.0 mg, 1.16 mmol).

¹H NMR (400 MHz, DMSO-*d*₆) δ: 8.50 - 8.35 (m, 1H), 8.14 (s, 1H), 7.94 (s, 1H), 7.46 - 7.38 (m, 1H), 3.85 - 3.50 (m, 4H), 3.15 - 2.90 (m, 4H), 2.65 - 2.55 (m, 3H).

MS calculated: 322.08; MS found: 323.1 [M+H]⁺.

### Example 41

### Synthesis of 5-chloro-4-(4,4-dimethylpiperidin-1-yl)-7-nitroquinolin-8-ol (41)

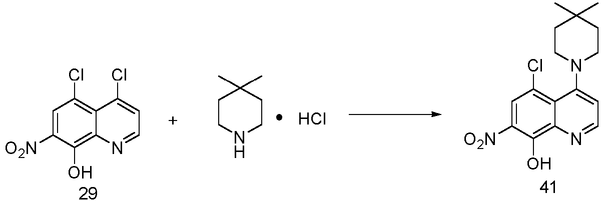

At room temperature, 4,5-dichloro-7-nitroquinolin-8-ol (29) (50.0 mg, 0.19 mmol), 4,4-dimethylpiperidine hydrochloride (87.0 mg, 0.58 mmol) and triethylamine (39.0 mg, 0.38 mmol) were added successively to acetonitrile (1.0 mL). The reaction mixture was stirred at 80°C for 1 hour, cooled to room temperature, and concentrated under reduced pressure to remove the organic solvent. Water (5.0 mL) was added to the resulting residue, stirred for 3 minutes, and filtered under reduced pressure. The filter cake was washed successively with water (0.5 mL x 2) and ethanol (0.5 mL x 2), and dried under vacuum to obtain 5-chloro-4-(4,4-dimethylpiperidin-1-yl)-7-nitroquinolin-8-ol (41) (52.0 mg, yield 80%).

¹H NMR (400 MHz, DMSO-*d₆*) δ 8.23 (d, *J* = 6.6 Hz, 1H), 7.86 (s, 1H), 7.35 (d, *J* = 6.8 Hz, 1H), 3.76 - 3.54 (m, 4H), 1.59 - 1.50 (m, 2H), 1.48- 1.40 (m, 2H) 1.06 (s, 3H), 0.91 (s, 3H).

MS calculated: 335.1; MS found: 336.1[M+H]⁺.

### Example 42

### Synthesis of 5-chloro-7-nitro-4-(pyrrolidin-1-yl)quinolin-8-ol (42)

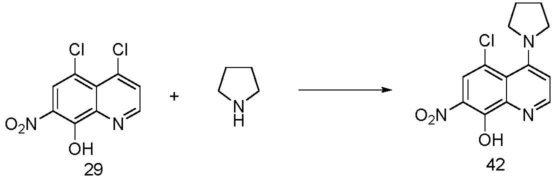

At room temperature, 4,5-dichloro-7-nitroquinolin-8-ol (29) (60.0 mg, 0.23 mmol) and tetrahydropyrrole (66.0 mg, 0.93 mmol) were added to acetonitrile (6.0 mL). The reaction mixture was stirred at 100°C for 1 hour, cooled to room temperature, and concentrated under reduced pressure to remove the organic solvent. Water (3.0 mL) was added to the resulting residue, stirred for 3 minutes, and filtered under reduced pressure. The filter cake was washed successively with water (0.5 mL x 2) and ethanol (0.5 mL x 2), and dried under vacuum to obtain 5-chloro-7-nitro-4-(pyrrolidin-1-yl)quinolin-8-ol (42) (58.0 mg, yield 86%).

¹H NMR (400 MHz, DMSO-*d*₆) δ: 8.30 - 8.10 (m, 1H), 7.74 (s, 1H), 7.00 - 6.85 (m, 1H), 7.46 - 7.38 (m, 1 H), 2.00 - 1.75 (m, 8 H).

MS calculated: 293.06; MS found: 294.0 [M+H]⁺.

### Example 43

### Preparation of 5-chloro-4-(2-fluoroethoxy)-7-nitroquinolin-8-ol (43)

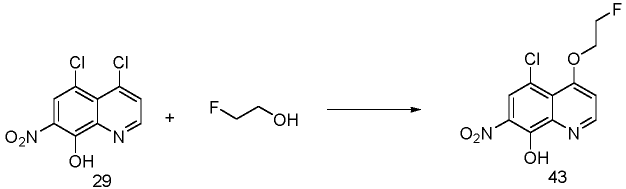

At room temperature, sodium hydride (60 weight%, a mixture in mineral oil) (46.0 mg, 1.16 mmol) was added to a solution of 2-fluoroethanol (147.0 mg, 2.3 mmol) in dimethyl sulfoxide (4.0 mL). The resulting mixture was stirred at room temperature for 1 hour, followed by the addition of 4,5-dichloro-7-nitroquinolin-8-ol (29) (60.0 mg, 0.23 mmol). The resulting mixture was stirred at 100°C for 2 hours, cooled to room temperature, and concentrated under reduced pressure to remove the organic solvent. Water (3.0 mL) was added to the resulting residue, stirred for 3 minutes, and filtered under reduced pressure. The filter cake was washed successively with water (0.5 mL x 2) and ethanol (0.5 mL x 2), and dried under vacuum to obtain 5-chloro-4-(2-fluoroethoxy)-7-nitroquinolin-8-ol (43) (40.0 mg, yield 61%).

¹H NMR (400 MHz, DMSO-*d*₆) δ: 8.46 (d, *J* = 4.4Hz, 1H), 7.85 (s, 1H), 7.13 (d, *J* = 5.2Hz, 1H), 4.91 - 4.88 (m, 1H), 4.79 - 4.76 (m, 1H), 4.46 - 4.43 (m, 1H), 4.39 - 4.36 (m, 1H).

MS calculated: 286.02; MS found: 287.0[M+H]⁺.

### Example 44

### Synthesis of 4-(5-chloro-8-hydroxy-7-nitroquinolin-4-yl)-N-methylpiperazine-1-carboxamide (44)

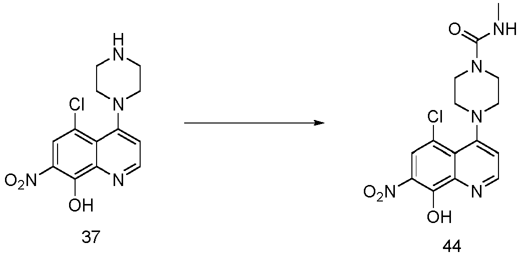

At 0°C, triethylamine (60.0 mg, 0.58 mmol), methylaminoformyl chloride (55.0 mg, 0.58 mmol) and N,N-dimethylformamide (0.1 mL) were added successively to a solution of 5-chloro-7-nitro-4-(piperazin-1-yl)quinolin-8-ol (37) (60.0 mg, 0.19 mmol) in dichloromethane (5.0 mL). The reaction mixture was warmed up to room temperature, stirred for 1 hour, and diluted with water (10.0 mL). The resulting mixture was extracted with dichloromethane (10.0 mL x 3). The organic phases were combined, dried over anhydrous sodium sulfate and filtered, and the filtrate was concentrated under reduced pressure. The resulting residue was purified by reversed-phase high performance liquid chromatography (the chromatographic column was Eclipse XDB-C18 (21.2 mm×250 mm, 7µm), the mobile phase was water/acetonitrile=100%/0 - 70%/30%, gradient elution, 0.1% formic acid was added to the mobile phase by volume percentage, and the flow rate was 20.0 mL/min) to obtain 4-(5-chloro-8-hydroxy-7-nitroquinolin-4-yl)-N-methylpiperazine-1-carboxamide (44) (46.0 mg, yield 66%).

¹H NMR (400 MHz, DMSO-*d*₆) δ 8.34 (d, *J =* 8.0 Hz, 1H), 7.91 (s, 1H), 7.39 (d, *J* = 8.0 Hz, 1H), 6.63 - 6.55 (m, 1H), 3.68 - 3.60 (m, 4H), 3.56 - 3.51 (m, 4H), 2.58 (d, *J* = 4.0 Hz, 3H).

MS calculated: 365.09; MS found: 366.1 [M+H]⁺.

### Example 45

### Synthesis of 4-(5-chloro-8-hydroxy-7-nitroquinolin-4-yl)-1-(2,2,2-trimethylacetyl)piperazine (45)

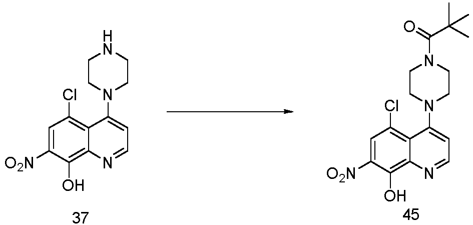

In accordance with the same synthesis method of Example 44, 4-(5-chloro-8-hydroxy-7-nitroquinolin-4-yl)-1-(2,2,2-trimethylacetyl)piperazine (45) (56.0 mg, yield 75%) was obtained from 5-chloro-7-nitro-4-(piperazin-1-yl)quinolin-8-ol (37) (60.0 mg, 0.19 mmol), trimethylacetyl chloride (73.0 mg, 0.60 mmol) and triethylamine (70.0 mg, 0.60 mmol).

¹H NMR (400 MHz, DMSO-*d*₆) δ 8.38 (d, *J* = 8.0 Hz, 1H), 7.93 (s, 1H), 7.37 (d, *J* = 8.0 Hz, 1H), 4.02 - 3.84 (m, 4H), 3.72 - 3.62 (m, 4H), 1.21 (s, 9H).

MS calculated: 392.13; MS found: 393.1 [M+H]⁺.

### Example 46

### Synthesis of ethyl 1-(5-chloro-8-hydroxy-7-nitroquinolin-4-yl)piperidine-4-carboxylate (46)

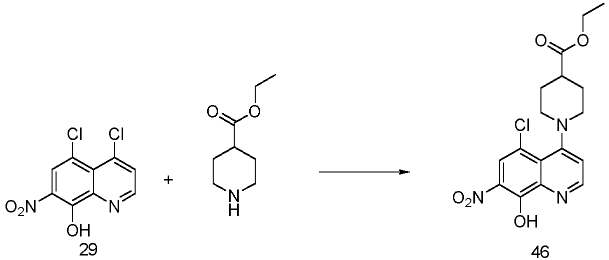

At room temperature, 4,5-dichloro-7-nitroquinolin-8-ol (29) (50.0 mg, 0.19 mmol) and ethyl 4-piperidinecarboxylate (91.0 mg, 0.58 mmol) were added to acetonitrile (1.0 mL). The reaction mixture was stirred at 80°C for 1 hour, cooled to room temperature, and concentrated under reduced pressure to remove the organic solvent. Water (5.0 mL) was added to the resulting residue, stirred for 30 minutes, and filtered under reduced pressure. The filter cake was washed with water (0.5 mL x 2), and dried under vacuum to obtain ethyl 1-(5-chloro-8-hydroxy-7-nitroquinolin-4-yl)piperidine-4-carboxylate (46) (47.0 mg, yield 64%).

¹H NMR (400 MHz, DMSO-*d₆*) δ: 8.37 (d, *J=* 6.0 Hz, 1H), 8.20 (d, *J=* 6.8 Hz, 1H), 7.92 (s, 1H), 4.12 (q, *J=* 6.8 Hz, 2H), 3.92 (d, *J=* 13.6 Hz, 1H), 3.73 (d, *J=* 11.4 Hz, 1H), 3.62 - 3.57 (m, 1H), 3.06 - 2.98 (m, 1H), 2.81 (m, 1H), 1.95 (m, 3H), 1.57 (m, 1H), 1.15 (t, *J* = 6.8 Hz, 3H).

MS calculated: 379.1; MS found: 380.1[M+H]⁺.

### Example 47

### Synthesis of 5-chloro-7-nitro-4-phenoxyquinolin-8-ol (47)

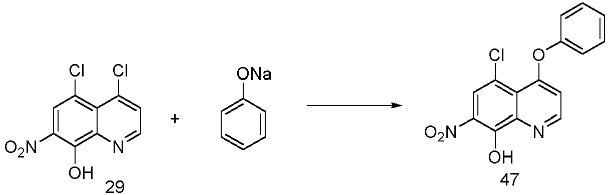

In accordance with the same synthesis method of Example 31, 5-chloro-7-nitro-4-phenoxyquinolin-8-ol (47) (71.9 mg, yield 99%) was obtained from 4,5-dichloro-7-nitroquinolin-8-ol (29) (60.0 mg, 0.23 mmol) and sodium phenolate (107.5 mg, 0.93 mmol).

¹H NMR (400 MHz, DMSO-*d*₆) δ: 8.53 - 8.48 (m, 2H), 7.94 (s, 1H), 7.52 - 7.45 (m, 2H), 7.30 - 7.22 (m, 1H), 7.16 - 7.10 (m, 2H), 6.92 - 6.88 (m, 1H).

MS calculated: 316.03; MS found: 317.1 [M+H]⁺.

### Example 48

### Synthesis of 5-chloro-4-(4-(methylsulfonyl)piperazin-1-yl)-7-nitroquinolin-8-ol (48)

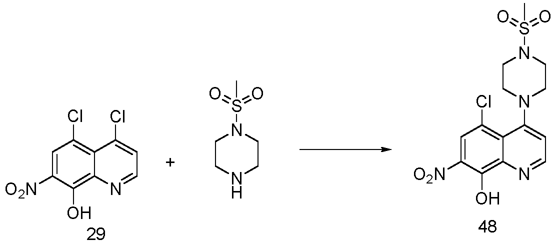

In accordance with the same synthesis method of Example 31, 5-chloro-4-(4-(methylsulfonyl)piperazin-1-yl)-7-nitroquinolin-8-ol (48) (62.0 mg, yield 70%) was obtained from 4,5-dichloro-7-nitroquinolin-8-ol (29) (60.0 mg, 0.23 mmol) and 1-(methylsulfonyl)piperazine (113.0 mg, 0.69 mmol).

¹H NMR (400 MHz, DMSO-*d₆*) δ 8.44 (d, *J* = 5.6 Hz, 1H), 7.88 (s, 1H), 7.25 (d, *J* = 5.8 Hz, 1H), 3.20 - 3.17 (m, 4H), 3.02 - 2.99 (m, 4H), 2.91 (s, 3H).

MS calculated: 386.05; MS found: 387.1 [M+H]⁺.

### Example 49

### Synthesis of 5-chloro-4-cyclopropyl-7-nitroquinolin-8-ol (49)

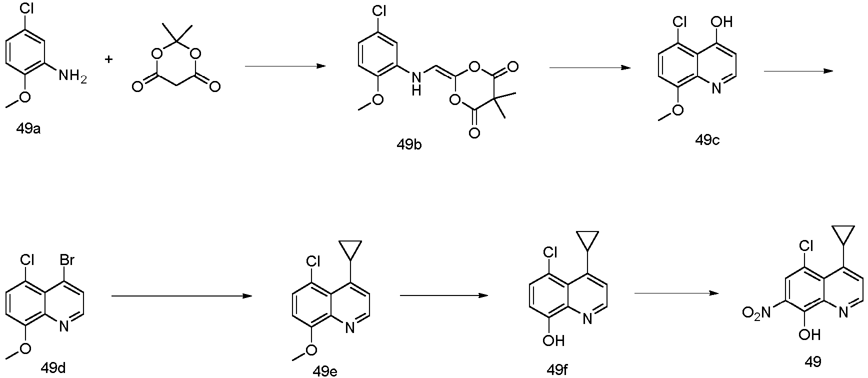

At room temperature, 2,2-dimethyl-1,3-dioxane-4,6-dione (12.0 g, 83.3 mmol) and triethyl orthoformate (13.7 g, 95.1 mmol) were added to ethanol (83.0 mL). The reaction mixture was stirred at 90°C for 4 hours, followed by the addition of 5-chloro-2-methoxyaniline (49a) (12.0 g, 76.4 mmol), stirred for 1 hour, and cooled to room temperature. The resulting mixture was filtered under reduced pressure, the filter cake was washed with ethanol (15.0 mL x 2), and dried under vacuum to obtain 5-(((5-chloro-2-methoxyphenyl)amino)methylene)-2,2-dimethyl-1,3-dioxane-4,6-dione (49b) (23.0 g, yield 97%).

At room temperature, 5-(((5-chloro-2-methoxyphenyl)amino)methylene)-2,2-dimethyl-1,3-dioxane-4,6-dione (49b) (23.0 g, 74.0 mmol) was added to diphenyl ether (125.0 mL). The reaction mixture was warmed up to 240°C, stirred for 3.5 hours, cooled to room temperature, diluted with petroleum ether (boiling range: 60°C to 90°C) (600.0 mL), and stirred for 2 hours. The resulting mixture was filtered under reduced pressure, the filter cake was washed with petroleum ether (boiling range: 60°C to 90°C) (50.0 mL x 3), and dried under vacuum to obtain 5-chloro-8-methoxyquinolin-4-ol (49c) (12.5 g, yield 81%).

At room temperature, 5-chloro-8-methoxyquinolin-4-ol (49c) (2.0 g, 7.35 mmol) was added to a mixed solvent of chloroform (20.0 mL) and N,N-dimethylformamide (2.0 mL), followed by the slowly dropwise addition of phosphorus oxybromide (4.2 g, 14.7 mmol). The reaction mixture was warmed up to 60°C, stirred for 2 hours, and cooled to room temperature, followed by the addition of ice water (30.0 mL). The pH of the aqueous phase was adjusted to about 9 to 10 with ammonia (25 to 28 weight%, NH₃ content). The resulting mixture was extracted with dichloromethane (100.0 mL x 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 1/1) to obtain 4-bromo-5-chloro-8-methoxyquinoline (49d) (1.4 g, yield 70%).

At room temperature, 4-bromo-5-chloro-8-methoxyquinoline (49d) (225.0 mg, 0.83 mmol), cyclopropylboronic acid (107.4 mg, 1.25 mmol), tetrakis(triphenylphosphine)palladium (129.1 mg, 0.083 mmol) and potassium carbonate (229.1 mg, 1.66 mmol) were added successively to a mixed solvent of toluene (10.0 mL), ethanol (1.0 mL) and water (2.0 mL). The reaction mixture was stirred at 90°C for 12 hours, cooled to room temperature, and diluted with water (20.0 mL). The resulting mixture was extracted with ethyl acetate (20.0 mL x 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 1/1) to obtain 5-chloro-4-cyclopropyl-8-methoxyquinoline (49e) (100.0 mg, yield 52%).

At room temperature, 5-chloro-4-cyclopropyl-8-methoxyquinoline (49e) (100.0 mg, 0.42 mmol) was added to a solution of boron tribromide in dichloromethane (1.0 M) (1.3 mL, 1.3 mmol). The reaction mixture was stirred at 50°C for 5 hours, and cooled to room temperature. The pH of the aqueous phase was adjusted to about 8 to 9 with saturated aqueous sodium bicarbonate solution. The resulting mixture was extracted with dichloromethane (10.0 mL x 3). The organic phases were combined, washed with saturated brine (10.0 mL), dried over anhydrous sodium sulfate, filtered, and concentrated under reduced pressure. The resulting residue was purified by silica gel column chromatography (eluent: dichloromethane/methanol = 80/1) to obtain 5-chloro-4-cyclopropylquinolin-8-ol (49f) (60.0 mg, yield 65%).

At room temperature, sodium nitrite (186.3 mg, 2.7 mmol) was added in batches to a suspension of 5-chloro-4-cyclopropylquinolin-8-ol (49f) (60 mg, 0.27 mmol) in hydrochloric acid (2.0 M) (5.0 mL). The reaction mixture was stirred at room temperature for 2 hours, and concentrated under reduced pressure to remove the solvent. The resulting residue was purified by reversed-phase high performance liquid chromatography (the chromatographic column was Eclipse XDB-C18 (21.2 mm×250 mm, 7µm), the mobile phase was water/acetonitrile=100% - 70%/30%, gradient elution, 0.1% formic acid was added to the mobile phase by volume percentage, and the flow rate was 20.0 mL/min) to obtain the product 5-chloro-4-cyclopropyl-7-nitroquinolin-8-ol (49) (8.5 mg, yield 12%).

¹H NMR (400 MHz, DMSO-*d*₆) δ: 9.04 - 8.79 (m, 1H), 8.22 - 7.93 (m, 1H), 7.75 - 7.41 (m, 1H), 3.00 - 2.92 (m, 1H), 1.24 - 1.17 (m, 2H), 1.03 - 0.95 (m, 2H).

MS calculated: 264.03; MS found: 265.0 [M+H]⁺.

### Example 50

### Synthesis of 4-(5-chloro-8-hydroxy-7-nitroquinolin-4-yl)-1-(2,2-dimethylacetyl)piperazine (50)

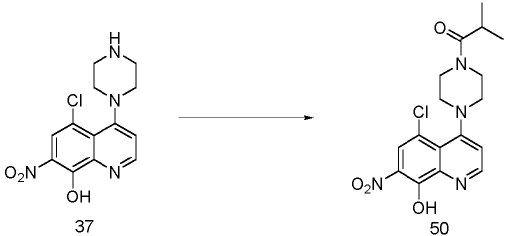

In accordance with the same synthesis method of Example 44, 4-(5-chloro-8-hydroxy-7-nitroquinolin-4-yl)-1-(2,2-dimethylacetyl)piperazine (50) (15.0 mg, yield 22%) was obtained from 5-chloro-7-nitro-4-(piperazin-1-yl)quinolin-8-ol (37) (56.0 mg, 0.18 mmol), isobutyryl chloride (38.4 mg, 0.36 mmol) and triethylamine (36.4 mg, 0.36 mmol).

¹H NMR (400 MHz, DMSO-*d₆*) δ: 8.15 (s, 1H), 7.91 (s, 1H), 7.25 (s, 1H), 4.35 - 4.21 (br s, 2H), 4.01 - 3.90 (br s, 2H), 2.95 - 2.88 (br s, 2H), 2.81 - 2.69 (br s, 2H), 2.05 - 1.94 (m, 1H), 1.01 (overlapping s, 6H).

MS calculated: 394.10; MS found: 395.1, 396.1 [M+H]⁺.

### Example 51

### Synthesis of 5-chloro-7-nitro-4-phenylquinolin-8-ol hydrochloride (51)

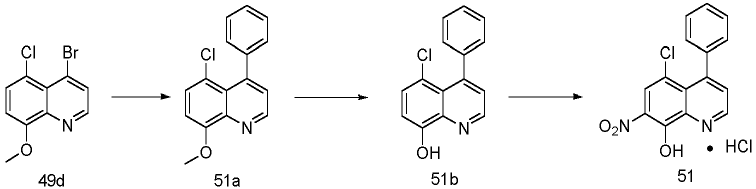

At room temperature, 4-bromo-5-chloro-8-methoxyquinoline (49d) (200.0 mg, 0.73 mmol), phenylboronic acid (134.0 mg, 1.10 mmol), tetrakis(triphenylphosphine)palladium (156.0 mg, 0.10 mmol) and potassium carbonate (207.0 mg, 1.50 mmol) were added successively to 1,4-dioxane (10.0 mL). The reaction mixture was stirred at 90°C for 12 hours, cooled to room temperature, and diluted with water (20.0 mL). The resulting mixture was extracted with dichloromethane (20.0 mL x 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 7/3) to obtain 5-chloro-8-methoxy-4-phenylquinoline (51a) (150.0 mg, yield 76%).

At 0°C, a solution of boron tribromide in dichloromethane (1.0 M) (1.5 mL, 1.5 mmol) was slowly added to a solution of 5-chloro-8-methoxy-4-phenylquinoline (51a) (150.0 mg, 0.56 mmol) in dichloromethane (2.0 mL). The reaction solution was warmed up to room temperature, stirred for 1 hour, stirred at 50°C for 3 hours, and cooled to room temperature. The pH of the aqueous phase was adjusted to about 8 to 9 with saturated aqueous sodium bicarbonate solution. The resulting mixture was extracted with dichloromethane (20.0 mL x 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 1/1) to obtain 5-chloro-4-phenylquinolin-8-ol (51b) (80.0 mg, yield 56%).

At 0°C, sodium nitrite (162.0 mg, 2.3 mmol) was added in batches to a suspension of 5-chloro-4-phenylquinolin-8-ol (60.0 mg, 0.23 mmol) in hydrochloric acid (3.0 M) (5.0 mL). The reaction mixture was warmed up to room temperature, stirred for 12 hours, and filtered under reduced pressure. The filter cake was washed with ethanol (0.5 mL x 2), and dried under vacuum to obtain 5-chloro-7-nitro-4-phenylquinolin-8-ol hydrochloride (51) (8.5 mg, yield 12%).

¹H NMR (400 MHz, DMSO-*d*₆) δ: 9.05 (d, *J* = 4.0 Hz, 1H), 8.09 (s, 1H), 7.70 (d, *J* = 4.0 Hz, 1H), 7.51 - 7.43 (m, 3H), 7.40 - 7.35 (m, 2H).

MS calculated: 300.03; MS found: 301.0 [M+H]⁺.

### Example 52

### Synthesis of ethyl 4-(5-chloro-8-hydroxy-7-nitroquinolin-4-yl)piperazine-1-carboxylate (52)

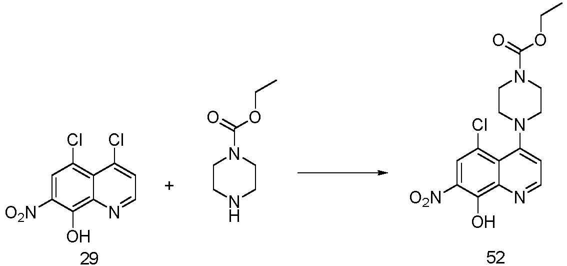

In accordance with the same synthesis method of Example 31, ethyl 4-(5-chloro-8-hydroxy-7-nitroquinolin-4-yl)piperazine-1-carboxylate (52) (73.0 mg, yield 83%) was obtained from 4,5-dichloro-7-nitroquinolin-8-ol (29) (60.0 mg, 0.23 mmol) and ethyl piperazine-1-carboxylate (110.0 mg, 0.70 mmol).

¹H NMR (400 MHz, DMSO-*d*₆) δ 8.38 (d, *J* = 4.0 Hz, 1H), 7.92 (s, 1H), 7.38 (d, *J* = 8.0 Hz, 1H), 4.13 - 4.05 (m, 2H), 3.74 - 3.62 (m, 8H), 1.20 (t, *J* = 7.0 Hz, 3H).

MS calculated: 380.09; MS found: 381.1 [M+H]⁺.

### Example 53

### Synthesis of 5-chloro-4-(cyclopropylmethoxy)-7-nitroquinolin-8-ol (53)

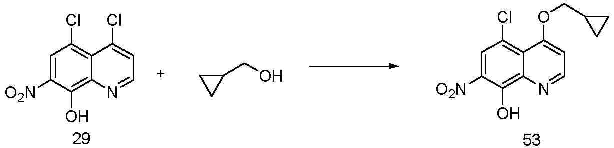

In accordance with the same synthesis method of Example 43, 5-chloro-4-(cyclopropylmethoxy)-7-nitroquinolin-8-ol (53) (14.0 mg, yield 21%) was obtained from 4,5-dichloro-7-nitroquinolin-8-ol (29) (60.0 mg, 0.23 mmol), cyclopropylmethanol (250.0 mg, 1.16 mmol) and sodium hydride (60 weight%, a mixture in mineral oil) (46.0 mg, 1.16 mmol).

¹H NMR (400 MHz, DMSO-*d*₆) δ: 8.45 - 8.35 (m, 1H), 7.83 (s, 1H), 7.10 - 7.00 (m, 1H), 4.10 - 3.95 (m, 2H), 1.40 - 1.25 (m, 1H), 0.65 - 0.55 (m, 2H), 0.46 - 0.35 (m, 2H).

MS calculated: 294.04; MS found: 295.0 [M+H]⁺.

### Example 54

### Synthesis of 5-chloro-4-(4-(3-methoxypropoxy)piperidin-1-yl)-7-nitroquinolin-8-ol dihydrochloride (54)

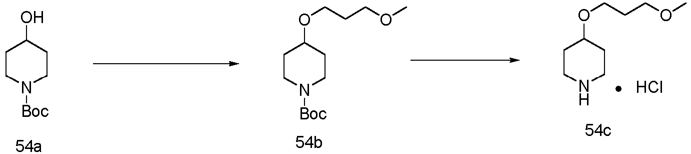

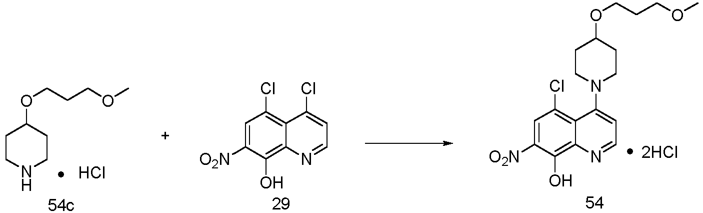

At room temperature, tert-butyl 4-hydroxypiperidine-1-carboxylate (1.0 g, 5.0 mmol) (54a) and sodium hydride (60 weight%, a mixture in mineral oil) (0.50 g, 12.5 mmol) were added successively to N,N-dimethylformamide (5.0 mL). The reaction mixture was stirred at room temperature for 1.5 hours, followed by the addition of 1-bromo-3-methoxypropane (2.3 g, 15.0 mmol). The reaction mixture was warmed up to 50°C, stirred for 4 hours, cooled to room temperature, and diluted with water (20.0 mL) and dichloromethane (20.0 mL). The organic phase was separated, and the aqueous phase was extracted with dichloromethane (20.0 mL x 3). The organic phases were combined, dried over anhydrous sodium sulfate and filtered, and the filtrate was concentrated under reduced pressure. The resulting residue was purified by silica gel column chromatography (gradient elution, eluent: petroleum ether/ethyl acetate = 30/1-20/1) to obtain *tert*-butyl 4-(3-methoxypropoxy)piperidine-1-carboxylate (54b) (0.34 g, yield 25%).

At room temperature, a solution of hydrogen chloride in 1,4-dioxane (4.0 M) (3.1 mL, 12.4 mmol) was added to *tert*-butyl 4-(3-methoxypropoxy)piperidine-1-carboxylate (54b) (340.0 mg, 1.24 mmol). The reaction mixture was stirred at room temperature for 2 hours, and concentrated to dryness under reduced pressure to obtain the crude product 4-(3-methoxypropoxy)piperidine hydrochloride (54c) (310.0 mg, crude yield >100%).

At room temperature, 4,5-dichloro-7-nitroquinolin-8-ol (29) (40.0 mg, 0.15 mmol) and 4-(3-methoxypropoxy)piperidine hydrochloride (54c) (310.0 mg, 1.8 mmol) were added to N,N-dimethylformamide (3.0 mL). The reaction mixture was stirred at 100°C for 4 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/acetonitrile=100% - 70%/30%, gradient elution, 0.1% formic acid was added to the mobile phase by volume percentage, and the flow rate was 20.0 mL/min) to obtain 5-chloro-4-(4-(3-methoxypropoxy)piperidin-1-yl)-7-nitroquinolin-8-ol dihydrochloride (54) (14.0 mg, yield 24%).

¹H NMR (400 MHz, DMSO-*d*₆) δ: 8.35 - 8.20 (m, 1H), 7.90 - 7.82 (m, 1H), 7.42 - 7.30 (m, 1H), 3.40 - 3.28 (m, 12 H), 2.10 - 1.60 (m, 6 H).

MS calculated: 395.12; MS found: 396.1 [M+H]⁺.

### Example 55

### Synthesis of 5-chloro-4-(4,4-difluoropiperidin-1-yl)-7-nitroquinolin-8-ol (55)

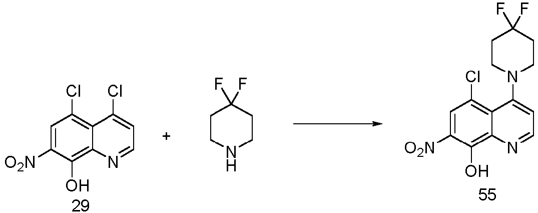

In accordance with the same synthesis method of Example 31, 5-chloro-4-(4,4-difluoropiperidin-1-yl)-7-nitroquinolin-8-ol (55) (43.0 mg, yield 54%) was obtained from 4,5-dichloro-7-nitroquinolin-8-ol (29) (60.0 mg, 0.23 mmol) and 4,4-difluoroperidine (140.0 mg, 1.16 mmol).

¹H NMR (400 MHz, DMSO-*d*₆) δ: 8.39 (d, *J =* 8.0 Hz, 1H), 7.92 (s, 1H), 7.44 (d, *J* = 8.0 Hz, 1H), 3.80 - 3.60 (m, 4 H), 2.35 - 2.05 (m, 4 H).

MS calculated: 343.05; MS found: 344.1 [M+H]⁺.

### Example 56

### Synthesis of 1-(5-chloro-8-hydroxy-7-nitroquinolin-4-yl)piperidin-4-one (56)

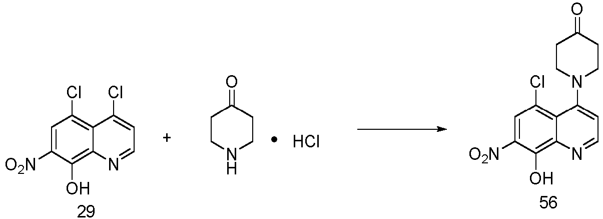

At room temperature, 4,5-dichloro-7-nitroquinolin-8-ol (29) (40.0 mg, 0.15 mmol), 4-piperidone hydrochloride (37.0 mg, 0.37 mmol) and triethylamine (45.0 mg, 0.45 mmol) were added successively to N,N-dimethylformamide (2.0 mL). The reaction mixture was stirred at 100°C for 2 hours, cooled to room temperature, and concentrated under reduced pressure to remove the organic solvent. Water (2.0 mL) was added to the resulting residue, stirred for 3 minutes, and filtered under reduced pressure. The filter cake was washed with water (0.5 mL x 2), and dried under vacuum to obtain 1-(5-chloro-8-hydroxy-7-nitroquinolin-4-yl)piperidin-4-one (56) (20.0 mg, yield 41%).

¹H NMR (400 MHz, DMSO-*d*₆) δ 8.39 - 8.34 (m, 1H), 7.93 - 7.86 (m, 1H), 7.42 - 7.35 (m, 1H), 3.95 - 3.64 (m, 8H).

MS calculated: 321.05; MS found: 322.1 [M+H]⁺.

### Example 57

### Synthesis of (1-methyl)propyl 4-(5-chloro-8-hydroxy-7-nitroquinolin-4-yl)piperazine-1-carboxylate (57)

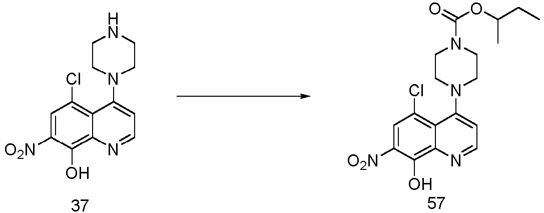

In accordance with the same synthesis method of Example 44, (1-methyl)propyl 4-(5-chloro-8-hydroxy-7-nitroquinolin-4-yl)piperazine-1-carboxylate (57) (50.0 mg, yield 68%) was obtained from 5-chloro-7-nitro-4-(piperazin-1-yl)quinolin-8-ol (37) (56.0 mg, 0.18 mmol), *sec*-butyl chloroformate (75.0 mg, 0.55 mmol) and triethylamine (54.6 mg, 0.54 mmol).

¹H NMR (400 MHz, DMSO-*d*₆) δ 8.38 (d, *J =* 4.0 Hz, 1H), 7.92 (s, 1H), 7.39 (d, *J* = 8.0 Hz, 1H), 4.68 - 4.62 (m, 1H), 3.76 - 3.53 (m, 8H), 1.56 - 1.49 (m, 2H), 1.20 - 1.14 (m, 3H), 0.90 - 0.81 (m, 3H).

MS calculated: 408.12; MS found: 409.1 [M+H]⁺.

### Example 58

### Synthesis of isopropyl 4-(5-chloro-8-hydroxy-7-nitroquinolin-4-yl)piperazine-1-carboxylate (58)

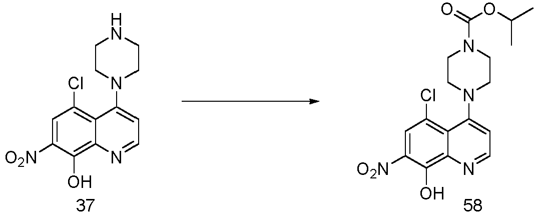

In accordance with the same synthesis method of Example 44, isopropyl 4-(5-chloro-8-hydroxy-7-nitroquinolin-4-yl)piperazine-1-carboxylate (58) (46.0 mg, yield 61%) was obtained from 5-chloro-7-nitro-4-(piperazin-1-yl)quinolin-8-ol (37) (60.0 mg, 0.19 mmol), isopropyl chloroformate (73.8 mg, 0.60 mmol) and triethylamine (67.0 mg, 0.60 mmol).

¹H NMR (400 MHz, DMSO-*d*₆) δ 8.38 (d, *J* = 4.0 Hz, 1H), 7.92 (s, 1H), 7.38 (d, *J* = 8.0 Hz, 1H), 4.86 - 4.74 (m, 1H), 3.73 - 3.45 (m, 8H), 1.25 - 1.16 (m, 6H).

MS calculated: 394.10; MS found: 395.1 [M+H]⁺.

### Example 59

### Synthesis of 5-chloro-4-(cyclopentyloxy)-7-nitroquinolin-8-ol (59)

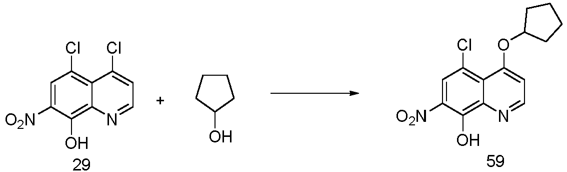

At room temperature, cyclopentanol (166.0 mg, 1.93 mmol) and sodium hydride (60 weight%, a mixture in mineral oil) (38.8 mg, 0.97 mmol) were added successively to N,N-dimethylformamide (2.0 mL). The reaction mixture was stirred at room temperature for 1 hour, followed by the addition of 4,5-dichloro-7-nitroquinolin-8-ol (29) (50.0 mg, 0.19 mmol). The resulting mixture was stirred at 100°C for 2 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/acetonitrile=100% - 70%/30%, gradient elution, 0.1% formic acid was added to the mobile phase by volume percentage, and the flow rate was 20.0 mL/min) to obtain 5-chloro-4-(cyclopentyloxy)-7-nitroquinolin-8-ol (59) (11.0 mg, yield 19%).

¹H NMR (400 MHz, DMSO-*d*₆) δ: 8.80-8.60 (m, 1H), 8.05-7.95 (m, 1H), 7.50-7.35 (m, 1H), 5.38-5.22 (m, 1 H), 2.10-1.60(m, 9 H).

MS calculated: 308.06; MS found: 309.0 [M+H]⁺.

### Example 60

### Synthesis of 5-chloro-4-(cyclohexyloxy)-7-nitroquinolin-8-ol (60)

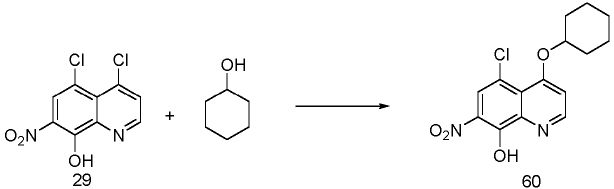

In accordance with the same synthesis method of Example 59, 5-chloro-4-(cyclohexyloxy)-7-nitroquinolin-8-ol (60) (30.0 mg, yield 16%) was obtained from 4,5-dichloro-7-nitroquinolin-8-ol (29) (150.0 mg, 0.58 mmol), cyclohexanol (117.0 mg, 1.16 mmol) and sodium hydride (60 weight%, a mixture in mineral oil) (47.0 mg, 1.16 mmol).

¹H NMR (400 MHz, DMSO-*d*₆) δ: 8.65 (d, *J =* 8.0 Hz, 1H), 7.98 (s, 1H), 7.54 (d, *J* = 8.0 Hz, 1H), 5.13 - 4.85 (m, 1H), 2.10 - 1.86 (m, 2H), 1.84 - 1.62 (m, 4H), 1.56 - 1.38 (m, 4H).

MS calculated: 322.07; MS found: 323.1 [M+H]⁺.

### Example 61

### Synthesis of 5-chloro-4-ethoxy-7-nitroquinolin-8-ol (61)

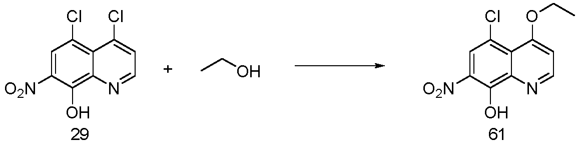

In accordance with the same synthesis method of Example 43, 5-chloro-4-ethoxy-7-nitroquinolin-8-ol (61) (23.3 mg, yield 38%) was obtained from 4,5-dichloro-7-nitroquinolin-8-ol (29) (60.0 mg, 0.23 mmol), ethanol (6.0 mL, 102.7 mmol) and sodium hydride (60 weight%, a mixture in mineral oil) (50.6 mg, 1.26 mmol).

¹H NMR (400 MHz, DMSO-*d*₆) δ: 8.75 - 8.70 (m, 1H), 8.00 (s, 1H), 7.48 - 7.41 (m, 1H), 4.50 - 4.40 (m, 2H), 1.57 - 1.45 (m, 3H).

MS calculated: 268.03; MS found: 269.0 [M+H]⁺.

### Example 62

### Synthesis of 7-nitro-4-trifluoromethylquinolin-8-ol (62)

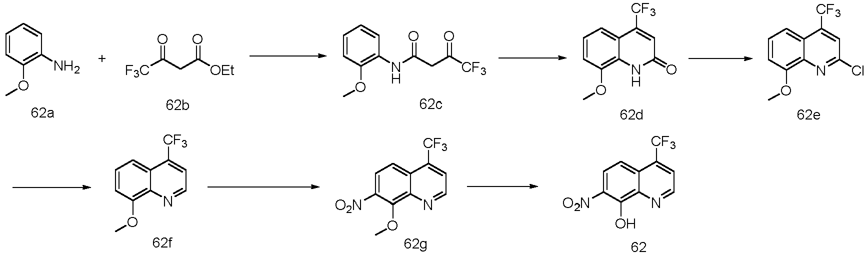

At room temperature, o-methoxyaniline (62a) (2.36 g, 19.2 mmol), ethyl 4,4,4-trifluoroacetoacetate (62b) (2.95 g, 16.0 mmol) and triethylamine (3.24 g, 32.0 mmol) were added to toluene (16.0 mL). The reaction mixture was stirred at 125°C for 4 hours, cooled to room temperature, and concentrated under reduced pressure to remove the organic solvent. The resulting residue was dissolved in dichloromethane (50.0 mL), and washed successively with hydrochloric acid (2.0 M) (15.0 mL x 2), saturated aqueous sodium bicarbonate solution (20.0 mL) and water (15.0 mL). The organic phase was dried over anhydrous sodium sulfate, filtered, and concentrated under reduced pressure to obtain the crude product 4,4,4-trifluoro-N-(2-methoxyphenyl)-3-oxobutanamide (62c) (3.48 g, crude yield 78%).

At 90°C, 4,4,4-trifluoro-N-(2-methoxyphenyl)-3-oxobutanamide (62c) (2.81 g, 10.7 mmol) was completely dissolved in polyphosphoric acid (14.0 g). The reaction mixture was stirred at 90°C for 3 hours, cooled to room temperature, and diluted with water (100.0 mL) until the polyphosphoric acid was completely dissolved. The resulting mixture was extracted with dichloromethane (40.0 mL x 3). The organic phases were combined, washed with saturated aqueous sodium bicarbonate solution (30.0 mL x 3), dried over anhydrous sodium sulfate, filtered, and concentrated under reduced pressure. The resulting residue was purified by silica gel column chromatography (eluent: petroleum ether/ethyl acetate = 40/1) to obtain 8-methoxy-4-trifluoromethyl-2(1*H*)-quinolinone (62d) (2.21 g, yield 84%).

At room temperature, 8-methoxy-4-trifluoromethyl-2(1*H*)-quinolinone (62d) (2.09 g, 8.6 mmol) was added to phosphorus oxychloride (8.6 mL). The reaction mixture was stirred at 100°C for 2 hours, cooled to room temperature, and diluted with dichloromethane (40.0 mL). The pH of the aqueous phase was adjusted to about 9 to 10 with saturated aqueous sodium carbonate solution. The organic phase was separated, and the aqueous phase was extracted with dichloromethane (15.0 mL x 2). The combined organic phase was washed successively with saturated aqueous sodium carbonate solution (25.0 mL), water (25.0 mL) and saturated brine (25.0 mL), dried over anhydrous sodium sulfate, filtered, and concentrated under reduced pressure to obtain the crude product 2-chloro-8-methoxy-4-trifluoromethylquinoline (62e) (2.26 g, crude yield 100%).

At room temperature, 2-chloro-8-methoxy-4-trifluoromethylquinoline (62e) (520.0 mg, 2.0 mmol) was added to a mixed solvent of tetrahydrofuran (10.0 mL) and methanol (10.0 mL), followed by the addition of palladium/carbon (palladium loading: 10 weight%) (210.0 mg, 0.2 mmol). The reaction mixture was stirred under a hydrogen atmosphere for 1 hour, and filtered through diatomite. The filter cake was washed with a mixed solvent of dichloromethane and methanol (volume ratio, dichloromethane/methanol = 20/1) (30.0 mL). The filtrate was concentrated under reduced pressure. The resulting residue was dissolved in dichloromethane (20.0 mL), washed with saturated aqueous sodium bicarbonate solution (10.0 mL x 2), dried over anhydrous sodium sulfate, filtered, and concentrated under reduced pressure. The resulting residue was purified by silica gel column chromatography (eluent: petroleum ether/ethyl acetate = 3/1) to obtain 8-methoxy-4-trifluoromethylquinoline (62f) (380.0 mg, yield 84%).

At 0°C, nitric acid (65 weight%) (0.69 mL, 10.0 mmol) was slowly added dropwise to a solution of 8-methoxy-4-trifluoromethylquinoline (62f) (380.0 mg, 1.7 mmol) in acetic anhydride (5.0 mL), and stirred for 10 minutes. Sulfuric acid (98 weight%) (0.12 mL, 2.2 mmol) was slowly added dropwise. The reaction mixture was stirred at 0°C for 30 minutes, and the pH of the aqueous phase was adjusted to about 9 to 10 with aqueous sodium hydroxide solution (1.0 M). The resulting mixture was extracted with dichloromethane (10.0 mL x 3). The organic phases were combined, dried over anhydrous sodium sulfate, filtered, and concentrated under reduced pressure. The resulting residue was purified by silica gel column chromatography (eluent: petroleum ether/ethyl acetate = 2/1) to obtain 8-methoxy-7-nitro-4-trifluoromethylquinoline (62g) (93.5 mg, yield 20%).

At room temperature, 8-methoxy-7-nitro-4-trifluoromethylquinoline (62g) (93.5 mg, 0.34 mmol) and lithium chloride (144.0 mg, 3.4 mmol) were added to N,N-dimethylformamide (0.4 mL). The reaction mixture was stirred at 170°C for 1 hour, cooled to room temperature, and concentrated under reduced pressure to remove the organic solvent. Water (1.0 mL) was added to the resulting residue, stirred for 3 minutes, and filtered under reduced pressure. The filter cake was washed with water (0.2 mL x 2), and dried under vacuum to obtain 7-nitro-4-trifluoromethylquinolin-8-ol (62) (70.0 mg, yield 79%).

¹H NMR (400 MHz, DMSO-*d₆*) δ: 8.79 (d, *J =* 3.6 Hz, 1H), 8.10 (d, *J =* 9.6 Hz, 1H), 7.88 (d, *J* = 4.4 Hz, 1H), 6.52 (dd, *J =* 9.6, 2.4 Hz, 1H).

MS calculated: 258.03; MS found: 259.1 [M+H]⁺.

### Example 63

### Synthesis of 5-chloro-7-nitro-4-(6-azaspiro[2.5]octan-6-yl)quinolin-8-ol (63)

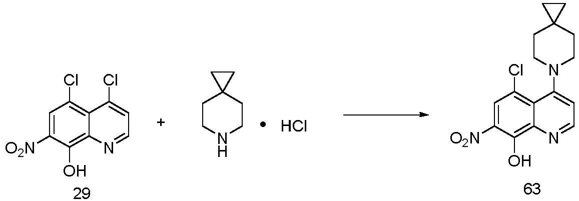

At room temperature, 6-azaspiro[2.5]octane hydrochloride (85.4 mg, 0.58 mmol) and N,N-diisopropylethylamine (125.0 mg, 0.96 mmol) were added to N,N-dimethylformamide (2.0 mL). The resulting mixture was stirred at room temperature for 1 hour, followed by the addition of 4,5-dichloro-7-nitroquinolin-8-ol (29) (50.0 mg, 0.19 mmol). The reaction mixture was stirred at 80°C for 4 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/acetonitrile=100% - 70%/30%, gradient elution, 0.1% formic acid was added to the mobile phase by volume percentage, and the flow rate was 20.0 mL/min) to obtain 5-chloro-7-nitro-4-(6-azaspiro[2.5]octan-6-yl)quinolin-8-ol (63) (3.5 mg, yield 6%).

¹H NMR (400 MHz, DMSO-*d*₆) δ: 8.32 - 8.20 (m, 1H), 7.90 - 7.85 (m, 1H), 7.44 - 7.33 (m, 1H), 3.75 - 3.60 (m, 4H), 2.05 - 1.95 (m, 1H), 1.65 - 1.35 (m, 4H), 0.50 - 0.28 (m, 4H).

MS calculated: 333.09; MS found: 334.1 [M+H]⁺.

### Example 64

### Synthesis of 5-chloro-4-cyclohexyl-7-nitroquinolin-8-ol (64)

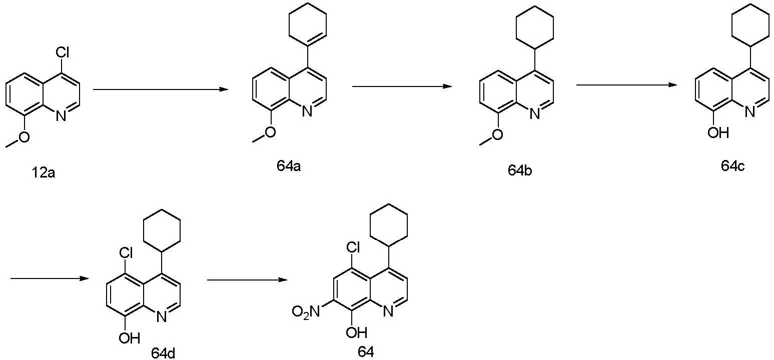

At room temperature, 4-chloro-8-methoxyquinoline (12a) (1.0 g, 5.2 mmol), cyclohexenylboronic acid (0.975 g, 7.7 mmol), tetrakis(triphenylphosphine)palladium (0.600 g, 0.50 mmol) and potassium carbonate (1.5 g, 11.0 mmol) were added successively to a mixed solvent of toluene (10.0 mL), ethanol (1.0 mL) and water (2.0 mL). The reaction mixture was stirred at 100°C for 12 hours, cooled to room temperature, and diluted with water (20.0 mL). The resulting mixture was extracted with ethyl acetate (30.0 mL x 2). The organic phases were combined, dried over anhydrous sodium sulfate and filtered, and the filtrate was concentrated under reduced pressure. The resulting residue was purified by silica gel column chromatography (eluent: petroleum ether/ethyl acetate = 1/1) to obtain 4-cyclohexenyl-8-methoxyquinoline (64a) (1.1 g, yield 89%).

At room temperature, 4-cyclohexenyl-8-methoxyquinoline (64a) (850.0 mg, 3.5 mmol) and palladium/carbon (palladium loading: 10 weight%) (85.0 mg, 0.080 mmol) were successively added to methanol (20.0 mL). The resulting mixture was stirred under a hydrogen atmosphere for 3 hours, and filtered through diatomite, and the filter cake was washed with ethyl acetate (10.0 mL x 3). The filtrate was concentrated to dryness under reduced pressure to obtain 4-cyclohexyl-8-methoxyquinoline (64b) (850.0 mg, yield 99%).

At 0°C, a solution of boron tribromide in dichloromethane (1.0 M) (7.0 mL, 7.0 mmol) was slowly added dropwise to a solution of 4-cyclohexyl-8-methoxyquinoline (64b) (420.0 mg, 1.7 mmol) in dichloromethane (2.0 ml). The reaction mixture was warmed up to room temperature, stirred at 50°C for 5 hours, and cooled to room temperature. The pH of the aqueous phase was adjusted to about 8 to 9 with saturated aqueous sodium bicarbonate solution. The resulting mixture was extracted with ethyl acetate (10.0 mL x 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 2/1) to obtain 4-cyclohexylquinolin-8-ol (64c) (250.0 mg, yield 65%).

At 0°C, N-chlorosuccinimide (147.0 mg, 1.1 mmol) was added to a solution of 4-cyclohexylquinolin-8-ol (64c) (250.0 mg, 1.1 mmol) in sulfuric acid (98 weight%) (7.0 mL). The reaction mixture was stirred at 0°C for 1 hour, and the pH of the aqueous phase was adjusted to about 8 to 9 with saturated aqueous sodium bicarbonate solution. The resulting mixture was extracted with ethyl acetate (10.0 mL x 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 3/1) to obtain 5-chloro-4-cyclohexylquinolin-8-ol (64d) (230.0 mg, yield 80%).

At room temperature, sodium nitrite (600.0 mg, 7.6 mmol) was added in batches to a suspension of 5-chloro-4-cyclohexylquinolin-8-ol (64d) (200.0 mg, 0.76 mmol) in hydrochloric acid (2.0 M) (10.0 mL). The reaction mixture was stirred at room temperature for 5 hours, and the pH of the aqueous phase was adjusted to about 8 to 9 with ammonia (25 to 28 weight%, NH₃ content). The resulting mixture was filtered under reduced pressure, and the filter cake was purified by reversed-phase high performance liquid chromatography (the chromatographic column was Eclipse XDB-C18 (21.2 mm×250 mm, 7µm), the mobile phase was water/acetonitrile=100% - 70%/30%, gradient elution, 0.1% formic acid was added to the mobile phase by volume percentage, and the flow rate was 20.0 mL/min) to obtain 5-chloro-4-cyclohexyl-7-nitroquinolin-8-ol (64) (60.0 mg, yield 26%).

¹H NMR (400 MHz, DMSO-*d*₆) δ: 8.85 (d, *J =* 4.0 Hz, 1H), 7.72 (d, *J =* 4.0 Hz, 1H), 7.68 - 7.63 (m, 1H), 7.17 - 7.11 (m, 1H), 4.36 - 4.26 (m, 1H), 1.97 - 1.73 (m, 4H), 1.61 - 1.22 (m, 6H).

MS calculated: 261.09; MS found: 262.1 [M+H]⁺.

### Example 65

### Synthesis of 5-chloro-4-fluoro-7-nitroquinolin-8-ol (65)

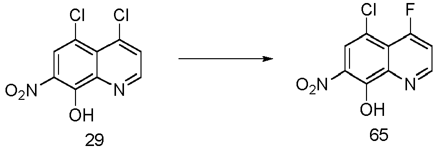

At room temperature, 4,5-dichloro-7-nitroquinolin-8-ol (29) (60.0 mg, 0.23 mmol) and cesium fluoride (350.0 mg, 2.3 mmol) were added to dimethyl sulfoxide (2.0 mL). The resulting mixture was stirred at 180°C for 2 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/acetonitrile=100% - 70%/30%, gradient elution, 0.1% formic acid was added to the mobile phase by volume percentage, and the flow rate was 20.0 mL/min) to obtain 5-chloro-4-fluoro-7-nitroquinolin-8-ol (65) (12.0 mg, yield 22%).

¹H NMR (400 MHz, DMSO-*d*₆) δ: 9.13 - 9.01 (m, 1H), 8.18 (s, 1H), 7.88 - 7.73 (m, 1H).

MS calculated: 241.99; MS found: 243.0 [M+H]⁺.

### Example 66

### Synthesis of 5-chloro-4-(3-methylpiperidin-1-yl)-7-nitroquinolin-8-ol (66)

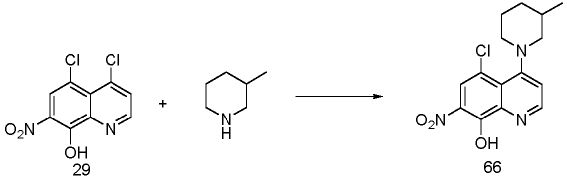

In accordance with the same synthesis method of Example 46, 5-chloro-4-(3-methylpiperidin-1-yl)-7-nitroquinolin-8-ol (66) (62.0 mg, yield 99.8%) was obtained from 4,5-dichloro-7-nitroquinolin-8-ol (29) (50.0 mg, 0.19 mmol) and 3-methylpiperidine (57.0 mg, 0.57 mmol).

¹H NMR (400 MHz, DMSO-*d₆*) δ 7.82 (dd, *J* = 16.2, 1.6 Hz, 1H), 7.27 - 7.04 (m, 2H), 3.28 - 3.13 (m, 3H), 2.76 (td, *J =* 12.7, 3.0 Hz, 1H), 2.26 - 2.12 (m, 1H), 2.07 - 1.95 (m, 1H), 1.81 - 1.71 (m, 3H), 0.91 (d, *J =* 6.5 Hz, 3H).

MS calculated: 321.1; MS found: 322.1[M+H]⁺.

### Example 67

### Synthesis of 4-(benzyloxy)-5-chloro-7-nitroquinolin-8-ol hydrochloride (67)

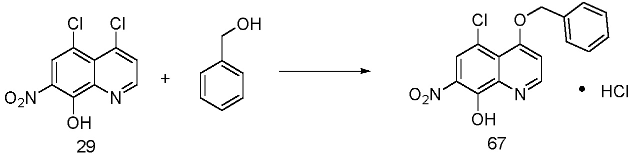

At room temperature, sodium hydride (60 weight%, a mixture in mineral oil) (55.0 mg, 1.38 mmol) was added to a solution of benzyl alcohol (100.0 mg, 0.93 mmol) in dimethyl sulfoxide (6.0 mL). The resulting mixture was stirred at room temperature for 1 hour, followed by the addition of 4,5-dichloro-7-nitroquinolin-8-ol (29) (60.0 mg, 0.23 mmol). The reaction mixture was stirred at room temperature for 3 hours, followed by the addition of hydrochloric acid (36 weight%) (1.5 mL), and filtered under reduced pressure. The filter cake was washed successively with water (0.5 mL x 2) and ethanol (0.5 mL x 2), and dried under vacuum to obtain 4-(benzyloxy)-5-chloro-7-nitroquinolin-8-ol hydrochloride (67) (29.5 mg, yield 35%).

¹H NMR (400 MHz, DMSO-*d*₆) δ: 8.83 - 8.76 (m, 1H), 8.05 - 8.00 (m, 1H), 7.62 - 7.55 (m, 3H), 7.49 - 7.35 (m, 3 H), 5.58 - 5.51 (m, 2 H).

MS calculated: 330.04; MS found: 331.0 [M+H]⁺.

### Example 68

### Synthesis of 4-chloro-5-fluoro-7-nitroquinolin-8-ol (68)

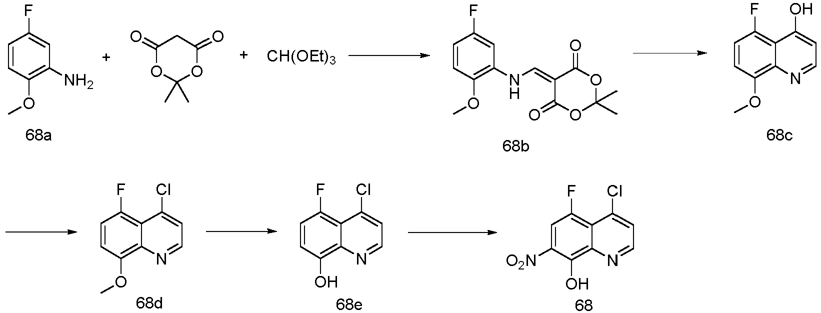

At room temperature, 5-fluoro-2-methoxyaniline (68a) (1.41 g, 10.0 mmol), 2,2-dimethyl-1,3-dioxane-4,6-dione (1.73 g, 12.0 mmol) and triethyl orthoformate (3.56 g, 24.0 mmol) were added to ethanol (10.0 mL). The resulting mixture was stirred at 95°C for 3 hours, cooled to room temperature, and filtered under reduced pressure. The filter cake was washed with ethanol (5.0 mL x 3), and dried under vacuum to obtain 5-(((5-fluoro-2-methoxyphenyl)amino)methylene)-2,2-dimethyl-1,3-dioxane-4,6-dione (68b) (3.21 g, yield 99%).

At room temperature, 5-(((5-fluoro-2-methoxyphenyl)amino)methylene)-2,2-dimethyl-1,3-dioxane-4,6-dione (68b) (2.70 g, 9.2 mmol) was added to diphenyl ether (46.0 mL). The reaction mixture was stirred at 210°C for 4 hours, cooled to room temperature, diluted with petroleum ether (boiling range: 60°C to 90°C) (250.0 mL), and filtered under reduced pressure. The filter cake was washed with petroleum ether (boiling range: 60°C to 90°C) (8.0 mL x 4), and dried under vacuum to obtain 5-fluoro-8-methoxyquinolin-4-ol (68c) (1.49 g, yield 84%).

At room temperature, phosphorus oxychloride (7.7 mL) was added to 5-fluoro-8-methoxyquinolin-4-ol (68c) (1.49 g, 7.7 mmol). The reaction mixture was stirred at 100°C for 3 hours, cooled to room temperature, and diluted with dichloromethane (40.0 mL). The pH of the aqueous phase was adjusted to about 9 to 10 with aqueous sodium hydroxide solution (1.0 M). The organic phase was separated, and the aqueous phase was extracted with dichloromethane (20.0 mL x 2). The combined organic phase was washed successively with saturated aqueous sodium carbonate solution (20.0 mL), water (20.0 mL) and saturated brine (20.0 mL), dried over anhydrous sodium sulfate and filtered, and the filtrate was concentrated under reduced pressure. The resulting residue was purified by silica gel column chromatography (eluent: dichloromethane/petroleum ether = 2/1) to obtain 4-chloro-5-fluoro-8-methoxyquinoline (68d) (1.33 g, yield 81%).

At 0°C, a solution of boron tribromide in dichloromethane (1.0 M) (31.4 mL, 31.4 mmol) was slowly added dropwise to 4-chloro-5-fluoro-8-methoxyquinoline (68d) (1.33 g, 6.3 mmol). The reaction mixture was warmed up to room temperature, stirred for 5 hours, followed by the addition of ice water (150.0 mL). The pH of the aqueous phase was adjusted to about 9 to 10 with solid sodium carbonate. The organic phase was separated, and the aqueous phase was extracted with dichloromethane (30.0 mL x 4). The combined organic phase was washed with saturated aqueous sodium carbonate solution (40.0 mL), dried over anhydrous sodium sulfate and filtered, and the filtrate was concentrated under reduced pressure. The resulting residue was purified by silica gel column chromatography (eluent: dichloromethane/ethyl acetate = 100/1) to obtain 4-chloro-5-fluoroquinolin-8-ol (68e) (0.65 g, yield 52%).

At 0°C, a solution of sodium nitrite (2.27 g, 32.9 mmol) in water (2.7 mL) was added dropwise to a suspension of 4-chloro-5-fluoroquinolin-8-ol (68e) (0.65 g, 3.3 mmol) in hydrochloric acid (2.4 M) (13.7 mL). The reaction mixture was warmed up to room temperature, and stirred for 10 hours. The pH of the aqueous phase was adjusted to about 9 to 10 with solid sodium carbonate, and the mixture was filtered under reduced pressure. The filter cake was washed with water (3.0 mL x 3), and dried under vacuum to obtain 4-chloro-5-fluoro-7-nitroquinolin-8-ol (68) (0.354 g, yield 44%).

¹H NMR (400 MHz, DMSO-*d*₆) δ: 8.62 (s, 1H), 7.79 (overlapping s, 2H).

MS calculated: 241.99; MS found: 243.1, 245.1 [M+H]⁺.

### Example 69

### Synthesis of 4-(4,4-difluoropiperidin-1-yl)-5-fluoro-7-nitroquinolin-8-ol (69)

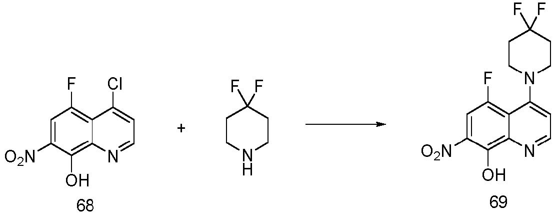

At room temperature, 4-chloro-5-fluoro-7-nitroquinolin-8-ol (68) (42.0 mg, 0.17 mmol) and 4,4-difluoroperidine (63.0 mg, 0.52 mmol) were added to N,N-dimethylformamide (3.0 mL). The reaction mixture was stirred at 90°C for 4 hours, cooled to room temperature, and concentrated under reduced pressure to remove the organic solvent. Water (2.0 mL) was added to the resulting residue, stirred for 3 minutes, and filtered under reduced pressure. The filter cake was washed with water (0.5 mL x 2), and dried under vacuum to obtain 4-(4,4-difluoropiperidin-1-yl)-5-fluoro-7-nitroquinolin-8-ol (69) (30.0 mg, yield 54%).

¹H NMR (400 MHz, DMSO-*d*₆) δ: 8.63 - 8.50 (m, 1 H), 7.78 - 7.70 (m, 1 H), 7.35 - 7.28 (m, 1 H), 3.50 - 3.40 (m, 4 H), 2.30 - 2.15 (m, 4 H).

MS calculated: 327.08; MS found: 328.1 [M+H]⁺.

### Example 70

### Synthesis of 5-fluoro-7-nitro-4-(4-(trifluoromethyl)piperidin-1-yl)quinolin-8-ol (70)

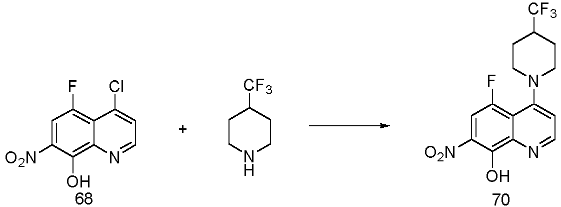

In accordance with the same synthesis method of Example 69, 5-fluoro-7-nitro-4-(4-(trifluoromethyl)piperidin-1-yl)quinolin-8-ol (70) (40.0 mg, yield 56%) was obtained from 4-chloro-5-fluoro-7-nitroquinolin-8-ol (68) (48.0 mg, 0.20 mmol) and 4-(trifluoromethyl)piperidine (153.0 mg, 1.0 mmol).

¹H NMR (400 MHz, DMSO-*d*₆) δ: 8.58 - 8.40 (m, 1 H), 7.68 - 7.55 (m, 1 H), 7.22 - 7.11 (m, 1 H), 3.74 - 3.61 (m, 2 H), 3.46 - 3.39 (m, 2 H), 3.01 - 2.92 (m, 1 H), 2.00 - 1.95 (m, 2 H), 1.72 - 1.65 (m, 2 H).

MS calculated: 359.09; MS found: 360.1 [M+H]⁺.

### Example 71

### Synthesis of ethyl 1-(5-fluoro-8-hydroxy-7-nitroquinolin-4-yl)piperidine-4-carboxylate (71)

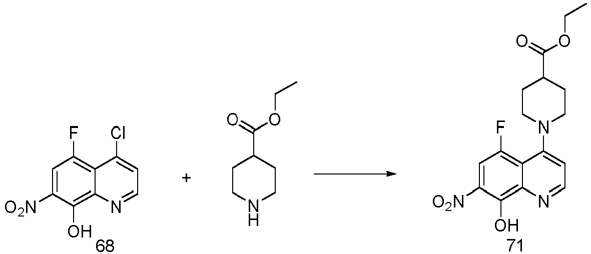

In accordance with the same synthesis method of Example 69, ethyl 1-(5-fluoro-8-hydroxy-7-nitroquinolin-4-yl)piperidine-4-carboxylate (71) (33.0 mg, yield 48%) was obtained from 4-chloro-5-fluoro-7-nitroquinolin-8-ol (68) (45.0 mg, 0.19 mmol) and ethyl 4-piperidinecarboxylate (210 mg, 1.33 mmol).

¹H NMR (400 MHz, DMSO-*d*₆) δ: 8.45 - 8.35 (m, 1H), 7.52 - 7.44 (m, 1H), 7.05 - 6.98 (m, 1H), 4.14 - 4.04 (m, 2H), 3.30 - 3.15 (m, 4H), 2.85 - 2.70 (m, 1H), 2.00 - 1.90 (m, 2H), 1.80 - 1.60 (m, 2H), 1.25 - 1.15 (m, 3H).

MS calculated: 363.12; MS found: 364.1 [M+H]⁺.

### Example 72

### Synthesis of 4-(cyclopropylmethoxy)-5-fluoro-7-nitroquinolin-8-ol (72)

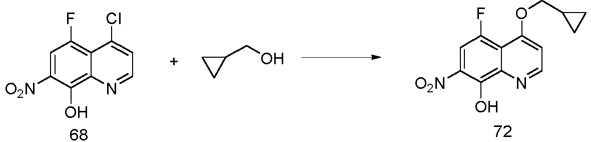

In accordance with the same synthesis method of Example 69, 4-(cyclopropylmethoxy)-5-fluoro-7-nitroquinolin-8-ol (72) (38.0 mg, yield 65%) was obtained from 4-chloro-5-fluoro-7-nitroquinolin-8-ol (68) (50.0 mg, 0.21 mmol) and cyclopropylmethanol (149.0 mg, 2.1 mmol).

¹H NMR (400 MHz, DMSO-*d*₆) δ: 8.82 - 8.78 (m, 1H), 7.80 - 7.73 (m, 1H), 7.39 - 7.33 (m, 1H), 4.23 - 4.18 (m, 2H), 0.88 - 0.80 (m, 1H), 0.62 - 0.60 (m, 2H), 0.46 - 0.40 (m, 2H).

MS calculated: 278.07; MS found: 279.1 [M+H]⁺.

### Example 73

### Synthesis of 5-chloro-7-nitro-4-(trifluoromethyl)-quinolin-8-ol hydrochloride (73)

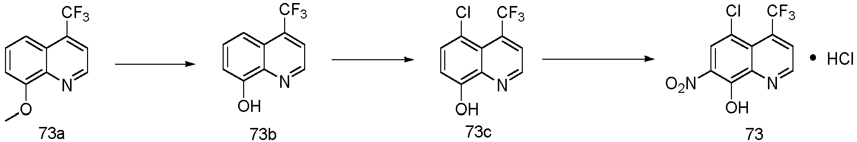

At room temperature, 8-methoxy-4-(trifluoromethyl)quinoline (73a) (230.0 mg, 1.0 mmol) was added to a solution of boron tribromide in dichloromethane (1.0 M) (5.0 mL, 5.0 mmol). The reaction mixture was stirred at room temperature for 7 hours, and diluted with dichloromethane (20.0 mL). The pH of the aqueous phase was adjusted to about 9 to 10 with saturated aqueous sodium carbonate solution. The organic phase was separated, and the aqueous phase was extracted with dichloromethane (10.0 mL x 4). The combined organic phase was dried over anhydrous sodium sulfate, filtered, and concentrated under reduced pressure. The resulting residue was purified by silica gel column chromatography (eluent: dichloromethane/methanol = 50/1) to obtain 4-trifluoromethyl-8-hydroxyquinoline (73b) (190.0 mg, yield 88%).

At room temperature, N-chlorosuccinimide (64.0 mg, 0.48 mmol) was added to a solution of 4-trifluoromethyl-8-hydroxyquinoline (73b) (85.0 mg, 0.4 mmol) in sulfuric acid (98 weight%) (2.0 mL). The reaction mixture was stirred at room temperature for 24 hours, and the pH of the aqueous phase was adjusted to about 9 to 10 with saturated aqueous sodium carbonate solution. The resulting mixture was extracted with dichloromethane (5.0 mL x 4), the combined organic phase was dried over anhydrous sodium sulfate, filtered, and concentrated under reduced pressure. The resulting residue was purified by silica gel column chromatography (eluent: dichloromethane/methanol = 50/1) to obtain 5-chloro-4-(trifluoromethyl)quinolin-8-ol (73c) (87.0 mg, yield 88%).

At 0°C, sodium nitrite (240.0 mg, 3.5 mmol) was added in batches to a suspension of 5-chloro-4-(trifluoromethyl)quinolin-8-ol (73c) (87.0 mg, 0.35 mmol) in hydrochloric acid (2.0 M) (1.75 mL). The reaction mixture was warmed up to room temperature, stirred for 54 hours, and filtered under reduced pressure. The filter cake was washed with a mixed solvent of ethanol and water (volume ratio, ethanol/water = 1/1) (1.0 mL x 2), and dried under vacuum to obtain 5-chloro-7-nitro-4-(trifluoromethyl)-quinolin-8-ol hydrochloride (73) (69.0 mg, yield 60%).

¹H NMR (400 MHz, DMSO-*d₆*) δ: 9.28 (d, *J =* 4.4 Hz, 1H), 8.41 (overlapping s, 2H).

MS calculated: 291.99; MS found: 293.0, 295.0 [M+H]⁺.

### Example 74

### Synthesis of 4-chloro-3-cyclopropyl-7-nitroquinolin-8-ol

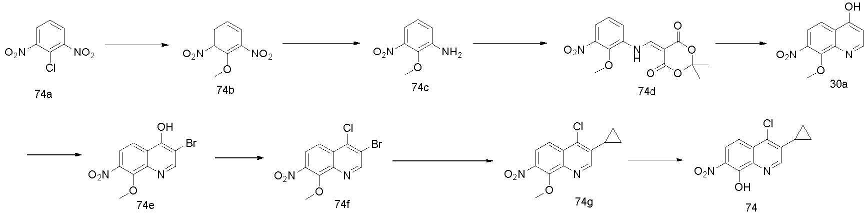

At room temperature, sodium methoxide (10.7 g, 198.0 mmol) was slowly added to a solution of 2,6-dinitrochlorobenzene (74a) (10.0 g, 49.5 mmol) in methanol (100.0 mL). The reaction mixture was stirred at room temperature for 12 hours, and concentrated under reduced pressure to remove the organic solvent. The resulting residue was dissolved in water (30.0 mL) and dichloromethane (30.0 mL). The organic phase was separated, and the aqueous phase was extracted with dichloromethane (30.0 mL x 2). The combined organic phase was dried over anhydrous sodium sulfate, filtered, and concentrated to dryness under reduced pressure to obtain 2-methoxy-1,3-dinitrobenzene (74b) (9.8 g, yield 99%).

At room temperature, reduced iron powder (3.38 g, 60.6 mmol) was added to a solution of 2-methoxy-1,3-dinitrobenzene (74b) (4.0 g, 20.2 mmol) in acetic acid (50.0 mL). The reaction mixture was stirred at 65°C for 1.5 hours, cooled to room temperature, and concentrated under reduced pressure to remove the organic solvent. Dichloromethane (30.0 mL) was added to the resulting residue, and the pH of the aqueous phase was adjusted to about 8 to 9 with saturated aqueous sodium bicarbonate solution. The resulting mixture was filtered under reduced pressure. The organic phase was separated from the filtrate, washed with saturated brine (15.0 mL), dried over anhydrous sodium sulfate, filtered, and concentrated under reduced pressure. The resulting residue was purified by silica gel column chromatography (eluent: petroleum ether/dichloromethane/ethyl acetate = 8/1/1) to obtain 2-methoxy-3-nitroaniline (74c) (2.06 g, yield 61%).

At room temperature, 2-methoxy-3-nitroaniline (74c) (2.06 g, 12.3 mmol), 2,2-dimethyl-1,3-dioxane-4,6-dione (2.12 g, 14.7 mmol) and triethyl orthoformate (4.36 g, 29.4 mmol) were added to ethanol (15.0 mL). The reaction mixture was stirred at 98°C for 4 hours, cooled to room temperature, and filtered under reduced pressure. The filter cake was washed with petroleum ether (boiling range: 60°C to 90°C) (10.0 mL x 3), and dried under vacuum to obtain 5-(((2-methoxy-3-nitrophenyl)amino)methylene)-2,2-dimethyl-1,3-dioxane-4,6-dione (74d) (3.85 g, yield 97%).

At room temperature, 5-(((2-methoxy-3-nitrophenyl)amino)methylene)-2,2-dimethyl-1,3-dioxane-4,6-dione (74d) (3.85 g, 11.9 mmol) was added to diphenyl ether (50.0 mL). The reaction mixture was stirred at 265°C for 2 hours, cooled to room temperature, diluted with petroleum ether (boiling range: 60°C to 90°C) (250.0 mL), and filtered under reduced pressure. The filter cake was washed with petroleum ether (boiling range: 60°C to 90°C) (10.0 mL x 4), and dried under vacuum to obtain 8-methoxy-7-nitroquinolin-4-ol (30a) (2.33 g, yield 89%).

At room temperature, N-bromosuccinimide (404.0 mg, 2.3 mmol) was added to a suspension of 8-methoxy-7-nitroquinolin-4-ol (30a) (500.0 mg, 2.3 mmol) in acetonitrile (6.0 mL). The reaction mixture was stirred at room temperature for 2 hours, and concentrated under reduced pressure to remove the organic solvent. Water (30.0 mL) was added to the resulting residue, stirred for 15 minutes, and filtered under reduced pressure. The filter cake was washed with water (5.0 mL x 2), and dried under vacuum to obtain the crude product 3-bromo-8-methoxy-7-nitroquinolin-4-ol (74e) (628.0 mg, crude yield 93%).

At room temperature, 3-bromo-8-methoxy-7-nitroquinolin-4-ol (74e) (628.0 mg, 0.45 mmol) was added to phosphorus oxychloride (8.0 mL). The reaction mixture was stirred at 100°C for 2 hours, cooled to room temperature, and concentrated under reduced pressure to remove phosphorus oxychloride. The resulting residue was dissolved in dichloromethane (15.0 mL), and the pH of the aqueous phase was adjusted to about 9 to 10 with saturated aqueous sodium carbonate solution. The organic phase was separated, washed with saturated brine (10.0 mL), dried over anhydrous sodium sulfate, filtered, and concentrated under reduced pressure to obtain the crude product 3-bromo-4-chloro-8-methoxy-7-nitroquinoline (74f) (670.0 mg, crude yield 100%).

At room temperature, 3-bromo-4-chloro-8-methoxy-7-nitroquinoline (74f) (350.0 mg, 1.1 mmol), cyclopropylboronic acid (123.0 mg, 1.43 mmol), palladium acetate (25.0 mg, 0.11 mmol), tricyclohexylphosphine (62.0 mg, 0.22 mmol) and potassium phosphate (817.0 mg, 3.85 mmol) were added successively to a mixed solvent of toluene (7.0 mL) and water (0.70 mL). The reaction solution was stirred at 95°C for 3 hours, cooled to room temperature, and diluted with ethyl acetate (10.0 mL) and water (10.0 mL). The organic phase was separated, and the aqueous phase was extracted with ethyl acetate (10.0 mL x 2). The organic phases were combined, washed with saturated brine (20.0 mL), dried over anhydrous sodium sulfate, filtered, and concentrated under reduced pressure. The resulting residue was purified by silica gel column chromatography (eluent: petroleum ether/dichloromethane/ethyl acetate = 7/2/1) to obtain 4-chloro-3-cyclopropyl-8-methoxy-7-nitroquinoline (74g) (117.0 mg, yield 38%).

At room temperature, 4-chloro-3-cyclopropyl-8-methoxy-7-nitroquinoline (74g) (45.0 mg, 0.16 mmol) and lithium chloride (68.0 mg, 1.6 mmol) were added to 1-methyl-2-pyrrolidone (2.0 mL). The reaction mixture was stirred at 180°C for 30 minutes, cooled to room temperature, and concentrated under reduced pressure to remove the organic solvent. Water (2.0 mL) was added to the resulting residue, stirred for 3 minutes, and filtered under reduced pressure. The filter cake was washed with a mixed solvent of ethanol and water (volume ratio, ethanol/water = 1/1) (2.0 mL x 2), and dried under vacuum to obtain 4-chloro-3-cyclopropyl-7-nitroquinolin-8-ol (74) (50.7 mg, yield 91%).

¹H NMR (400 MHz, DMSO-*d*₆) δ: 8.22 (s, 1H), 8.01 (d, *J* = 10.0 Hz, 1H), 6.63 (d, *J* = 9.6 Hz, 1H), 2.30-2.24 (m, 1H), 1.17 - 1.12 (m, 2H), 0.97 - 0.91(m, 2H).

MS calculated: 264.03; MS found: 265.1, 267.0 [M+H]⁺.

### Example 75

### Synthesis of 3-cyclopropyl-4-methoxy-7-nitroquinolin-8-ol (75)

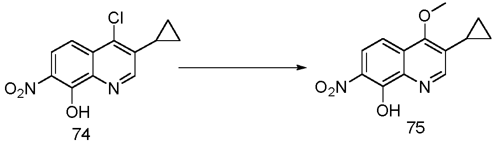

At room temperature, 4-chloro-3-cyclopropyl-7-nitroquinolin-8-ol (74) (42.0 mg, 0.16 mmol) and sodium methoxide (43.0 mg, 0.80 mmol) were added to a mixed solvent of 1-methyl-2-pyrrolidone (1.0 mL) and dimethyl sulfoxide (1.0 mL). The resulting mixture was stirred at 120°C for 2 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/methanol = 100%/0 - 10%/90%, gradient elution, 0.1% formic acid was added to the mobile phase by volume percentage, and the flow rate was 20.0 mL/min) to obtain 3-cyclopropyl-4-methoxy-7-nitroquinolin-8-ol (75) (10.7 mg, yield 26%).

¹H NMR (400 MHz, DMSO-*d*₆) δ: 7.97 (d, *J* = 9.6 Hz, 1H), 7.86 (s, 1H), 6.80 (d, *J* = 10.0 Hz, 1H), 3.98 (s, 3H), 2.29 - 2.27 (m, 1H), 1.13 - 1.07 (m, 2H), 1.06 - 1.01 (m, 2H).

MS calculated: 260.08; MS found: 261.1 [M+H]⁺.

### Example 76

### Synthesis of 8-hydroxy-7-nitroquinoline-4-carbonitrile (76)

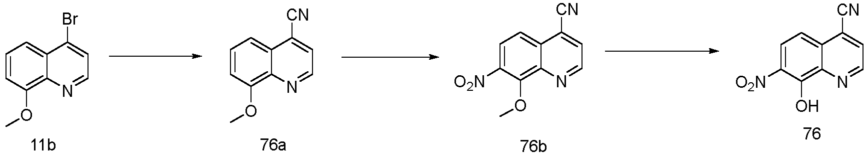

At room temperature, zinc cyanide (788.0 mg, 6.71 mmol) and tetrakis(triphenylphosphine)palladium (728.0 mg, 0.63 mmol) were added to a solution of 4-bromo-8-methoxyquinoline (11b) (1.0 g, 4.2 mmol) in N,N-dimethylformamide (15.0 mL). The reaction mixture was stirred at 100°C for 2.5 hours, cooled to room temperature, and filtered under reduced pressure. The filtrate was concentrated under reduced pressure to remove the organic solvent, and the resulting residue was dissolved in dichloromethane (15.0 mL) and water (15.0 mL). The organic phase was separated, washed with saturated brine (10.0 mL), dried over anhydrous sodium sulfate, filtered, and concentrated under reduced pressure. Ethyl acetate (5.0 mL) was added to the resulting residue, stirred for 3 minutes, and filtered under reduced pressure. The filter cake was washed with ethyl acetate (2.5 mL x 2), and dried under vacuum to obtain 8-methoxyquinoline-4-carbonitrile (76a) (650.0 mg, yield 84%).

At 0°C, nitric acid (65 weight%) (0.45 mL, 6.52 mmol) was slowly added to a solution of 8-methoxyquinoline-4-carbonitrile (76a) (200.0 mg, 1.08 mmol) in acetic anhydride (5.0 mL). The reaction mixture was stirred at 0°C for 10 minutes, followed by the slowly dropwise addition of sulfuric acid (98 weight%) (0.1 mL, 1.87 mmol), and continued to stir for 30 minutes. The pH of the aqueous phase was adjusted to about 9 to 10 with aqueous sodium hydroxide solution (1.0 M). The resulting mixture was extracted with dichloromethane (10.0 mL x 3). The organic phases were combined, washed with saturated brine (10.0 mL), dried over anhydrous sodium sulfate, filtered, and concentrated under reduced pressure. The resulting residue was purified by silica gel column chromatography (eluent: petroleum ether/ethyl acetate = 4/1) to obtain 8-methoxy-7-nitroquinoline-4-carbonitrile (76b) (65.0 mg, yield 26%).

At room temperature, 8-methoxy-7-nitroquinoline-4-carbonitrile (76b) (65.0 mg, 0.28 mmol) and lithium chloride (119.0 mg, 2.8 mmol) were added to N,N-dimethylformamide (2.0 mL). The reaction mixture was stirred at 160°C for 30 minutes, cooled to room temperature, and concentrated under reduced pressure to remove the organic solvent. Water (2.0 mL) was added to the resulting residue, stirred for 3 minutes, and filtered under reduced pressure. The filter cake was washed successively with ethanol (2.0 mL) and water (2.0 mL), and dried under vacuum to obtain 8-hydroxy-7-nitroquinoline-4-carbonitrile (76) (44.0 mg, yield 72%).

¹H NMR (400 MHz, DMSO-*d₆*) δ: 8.75 (d, *J* = 4.4 Hz, 1H), 8.15 (d, *J* = 9.2 Hz, 1H), 8.02 (d, *J =* 4.4 Hz, 1H), 6.54 (d, *J =* 9.6 Hz, 1H).

MS calculated: 215.03; MS found: 216.1 [M+H]⁺.

### Example 77

### Synthesis of 4-(difluoromethoxy)-7-nitroquinolin-8-ol (77)

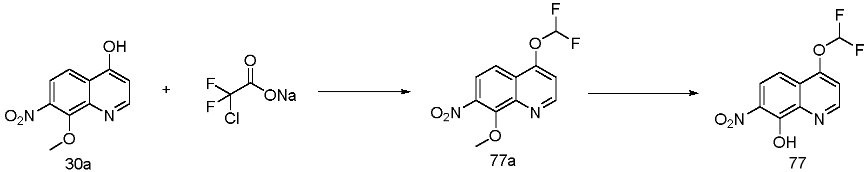

At room temperature, 8-methoxy-7-nitroquinolin-4-ol (30a) (66.1 mg, 0.30 mmol) and sodium difluorochloroacetate (137.0 mg, 0.90 mmol) were added successively to a suspension of cesium carbonate (293.0 mg, 0.30 mmol) in N,N-dimethylformamide (1.5 mL). The reaction mixture was stirred at room temperature for 15 minutes, and then at 80°C for 30 minutes, cooled to room temperature, and diluted with water (10.0 mL) and dichloromethane (10.0 mL). The organic phase was separated, and the aqueous phase was extracted with dichloromethane (5.0 mL x 3). The combined organic phase was dried over anhydrous sodium sulfate, filtered, and concentrated under reduced pressure. The resulting residue was purified by silica gel column chromatography (eluent: dichloromethane/petroleum ether = 3/1) to obtain 4-(difluoromethoxy)-8-methoxy-7-nitroquinoline (77a) (52.6 mg, yield 65%).

At room temperature, 4-(difluoromethoxy)-8-methoxy-7-nitroquinoline (77a) (52.6 mg, 0.195 mmol) and lithium chloride (82.5 mg, 1.95 mmol) were added to N,N-dimethylformamide (0.24 mL). The reaction mixture was stirred at 160°C for 30 minutes, cooled to room temperature, and concentrated under reduced pressure to remove the organic solvent. Water (1.0 mL) was added to the resulting residue, stirred for 3 minutes, and filtered under reduced pressure. The filter cake was washed with a mixed solvent of ethanol and water (volume ratio, ethanol/water = 1/1) (0.5 mL x 3), and dried under vacuum to obtain 4-(difluoromethoxy)-7-nitroquinolin-8-ol (77) (37.2 mg, yield 74%).

¹H NMR (400 MHz, DMSO-*d₆*) δ: 8.59 (d, *J =* 5.2 Hz, 1H), 7.98 (d, *J =* 9.6 Hz, 1H), 7.62 (t, *J* = 72.8 Hz, 1H), 7.32 (d, *J =* 5.2 Hz, 1H), 6.59 (d, *J* = 9.6 Hz, 1H).

MS calculated: 256.03; MS found: 257.1 [M+H]⁺.

### Example 78

### Synthesis of 3-(8-hydroxy-7-nitroquinolin-4-yl)-N-acryloylmorpholine (78)

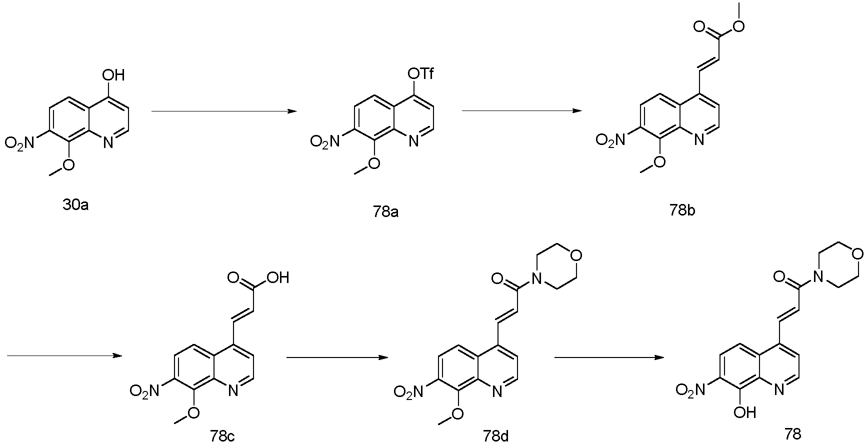

At room temperature, 8-methoxy-7-nitroquinolin-4-ol (30a) (5.07 g, 23.0 mmol) and N,N-diisopropylethylamine (12.3 mL, 70.3 mmol) were added to N,N-dimethylformamide (75.0 mL), followed by the slow addition of N-phenylbis(trifluoromethanesulfonyl)imide (11.1 g, 31.1 mmol). The reaction mixture was stirred at room temperature for 12 hours, and diluted with ethyl acetate (100.0 mL) and water (100.0 mL). The organic phase was separated, and the aqueous phase was extracted with ethyl acetate (50.0 mL x 3). The combined organic phase was dried over anhydrous sodium sulfate, filtered, and concentrated under reduced pressure. The resulting residue was purified by silica gel column chromatography (eluent: petroleum ether/ethyl acetate = 8/1) to obtain 8-methoxy-7-nitroquinolin-4-yl trifluoromethanesulfonate (78a) (3.99 g, yield 50%).

At room temperature, 8-methoxy-7-nitroquinolin-4-yl trifluoromethanesulfonate (78a) (3.99 g, 11.3 mmol), methyl acrylate (2.2 mL, 24.3 mmol), palladium acetate (254.0 mg, 1.13 mmol), tris(*o*-methylphenyl)phosphine (516.0 mg, 1.7 mmol) and triethylamine (4.3 mL, 31.5 mmol) were added successively to N,N-dimethylformamide (75.0 mL). The reaction mixture was stirred at 100°C for 12 hours, cooled to room temperature, and concentrated under reduced pressure to remove the organic solvent. Ethyl acetate (40.0 mL) and water (40.0 mL) were added to the resulting residue. The organic phase was separated, and the aqueous phase was extracted with ethyl acetate (20.0 mL x 2). The combined organic phase was dried over anhydrous sodium sulfate, filtered, and concentrated under reduced pressure. The resulting residue was purified by silica gel column chromatography (eluent: petroleum ether/dichloromethane/ethyl acetate = 8/1/1) to obtain methyl 3-(8-methoxy-7-nitroquinolin-4-yl)acrylate (78b) (618.9 mg, yield 19%).

At room temperature, methyl 3-(8-methoxy-7-nitroquinolin-4-yl)acrylate (78b) (618.9 mg, 2.15 mmol) was added to a mixed solvent of tetrahydrofuran (8.0 mL), methanol (8.0 mL) and water (2.7 mL), followed by the addition of lithium hydroxide (77.4 mg, 3.23 mmol). The reaction mixture was stirred at room temperature for 12 hours, and concentrated under reduced pressure to remove the organic solvent. Ethyl acetate (15.0 mL) and water (15.0 mL) were added to the resulting residue, and the aqueous phase was separated. The pH of the aqueous phase was adjusted to about 3 to 4 with hydrochloric acid (1.0 M) solution. The resulting mixture was filtered under reduced pressure, the filter cake was washed with water (2.0 mL x 2), and dried under vacuum to obtain 3-(8-methoxy-7-nitroquinolin-4-yl)acrylic acid (78c) (560.1 mg, yield 95%).

At room temperature, 3-(8-methoxy-7-nitroquinolin-4-yl)acrylic acid (78c) (143.0 mg, 0.52 mmol), morpholine (68.0 mg, 0.78 mmol), O-(7-azabenzotriazol-1-yl)-N,N,N',N'-tetramethyluronium hexafluorophosphate (297.0 mg, 0.78 mmol) and N,N-diisopropylethylamine (0.28 mL, 1.56 mmol) were added successively to N,N-dimethylformamide (2.0 mL). The reaction mixture was stirred at room temperature for 12 hours, and concentrated under reduced pressure to remove the organic solvent. Ethyl acetate (10.0 mL) and water (10.0 mL) were added to the resulting residue. The organic phase was separated, and the aqueous phase was extracted with ethyl acetate (10.0 mL x 2). The combined organic phase was dried over anhydrous sodium sulfate, filtered, and concentrated under reduced pressure. The resulting residue was purified by silica gel column chromatography (eluent: dichloromethane/ethyl acetate = 7/3) to obtain 3-(8-methoxy-7-nitroquinolin-4-yl)-N-acryloylmorpholine (78d) (88.0 mg, yield 49%).

At room temperature, 3-(8-methoxy-7-nitroquinolin-4-yl)-N-acryloylmorpholine (78d) (44.0 mg, 0.13 mmol) and lithium chloride (53.8 mg, 1.3 mmol) were added to N,N-dimethylformamide (1.0 mL). The reaction mixture was stirred at 160°C for 1 hour, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/methanol = 100%/0 - 80%/20%, gradient elution, 0.1% formic acid was added to the mobile phase by volume percentage, and the flow rate was 20.0 mL/min) to obtain 3-(8-hydroxy-7-nitroquinolin-4-yl)-N-acryloylmorpholine (78) (20.2 mg, yield 24%).

¹H NMR (400 MHz, DMSO-*d₆*) δ: 8.70 (d, *J =* 4.6 Hz, 1H), 8.09 (d, *J =* 15.6 Hz, 1H), 8.02 (d, *J* = 9.6 Hz, 1H), 7.99 (d, *J* = 4.6 Hz, 1H), 7.51 (d, *J* = 15.2 Hz, 1H), 6.87 (d, *J* = 10.0 Hz, 1H), 3.81 - 3.74 (m, 2H), 3.68 - 3.60 (m, 4H), 3.44 - 3.37 (m, 2H).

MS calculated: 329.10; MS found: 330.2 [M+H]⁺.

### Example 79

### Synthesis of 3-(8-hydroxy-7-nitroquinolin-4-yl)-N-methacrylamide (79)

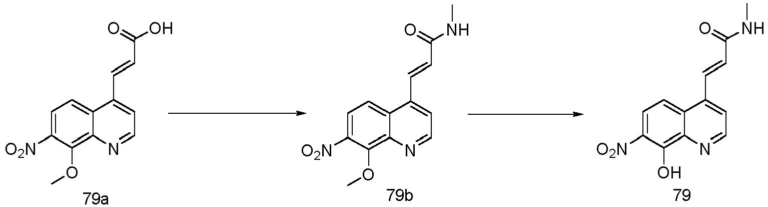

At room temperature, 3-(8-methoxy-7-nitroquinolin-4-yl)acrylic acid (79a) (150.0 mg, 0.55 mmol), methylamine hydrochloride (55.0 mg, 0.82 mmol), O-(7-azabenzotriazol-1-yl)-N,N,N',N'-tetramethyluronium hexafluorophosphate (312.0 mg, 0.82 mmol) and N,N-diisopropylethylamine (0.30 mL, 1.64 mmol) were added successively to N,N-dimethylformamide (2.0 mL). The reaction mixture was stirred at room temperature for 12 hours, and concentrated under reduced pressure to remove the organic solvent. Ethyl acetate (10.0 mL) and water (10.0 mL) were added to the resulting residue. The organic phase was separated, and the aqueous phase was extracted with ethyl acetate (10.0 mL x 2). The combined organic phase was dried over anhydrous sodium sulfate, filtered, and concentrated under reduced pressure. The resulting residue was purified by silica gel column chromatography (eluent: dichloromethane/ethyl acetate = 7/3) to obtain 3-(8-methoxy-7-nitroquinolin-4-yl)-N-methylacrylamide (79b) (105.0 mg, yield 67%).

At room temperature, 3-(8-methoxy-7-nitroquinolin-4-yl)-N-methylacrylamide (79b) (105.0 mg, 0.37 mmol) and lithium chloride (157.0 mg, 3.7 mmol) were added to N,N-dimethylformamide (3.0 mL). The reaction mixture was stirred at 160°C for 1 hour, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/methanol = 100%/0 - 80%/20%, gradient elution, 0.1% formic acid was added to the mobile phase by volume percentage, and the flow rate was 20.0 mL/min) to obtain 3-(8-hydroxy-7-nitroquinolin-4-yl)-N-methacrylamide (79) (18.7 mg, yield 19%).

¹H NMR (400 MHz, DMSO-*d₆*) δ: 9.03 (d, *J =* 4.8 Hz, 1H), 8.44 (d, *J =* 4.4 Hz, 1H), 8.12 (d, *J =* 9.6 Hz, 1H), 8.08 (d, *J* = 16.0 Hz, 1H), 7.97 (d, *J* = 4.4 Hz, 1H), 7.70 (d, *J* = 9.2 Hz, 1H), 6.92 (d, *J =* 15.6 Hz, 1H), 2.79 (d, *J =* 4.8 Hz, 3H).

MS calculated: 273.07; MS found: 274.1 [M+H]⁺.

### Example 80

### Synthesis of 4-ethynyl-7-nitroquinolin-8-ol (80)

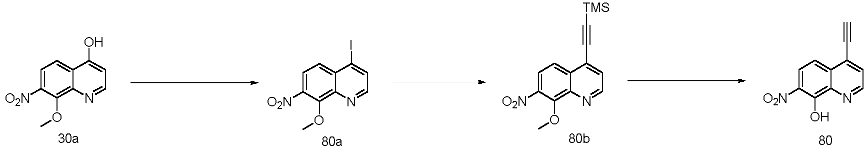

At 0°C, 8-methoxy-7-nitroquinolin-4-ol (30a) (300.0 mg, 1.36 mmol) and pyridine (129.0 mg, 1.63 mmol) were added to dichloromethane (6.0 mL), followed by the slowly dropwise addition of trifluoromethanesulfonic anhydride (751.0 mg, 2.66 mmol). The reaction mixture was warmed up to room temperature, and stirred for 3.5 hours, followed by the addition of sodium iodide (1.02 g, 6.8 mmol). Trifluoromethanesulfonic acid (0.2 mL, 2.26 mmol) was slowly added dropwise at 0°C. The reaction mixture was stirred at room temperature for 16 hours, and the pH of the aqueous phase was adjusted to about 9 to 10 with saturated aqueous sodium carbonate solution. The resulting mixture was extracted with dichloromethane (10.0 mL x 3). The organic phases were combined, dried over anhydrous sodium sulfate, filtered, and concentrated under reduced pressure. The resulting residue was purified by silica gel column chromatography (eluent: petroleum ether/ethyl acetate = 5/1) to obtain 4-iodo-8-methoxy-7-nitroquinoline (80a) (210.0 mg, yield 47%).

At room temperature, (trimethylsilyl)acetylene (188.0 mg, 1.9 mmol), bis(triphenylphosphine)palladium(II) chloride (45.0 mg, 0.06 mmol), copper iodide (6.0 mg, 0.03 mmol) and triethylamine (0.27 mL, 1.9 mmol) were added successively to a solution of 4-iodo-8-methoxy-7-nitroquinoline (80a) (210.0 mg, 0.64 mmol) in N,N-dimethylformamide (5.0 mL). The reaction mixture was stirred at 60°C for 16 hours, cooled to room temperature, and concentrated under reduced pressure to remove the organic solvent. The resulting residue was purified by silica gel column chromatography (eluent: petroleum ether/ethyl acetate = 4/1) to obtain 8-methoxy-7-nitro-4-((trimethylsilyl)ethynyl)quinoline (80b) (91.0 mg, yield 48%).

At room temperature, 8-methoxy-7-nitro-4-((trimethylsilyl)ethynyl)quinoline (80b) (81.0 mg, 0.27 mmol) and lithium chloride (113.0 mg, 2.7 mmol) were added to N,N-dimethylformamide (2.0 mL). The reaction mixture was stirred at 150°C for 1 hour, cooled to room temperature, and concentrated under reduced pressure to remove the organic solvent. Potassium carbonate (372.0 mg, 2.7 mmol) and methanol (5.0 mL) were added to the resulting residue, stirred at room temperature for 2 hours, and filtered under reduced pressure. The filtrate was concentrated under reduced pressure. Water (2.0 mL) was added to the resulting residue, stirred for 3 minutes, and filtered under reduced pressure. A mixed solvent of ethanol (2.0 mL) and water (2.0 mL) was added to the resulting filter cake, stirred for 10 minutes, and filtered under reduced pressure. The filter cake was dried under vacuum to obtain 4-ethynyl-7-nitroquinolin-8-ol (80) (57.0 mg, yield 99%).

¹H NMR (400 MHz, DMSO-*d₆*) δ: 8.58 (s, 1H), 8.01 (d, *J* = 9.5 Hz, 1H), 7.62 (s, 1H), 6.67 (d, *J =* 9.5 Hz, 1H), 4.96 (s, 1H).

MS calculated: 214.04; MS found: 215.1 [M+H]⁺.

### Example 81

### Synthesis of 4-(difluoromethyl)-7-nitroquinolin-8-ol (81)

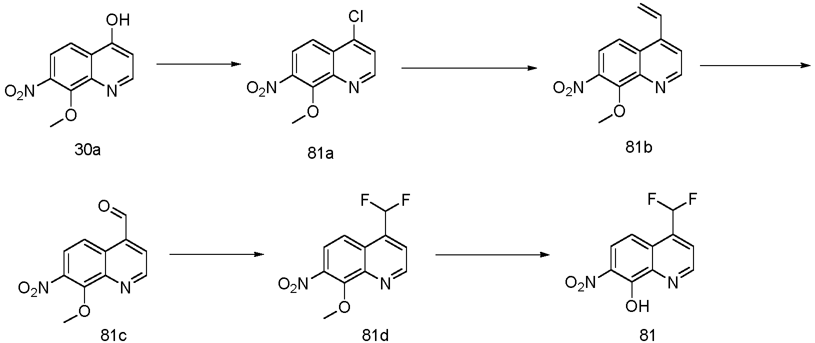

At room temperature, 8-methoxy-7-nitroquinolin-4-ol (30a) (1.5 g, 6.81 mmol) was added to phosphorus oxychloride (15.0 mL). The reaction mixture was stirred at 100°C for 2 hours, cooled to room temperature, and concentrated under reduced pressure. Dichloromethane (20.0 mL) was added to the resulting residue, and the pH of the aqueous phase was adjusted to about 8 to 9 with saturated aqueous sodium bicarbonate solution. The organic phase was separated, and the aqueous phase was extracted with dichloromethane (20.0 mL x 2). The combined organic phase was dried over anhydrous sodium sulfate, filtered, and concentrated under reduced pressure. The resulting residue was purified by silica gel column chromatography (eluent: petroleum ether/ethyl acetate = 4/1) to obtain 4-chloro-8-methoxy-7-nitroquinoline (81a) (510.0 mg, yield 32%).

At room temperature, 4-chloro-8-methoxy-7-nitroquinoline (81a) (510.0 mg, 2.13 mmol), potassium vinyl trifluoroborate (458.0 mg, 3.42 mmol), [1,1'-bis(diphenylphosphino)ferrocene]dichloropalladium(II) (234.0 mg, 0.32 mmol) and potassium carbonate (885.0 mg, 6.41 mmol) were added successively to a mixed solvent of 1,4-dioxane (8.0 mL) and water (2.0 mL). The reaction mixture was stirred at 95°C for 16 hours, cooled to room temperature, and concentrated under reduced pressure. Dichloromethane (10.0 mL) and water (10.0 mL) were added to the resulting residue. The organic phase was separated, and the aqueous phase was extracted with dichloromethane (10.0 mL x 2). The combined organic phase was dried over anhydrous sodium sulfate, filtered, and concentrated under reduced pressure. The resulting residue was purified by silica gel column chromatography (eluent: petroleum ether/ethyl acetate = 4/1) to obtain 8-methoxy-7-nitro-4-vinylquinoline (81b) (350.0 mg, yield 71%).

At 0°C, 8-methoxy-7-nitro-4-vinylquinoline (81b) (350.0 mg, 1.52 mmol) and potassium osmate dihydrate (56.0 mg, 0.15 mmol) were added successively to a mixed solvent of tetrahydrofuran (18.0 mL) and water (4.5 mL). The reaction mixture was stirred at 0°C for 10 minutes, followed by the addition of sodium periodate (975.0 mg, 4.56 mmol). The resulting mixture was warmed up to room temperature, stirred for 16 hours, and diluted with ethyl acetate (10.0 mL) and water (10.0 mL). The organic phase was separated, and the aqueous phase was extracted with ethyl acetate (10.0 mL x 2). The combined organic phase was dried over anhydrous sodium sulfate, filtered, and concentrated under reduced pressure to obtain the crude product 8-methoxy-7-nitroquinoline-4-carbaldehyde (81c) (355.0 mg, crude yield 100%).

At 0°C, diethylaminosulfur trifluoride (180 mg, 1.11 mmol) was slowly added dropwise to a solution of 8-methoxy-7-nitroquinoline-4-carbaldehyde (81c) (86.0 mg, 0.37 mmol) in dichloromethane (18.0 mL). The reaction mixture was stirred at 0°C for 10 minutes, and at room temperature for 3 hours. The pH of the aqueous phase was adjusted to about 8 to 9 with saturated aqueous sodium bicarbonate solution. The organic phase was separated, and the aqueous phase was extracted with dichloromethane (10.0 mL x 2). The combined organic phase was dried over anhydrous sodium sulfate, filtered, and concentrated under reduced pressure. The resulting residue was purified by silica gel column chromatography (eluent: petroleum ether/ethyl acetate = 4/1) to obtain 4-(difluoromethyl)-8-methoxy-7-nitroquinoline (81d) (65.0 mg, yield 69%).

At room temperature, 4-(difluoromethyl)-8-methoxy-7-nitroquinoline (81d) (65.0 mg, 0.256 mmol) and lithium chloride (107.0 mg, 2.56 mmol) were added to N,N-dimethylformamide (2.0 mL). The reaction mixture was stirred at 160°C for 40 minutes, cooled to room temperature, and concentrated under reduced pressure to remove the organic solvent. Water (2.0 mL) was added to the resulting residue, stirred for 3 minutes, and filtered under reduced pressure. A mixed solvent of ethanol (2.0 mL) and water (2.0 mL) was added to the resulting filter cake, stirred for 10 minutes, and filtered under reduced pressure. The filter cake was dried under vacuum to obtain 4-(difluoromethyl)-7-nitroquinolin-8-ol (81) (47.3 mg, yield 77%).

¹H NMR (400 MHz, DMSO-*d₆*) δ: 8.74 (d, *J =* 4.3 Hz, 1H), 8.03 (d, *J =* 9.6 Hz, 1H), 7.72 (d, *J* = 4.3 Hz, 1H), 7.55 (t, *J =* 54.0 Hz, 1H), 6.62 (d, *J =* 9.6 Hz, 1H).

MS calculated: 240.03; MS found: 241.1 [M+H]⁺.

### Example 82

### Synthesis of 4-chloro-7-nitro-3-phenylquinolin-8-ol (82)

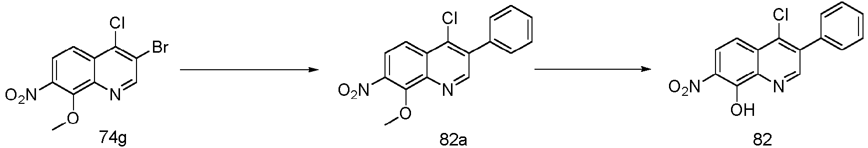

At room temperature, 3-bromo-4-chloro-8-methoxy-7-nitroquinoline (74g) (120.0 mg, 0.38 mmol), phenylboronic acid (48.0 mg, 0.40 mmol), palladium acetate (8.5 mg, 0.038 mmol), tricyclohexylphosphine (21.2 mg, 0.076 mmol) and potassium phosphate (281.0 mg, 1.32 mmol) were added successively to a mixed solvent of toluene (3.0 mL) and water (0.5 mL). The reaction mixture was stirred at 92°C for 3 hours, cooled to room temperature, and diluted with ethyl acetate (10.0 mL) and water (10.0 mL). The organic phase was separated, and the aqueous phase was extracted with ethyl acetate (10.0 mL x 2). The combined organic phase was dried over anhydrous sodium sulfate, filtered, and concentrated under reduced pressure. The resulting residue was purified by silica gel column chromatography (eluent: petroleum ether/dichloromethane/ethyl acetate = 8/1/1) to obtain 4-chloro-8-methoxy-7-nitro-3-phenylquinoline (82a) (61.0 mg, yield 51%).

At room temperature, 4-chloro-8-methoxy-7-nitro-3-phenylquinoline (82a) (61.0 mg, 0.194 mmol) and lithium chloride (81.0 mg, 1.93 mmol) were added to N,N-dimethylformamide (2.0 mL). The reaction mixture was stirred at 160°C for 50 minutes, cooled to room temperature, and concentrated under reduced pressure to remove the organic solvent. Water (2.0 mL) was added to the resulting residue, stirred for 3 minutes, and filtered under reduced pressure. A mixed solvent of ethanol (2.0 mL) and water (2.0 mL) was added to the resulting filter cake, stirred for 10 minutes, and filtered under reduced pressure. The filter cake was dried under vacuum to obtain 4-chloro-7-nitro-3-phenylquinolin-8-ol (82) (57.0 mg, yield 98%).

¹H NMR (400 MHz, DMSO-*d₆*) δ: 8.54 (s, 1H), 8.09 (d, *J* = 9.7 Hz, 1H), 7.61 - 7.50 (m, 5H), 6.73 (d, *J* = 9.7 Hz, 1H).

MS calculated: 300.03; MS found: 301.1/303.0 [M+H]⁺.

### Example 83

### Synthesis of 5-chloro-3-methyl-7-nitroquinolin-8-ol (83)

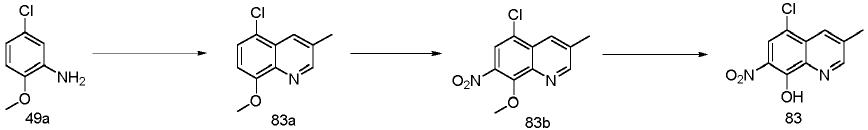

At room temperature, 5-chloro-2-methoxyaniline (49a) (3.0 g, 19.0 mmol) was added to hydrochloric acid solution (6.0 M) (30.0 mL). The reaction mixture was warmed up to 100°C, followed by the slowly dropwise addition of 2-methacrolein (3.34 g, 47.7 mmol). The reaction mixture was stirred at 100°C for 2 hours, and cooled to room temperature. The pH of the aqueous phase was adjusted to about 7 to 8 with aqueous sodium hydroxide solution (1.0 M). The resulting mixture was extracted with dichloromethane (50.0 mL x 3). The organic phases were combined, dried over anhydrous sodium sulfate and filtered, and the filtrate was concentrated under reduced pressure. The resulting residue was purified by silica gel column chromatography (gradient elution, eluent: petroleum ether/ethyl acetate = 7/1 to 3/1) to obtain 3-methyl-5-chloro-8-methoxyquinoline (83a) (1.35 g, yield 34%).

At 0°C, nitric acid (65 weight%) (1.35 mL, 19.6 mmol) was slowly added to a solution of 3-methyl-5-chloro-8-methoxyquinoline (83a) (1.35 g, 6.5 mmol) in acetic anhydride (27.0 mL). The reaction mixture was stirred at 0°C for 5 minutes, followed by the slowly dropwise addition of sulfuric acid (98 weight%) (0.35 mL, 6.5 mmol), and continued to stir for 30 minutes. The pH of the aqueous phase was adjusted to about 9 to 10 with aqueous sodium hydroxide solution (1.0 M). The resulting mixture was extracted with dichloromethane (10.0 mL x 3). The organic phases were combined, washed with saturated brine (10.0 mL), dried over anhydrous sodium sulfate, filtered, and concentrated under reduced pressure. The resulting residue was purified by silica gel column chromatography (eluent: petroleum ether/ethyl acetate = 10/1) to obtain 3-methyl-5-chloro-7-nitro-8-methoxyquinoline (83b) (0.64 g, yield 39%).

At room temperature, 3-methyl-5-chloro-7-nitro-8-methoxyquinoline (83b) (30.0 mg, 0.12 mmol) and lithium chloride (50.0 mg, 1.2 mmol) were added to N,N-dimethylformamide (1.0 mL). The reaction mixture was stirred at 140°C for 2 hours, cooled to room temperature, and concentrated under reduced pressure to remove the organic solvent. Water (1.0 mL) was added to the resulting residue, stirred for 3 minutes, and filtered under reduced pressure. The filter cake was washed with a mixed solvent of water and ethanol (volume ratio, water/ethanol = 1/1) (0.2 mL x 2), and dried under vacuum to obtain 3-methyl-5-chloro-7-nitro-8-hydroxyquinoline (83) (19.0 mg, yield 67%).

¹H NMR (400 MHz, DMSO-*d₆*) δ: 8.61 (s, 1H), 8.03 (d, *J* = 12.3 Hz, 2H), 2.50 (s, 3H).

MS calculated: 238.01; MS found: 239.0 [M+H]⁺.

### Example 84

### Synthesis of 5-cyclopropyl-3-methyl-7-nitroquinolin-8-ol (84)

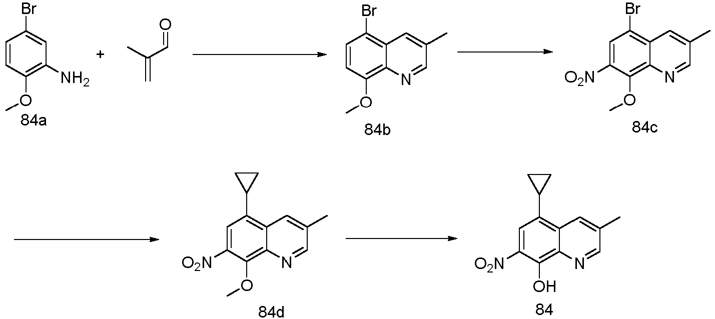

At room temperature, 5-bromo-2-methoxyaniline (84a) (1.5 g, 7.43 mmol) was added to hydrochloric acid solution (6.0 M) (40.0 mL). The reaction mixture was warmed up to 110°C, followed by the slowly dropwise addition of 2-methacrolein (1.8 mL, 22.2 mmol), and stirred for 3 hours. The reaction mixture was cooled to room temperature, and the pH of the aqueous phase was adjusted to about 7 to 8 with aqueous sodium hydroxide solution (6.0 M). The resulting mixture was extracted with ethyl acetate (50.0 mL x 3). The organic phases were combined, dried over anhydrous sodium sulfate, filtered, and concentrated under reduced pressure. The resulting residue was purified by silica gel column chromatography (eluent: petroleum ether/ethyl acetate = 7/3) to obtain 5-bromo-8-methoxy-3-methylquinoline (84b) (1.0 g, yield 53%).

At room temperature, nitric acid (65 weight%) (0.51 mL, 12.0 mmol) was slowly added dropwise to a solution of 5-bromo-8-methoxy-3-methylquinoline (84b) (251.0 mg, 1.0 mmol) in acetic anhydride (5.5 mL). The reaction mixture was stirred at room temperature for 10 minutes, followed by the addition of sulfuric acid (98 weight%) (0.12 mL, 2.4 mmol), and continued to stir for 2 hours. The reaction mixture was diluted with water (30.0 mL), and the pH of the aqueous phase was adjusted to about 8 to 9 with aqueous sodium hydroxide solution (1.0 M). The resulting mixture was extracted with dichloromethane (10.0 mL x 3). The organic phases were combined, dried over anhydrous sodium sulfate, filtered, and concentrated under reduced pressure. The resulting residue was purified by silica gel column chromatography (eluent: petroleum ether/ethyl acetate = 85%/15%) to obtain 5-bromo-8-methoxy-3-methyl-7-nitroquinoline (84c) (50.0 mg, yield 17%).

At room temperature, 5-bromo-8-methoxy-3-methyl-7-nitroquinoline (84c) (50.0 mg, 0.17 mmol), cyclopropylboronic acid (29.0 mg, 0.34 mmol), tetrakis(triphenylphosphine) palladium (39.0 mg, 0.034 mmol) and potassium carbonate (70.4 mg, 0.51 mmol) were added successively to a mixed solvent of toluene (3.0 mL) and water (0.3 mL). The reaction mixture was stirred at 105 °C for 3 hours, cooled to room temperature, and extracted with ethyl acetate (10.0 mL x 3). The combined organic phase was dried over anhydrous sodium sulfate, filtered, and concentrated under reduced pressure. The resulting residue was purified by silica gel column chromatography (eluent: petroleum ether/ethyl acetate = 4/1) to obtain 5-cyclopropyl-8-methoxy-3-methyl-7-nitroquinoline (84d) (28.0 mg, yield 64%).

At room temperature, 5-cyclopropyl-8-methoxy-3-methyl-7-nitroquinoline (84d) (28.0 mg, 0.11 mmol) and lithium chloride (45.6 mg, 1.1 mmol) were added to N,N-dimethylformamide (1.0 mL). The reaction mixture was stirred at 150°C for 1 hour, cooled to room temperature, and concentrated under reduced pressure to remove the organic solvent. Water (2.0 mL) was added to the resulting residue, stirred for 3 minutes, and filtered under reduced pressure. The filter cake was washed successively with water (0.5 mL x 2) and ethanol (0.5 mL x 2), and dried under vacuum to obtain 5-cyclopropyl-3-methyl-7-nitroquinolin-8-ol (84) (16.0 mg, yield 60%).

¹H NMR (400 MHz, DMSO-*d*₆) δ: 8.55 - 8.48 (m, 1H), 8.36 - 8.30 (m, 1H), 7.71 - 7.64 (m, 1H), 2.52 - 2.50 (m, 3H), 2.05 - 1.97 (m, 1H), 0.98 - 0.90 (m, 2H), 0.58 - 0.52 (m, 2H).

MS calculated: 244.08; MS found: 245.1 [M+H]⁺.

### Example 85

### Synthesis of 7-nitro-4-(piperidin-1-yl-methyl)quinolin-8-ol (85)

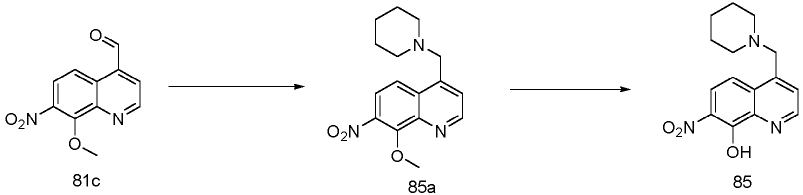

At room temperature, 8-methoxy-7-nitroquinoline-4-carbaldehyde (81c) (110.0 mg, 0.47 mmol) and piperidine (52.0 mg, 0.61 mmol) were added successively to a mixed solvent of tetrahydrofuran (3.0 mL) and dichloromethane (3.0 mL). The reaction mixture was stirred at room temperature for 90 minutes, followed by the addition of sodium triacetoxyborohydride (301.0 mg, 1.42 mmol), and continued to stir for 3 hours. The pH of the aqueous phase was adjusted to about 7 to 8 with saturated aqueous sodium bicarbonate solution. The resulting mixture was extracted with dichloromethane (10.0 mL x 3). The organic phases were combined, washed with saturated brine (10.0 mL), dried over anhydrous sodium sulfate, filtered, and concentrated under reduced pressure. The resulting residue was purified by silica gel thin layer chromatography (developing solvent: dichloromethane/methanol=16/1) to obtain 8-methoxy-7-nitro-4-(piperidin-1-ylmethyl)quinoline (85a) (130.0 mg, yield 72%).

At room temperature, 8-methoxy-7-nitro-4-(piperidin-1-yl-methyl)quinoline (85a) (130.0 mg, 0.43 mmol) and lithium chloride (180 mg, 4.3 mmol) were added to N,N-dimethylformamide (2.0 mL). The reaction mixture was stirred at 160°C for 45 minutes, cooled to room temperature, and concentrated under reduced pressure to remove the organic solvent. Water (2.0 mL) was added to the resulting residue, stirred for 3 minutes, and filtered under reduced pressure. A mixed solvent of ethanol (2.0 mL) and water (2.0 mL) was added to the resulting filter cake, stirred for 10 minutes, and filtered under reduced pressure. The filter cake was dried under vacuum to obtain 7-nitro-4-(piperidin-1-yl-methyl)quinolin-8-ol (85) (112.0 mg, yield 91%).

¹H NMR (400 MHz, DMSO-*d₆*) δ 8.56 (d, *J =* 4.3 Hz, 1H), 7.92 (d, *J =* 9.7 Hz, 1H), 7.54 (d, *J* = 4.3 Hz, 1H), 6.76 (d, *J =* 9.7 Hz, 1H), 3.76 (s, 2H), 2.43 (s, 4H), 1.53 (d, *J =* 4.8 Hz, 4H), 1.43 (d, *J* = 3.6 Hz, 2H).

MS calculated: 287.13; MS found: 288.1[M+H]⁺.

### Example 86

### Synthesis of 5-fluoro-3-methyl-7-nitroquinolin-8-ol (86)

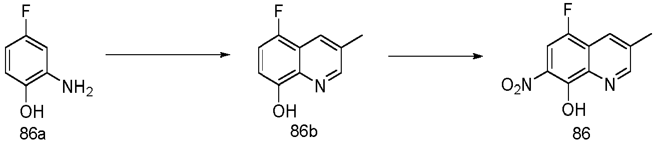

At room temperature, 2-amino-4-fluorophenol (86a) (2.00 g, 15.7 mmol) was added to hydrochloric acid solution (6.0 M) (20.0 mL). The reaction mixture was warmed up to 100°C, followed by the slowly dropwise addition of 2-methacrolein (3.3 mL, 39.4 mmol), and stirred for 2 hours. The reaction mixture was cooled to room temperature, and the pH of the aqueous phase was adjusted to about 7 to 8 with aqueous sodium hydroxide solution (6.0 M). The resulting mixture was extracted with dichloromethane (50.0 mL x 3). The organic phases were combined, dried over anhydrous sodium sulfate, filtered, and concentrated under reduced pressure. The resulting residue was purified by silica gel column chromatography (gradient elution, eluent: petroleum ether/ethyl acetate=10/1 to 5/1) to obtain 5-fluoro-3-methylquinolin-8-ol (86b) (400 mg, yield 14.3%).

At room temperature, sodium nitrite (350.0 mg, 4.1 mmol) was added to a solution of 5-fluoro-3-methylquinolin-8-ol (86b) (90.0 mg, 0.51 mmol) in acetic acid (2.0 mL). The reaction mixture was stirred at room temperature for 1 hour, and the pH of the aqueous phase was adjusted to about 8 to 9 with saturated aqueous sodium bicarbonate solution. The resulting mixture was filtered under reduced pressure, the filter cake was washed with a mixed solvent of ethanol and water (volume ratio, ethanol/water = 1/1) (1.0 mL x 2), and dried under vacuum to obtain 5-fluoro-3-methyl-7-nitroquinolin-8-ol (86) (65.0 mg, yield 58%).

¹H NMR (400 MHz, DMSO-*d₆*) δ 8.77 (s, 1H), 8.03 (s, 1H), 7.69 (d, *J* = 12.5 Hz, 1H), 2.49 (s, 3H).

MS calculated: 222.04; MS found: 223.0 [M+H]⁺.

### Example 87

### Synthesis of 5-(morpholinomethyl)-7-nitroquinolin-8-ol acetate (87)

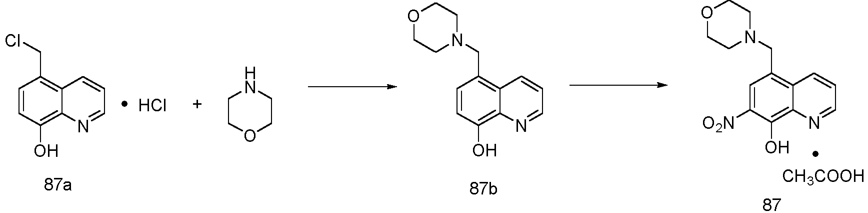

At 0°C, 5-(chloromethyl)quinolin-8-ol hydrochloride (87a) (120.0 mg, 0.62 mmol) and morpholine (270.0 mg, 2.5 mmol) were added successively to dichloromethane (3.0 mL). The reaction mixture was stirred at room temperature for 2 hours, and concentrated under reduced pressure. The resulting residue was purified by silica gel column chromatography (eluent: dichloromethane/methanol = 49/1, and adding 0.5% ammonia (25 to 28weight%, NH₃ content) by the total volume of the eluent) to obtain 5-(morpholinomethyl)quinolin-8-ol (87b) (80.0 mg, yield 53%).

At room temperature, sodium nitrite (34.0 mg, 0.49 mmol) was added to a solution of 5-(morpholinomethyl)quinolin-8-ol (87a) (80.0 mg, 0.33 mmol) in acetic acid (1.5 mL). The reaction mixture was stirred at room temperature for 2 hours, and concentrated under reduced pressure to remove the organic solvent. Water (1.0 mL) was added to the resulting residue, stirred for 10 minutes, and filtered under reduced pressure. The filter cake was washed with water (0.5 mL x 2), and dried under vacuum to obtain 5-(morpholinomethyl)-7-nitroquinolin-8-ol acetate (87) (76.0 mg, yield 45%).

¹H NMR (400 MHz, DMSO-*d*₆) δ: 9.08 - 9.035 (m, 1H), 9.02 - 8.97 (m, 1H), 8.42 - 8.36 (s, 1H), 8.01 - 7.95 (m, 1H), 4.80 - 4.70 (m, 2H), 3.96 - 3.64 (m, 4H), 3.35 - 3.22 (m, 4H).

MS calculated: 289.11; MS found: 290.1 [M+H]⁺.

### Example 88

### Synthesis of ethyl 8-hydroxy-7-nitroquinoline-3-carboxylate (88)

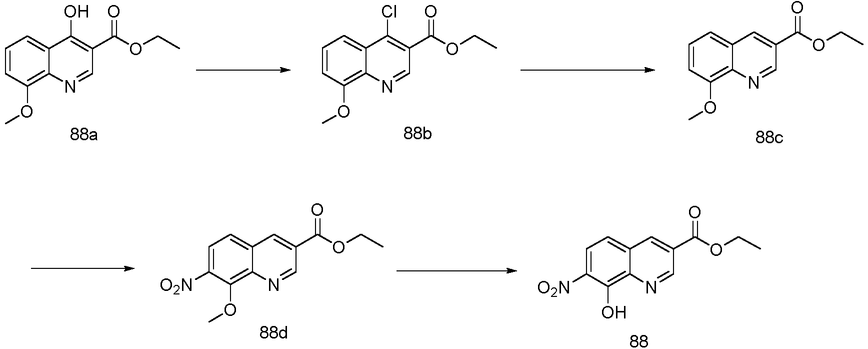

At room temperature, phosphorus oxychloride (2.3 mL, 24.3 mmol) was added to a solution of ethyl 4-hydroxy-8-methoxyquinoline-3-carboxylate (88a) (2.0 g, 8.1 mmol) in acetonitrile (20.0 mL). The reaction mixture was stirred at 90°C for 2.5 hours, cooled to room temperature, and concentrated under reduced pressure. The resulting residue was diluted with dichloromethane (30.0 mL), followed by the addition of triethylamine (4.91 g, 48.5 mmol), stirred for 30 minutes and filtered under reduced pressure. The filter cake was washed with dichloromethane (5.0 mL x 3), and the filtrate was concentrated under reduced pressure. The resulting residue was purified by silica gel column chromatography (eluent: petroleum ether/ethyl acetate = 3/1) to obtain ethyl 4-chloro-8-methoxyquinoline-3-carboxylate (88b) (2.1 g, yield 98%).

At room temperature, ethyl 4-chloro-8-methoxyquinoline-3-carboxylate (88b) (2.1 g, 7.9 mmol), triethylamine (1.2 g, 11.9 mmol), palladium/carbon (palladium loading: 10 weight%) (0.42 g, 0.39 mmol) were added successively to a mixed solvent of tetrahydrofuran (10.0 mL) and methanol (10.0 mL). The reaction mixture was stirred under a hydrogen atmosphere for 2 hours, and filtered through diatomite. The filter cake was washed with tetrahydrofuran (10.0 mL x 3), and the filtrate was concentrated under reduced pressure. The resulting residue was diluted with dichloromethane (20.0 mL), washed with saturated brine (10.0 mL), dried over anhydrous sodium sulfate, filtered, and concentrated under reduced pressure to obtain the crude product ethyl 8-methoxyquinoline-3-carboxylate (88c) (1.8 g, yield 99%).

At 0°C, nitric acid (65 weight%) (0.45 mL, 6.5 mmol) was slowly added to a solution of ethyl 8-methoxyquinoline-3-carboxylate (88c) (500.0 mg, 2.2 mmol) in acetic anhydride (10.0 mL), and stirred for 10 minutes. Concentrated sulfuric acid (98 weight%) (0.12 mL, 2.2 mmol) was slowly added dropwise. The reaction mixture was stirred at 0°C for 2 hours, and diluted with water (20.0 mL). The pH of the aqueous phase was adjusted to about 8 to 9 with saturated aqueous potassium carbonate solution. The resulting mixture was extracted with dichloromethane (30.0 mL x 3). The organic phases were combined, dried over anhydrous sodium sulfate and filtered, and the filtrate was concentrated under reduced pressure. The resulting residue was purified by silica gel column chromatography (gradient elution, eluent: petroleum ether/ethyl acetate = 10/1 to 2/1) to obtain ethyl 8-methoxy-7-nitroquinoline-3-carboxylate (88d) (88.0 mg, yield 15%).

At room temperature, ethyl 8-methoxy-7-nitroquinoline-3-carboxylate (88d) (88.0 mg, 0.32 mmol) and lithium chloride (135.0 mg, 3.2 mmol) were added to N,N-dimethylformamide (2.0 mL). The reaction mixture was stirred at 130°C for 45 minutes, cooled to room temperature, and concentrated under reduced pressure to remove the organic solvent. Water (2.0 mL) was added to the resulting residue, stirred for 3 minutes, and filtered under reduced pressure. The filter cake was washed with water (0.5 mL x 2), and dried under vacuum to obtain ethyl 8-hydroxy-7-nitroquinoline-3-carboxylate (88) (71.0 mg, yield 85%).

¹H NMR (400 MHz, DMSO-*d₆*) δ 9.01 (s, 1H), 8.63 (d, *J* = 1.5 Hz, 1H), 7.96 (d, *J* = 9.3 Hz, 1H), 6.60 (d, *J* = 9.1 Hz, 1H), 4.39 (q, *J =* 7.1 Hz, 2H), 1.37 (t, *J =* 7.1 Hz, 3H).

MS calculated: 262.06; MS found: 263.1 [M+H]⁺.

### Example 89

### Synthesis of 4-(morpholinomethyl)-7-nitroquinolin-8-ol (89)

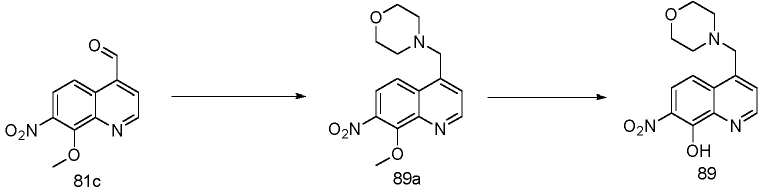

At room temperature, 8-methoxy-7-nitroquinoline-4-carbaldehyde (81c) (77.0 mg, 0.33 mmol) and morpholine (58.0 mg, 0.61 mmol) were added successively to a mixed solvent of tetrahydrofuran (2.0 mL) and dichloromethane (2.0 mL). The reaction mixture was stirred at room temperature for 2 hours, followed by the addition of sodium triacetoxyborohydride (210.0 mg, 0.99 mmol), and continued to stir for 4.5 hours. The pH of the aqueous phase was adjusted to about 7 to 8 with saturated aqueous sodium bicarbonate solution. The resulting mixture was extracted with dichloromethane (10.0 mL x 3). The organic phases were combined, washed with saturated brine (10.0 mL), dried over anhydrous sodium sulfate, filtered, and concentrated under reduced pressure. The resulting residue was purified by silica gel column chromatography (gradient elution, eluent: dichloromethane/methanol=80/1 to 70/1) to obtain 4-((8-methoxy-7-nitroquinolin-4-yl)methyl)morpholine (89a) (61.0 mg, yield 61%).

At room temperature, 4-((8-methoxy-7-nitroquinolin-4-yl)methyl)morpholine (89a) (61.0 mg, 0.2 mmol) and lithium chloride (85.0 mg, 2.0 mmol) were added to N,N-dimethylformamide (2.0 mL). The reaction mixture was stirred at 160°C for 40 minutes, cooled to room temperature, and concentrated under reduced pressure to remove the organic solvent. Water (2.0 mL) was added to the resulting residue, stirred for 3 minutes, and filtered under reduced pressure. A mixed solvent of ethanol (2.0 mL) and water (2.0 mL) was added to the resulting filter cake, stirred for 10 minutes, and filtered under reduced pressure. The filter cake was dried under vacuum to obtain 4-(morpholinomethyl)-7-nitroquinolin-8-ol (89) (38.9 mg, yield 67%).

¹H NMR (400 MHz, DMSO-*d₆*) δ 8.57 (d, *J =* 4.4 Hz, 1H), 7.94 (d, *J* = 9.7 Hz, 1H), 7.56 (d, *J* = 4.5 Hz, 1H), 6.78 (d, *J =* 9.7 Hz, 1H), 3.82 (s, 2H), 3.66 - 3.58 (m, 4H), 2.47 (br s, 4H).

MS calculated: 289.11; MS found: 290.1[M+H]⁺.

### Example 90

### Synthesis of 5-chloro-3-((diethylamino)methyl)-7-nitroquinolin-8-ol ditrifluoroacetate (90)

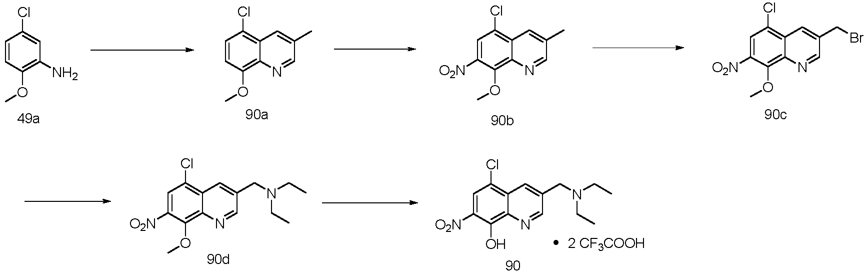

At room temperature, 5-chloro-2-methoxyaniline (49a) (6.0 g, 38.0 mmol) was added to hydrochloric acid solution (6.0 M) (60.0 mL). The reaction mixture was warmed up to 100°C, followed by the slowly dropwise addition of 2-methacrolein (7.8 mL, 95.4 mmol), and stirred for 2 hours. The reaction mixture was cooled to room temperature, and the pH of the aqueous phase was adjusted to about 7 to 8 with aqueous sodium hydroxide solution (6.0 M). The resulting mixture was extracted with dichloromethane (100.0 mL x 3). The organic phases were combined, dried over anhydrous sodium sulfate, filtered, and concentrated under reduced pressure. The resulting residue was purified by silica gel column chromatography (gradient elution, eluent: petroleum ether/ethyl acetate = 7/1 to 3/1) to obtain 5-chloro-8-methoxy-3-methylquinoline (90a) (2.7 g, yield 34%).

At room temperature, nitric acid (65 weight%) (1.50 mL, 21.9 mmol) was slowly added dropwise to a solution of 5-chloro-8-methoxy-3-methylquinoline (90a) (1.5 g, 7.2 mmol) in acetic anhydride (30.0 mL). The reaction mixture was stirred at room temperature for 10 minutes, followed by the slowly dropwise addition of sulfuric acid (98 weight%) (0.39 mL, 7.2 mmol), and continued to stir for 2 hours. The reaction mixture was diluted with water (50.0 mL), and the pH of the aqueous phase was adjusted to about 8 to 9 with saturated aqueous potassium carbonate solution. The resulting mixture was extracted with dichloromethane (50.0 mL x 3). The organic phases were combined, dried over anhydrous sodium sulfate, filtered, and concentrated under reduced pressure. The resulting residue was purified by silica gel column chromatography (eluent: petroleum ether/ethyl acetate = 10/1) to obtain 5-chloro-8-methoxy-3-methyl-7-nitroquinoline (90b) (660.0 mg, yield 36%).

At room temperature, 5-chloro-8-methoxy-3-methyl-7-nitroquinoline (90b) (660.0 mg, 2.5 mmol), N-bromosuccinimide (473.0 mg, 2.6 mmol) and 2,2'-azobisisobutyronitrile (250.0 mg, 1.5 mmol) were added successively to carbon tetrachloride (20.0 mL). The reaction mixture was warmed up to 80°C, stirred for 12 hours, cooled to room temperature, and filtered under reduced pressure. The filter cake was washed with carbon tetrachloride (10.0 mL x 3), and the filtrate was concentrated under reduced pressure to obtain the crude product 3-(bromomethyl)-5-chloro-8-methoxy-7-nitroquinoline (90c) (838.0 mg, crude yield >100%).

At room temperature, the crude 3-(bromomethyl)-5-chloro-8-methoxy-7-nitroquinoline (90c) (102.0 mg, 0.31 mmol) and diethylamine (225.0 mg, 3.1 mmol) were added successively to tetrahydrofuran (4.0 mL). The reaction mixture was stirred at room temperature for 2 hours, and concentrated under reduced pressure. The resulting residue was purified by silica gel thin layer chromatography (developing solvent: petroleum ether/ethyl acetate=3/1) to obtain 5-chloro-3-(diethylamino)methyl-8-methoxy-7-nitroquinoline (90d) (65.0 mg, yield 65%).

At room temperature, 5-chloro-3-(diethylamino)methyl-8-methoxy-7-nitroquinoline (90d) (65.0 mg, 0.2 mmol) and lithium chloride (85.0 mg, 2.0 mmol) were added to N,N-dimethylformamide (1.5 mL). The reaction mixture was stirred at 130°C for 1.5 hours, cooled to room temperature, and concentrated under reduced pressure to remove the organic solvent. The resulting residue was completely dissolved in a mixed solvent of trifluoroacetic acid (0.5 mL) and water (2.0 mL), and purified by reversed-phase high performance liquid chromatography (the chromatographic column was Eclipse XDB-C18 (21.2 mm×250 mm, 7µm), the mobile phase was water/acetonitrile=100%/0 - 75%/25%, gradient elution, and the flow rate was 20.0 mL/min) to obtain 5-chloro-3-((diethylamino)methyl)-7-nitroquinolin-8-ol ditrifluoroacetate (90) (17.0 mg, yield 16%).

¹H NMR (400 MHz, DMSO-*d₆*) δ: 8.87 (s, 1H), 8.49 (s, 1H), 8.11 (s, 1H), 4.51 (s, 2H), 3.13 (dd, *J =* 13.9, 6.8 Hz, 4H), 1.25 (t, *J =* 7.1 Hz, 6H).

MS calculated: 309.09; MS found: 310.1[M+H]⁺.

### Example 91

### Synthesis of 7-nitro-5-(piperidin-1-ylmethyl)quinolin-8-ol acetate (91)

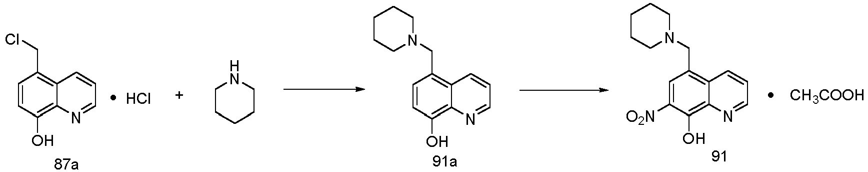

At 0°C, 5-(chloromethyl)quinolin-8-ol hydrochloride (87a) (120.0 mg, 0.62 mmol) and piperidine (264.0 mg, 3.1 mmol) were added successively to dichloromethane (3.0 mL). The reaction mixture was stirred at room temperature for 2 hours, and concentrated under reduced pressure. The resulting residue was purified by silica gel column chromatography (eluent: dichloromethane/methanol = 49/1, and adding 0.5% ammonia (25 to 28weight%, NH₃ content) by the total volume of the eluent) to obtain 5-(piperidin-1-ylmethyl)quinolin-8-ol (91a) (100.0 mg, yield 67%).

At room temperature, sodium nitrite (68.0 mg, 0.99 mmol) was added to a solution of 5-(piperidin-1-ylmethyl)quinolin-8-ol (91a) (100.0 mg, 0.41 mmol) in acetic acid (2.0 mL). The reaction mixture was stirred at room temperature for 2 hours, and filtered under reduced pressure. The filter cake was washed with water (0.5 mL x 2), and dried under vacuum to obtain 7-nitro-5-(piperidin-1-ylmethyl)quinolin-8-ol acetate (91) (129.0 mg, yield 88%).

¹H NMR (400 MHz, DMSO-*d*₆) δ: 9.01-8.97 (m, 1H), 8.92-8.87 (m, 1H), 8.38-8.35 (s, 1H), 7.95-7.89 (m, 1H), 4.71 - 4.65 (m, 2H), 3.35 - 3.10 (m, 4H), 1.93 (s, 3.57 H, the methyl signal of acetic acid)), 1.80 - 1.65 (m, 4H), 1.60 - 1.45 (m, 2H).

MS calculated: 287.11; MS found: 288.1 [M+H]⁺.

### Example 92

### Synthesis of 5-chloro-7-nitro-3-(piperidin-1-ylmethyl)quinolin-8-ol (92)

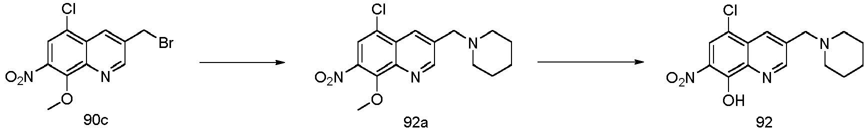

At room temperature, the crude 3-(bromomethyl)-5-chloro-8-methoxy-7-nitroquinoline (90c) (88.0 mg, 0.27 mmol) and piperidine (113.0 mg, 1.33 mmol) were added successively to tetrahydrofuran (2.0 mL). The reaction mixture was stirred at room temperature for 2 hours, and concentrated under reduced pressure. The resulting residue was purified by silica gel thin layer chromatography (developing solvent: dichloromethane/methanol=25/1) to obtain 5-chloro-8-methoxy-7-nitro-3-(piperidin-1-ylmethyl)quinoline (92a) (35.0 mg, yield 39%).

At room temperature, 5-chloro-8-methoxy-7-nitro-3-(piperidin-1-ylmethyl)quinoline (92a) (35.0 mg, 0.10 mmol) and lithium chloride (44.0 mg, 1.0 mmol) were added to N,N-dimethylformamide (1.0 mL). The reaction mixture was stirred at 130°C for 45 minutes, cooled to room temperature, and concentrated under reduced pressure to remove the organic solvent. Water (1.0 mL) was added to the resulting residue, stirred for 3 minutes, and filtered under reduced pressure. The filter cake was washed with water (0.5 mL x 3), and dried under vacuum to obtain 5-chloro-7-nitro-3-(piperidin-1-ylmethyl)quinolin-8-ol (92) (30.7 mg, yield 92%).

¹H NMR (400 MHz, DMSO-*d₆*) δ: 8.62 (s, 1H), 8.09 (s, 1H), 8.02 (s, 1H), 3.65 (s, 2H), 2.37 (s, 4H), 1.54 - 1.47 (m, 4H), 1.39 (d, *J* = 4.4 Hz, 2H).

MS calculated: 321.09; MS found: 322.1 [M+H]⁺.

### Example 93

### Synthesis of 7-nitro-5-(pyrrolidin-1-ylmethyl)quinolin-8-ol acetate (93)

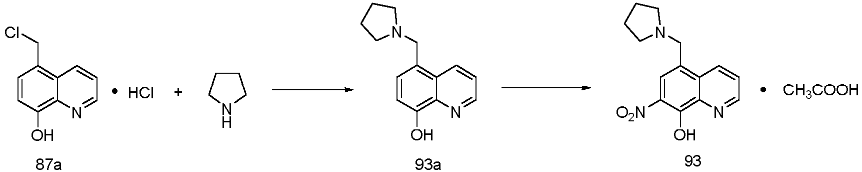

At 0°C, 5-(chloromethyl)quinolin-8-ol hydrochloride (87a) (100.0 mg, 0.43 mmol) and tetrahydropyrrole (147.0 mg, 2.1 mmol) were added successively to dichloromethane (2.0 mL). The reaction mixture was stirred at room temperature for 2 hours, and concentrated under reduced pressure. The resulting residue was purified by silica gel column chromatography (eluent: dichloromethane/methanol = 49/1, and adding 0.5% ammonia (25 to 28weight%, NH₃ content) by the total volume of the eluent) to obtain 5-(pyrrolidin-1-ylmethyl)quinolin-8-ol (93a) (55.0 mg, yield 47%).

At room temperature, sodium nitrite (50.0 mg, 0.72 mmol) was added to a solution of 5-(pyrrolidin-1-ylmethyl)quinolin-8-ol (93a) (55.0 mg, 0.24 mmol) in acetic acid (2.0 mL). The reaction mixture was stirred at room temperature for 2 hours, and filtered under reduced pressure. The filter cake was washed with water (0.5 mL x 2), and dried under vacuum to obtain 7-nitro-5-(pyrrolidin-1-ylmethyl)quinolin-8-ol acetate (93) (22.7 mg, yield 31%).

¹H NMR (400 MHz, DMSO-*d*₆) δ: 9.08-9.04 (m, 1H), 8.96-8.92 (m, 1H), 8.42(s, 1 H), 8.01-7.95 (m, 1H), 4.82 (s, 2H), 3.75-3.40 (m, 5H), 2.04-1.93 (m, 3H), 1.94 (s, 1.34 H, overlapped with CH₃COOH in 1: 0.45 mol. ratio).

MS calculated: 273.11; MS found: 274.1 [M+H]⁺.

### Example 94

### Synthesis of 5-((diethylamino)methyl)-7-nitroquinolin-8-ol (94)

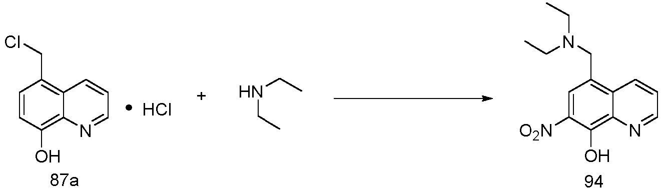

At 0°C, 5-(chloromethyl)quinolin-8-ol hydrochloride (87a) (110.0 mg, 0.48 mmol) and diethylamine (140.0 mg, 1.91 mmol) were added successively to dichloromethane (2.5 mL). The reaction mixture was stirred at room temperature for 2 hours, and concentrated under reduced pressure. Acetic acid (2.0 mL) was added to the resulting residue, followed by the addition of sodium nitrite (99.0 mg, 1.43 mmol). The resulting mixture was stirred at room temperature for 2 hours, and concentrated under reduced pressure. The resulting residue was purified by reversed-phase high performance liquid chromatography (the chromatographic column was Eclipse XDB-C18 (21.2 mm×250 mm, 7µm), the mobile phase was water/methanol=100%/0 - 70%/30%, gradient elution, and the flow rate was 20.0 mL/min) to obtain 5-((diethylamino)methyl)-7-nitroquinolin-8-ol (94) (27.0 mg, yield 31%).

¹H NMR (400 MHz, DMSO-*d*₆) δ: 8.82-8.75 (m, 1H), 8.56-8.49 (m, 1H), 8.07 (s, 1H), 7.67-7.62 (m, 1 H), 4.21 (s, 2 H), 2.95-2.88 (m, 4 H), 1.20-1.15 (m, 4 H).

MS calculated: 275.13; MS found: 276.1 [M+H]⁺.

### Example 95

### Synthesis of 7-nitro-3-(piperidin-1-ylmethyl)quinolin-8-ol (95)

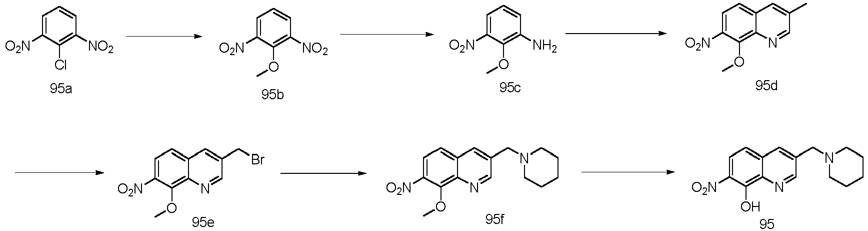

At room temperature, sodium methoxide (5.35 g, 99.0 mmol) was slowly added to a solution of 2,6-dinitrochlorobenzene (corrected in the reaction formula) (95a) (4.7 g, 23.2 mmol) in methanol (50.0 mL). The reaction mixture was stirred at room temperature for 16 hours, and concentrated under reduced pressure to remove the organic solvent. The resulting residue was dissolved in dichloromethane (20.0 mL) and water (20.0 mL). The organic phase was separated, and the aqueous phase was extracted with dichloromethane (20.0 mL x 2). The combined organic phase was washed with saturated brine (20.0 mL), dried over anhydrous sodium sulfate, filtered, and concentrated to dryness under reduced pressure to obtain the crude product 2-methoxy-1,3-dinitrobenzene (95b) (4.56 g, yield 99%).

At room temperature, reduced iron powder (3.86 g, 69.1 mmol) was added to a solution of 2-methoxy-1,3-dinitrobenzene (95b) (4.56 g, 23.0 mmol) in acetic acid (60.0 mL). The reaction mixture was stirred at 65°C for 1.5 hours. The reaction solution was concentrated under reduced pressure to remove the organic solvent. Dichloromethane (20.0 mL) was added to the resulting residue, and the pH of the aqueous phase was adjusted to about 8 to 9 with saturated aqueous sodium bicarbonate solution. The resulting mixture was filtered under reduced pressure. The organic phase was separated from the filtrate, washed with saturated brine (10.0 mL), dried over anhydrous sodium sulfate, filtered, and concentrated under reduced pressure. The resulting residue was purified by silica gel column chromatography (eluent: petroleum ether/dichloromethane/ethyl acetate = 8/1/1) to obtain 2-methoxy-3-nitroaniline (95c) (3.23 g, yield 84%).

At room temperature, 2-methoxy-3-nitroaniline (95c) (3.23 g, 19.2 mmol) was added to hydrochloric acid solution (6.0 M) (60.0 mL). The reaction mixture was warmed up to 100°C, followed by the slowly dropwise addition of 2-methacrolein (3.96 mL, 48.0 mmol), and stirred for 2 hours. The reaction mixture was cooled to room temperature, and the pH of the aqueous phase was adjusted to about 7 to 8 with aqueous sodium hydroxide solution (6.0 M). The resulting mixture was extracted with dichloromethane (50.0 mL x 3). The organic phases were combined, dried over anhydrous sodium sulfate, filtered, and concentrated under reduced pressure. The resulting residue was purified by silica gel column chromatography (eluent: petroleum ether/ethyl acetate = 85%/15%) to obtain 8-methoxy-3-methyl-7-nitroquinoline (95d) (1.67 g, yield 40%).

At room temperature, 8-methoxy-3-methyl-7-nitroquinoline (95d) (106.0 mg, 0.48 mmol), N-bromosuccinimide (95.0 mg, 0.53 mmol), and 2,2'-azobisisobutyronitrile (16.0 mg, 0.1 mmol) were added successively to carbon tetrachloride (6.0 mL). The reaction mixture was warmed up to 90°C, stirred for 16 hours, cooled to room temperature, and filtered under reduced pressure. The filter cake was washed with carbon tetrachloride (5.0 mL x 3), and the filtrate was concentrated under reduced pressure to obtain the crude product 3-(bromomethyl)-8-methoxy-7-nitroquinoline (95e) (143.0 mg, crude yield >100%).

At room temperature, a solution of the crude 3-(bromomethyl)-8-methoxy-7-nitroquinoline (95e) (143.0 mg, 0.48 mmol) in dichloromethane (3.0 mL) was slowly added dropwise to a solution of piperidine (123.0 mg, 1.44 mmol) in dichloromethane (2.0 mL). The reaction mixture was stirred at room temperature for 2 hours, and diluted with dichloromethane (20.0 mL) and water (20.0 mL). The organic phase was separated, and the aqueous phase was extracted with dichloromethane (10.0 mL x 2). The combined organic phase was washed with saturated brine (10.0 mL), dried over anhydrous sodium sulfate, filtered, and concentrated under reduced pressure. The resulting residue was purified by silica gel column chromatography (gradient elution, eluent: dichloromethane/methanol=80/1 to 60/1) to obtain 8-methoxy-7-nitro-3-(piperidin-1-ylmethyl)quinoline (95f) (37.0 mg, yield 25%).

At room temperature, 8-methoxy-7-nitro-3-(piperidin-1-ylmethyl)quinoline (95f) (37.0 mg, 0.12 mmol) and lithium chloride (51.6 mg, 1.22 mmol) were added to N,N-dimethylformamide (2.0 mL). The reaction mixture was stirred at 150°C for 50 minutes, cooled to room temperature, and concentrated under reduced pressure to remove the organic solvent. Water (2.0 mL) was added to the resulting residue, stirred for 3 minutes, and filtered under reduced pressure. Water (2.0 mL) was added to the resulting filter cake, stirred for 10 minutes, and filtered under reduced pressure. The filter cake was dried under vacuum to obtain 7-nitro-3-(piperidin-1-ylmethyl)quinolin-8-ol (95) (21.3 mg, yield 60%).

¹H NMR (400 MHz, DMSO-*d₆*) δ: 8.53 (s, 1H), 7.97 (s, 1H), 7.90 (d, *J* = 9.4 Hz, 1H), 6.50 (d, *J =* 9.4 Hz, 1H), 3.60 (s, 2H), 2.38 (s, 4H), 1.59 - 1.48 (m, 4H), 1.42 (d, *J =* 4.4 Hz, 2H).

MS calculated: 287.13; MS found: 288.1 [M+H]⁺.

### Example 96

### Synthesis of 5-chloro-3-(morpholinomethyl)-7-nitroquinolin-8-ol (96)

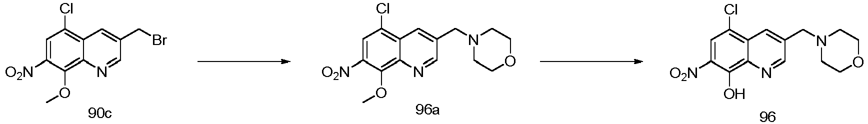

At room temperature, the crude 3-(bromomethyl)-5-chloro-8-methoxy-7-nitroquinoline (90c) (113.0 mg, 0.34 mmol) and morpholine (148.0 mg, 1.70 mmol) were added successively to tetrahydrofuran (2.5 mL). The reaction mixture was stirred at room temperature for 2 hours, and concentrated under reduced pressure. The resulting residue was purified by silica gel thin layer chromatography (developing solvent: dichloromethane/methanol=24/1) to obtain 5-chloro-8-methoxy-7-nitro-3-(morpholinmethyl)quinoline (96a) (52.0 mg, yield 45%).

At room temperature, 5-chloro-8-methoxy-7-nitro-3-(morpholinmethyl)quinoline (96a) (52.0 mg, 0.15 mmol) and lithium chloride (65.0 mg, 1.5 mmol) were added to N,N-dimethylformamide (1.5 mL). The reaction mixture was stirred at 130°C for 1 hour, cooled to room temperature, and concentrated under reduced pressure to remove the organic solvent. Water (1.0 mL) was added to the resulting residue, stirred for 3 minutes, and filtered under reduced pressure. The filter cake was washed with a mixed solvent of ethanol and water (volume ratio, ethanol/water = 1/1) (0.5 mL x 2), and dried under vacuum to obtain 5-chloro-3-(morpholinomethyl)-7-nitroquinolin-8-ol (96) (38.7 mg, yield 78%).

¹H NMR (400 MHz, DMSO-*d₆*) δ: 8.76 (s, 1H), 8.13 (s, 1H), 8.03 (s, 1H), 3.69 (s, 2H), 3.58 (br s, 4H), 2.40 (br s, 4H).

MS calculated: 323.07; MS found: 324.1 [M+H]⁺.

### Example 97

### Synthesis of 3-(morpholinomethyl)-7-nitroquinolin-8-ol (97)

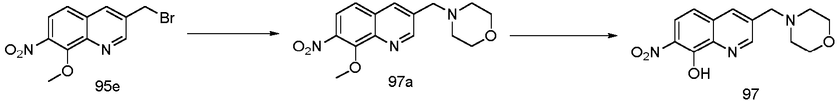

At room temperature, a solution of the crude 3-(bromomethyl)-8-methoxy-7-nitroquinoline (95e) (238.0 mg, 0.8 mmol) in dichloromethane (3.0 mL) was slowly added dropwise to a solution of morpholine (209.0 mg, 2.4 mmol) in dichloromethane (2.0 mL). The reaction mixture was stirred at room temperature for 2 hours, and diluted with dichloromethane (20.0 mL) and water (20.0 mL). The organic phase was separated, and the aqueous phase was extracted with dichloromethane (10.0 mL x 2). The combined organic phase was washed with saturated brine (10.0 mL), dried over anhydrous sodium sulfate, filtered, and concentrated under reduced pressure. The resulting residue was purified by silica gel column chromatography (eluent: dichloromethane/methanol = 61.5/1) to obtain 8-methoxy-7-nitro-3-(morpholinmethyl)quinoline (97a) (61.0 mg, yield 25%).

At room temperature, 8-methoxy-7-nitro-3-(morpholinmethyl)quinoline (97a) (61.0 mg, 0.2 mmol) and lithium chloride (84.8 mg, 2.0 mmol) were added to N,N-dimethylformamide (2.0 mL). The reaction mixture was stirred at 150°C for 1 hour, cooled to room temperature, and concentrated under reduced pressure to remove the organic solvent. Water (2.0 mL) was added to the resulting residue, stirred for 3 minutes, and filtered under reduced pressure. Water (2.0 mL) was added to the resulting filter cake, stirred for 10 minutes, and filtered under reduced pressure. The filter cake was dried under vacuum to obtain 3-(morpholinomethyl)-7-nitroquinolin-8-ol (97) (48.5 mg, yield 83%).

¹H NMR (400 MHz, DMSO-*d₆*) δ: 8.55 (s, 1H), 8.01 (s, 1H), 7.91 (d, *J =* 9.4 Hz, 1H), 6.51 (d, *J* = 9.4 Hz, 1H), 3.64 (s, 2H), 3.62 - 3.58 (m, 4H), 2.41 (s, 4H).

MS calculated: 289.11; MS found: 290.2 [M+H]⁺.

### Example 98

### Synthesis of 3-((diethylamino)methyl)-7-nitroquinolin-8-ol (98)

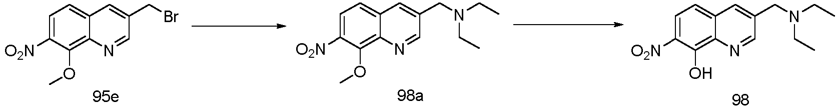

At room temperature, a solution of the crude 3-(bromomethyl)-8-methoxy-7-nitroquinoline (95e) (238.0 mg, 0.80 mmol) in dichloromethane (3.0 mL) was slowly added dropwise to a solution of diethylamine (176.8 mg, 2.4 mmol) in dichloromethane (2.0 mL). The reaction mixture was stirred at room temperature for 2 hours, and diluted with dichloromethane (20.0 mL) and water (20.0 mL). The organic phase was separated, and the aqueous phase was extracted with dichloromethane (10.0 mL x 2). The combined organic phase was washed with saturated brine (10.0 mL), dried over anhydrous sodium sulfate, filtered, and concentrated under reduced pressure. The resulting residue was purified by silica gel column chromatography (eluent: petroleum ether/ethyl acetate = 5.7/1) to obtain 8-methoxy-7-nitro-3-((diethylamino)methyl)quinoline (98a) (47.0 mg, yield 20%).

At room temperature, 8-methoxy-7-nitro-3-((diethylamino)methyl)quinoline (98a) (47.0 mg, 0.16 mmol) and lithium chloride (67.8 mg, 1.6 mmol) were added to N,N-dimethylformamide (2.0 mL). The reaction mixture was stirred at 150°C for 1 hour, cooled to room temperature, and concentrated under reduced pressure to remove the organic solvent. Water (2.0 mL) was added to the resulting residue, stirred for 3 minutes, and filtered under reduced pressure. Water (2.0 mL) was added to the resulting filter cake, stirred for 10 minutes, and filtered under reduced pressure. The filter cake was dried under vacuum to obtain 3-((diethylamino)methyl)-7-nitroquinolin-8-ol (98) (25.8 mg, yield 58%).

¹H NMR (400 MHz, DMSO-*d₆*) δ: 8.55 (s, 1H), 7.99 (s, 1H), 7.90 (d, *J =* 9.4 Hz, 1H), 6.50 (d, *J =* 9.4 Hz, 1H), 3.69 (s, 2H), 2.52 - 2.47 (m, 4H), 1.02 (t, *J =* 7.1 Hz, 6H).

MS calculated: 275.13; MS found: 276.2[M+H]⁺.

### Example 99

### Synthesis of 5-((methylamino)methyl)-7-nitroquinolin-8-ol dihydrochloride (99)

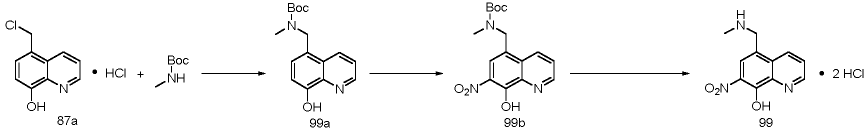

At 0°C, 5-(chloromethyl)quinolin-8-ol hydrochloride (87a) (135.0 mg, 0.59 mmol) and tert-butyl N-methylcarbamate (384.0 mg, 2.93 mmol) were added successively to acetonitrile (4.0 mL). The reaction mixture was stirred at 85°C for 2 hours, cooled to room temperature, and concentrated to dryness under reduced pressure to obtain the crude product *tert*-butyl N-((8-hydroxyquinolin-5-yl)methyl)-N-methylcarbamate (99a) (436.6 mg, crude yield >100%).

At room temperature, sodium nitrite (108.0 mg, 1.56 mmol) was added to a solution of the crude *tert*-butyl N-((8-hydroxyquinolin-5-yl)methyl)-N-methylcarbamate (99a) (436.6 mg, 0.59 mmol) in acetic acid (2.0 mL). The reaction mixture was stirred at room temperature for 2 hours, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/acetonitrile = 100%/0 - 30%/70%, gradient elution, and the flow rate was 20.0 mL/min) to obtain *tert*-butyl N-(8-hydroxy-7-nitroquinolin-5-yl)methyl-N-methylcarbamate (99b) (30.0 mg, yield 17%).

At room temperature, a solution of hydrogen chloride in 1,4-dioxane (4.0 M) (1.5 mL, 6.0 mmol) was added dropwise to a solution of *tert*-butyl N-(8-hydroxy-7-nitroquinolin-5-yl)methyl-N-methylcarbamate (99b) (30.0 mg, 0.090 mmol) in dichloromethane (1.0 mL). The reaction mixture was stirred at room temperature for 2 hours, and concentrated to dryness under reduced pressure to obtain 5-((methylamino)methyl)-7-nitroquinolin-8-ol dihydrochloride (99) (27.0 mg, yield 98%).

¹H NMR (400 MHz, DMSO-*d₆*) δ 9.22 (br s, 2H), 9.07 (d, *J =* 3.2 Hz, 1H), 8.96 (d, *J =* 8.4 Hz, 1H), 8.40 (s, 1H), 7.97 (dd, *J =* 8.8, 4.4 Hz, 1H), 4.59 (t, *J =* 4.8 Hz, 2H), 2.65 (t, *J =* 4.8 Hz, 3H).

MS calculated: 233.08; MS found: 234.1 [M+H]⁺.

### Example 100

### Synthesis of 4-((8-hydroxy-7-nitroquinolin-3-yl)methyl)piperazin-2-one (100)

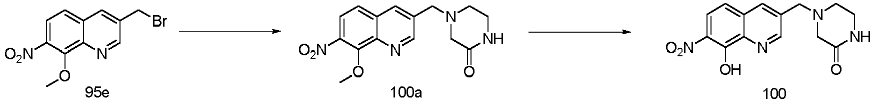

At room temperature, 2-piperazinone (275.3 mg, 2.8 mmol) was added to a mixed solvent of dichloromethane (3.0 mL) and N,N-dimethylformamide (2.0 mL). A solution of the crude 3-(bromomethyl)-8-methoxy-7-nitroquinoline (95e) (272.0 mg, 0.92 mmol) in dichloromethane (3.0 mL) was slowly added dropwise. The reaction mixture was stirred at room temperature for 2 hours, and diluted with dichloromethane (20.0 mL) and water (20.0 mL). The organic phase was separated, and the aqueous phase was extracted with dichloromethane (10.0 mL x 2). The combined organic phase was washed with saturated brine (10.0 mL), dried over anhydrous sodium sulfate, filtered, and concentrated under reduced pressure. The resulting residue was purified by silica gel column chromatography (eluent: dichloromethane/methanol=15/1) to obtain 4-((8-methoxy-7-nitroquinolin-3-yl)methyl)piperazin-2-one (100a) (75.0 mg, yield 26%).

At room temperature, 4-((8-methoxy-7-nitroquinolin-3-yl)methyl)piperazin-2-one (100a) (75.0 mg, 0.24 mmol) and lithium chloride (99.6 mg, 2.4 mmol) were added to N,N-dimethylformamide (2.0 mL). The reaction mixture was stirred at 150°C for 1 hour, cooled to room temperature, and concentrated under reduced pressure to remove the organic solvent. The resulting residue was dissolved in water (5.0 mL), and purified by reversed-phase high performance liquid chromatography (the chromatographic column was Eclipse XDB-C18 (21.2 mm×250 mm, 7µm), the mobile phase was water/acetonitrile = 100%/0 - 70%/30%, gradient elution, and the flow rate was 20.0 mL/min) to obtain 4-((8-hydroxy-7-nitroquinolin-3-yl)methyl)piperazin-2-one (100) (41.3 mg, yield 58%).

¹H NMR (400 MHz, DMSO-*d₆*) δ 8.57 (s, 1H), 8.05 (s, 1H), 7.92 (d, *J* = 9.4 Hz, 1H), 7.83 (s, 1H), 6.53 (d, *J =* 9.4 Hz, 1H), 3.73 (s, 2H), 3.18 (s, 2H), 3.00 (s, 2H), 2.60 (t, *J* = 5.2 Hz, 2H).

MS calculated: 302.10; MS found: 303.1[M+H]⁺.

### Example 101

### Synthesis of 7-nitro-5-(piperazin-1-ylmethyl)quinolin-8-ol trihydrochloride (101)

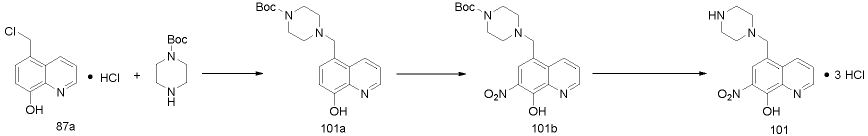

At 0°C, 5-(chloromethyl)quinolin-8-ol hydrochloride (87a) (69.0 mg, 0.3 mmol) and *tert-butyl* 1-piperazinecarboxylate (56.0 mg, 0.3 mmol) were added successively to acetonitrile (4.0 mL). The reaction mixture was stirred at 85°C for 2 hours, cooled to room temperature, and concentrated under reduced pressure. The resulting residue was purified by silica gel column chromatography (eluent: dichloromethane/methanol = 49/1, and adding 0.5% ammonia (25 to 28weight%, NH₃ content) by the total volume of the eluent) to obtain tert-butyl 4-((8-hydroxyquinolin-5-yl)methyl)piperazine-1-carboxylate (101a) (100.0 mg, yield 97%).

At room temperature, sodium nitrite (56.0 mg, 0.81 mmol) was added to a solution of *tert-butyl* 4-((8-hydroxyquinolin-5-yl)methyl)piperazine-1-carboxylate (101a) (100.0 mg, 0.27 mmol) in acetic acid (2.0 mL). The reaction mixture was stirred at room temperature for 2 hours, and filtered under reduced pressure. The filter cake was washed with ethanol (0.5 mL x 2), and dried under vacuum to obtain *tert*-butyl 4-((8-hydroxy-7-nitroquinolin-5-yl)methyl)piperazine-1-carboxylate (101b) (30.0 mg, yield 29%).

At room temperature, a solution of hydrogen chloride in 1,4-dioxane (4.0 M) (1.5 mL, 6.0 mmol) was added dropwise to a solution of *tert*-butyl 4-((8-hydroxy-7-nitroquinolin-5-yl)methyl)piperazine-1-carboxylate (101b) (30.0 mg, 0.077 mmol) in dichloromethane (0.5 mL). The reaction mixture was stirred at room temperature for 2 hours, and concentrated to dryness under reduced pressure to obtain 7-nitro-5-(piperazin-1-ylmethyl)quinolin-8-ol trihydrochloride (101) (24.0 mg, yield 78%).

¹H NMR (400 MHz, DMSO-*d₆*) δ: 10.09 (br s, 1H), 9.15 (s, 1H), 9.04 (d, *J =* 4.0 Hz, 1H), 8.49 (s, 1H), 7.95 (dd, *J =* 8.4, 4.2 Hz, 1H), 4.95 - 4.72 (m, 2H), 3.46 - 3.38 (m, 2H).

MS calculated: 288.12; MS found: 289.1 [M+H]⁺.

### Example 102

### Synthesis of 7-nitro-3-(piperazin-1-ylmethyl)quinolin-8-ol trihydrochloride (102)

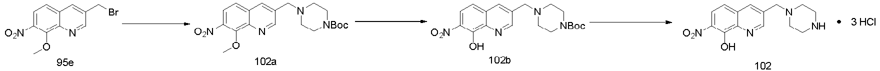

At room temperature, a solution of the crude 3-(bromomethyl)-8-methoxy-7-nitroquinoline (95e) (272.0 mg, 0.92 mmol) in dichloromethane (3.0 mL) was slowly added dropwise to a solution of tert-butyl 1-piperazinecarboxylate (853.0 mg, 4.6 mmol) in dichloromethane (3.0 mL). The reaction mixture was stirred at room temperature for 2 hours, and diluted with dichloromethane (20.0 mL) and water (20.0 mL). The organic phase was separated, and the aqueous phase was extracted with dichloromethane (10.0 mL x 2). The combined organic phase was washed with saturated brine (10.0 mL), dried over anhydrous sodium sulfate, filtered, and concentrated under reduced pressure. The resulting residue was purified by silica gel column chromatography (eluent: dichloromethane/methanol = 70/1) to obtain *tert*-butyl 4-((8-methoxy-7-nitroquinolin-3-yl)methyl)piperazine-1-carboxylate (102a) (83.0 mg, yield 23%).

At room temperature, *tert-butyl* 4-((8-methoxy-7-nitroquinolin-3-yl)methyl)piperazine-1-carboxylate (102a) (83.0 mg, 0.21 mmol) and lithium chloride (89.0 mg, 2.1 mmol) were added to N,N-dimethylformamide (2.0 mL). The reaction mixture was stirred at 150°C for 1 hour, cooled to room temperature, and concentrated under reduced pressure to remove the organic solvent. Water (2.0 mL) was added to the resulting residue, stirred for 3 minutes, and filtered under reduced pressure. Water (2.0 mL) was added to the resulting filter cake, stirred for 10 minutes, and filtered under reduced pressure. The filter cake was dried under vacuum to obtain *tert*-butyl 4-((8-hydroxy-7-nitroquinolin-3-yl)methyl)piperazine-1-carboxylate (102b) (80.0 mg, yield 99%).

At room temperature, *tert-butyl* 4-((8-hydroxy-7-nitroquinolin-3-yl)methyl)piperazine-1-carboxylate (102b) (80.0 mg, 0.21 mmol) was added to a solution of hydrogen chloride in 1,4-dioxane (4.0 M) (6.0 mL, 24.0 mmol). The reaction mixture was stirred at room temperature for 2 hours, and concentrated under reduced pressure to remove the organic solvent. Ethyl acetate (5.0 mL) was added to the resulting residue, stirred for 3 minutes, and filtered under reduced pressure. The filter cake was washed with ethyl acetate (0.5 mL x 2), and dried under vacuum to obtain 7-nitro-3-(piperazin-1-ylmethyl)quinolin-8-ol trihydrochloride (102) (62.7 mg, yield 77%).

¹H NMR (400 MHz, D₂O-*d₆*) δ: 10.03 (s, 2H), 9.31 (s, 1H), 8.76 (s, 1H), 8.07 (d, *J* = 9.2 Hz, 1H), 7.49 (d, *J =* 9.2 Hz, 1H), 4.66 (s, 2H), 3.45 (m, 8H).

MS calculated: 288.12; MS found: 289.1[M+H]⁺.

### Example 103

### Synthesis of 5-((ethylamino)methyl)-7-nitroquinolin-8-ol dihydrochloride (103)

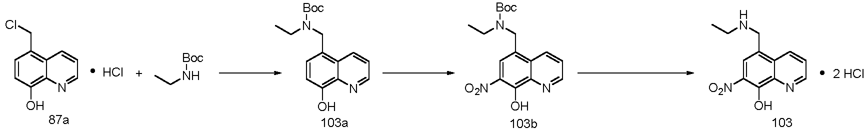

At 0°C, 5-(chloromethyl)quinolin-8-ol hydrochloride (87a) (115.0 mg, 0.5 mmol) and *tert*-butyl N-ethylcarbamate (290.0 mg, 2.0 mmol) were added successively to acetonitrile (4.0 mL). The reaction mixture was stirred at 85°C for 2 hours, cooled to room temperature, and filtered under reduced pressure. The filter cake was washed with acetonitrile (1.0 mL x 2), and the filtrate was concentrated to dryness under reduced pressure to obtain *tert*-butyl N-(8-hydroxyquinolin-5-yl)methyl-N-ethylcarbamate (103a) (94.0 mg, yield 60%).

At room temperature, sodium nitrite (37.0 mg, 0.54 mmol) was added to a solution of *tert*-butyl N-(8-hydroxyquinolin-5-yl)methyl-N-ethylcarbamate (103a) (54.0 mg, 0.18 mmol) in acetic acid (1.5 mL). The reaction mixture was stirred at room temperature for 2 hours, and filtered under reduced pressure. The resulting filtrate was purified by reversed-phase high performance liquid chromatography (the chromatographic column was Eclipse XDB-C18 (21.2 mm×250 mm, 7µm), the mobile phase was water/acetonitrile = 100%/0 - 30%/70%, gradient elution, and the flow rate was 20.0 mL/min) to obtain *tert-*butyl N-(8-hydroxy-7-nitroquinolin-5-yl)methyl-N-ethylcarbamate (103b) (28.0 mg, yield 45%).

At room temperature, a solution of hydrogen chloride in 1,4-dioxane (4.0 M) (1.5 mL, 6.0 mmol) was added dropwise to a solution of *tert*-butyl N-(8-hydroxy-7-nitroquinolin-5-yl)methyl-N-ethylcarbamate (103b) (28.0 mg, 0.081 mmol) in dichloromethane (1.0 mL). The reaction mixture was stirred at room temperature for 2 hours, and concentrated to dryness under reduced pressure to obtain 5-((ethylamino)methyl)-7-nitroquinolin-8-ol dihydrochloride (103) (26.0 mg, yield 88%).

¹H NMR (400 MHz, DMSO-*d₆*) δ: 9.30 (br s, 2H), 9.08 (d, *J =* 4.0 Hz, 1H), 8.97 (d, *J =* 8.6 Hz, 1H), 8.43 (s, 1H), 7.97 (dd, *J =* 8.4, 4.4 Hz, 1H), 4.59 (t, *J =* 5.6 Hz, 2H), 3.11 (dd, *J =* 12.4, 6.8 Hz, 2H), 1.29 (t, *J =* 7.2 Hz, 3H).

MS calculated: 247.25; MS found: 248.1 [M+H]⁺.

### Example 104

### Synthesis of 5-((dimethylamino)methyl)-7-nitro-4-(trifluoromethyl)quinolin-8-ol (104)

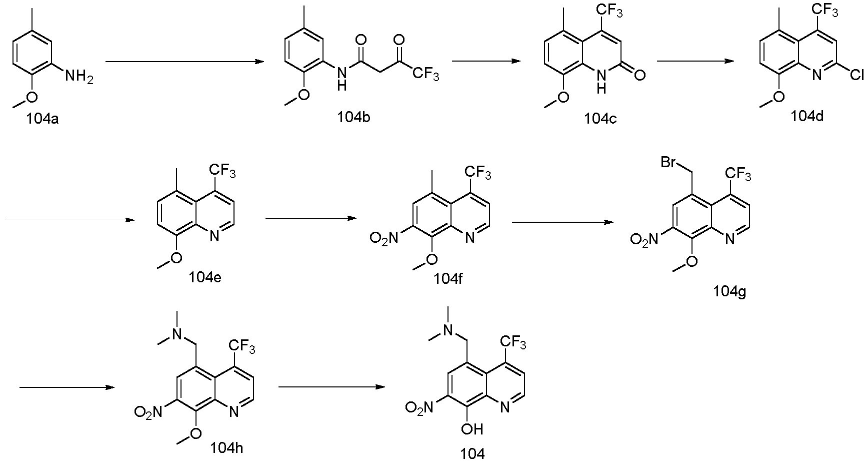

At room temperature, 2-methoxy-5-methylaniline (104a) (27.4 g, 199.73 mmol) was added to ethyl 4,4,4-trifluoroacetoacetate (75.0 g, 399.47 mmol). The reaction mixture was warmed up to 135°C, stirred for 1 hour, cooled to 0°C, followed by the addition of water (4.0 mL), and stirred for 30 minutes. The resulting mixture was filtered under reduced pressure, the filter cake was washed with petroleum ether (boiling range: 60°C to 90°C) (25.0 mL x 3), and dried under vacuum to obtain the crude product 4,4,4-trifluoro-N-(2-methoxy-5-methylphenyl)-3-oxobutanamide (104b) (28.0 g, crude yield 51%).

At room temperature, the crude 4,4,4-trifluoro-N-(2-methoxy-5-methylphenyl)-3-oxobutanamide (104b) (6.0 g, 21.80 mmol) was added to polyphosphoric acid (30.0 mL). The reaction mixture was warmed up to 150°C, stirred for 1 hour, cooled to about 60°C to 70°C, followed by the addition of ice water (100.0 mL), stirred vigorously for 30 minutes, and filtered under reduced pressure. The filter cake was washed with water (20.0 mL x 4), and dried under vacuum to obtain the crude product 8-methoxy-5-methyl-4-trifluoromethyl-2(*1H*)-quinolinone (104c) (5.0 g, crude yield 89%).

At room temperature, the crude 8-methoxy-5-methyl-4-trifluoromethyl-2(*1H*)-quinolinone (104c) (5.0 g, 19.4 mmol) was added to phosphorus oxychloride (20.0 mL). The reaction mixture was stirred at 100°C for 3 hours, cooled to room temperature, followed by the addition of ice water (100.0 mL), stirred vigorously for 30 minutes, and filtered under reduced pressure. The filter cake was washed with water (20.0 mL x 3), and dried under vacuum to obtain the crude product 2-chloro-8-methoxy-5-methyl-4-(trifluoromethyl)quinoline (104d) (4.5 g, crude yield 84%).

At room temperature, the crude 2-chloro-8-methoxy-5-methyl-4-(trifluoromethyl)quinoline (104d) (1.7 g, 6.17 mmol) and aqueous hydrazine hydrate solution (80 weight%) (2.2 g, 43.17 mmol) were added successively to ethanol (20.0 mL), followed by the addition of palladium/carbon (palladium loading: 10 weight%) (0.17 g, 0.16 mmol). The reaction mixture was stirred at 90°C for 5 hours, cooled to room temperature, and filtered through diatomite. The filter cake was washed with ethyl acetate (10.0 mL x 4), and the filtrate was concentrated under reduced pressure. Ethyl acetate (20.0 mL) and water (20.0 mL) were added to the resulting residue. The organic phase was separated, and the aqueous phase was extracted with ethyl acetate (20.0 mL x 2). The combined organic phase was washed with saturated brine (20.0 mL), dried over anhydrous sodium sulfate, filtered, and concentrated under reduced pressure. The resulting residue was purified by silica gel column chromatography (eluent: petroleum ether/ethyl acetate = 1/1) to obtain 8-methoxy-5-methyl-4-(trifluoromethyl)quinoline (104e) (1.0 g, yield 67%).

At 0°C, nitric acid (65 weight%) (2.6 mL, 37.3 mmol) was slowly added dropwise to a solution of 8-methoxy-5-methyl-4-(trifluoromethyl)quinoline (104e) (1.5 g, 6.22 mmol) in acetic anhydride (15.0 mL), and stirred for 10 minutes. Sulfuric acid (98 weight%) (0.41 mL, 7.50 mmol) was slowly added dropwise. The reaction mixture was stirred at 0°C for 10 minutes, and the pH of the aqueous phase was adjusted to about 9 to 10 with aqueous sodium hydroxide solution (1.0 M). The resulting mixture was extracted with ethyl acetate (50.0 mL x 2). The organic phases were combined, dried over anhydrous sodium sulfate, filtered, and concentrated under reduced pressure. The resulting residue was purified by silica gel column chromatography (eluent: petroleum ether/ethyl acetate = 20/1) to obtain 8-methoxy-5-methyl-7-nitro-4-(trifluoromethyl)quinoline (104f) (0.15 g, yield 8%).

At room temperature, N-bromosuccinimide (87.3 mg, 0.44 mmol) and 2,2'-azobisisobutyronitrile (120.4 mg, 0.74 mmol) were added successively to a solution of 8-methoxy-5-methyl-7-nitro-4-(trifluoromethyl)quinoline (104f) (105.0 mg, 0.37 mmol) in 1,2-dichloroethane (4.0 mL). The reaction mixture was warmed up to 90°C, stirred for 16 hours, cooled to room temperature, and concentrated under reduced pressure. The resulting residue was purified by silica gel column chromatography (eluent: petroleum ether/ethyl acetate = 3/1) to obtain 5-(bromomethyl)-8-methoxy-7-nitro-4-(trifluoromethyl)quinoline (104g) (100.0 mg, yield 74%).

At room temperature, dimethylamine hydrochloride (44.6 mg, 0.55 mmol) and triethylamine (110.9 mg, 1.10 mmol) were added successively to a solution of 5-(bromomethyl)-8-methoxy-7-nitro-4-(trifluoromethyl)quinoline (104g) (100.0 mg, 0.27 mmol) in dichloromethane (2.0 mL). The reaction mixture was stirred at room temperature for 2 hours, and concentrated under reduced pressure. The resulting residue was purified by silica gel column chromatography (eluent: petroleum ether/ethyl acetate = 3/1) to obtain 5-((dimethylamino)methyl)-8-methoxy-7-nitro-4-(trifluoromethyl)quinoline (104h) (70.0 mg, yield 79%).

At room temperature, 5-((dimethylamino)methyl)-8-methoxy-7-nitro-4-(trifluoromethyl)quinoline (104h) (70.0 mg, 0.21 mmol) and lithium chloride (26.5 mg, 0.63 mmol) were added to N,N-dimethylformamide (2.0 mL). The reaction mixture was stirred at 150°C for 1 hour, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/acetonitrile = 100%/0 - 70%/30%, gradient elution, and the flow rate was 20.0 mL/min) to obtain 5-((dimethylamino)methyl)-7-nitro-4-(trifluoromethyl)quinolin-8-ol (108) (15.1 mg, yield 23%).

¹H NMR (400 MHz, MeOH-*d₄*) δ 9.18 (s, 1H), 8.55 (s, 1H), 8.26 (s, 1H), 4.77 (s, 2H), 2.82 (s, 6H).

MS calculated: 315.08; MS found: 316.1 [M+H]⁺.

### Example 105

### Synthesis of 7-nitro-4-(piperidin-4-yl)quinolin-8-ol (105)

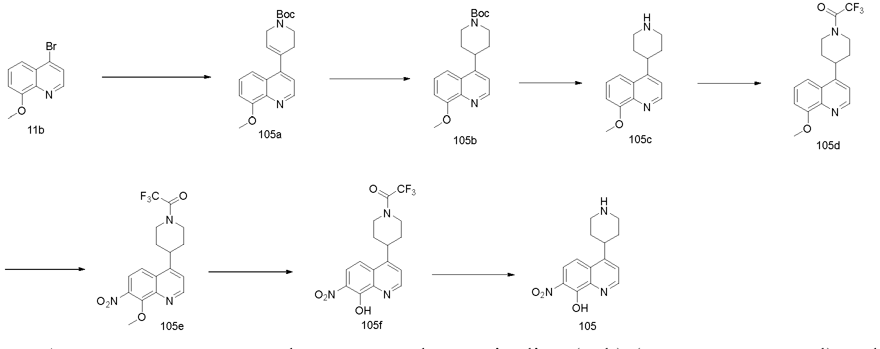

At room temperature, 4-bromo-8-methoxyquinoline (11b) (3.0 g, 12.68 mmol) and N-tert-butoxycarbonyl-1,2,5,6-tetrahydropyridine-4-boronic acid pinacol ester (4.7 g, 15.22 mmol) were added to a mixture of 1,4-dioxane (60.0 mL) and water (15.0 mL). Sodium carbonate (4.03 g, 38.04 mmol) and [1,1'-bis(diphenylphosphino)ferrocene]dichloropalladium(II) (1.03 g, 1.27 mmol) were added successively. The resulting reaction mixture was stirred at 80°C for 16 hours, cooled to room temperature, followed by the addition of ethyl acetate (100.0 mL) and water (100.0 mL). The organic phase was separated, and the aqueous phase was extracted with ethyl acetate (50.0 mL). The organic phases were combined, washed with saturated brine (100.0 mL), dried over anhydrous sodium sulfate, filtered, and concentrated under reduced pressure. The resulting residue was purified by silica gel column chromatography (eluent: petroleum ether/ethyl acetate = 3/1) to obtain tert-butyl 4-(8-methoxyquinolin-4-yl)-3,6-dihydropyridine-1(2H)-carboxylate (105a) (4.0 g, yield 93%).

At room temperature, tert-butyl 4-(8-methoxyquinolin-4-yl)-3,6-dihydropyridine-1(2H)-carboxylate (105a) (4.0 g, 11.76 mmol) and palladium/carbon (palladium loading: 10 weight%) (0.40 g, 0.376 mmol) were added successively to anhydrous methanol (40.0 mL). The resulting reaction mixture was stirred under a hydrogen atmosphere for 16 hours, and filtered through diatomite. The filter cake was washed with ethyl acetate (10.0 mL x 3), and the filtrate was concentrated under reduced pressure. The resulting residue was purified by silica gel column chromatography (eluent: petroleum ether/ethyl acetate = 4/1) to obtain tert-butyl 4-(8-methoxyquinolin-4-yl)piperidine-1-carboxylate (105b) (3.2 g, yield 80%).

At room temperature, *tert-butyl* 4-(8-methoxyquinolin-4-yl)piperidine-1-carboxylate (105b) (1.5 g, 4.37 mmol) was added to a solution of hydrogen chloride in ethyl acetate (3.0 M) (20.0 mL). The resulting reaction mixture was stirred at room temperature for 1 hour, and concentrated to dryness under reduced pressure to obtain the crude product 8-methoxy-4-(piperidin-4-yl)quinoline (105c) (1.1 g, crude yield 90%).

At room temperature, triethylamine (0.94 g, 9.26 mmol) and trifluoroacetic anhydride (0.778 g, 3.71 mmol) were added successively to a solution of 8-methoxy-4-(piperidin-4-yl)quinoline (105c) (0.86 g, 3.09 mmol) in dichloromethane (15.0 mL). The resulting reaction mixture was stirred at room temperature for 2 hours, followed by the addition of ethyl acetate (30.0 mL) and water (30.0 mL). The organic phase was separated, and the aqueous phase was extracted with ethyl acetate (20.0 mL). The organic phases were combined, washed with saturated brine (50.0 mL), dried over anhydrous sodium sulfate, filtered, and concentrated under reduced pressure. The resulting residue was purified by silica gel column chromatography (eluent: petroleum ether/ethyl acetate = 1/1) to obtain N-trifluoroacetyl-8-methoxy-4-(piperidin-4-yl)quinoline (105d) (0.67 g, yield 64%).

At 0°C, nitric acid (65 weight%) (0.79 mL, 11.89 mmol) was slowly added dropwise to a solution of N-trifluoroacetyl-8-methoxy-4-(piperidin-4-yl)quinoline (105d) (0.67 g, 1.98 mmol) in acetic anhydride (6.0 mL), and stirred for 10 minutes. Sulfuric acid (98 weight%) (0.13 mL, 2.38 mmol) was slowly added dropwise. The reaction mixture was stirred at 0°C for 10 minutes, and the pH of the aqueous phase was adjusted to about 9 to 10 with aqueous sodium hydroxide solution (1.0 M). The resulting mixture was extracted with ethyl acetate (50.0 mL x 2). The organic phases were combined, washed with saturated brine (30.0 mL), dried over anhydrous sodium sulfate, filtered, and concentrated under reduced pressure. The resulting residue was purified by silica gel column chromatography (eluent: petroleum ether/ethyl acetate = 2/1) to obtain N-trifluoroacetyl-7-nitro-8-methoxy-4-(piperidin-4-yl)quinoline (105e) (0.15 g, yield 20%).

At room temperature, N-trifluoroacetyl-7-nitro-8-methoxy-4-(piperidin-4-yl)quinoline (105e) (0.15 g, 0.39 mmol) and lithium chloride (0.050 g, 2.38 mmol) were added to N,N-dimethylformamide (10.0 mL). The reaction mixture was stirred at 150°C for 1 hour, cooled to room temperature, and concentrated under reduced pressure to remove the organic solvent to obtain the crude product N-trifluoroacetyl-7-nitro-4-(piperidin-4-yl)quinolin-8-ol (105f) (0.20 g, crude yield >100%).

At room temperature, the crude N-trifluoroacetyl-7-nitro-4-(piperidin-4-yl)quinolin-8-ol (105f) (0.20 g, 0.39 mmol (theoretical amount)) was added to a mixture of methanol (8.0 mL) and water (2.0 mL), followed by the addition of potassium hydroxide (0.11 g, 1.95 mmol). The resulting reaction mixture was stirred at room temperature for 2 hours, and concentrated to dryness under reduced pressure. The resulting residue was purified by reversed-phase high performance liquid chromatography (the chromatographic column was Eclipse XDB-C18 (21.2 mm×250 mm, 7µm), the mobile phase was water/acetonitrile=100%/0 - 30%/70%, gradient elution, and the flow rate was 20.0 mL/min) to obtain 7-nitro-4-(piperidin-4-yl)-quinolin-8-ol (105) (0.042 g, yield 29%).

¹H NMR (400 MHz, MeOH-*d₄*) δ 8.91 (d, *J =* 4.6 Hz, 1H), 8.18 (d, *J =* 9.6 Hz, 1H), 7.73 (d, *J =* 9.6 Hz, 1H), 7.65 (d, *J =* 4.6 Hz, 1H), 3.80 (s, 1H), 3.57 (d, *J =* 13.1 Hz, 2H), 3.33 (d, *J =* 13.2 Hz, 2H), 2.22 (d, *J =* 13.3 Hz, 2H), 2.07 - 1.96 (m, 3H).

MS calculated: 273.11; MS found: 274.35 [M+H]⁺.

### Example 106

### Synthesis of 5-fluoro-7-nitroquinolin-8-ol (106)

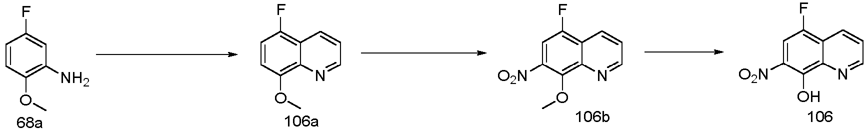

At room temperature, 5-fluoro-2-methoxyaniline (68a) (1.0 g, 7.08 mmol) was added to hydrochloric acid (6.0 M) (10.0 mL). The reaction mixture was warmed up to 110°C, followed by the slowly dropwise addition of acrolein diethyl acetal (2.31 g, 17.7 mmol), and stirred for 2 hours. The reaction mixture was cooled to room temperature, and the pH of the aqueous phase was adjusted to about 7 to 8 with saturated aqueous potassium carbonate solution. The resulting mixture was extracted with ethyl acetate (50.0 mL x 3). The organic phases were combined, dried over anhydrous sodium sulfate, filtered, and concentrated under reduced pressure. The resulting residue was purified by silica gel column chromatography (gradient elution, eluent: petroleum ether/ethyl acetate = 7/1 to 4/1) to obtain 5-fluoro-8-methoxyquinoline (106a) (0.20 g, yield 16%).

At 0°C, nitric acid (65 weight%) (0.23 mL, 3.56 mmol) was slowly added dropwise to a solution of 5-fluoro-8-methoxyquinoline (106a) (0.20 g, 1.13 mmol) in acetic anhydride (4.0 mL), and stirred for 10 minutes. Sulfuric acid (98 weight%) (0.061 mL, 1.13 mmol) was slowly added dropwise. The reaction mixture was stirred at 0°C for 2 hours, and the pH of the aqueous phase was adjusted to about 9 to 10 with aqueous sodium hydroxide solution (1.0 M). The resulting mixture was extracted with ethyl acetate (10.0 mL x 2). The organic phases were combined, dried over anhydrous sodium sulfate, filtered, and concentrated under reduced pressure. The resulting residue was purified by silica gel column chromatography (eluent: petroleum ether/ethyl acetate = 8/1) to obtain 5-fluoro-8-methoxy-7-nitroquinoline (106b) (0.165 g, yield 66%).

At room temperature, 5-fluoro-8-methoxy-7-nitroquinoline (106b) (65.0 mg, 0.29 mmol) and lithium chloride (124.0 mg, 2.90 mmol) were added to N,N-dimethylformamide (2.0 mL). The reaction mixture was stirred at 130°C for 1 hour, cooled to room temperature, and concentrated under reduced pressure to remove the organic solvent. Water (2.0 mL) was added to the resulting residue, stirred for 3 minutes, and filtered under reduced pressure. The resulting filter cake was washed with a mixed solvent of ethanol and water (volume ratio, ethanol/water = 1/1) (1.0 mL x 2), and dried under vacuum to obtain 5-fluoro-7-nitroquinolin-8-ol (106) (56.0 mg, yield 92%).

¹H NMR (400 MHz, DMSO-*d₆*) δ 8.76 (s, 1H), 8.21 (dd, *J =* 8.1, 0.9 Hz, 1H), 7.68 (d, *J =* 12.8 Hz, 1H), 7.65 (dd, *J =* 8.4, 4.5 Hz, 1H).

MS calculated: 208.03; MS found: 209.15 [M+H]⁺.

### Example 107

### Synthesis of 5-chloro-6-methyl-7-nitroquinolin-8-ol (107)

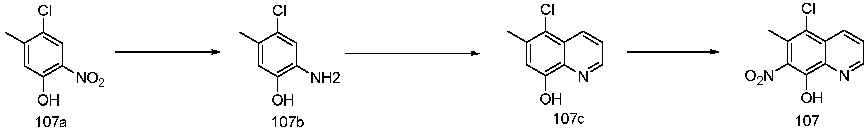

At room temperature, 4-chloro-5-methyl-2-nitrophenol (107a) (1.0 g, 5.35 mmol), iron powder (1.5 g, 26.74 mmol) and ammonium chloride (2.8 g, 53.78 mmol) were added successively to a mixture of ethanol (15.0 mL) and water (8.0 mL). The reaction mixture was stirred at 95°C for 2 hours, cooled to room temperature, and filtered through diatomite. The filter cake was washed with ethyl acetate (10.0 mL x 4), and the filtrates were combined. The organic phase was separated, and the aqueous phase was extracted with ethyl acetate (5.0 mL x 3). The organic phases were combined, dried over anhydrous sodium sulfate, filtered, and concentrated under reduced pressure to obtain the crude product 2-amino-4-chloro-5-methylphenol (107b) (0.57 g, crude yield 68%).

At room temperature, the crude 2-amino-4-chloro-5-methylphenol (107b) (0.435 g, 2.77 mmol) was added to hydrochloric acid (6.0 M) (10.0 mL). The reaction mixture was warmed up to 110°C, followed by the slowly dropwise addition of acrolein diethyl acetal (1.2 mL, 8.31 mmol), and stirred for 5 hours. The reaction mixture was cooled to room temperature, and the pH of the aqueous phase was adjusted to about 7 to 8 with saturated aqueous potassium carbonate solution. The resulting mixture was extracted with ethyl acetate (50.0 mL x 3). The organic phases were combined, dried over anhydrous sodium sulfate, filtered, and concentrated under reduced pressure. The resulting residue was purified by silica gel column chromatography (eluent: petroleum ether/ethyl acetate = 3/1) to obtain 5-chloro-6-methylquinolin-8-ol (107c) (0.41 g, yield 77%).

At room temperature, sodium nitrite (128.6 mg, 1.86 mmol) was added to a solution of 5-chloro-6-methylquinolin-8-ol (107c) (60.0 mg, 0.31 mmol) in acetic acid (3.0 mL). The resulting reaction mixture was stirred at room temperature for 3 hours, and filtered under reduced pressure. The filter cake was washed with a mixed solvent of ethanol and water (volume ratio, ethanol/water = 1/1) (1.0 mL x 2), and dried under vacuum to obtain 5-chloro-6-methyl-7-nitroquinolin-8-ol (107) (73.0 mg, yield 99%).

¹H-NMR (400 MHz, DMSO-*d₆*) δ: 10.22-10.00 (bs, 1 H), 8.93-8.86 (m, 1 H), 8.56-8.49 (m, 1 H), 7.75-7.67 (m, 1 H), 7.13 (s, 1 H), 2.56-2.51 (m, 3 H).

MS calculated: 238.63; MS found: 239.1 [M+H]⁺.

### Example 108

### Synthesis of 5-(difluoromethyl)-7-nitroquinolin-8-ol trifluoroacetate (108)

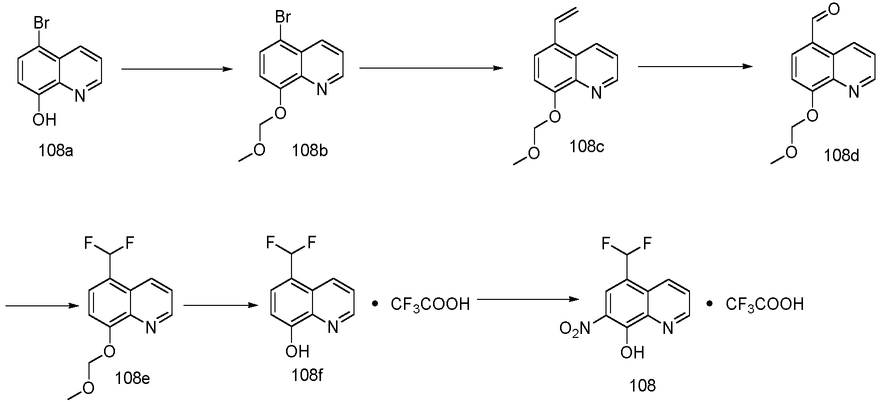

At 0°C, bromomethyl methyl ether (1.0 mL, 12.25 mmol) was slowly added dropwise to a solution of 5-bromo-8-hydroxyquinoline (108a) (2.04 g, 9.10 mmol) and diisopropylethylamine (4.4 mL, 24.90 mmol) in dichloromethane (40.0 mL). The resulting mixture was warmed up to room temperature, stirred for 5 hours, followed by the addition of water (20.0 mL). The organic phase was separated, and the aqueous phase was extracted with dichloromethane (20.0 mL x 3). The organic phases were combined, dried over anhydrous sodium sulfate, filtered, and concentrated under reduced pressure. The resulting residue was purified by silica gel column chromatography (gradient elution, eluent: petroleum ether/ethyl acetate = 100%/0 to 2/1) to obtain 5-bromo-8-(methoxymethoxy)quinoline (108b) (2.33 g, yield 96%).

At room temperature, 5-bromo-8-(methoxymethoxy)quinoline (108b) (616.0 mg, 2.30 mmol), potassium vinyltrifluoroborate (493.0 mg, 3.70 mmol), [1,1'-bis(diphenylphosphino)ferrocene]dichloropalladium(II) (168.0 mg, 0.23 mmol) and potassium carbonate (952.0 mg, 6.90 mmol) were added successively to a mixture of 1,4-dioxane (10.0 mL) and water (2.5 mL). The resulting mixture was stirred at 90°C for 18 hours, cooled to room temperature, and concentrated to dryness under reduced pressure. Dichloromethane (10.0 mL) and water (10.0 mL) were added to the resulting residue. The organic phase was separated, and the aqueous phase was extracted with dichloromethane (10.0 mL x 2). The organic phases were combined, dried over anhydrous sodium sulfate, filtered, and concentrated under reduced pressure. The resulting residue was purified by silica gel column chromatography (gradient elution, eluent: petroleum ether/ethyl acetate = 100%/0 to 4/1) to obtain 5-vinyl-8-(methoxymethoxy)quinoline (108c) (441.0 mg, yield 89%).

At 0°C, potassium osmate dihydrate (75.0 mg, 0.20 mmol) was added to a solution of 5-vinyl-8-(methoxymethoxy)quinoline (108c) (441.0 mg, 2.05 mmol) in tetrahydrofuran (36 mL), followed by the addition of water (9.0 mL). The reaction mixture was stirred at 0°C for 10 minutes, followed by the addition of sodium periodate (1.32 g, 6.15 mmol), and then stirred at room temperature for 16 hours. Ethyl acetate (15.0 mL) and water (10.0 mL) were added, the organic phase was separated, and the aqueous phase was extracted with ethyl acetate (10.0 mL x 3). The organic phases were combined, dried over anhydrous sodium sulfate, filtered, and concentrated to dryness under reduced pressure to obtain the crude product 8-(methoxymethoxy)quinoline-5-carbaldehyde (108d) (475.0 mg, crude yield 100%).

At 0°C, diethylaminosulfur trifluoride (610.0 mg, 3.77 mmol) was slowly added dropwise to a solution of 8-(methoxymethoxy)quinoline-5-carbaldehyde (108d) (230.0 mg, 1.06 mmol) in dichloromethane (10.0 mL). The reaction mixture was stirred at 0°C for 10 minutes, at room temperature for 16 hours, and then cooled to 0°C. The pH of the aqueous phase was adjusted to about 7 to 8 by the slowly dropwise addition of saturated aqueous sodium bicarbonate solution. The resulting mixture was extracted with dichloromethane (10.0 mL x 3). The organic phases were combined, dried over anhydrous sodium sulfate, filtered, and concentrated under reduced pressure. The resulting residue was purified by silica gel column chromatography (gradient elution, eluent: petroleum ether/ethyl acetate = 100%/0 to 1/1) to obtain 5-(difluoromethyl)-8-(methoxymethoxy)quinoline (108e) (132.0 mg, yield 52%).

At room temperature, 5-(difluoromethyl)-8-(methoxymethoxy)quinoline (108e) (55.0 mg, 0.23 mmol) was added to trifluoroacetic acid (3.0 mL). The resulting reaction solution was stirred at room temperature for 3 hours, and concentrated to dryness under reduced pressure to obtain the crude product 5-(difluoromethyl)-8-hydroxyquinoline trifluoroacetate (108f) (45.0 mg, crude yield >100%).

At room temperature, sodium nitrite (49.0 mg, 0.71 mmol) was added to a solution of 5-(difluoromethyl)-8-hydroxyquinoline trifluoroacetate (108f) (45.0 mg, 0.23 mmol) in acetic acid (3.0 mL). The reaction mixture was stirred at room temperature for 3 hours, and filtered under reduced pressure. The filter cake was washed with a mixed solvent of ethanol and water (volume ratio, ethanol/water = 1/1) (1.0 mL x 2), and dried under vacuum to obtain 5-(difluoromethyl)-7-nitroquinolin-8-ol trifluoroacetate (108) (75.6 mg, the total yield of the above two steps was 93%).

¹H NMR (400 MHz, DMSO-*d₆*) δ 9.07 (d, *J =* 4.6 Hz, 1H), 8.82 (t, *J =* 8.6 Hz, 1H), 8.45 (s, 1H), 8.03 (dd, *J =* 8.6, 4.6 Hz, 1H), 7.48 (t, *J =* 54.2 Hz, 1H).

MS calculated: 240.03; MS found: 241.1 [M+H]⁺.

### Example 109

### Synthesis of 5-(hydroxymethyl)-7-nitroquinolin-8-ol (109)

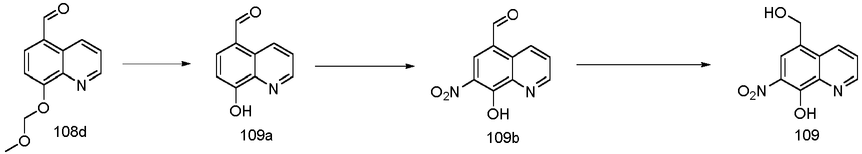

At room temperature, 8-(methoxymethoxy)quinoline-5-carbaldehyde (108d) (760.0 mg, 3.5 mmol) was added to trifluoroacetic acid (20.0 mL). The resulting reaction solution was stirred at room temperature for 3 hours, and concentrated to dryness under reduced pressure to obtain the crude product 8-hydroxyquinoline-5-carbaldehyde (109a) (606.0 mg, crude yield 100%).

At room temperature, the crude 8-hydroxyquinoline-5-carbaldehyde (109a) (606.0 mg, 3.5 mmol) was added to nitric acid (9 weight%) (10.0 mL). The reaction mixture was stirred at 90°C for 1 hour, cooled to room temperature, and filtered under reduced pressure. The filter cake was washed with water (3.0 mL x 3), and dried under vacuum to obtain 8-hydroxy-7-nitroquinoline-5-carbaldehyde (109b) (459.0 mg, the total yield of the above two steps was 60%).

At room temperature, 8-hydroxy-7-nitroquinoline-5-carbaldehyde (109b) (178.0 mg, 0.80 mmol) was added to a mixture of tetrahydrofuran (3.0 mL) and methanol (1.0 mL). The resulting reaction solution was cooled to 0°C, followed by the addition of sodium borohydride (34.0 mg, 0.90 mmol). The reaction mixture was stirred at 0°C for 1.5 hours, and concentrated to dryness under reduced pressure. The resulting residue was purified by reversed-phase high performance liquid chromatography (the chromatographic column was Eclipse XDB-C18 (21.2 mm×250 mm, 7µm), the mobile phase was water/acetonitrile=100%/0 - 99%/1%, gradient elution, and the flow rate was 20.0 mL/min) to obtain 5-(hydroxymethyl)-7-nitroquinolin-8-ol (109) (30.6 mg, yield 17%).

¹H NMR (400 MHz, DMSO-*d₆*) δ 8.62 (d, *J =* 4.2 Hz, 1H), 8.27 (d, *J =* 8.1 Hz, 1H), 7.92 (s, 1H), 7.54 (dd, *J =* 8.1, 4.2 Hz, 1H), 5.00 (s, 1H), 4.64 (s, 2H).

MS calculated: 220.05; MS found: 221.1 [M+H]⁺.

### Example 110

### Synthesis of 5-chloro-6-fluoro-7-nitroquinolin-8-ol (110)

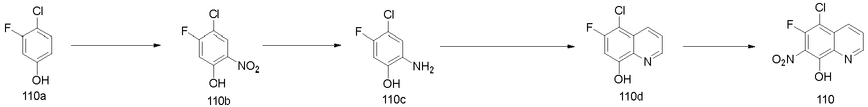

At room temperature, 3-fluoro-4-chlorophenol (110a) (1.5 g, 10.20 mmol) and tetrabutylammonium bromide (0.33 g, 0.102 mmol) were added successively to 1,2-dichloroethane (15.0 mL), followed by the slow addition of nitric acid (7 weight%) (18.0 mL). The reaction mixture was stirred at room temperature for 4 hours, and diluted with water (20.0 mL). The resulting mixture was extracted with dichloromethane (20.0 mL x 3). The organic phases were combined, dried over anhydrous sodium sulfate, filtered, and concentrated under reduced pressure. The resulting residue was purified by silica gel column chromatography (gradient elution, eluent: petroleum ether/ethyl acetate = 100%/0 to 10/1) to obtain 4-chloro-5-fluoro-2-nitrophenol (110b) (1.45 g, yield 74%).

At room temperature, 4-chloro-5-fluoro-2-nitrophenol (110b) (1.45 g, 7.57 mmol) was added to a mixture of acetic acid (14.5 mL) and water (4.5 mL), followed by the addition of reduced iron powder (2.11 g, 37.85 mmol). The resulting mixture was stirred at 100°C for 0.5 hour, cooled to room temperature, and filtered through diatomite, and the filter cake was washed with dichloromethane (10.0 mL x 4). The filtrates were combined, washed with saturated aqueous sodium bicarbonate solution (30.0 mL x 4) and saturated brine (30.0 mL) successively, dried over anhydrous sodium sulfate, filtered, and concentrated under reduced pressure. The resulting residue was purified by silica gel column chromatography (gradient elution, eluent: petroleum ether/ethyl acetate = 7/1 to 5/1) to obtain 2-amino-4-chloro-5-fluorophenol (110c) (0.247 g, yield 20%).

At room temperature, 2-amino-4-chloro-5-fluorophenol (110c) (0.247 g, 1.53 mmol), sodium 3-nitrobenzene sulfonate (0.413 g, 1.84 mmol) and glycerol (0.352 g, 3.83 mmol) were added successively to sulfuric acid (70 weight%) (2.0 mL). The resulting mixture was stirred at 140°C for 3 hours, and cooled to room temperature. The pH of the aqueous phase was adjusted to about 8 to 9 by the slowly dropwise addition of saturated aqueous sodium carbonate solution. The resulting mixture was extracted with dichloromethane (20.0 mL x 3). The organic phases were combined, dried over anhydrous sodium sulfate, filtered, and concentrated under reduced pressure. The resulting residue was purified by silica gel column chromatography (eluent: petroleum ether/ethyl acetate = 4/1) to obtain 5-chloro-6-fluoroquinolin-8-ol (110d) (0.12 g, yield 40%).

At room temperature, sodium nitrite (175.0 mg, 2.5 mmol) was added in batches to a suspension of 5-chloro-6-fluoroquinolin-8-ol (110d) (50.0 mg, 0.25 mmol) in hydrochloric acid (3.0 M) (1.67 mL). The reaction mixture was stirred at room temperature for 16 hours, and the pH of the aqueous phase was adjusted to about 8 to 9 by the slowly dropwise addition of saturated aqueous sodium carbonate solution. The resulting mixture was filtered under reduced pressure. The filter cake was washed with water (1.0 mL x 2) and a mixed solvent of ethanol and water (volume ratio, ethanol/water = 1/1) (0.5 mL x 2) successively, and dried under vacuum to obtain 5-chloro-6-fluoro-7-nitroquinolin-8-ol (110) (31.0 mg, yield 50%).

¹H NMR (400 MHz, DMSO-*d₆*) δ 9.06 (d, *J =* 3.7 Hz, 1H), 8.66 (d, *J =* 8.5 Hz, 1H), 7.96 (dd, *J* = 8.5, 4.2 Hz, 1H).

MS calculated: 241.99; MS found: 243.0, 245.0 [M+H]⁺.

### Example 111

### Synthesis of 5-chloro-7-nitro-4-(3-phenylpiperidin-1-yl)quinolin-8-ol (111)

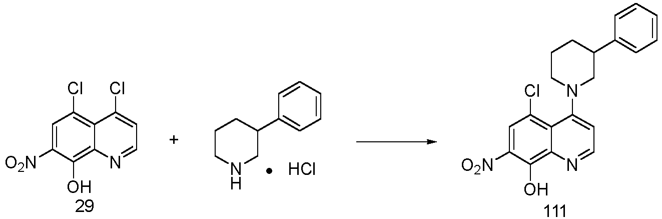

At room temperature, 4,5-dichloro-7-nitroquinolin-8-ol (29) (200.0 mg, 0.77 mmol) and 3-phenylpiperidine hydrochloride (161.0 mg, 1.0 mmol) were added to N,N-dimethylformamide (4.0 mL). The resulting mixture was stirred at 130°C for 5 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/methanol=100%/0 - 33%/67%, gradient elution, and the flow rate was 20.0 mL/min) to obtain 5-chloro-7-nitro-4-(3-phenylpiperidin-1-yl)quinolin-8-ol (111) (26.4 mg, yield 9%).

¹H NMR (400 MHz, DMSO-*d₆*) δ: 8.10-8.04 (m, 1H), 7.96-7.92 (m, 1H), 7.82-7.78 (m, 1H), 7.40-7.25 (m, 5H), 3.68-3.30 (m, 2 H), 3.08-2.92 (m, 1 H), 2.06-1.56 (m, 6 H).

MS calculated: 383.10; MS found: 384.1, 386.1 [M+H]⁺.

### Example 112

### Synthesis of 3-cyclopropyl-7-nitroquinolin-8-ol (112)

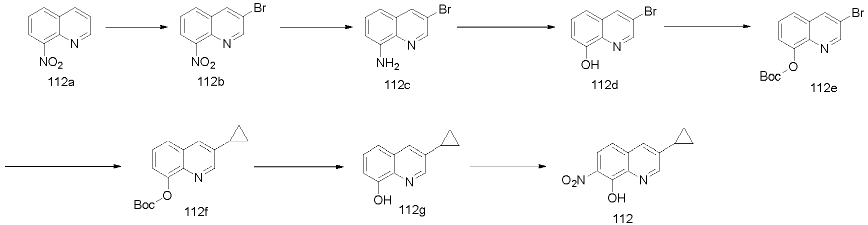

At 100°C, N-bromosuccinimide (5.34 g, 30.0 mmol) was added in three batches to a solution of 8-nitroquinoline (112a) (5.23 g, 30.0 mmol) in acetic acid (30.0 mL). The reaction mixture was stirred for 2 hours, cooled to room temperature, and concentrated under reduced pressure to remove the organic solvent. Dichloromethane (50.0 mL) was added to the resulting residue, and the pH of the aqueous phase was adjusted to about 7 to 8 with saturated aqueous sodium carbonate solution. The organic phase was separated, and the aqueous phase was extracted with dichloromethane (30.0 mL x 2). The organic phases were combined, dried over anhydrous sodium sulfate, filtered, and concentrated under reduced pressure. The resulting residue was purified by silica gel column chromatography (gradient elution, eluent: petroleum ether/ethyl acetate = 3/1 to 1.5/1) to obtain 3-bromo-8-nitroquinoline (112b) (4.61 g, yield 61%).

At room temperature, 3-bromo-8-nitroquinoline (112b) (4.23 g, 16.7 mmol), reduced iron powder (6.99 g, 125.3 mmol) and ammonium chloride (1.79 g, 33.4 mmol) were added successively to a mixture of ethanol (33.4 mL) and water (16.7 mL). The resulting reaction mixture was stirred at 100°C for 4 hours, cooled to room temperature, and filtered through diatomite. The filter cake was washed with ethyl acetate (10.0 mL x 4), and the filtrates were combined. The organic phase was separated, and the aqueous phase was extracted with ethyl acetate (20.0 mL x 3). The organic phases were combined, dried over anhydrous sodium sulfate, filtered, and concentrated under reduced pressure. The resulting residue was purified by silica gel column chromatography (gradient elution, eluent: petroleum ether/ethyl acetate= 1.5/1 to 1/1) to obtain 3-bromoquinolin-8-amine (112c) (3.16 g, yield 85%).

At 0°C, aqueous sodium nitrite (75.9 mg, 1.1 mmol) solution (1.0 mL) was added to a suspension of 3-bromoquinolin-8-amine (112c) (223.0 mg, 1.0 mmol) in sulfuric acid (26 weight%) (19.2 mL). The resulting mixture was stirred at 0°C for 20 minutes, added in batches to aqueous copper sulfate solution (11 weight%) (145.0 mL) at room temperature, followed by the addition of cuprous oxide (143.0 mg, 1.0 mmol). The reaction mixture was stirred at room temperature for 30 minutes, and extracted with dichloromethane (30.0 mL x 4). The organic phases were combined, dried over anhydrous sodium sulfate, filtered, and concentrated under reduced pressure. The resulting residue was purified by silica gel column chromatography (gradient elution, eluent: dichloromethane/methanol=200/1) to obtain 3-bromoquinolin-8-ol (112d) (143.0 mg, yield 64%).

At room temperature, 3-bromoquinolin-8-ol (112d) (143.0 mg, 0.64 mmol), triethylamine (0.13 mL, 0.96 mmol) and di-*tert*-butyl dicarbonate (168.0 mg, 0.768 mmol) were added successively to dichloromethane (3.2 mL). 4-(Dimethylamino)pyridine (7.8 mg, 0.064 mmol) was added. The reaction mixture was stirred for 2 hours and concentrated to dryness under reduced pressure. The resulting residue was purified by silica gel column chromatography (gradient elution, eluent: petroleum ether/ethyl acetate = 30/1) to obtain 3-bromoquinolin-8-yl *tert*-butyl carbonate (112e) (188.0 mg, yield 91%).

At room temperature, 3-bromoquinolin-8-yl *tert*-butyl carbonate (112e) (188.0 mg, 0.58 mmol), cyclopropylboronic acid (124.5 mg, 1.45 mmol), palladium acetate (26.0 mg, 0.116 mmol), tricyclohexylphosphine (65.0 mg, 0.232 mmol) and potassium phosphate (369.0 mg, 1.74 mmol) were added successively to a mixture of toluene (2.9 mL) and water (0.72 mL). The reaction mixture was stirred at 90°C for 26 hours, cooled to room temperature, and diluted with dichloromethane (10.0 mL) and water (10.0 mL). The organic phase was separated, and the aqueous phase was extracted with dichloromethane (10.0 mL x 2). The organic phases were combined, dried over anhydrous sodium sulfate, filtered, and concentrated under reduced pressure. The resulting residue was purified by silica gel column chromatography (gradient elution, eluent: petroleum ether/dichloromethane=1.5/1 to 1/1) to obtain *tert*-butyl (3-cyclopropylquinolin-8-yl) carbonate (112f) (134.0 mg, yield 81%).

At room temperature, a solution of *tert*-butyl (3-cyclopropylquinolin-8-yl) carbonate (112f) (134.0 mg, 0.47 mmol) in 1,4-dioxane (0.59 mL) was added to a solution of hydrogen chloride in 1,4-dioxane (4.0 M) (1.76 mL). The resulting mixture was stirred for 24 hours and concentrated to dryness under reduced pressure. Dichloromethane (10.0 mL) and saturated aqueous sodium bicarbonate solution (10.0 mL) were added to the resulting residue. The organic phase was separated, and the aqueous phase was extracted with dichloromethane (10.0 mL x 2). The organic phases were combined, dried over anhydrous sodium sulfate, filtered, and concentrated to dryness under reduced pressure to obtain the crude product 3-cyclopropylquinolin-8-ol (112g) (95.4 mg, crude yield >100%).

At room temperature, sodium nitrite (50.0 mg, 0.725 mmol) was added to a solution of the crude 3-cyclopropylquinolin-8-ol (112g) (54.6 mg, 0.29 mmol (theoretical amount)) in acetic acid (1.16 mL). The reaction mixture was stirred for 3 hours and concentrated to dryness under reduced pressure. A mixed solvent of ethanol and water (volume ratio, ethanol/water = 1/1) (1.0 mL) was added to the resulting residue, stirred for 5 minutes, and filtered under reduced pressure. The filter cake was washed with a mixed solvent of ethanol and water (volume ratio, ethanol/water = 2/1) (0.5 mL x 3), and dried under vacuum to obtain 3-cyclopropyl-7-nitroquinolin-8-ol (112) (42.1 mg, yield 63%).

¹H NMR (400 MHz, DMSO-*d₆*) δ: 9.24 (s, 1H), 8.82 (d, *J =* 1.2 Hz, 1H), 8.74 (d, *J* = 1.6 Hz, 1H), 8.51 (d, *J =* 8.8 Hz, 1H), 7.11 (d, *J =* 8.8 Hz, 1H), 2.34 - 2.26 (m, 1H), 1.21 (dd, *J =* 8.1, 1.8 Hz, 2H), 0.99 - 0.97 (m, 2H).

MS calculated: 230.07; MS found: 231.1 [M+H]⁺.

### Example 113

### Synthesis of 5-chloro-7-nitroquinolin-2-d-8-ol (113)

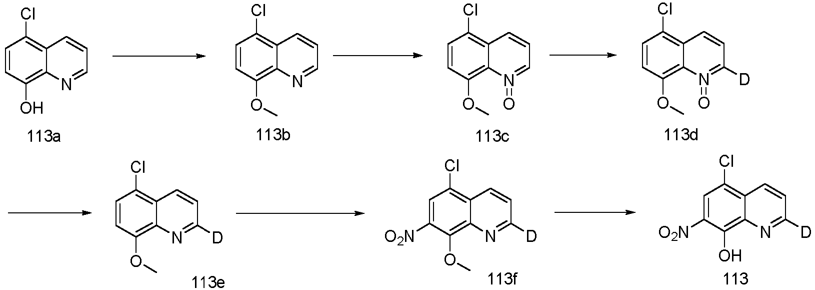

At 0°C, methyl iodide (0.68 mL, 10.8 mmol) was slowly added dropwise to a solution of 5-chloro-8-hydroxyquinoline (113a) (1.5 g, 8.35 mmol) and potassium carbonate (2.3 g, 16.7 mmol) in N,N-dimethylformamide (15.0 mL). The resulting mixture was warmed up to room temperature, stirred for 16 hours, diluted with water (50.0 mL), and extracted with dichloromethane (20.0 mL x 2). The organic phases were combined, washed with saturated brine (10.0 mL x 2), dried over anhydrous sodium sulfate and filtered, and the filtrate was concentrated under reduced pressure. The resulting residue was purified by silica gel column chromatography (gradient elution, eluent: petroleum ether/ethyl acetate = 8/1 to 2/1) to obtain 5-chloro-8-methoxyquinoline (113b) (1.48 g, yield 92%).

At room temperature, 3-chloroperoxybenzoic acid (85 weight%) (2.02 g, 9.95 mmol) was added in batches to a solution of 5-chloro-8-methoxyquinoline (113b) (1.288 g, 6.65 mmol) in dichloromethane (20.0 mL). The resulting mixture was stirred at room temperature for 16 hours, and cooled to 0°C. The pH of the aqueous phase was adjusted to about 8 to 9 with aqueous sodium hydroxide solution (1.0 M). The organic phase was separated, and the aqueous phase was extracted with dichloromethane (20.0 mL x 2). The organic phases were combined, washed with saturated brine (20.0 mL), dried over anhydrous sodium sulfate, filtered, and concentrated under reduced pressure. The resulting residue was purified by silica gel column chromatography (gradient elution, eluent: dichloromethane/methanol = 100%/0 to 91%/9%) to obtain 5-chloro-8-methoxyquinoline-1-oxide (113c) (1.0 g, yield 72%).

At room temperature, 5-chloro-8-methoxyquinoline-1-oxide (113c) (1.0 g, 4.77 mmol) was added in batches to a solution of sodium hydroxide (0.458 g, 11.45 mmol) in heavy water (5.0 mL). The reaction solution was stirred at 100°C for 6 hours, cooled to room temperature, and diluted with water (15.0 mL). The resulting mixture was extracted with dichloromethane (20.0 mL x 3). The organic phases were combined, washed with saturated brine (20.0 mL), dried over anhydrous sodium sulfate and filtered. The filtrate was concentrated to dryness under reduced pressure to obtain 5-chloro-8-methoxyquinoline-1-oxide-2-d (113d) (0.969 g, yield 96%).

At 0°C, phosphorus tribromide (0.43 mL, 4.6 mmol) was slowly added dropwise to a solution of 5-chloro-8-methoxyquinoline-1-oxide-2-d (113d) (0.486 g, 2.3 mmol) in N,N-dimethylformamide (6.0 mL). The reaction solution was stirred at room temperature for 2 hours, and cooled to 0°C. The pH of the aqueous phase was adjusted to about 8 to 9 with saturated aqueous sodium carbonate solution. The resulting mixture was extracted with dichloromethane (10.0 mL x 2). The organic phases were combined, washed with saturated brine (20.0 mL), dried over anhydrous sodium sulfate, filtered, and concentrated under reduced pressure. The resulting residue was purified by silica gel column chromatography (gradient elution, eluent: petroleum ether/ethyl acetate = 20/1 to 2/1) to obtain 5-chloro-8-methoxyquinoline-2-d (113e) (0.268 g, yield 60%).

At 0°C, nitric acid (65 weight%) (0.57 mL, 8.3 mmol) was slowly added dropwise to a solution of 5-chloro-8-methoxyquinoline-2-d (113e) (0.268 g, 1.37 mmol) in acetic anhydride (6.0 mL). The reaction mixture was stirred at 0°C for 10 minutes, followed by the slowly dropwise addition of sulfuric acid (98 weight%) (0.1 mL, 1.84 mmol). The reaction solution was warmed up to room temperature, stirred for 16 hours, and cooled to 0°C. The pH of the aqueous phase was adjusted to about 8 to 9 with aqueous sodium hydroxide solution (1.0 M). The resulting mixture was extracted with dichloromethane (10.0 mL x 3). The organic phases were combined, washed with saturated brine (10.0 mL), dried over anhydrous sodium sulfate, filtered, and concentrated under reduced pressure. The resulting residue was purified by silica gel column chromatography (gradient elution, eluent: petroleum ether/ethyl acetate = 20/1 to 10/1) to obtain 5-chloro-7-nitro-8-methoxyquinoline-2-d (113f) (0.094 g, yield 29%).

At room temperature, 5-chloro-7-nitro-8-methoxyquinoline-2-d (113f) (94.0 mg, 0.39 mmol) and lithium chloride (165.0 mg, 3.9 mmol) were added to N,N-dimethylformamide (3.0 mL). The reaction mixture was stirred at 140°C for 45 minutes, cooled to room temperature, and concentrated under reduced pressure to remove the organic solvent. Water (2.0 mL) was added to the resulting residue, stirred for 3 minutes, and filtered under reduced pressure. The resulting filter cake was washed with water (1.0 mL x 2), and dried under vacuum to obtain 5-chloro-7-nitroquinolin-2-d-8-ol (113) (84.0 mg, yield 95%).

¹H NMR (400 MHz, DMSO-*d₆*) δ: 8.30 (d, *J =* 8.3 Hz, 1H), 8.06 (s, 1H), 7.71 (d, *J* = 8.3 Hz, 1H).

MS calculated: 225.01; MS found: 226.0 [M+H]⁺.

### Example 114

### Synthesis of 6-bromo-5-chloro-7-nitroquinolin-8-ol (114)

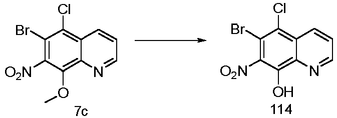

At room temperature, 6-bromo-5-chloro-7-nitro-8-methoxyquinoline (7c) (1.65 g, 5.21 mmol) and lithium chloride (2.2 g, 52.4 mmol) were added to N,N-dimethylformamide (15.0 mL). The reaction mixture was stirred at 120°C for 1 hour, cooled to room temperature, and concentrated under reduced pressure to remove the organic solvent. Water (25.0 mL) was added to the resulting residue, stirred for 5 minutes, and filtered under reduced pressure. The filter cake was washed with water (5.0 mL x 2), and dried under vacuum to obtain 6-bromo-5-chloro-7-nitroquinolin-8-ol (114) (1.41 g, yield 89%).

¹H NMR (400 MHz, DMSO-*d₆*) δ: 8.70 (dd, *J =* 4.4, 1.6 Hz, 1 H), 8.41 (dd, *J =* 8.6, 1.4 Hz, 1 H), 7.68 (dd, *J =* 8.4, 4.0 Hz, 1 H).

MS calculated: 301.91, 303.91; MS found: 303.0, 305.0 [M+H]⁺.

### Example 115

### Synthesis of 5,6-dichloro-7-nitroquinolin-8-ol (115)

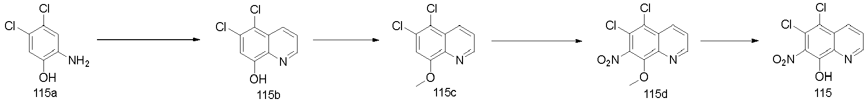

At room temperature, 2-amino-4,5-dichlorophenol (115a) (5.0 g, 28.1 mmol) was added to hydrochloric acid (6.0 M) (140.0 mL). The reaction mixture was warmed up to 110°C, followed by the slowly dropwise addition of acrolein diethyl acetal (12.0 mL, 73.8 mmol), and stirred for 1.5 hours. The reaction mixture was cooled to room temperature, and the pH of the aqueous phase was adjusted to about 9 to 10 by the slow addition of saturated aqueous sodium carbonate solution. The resulting mixture was extracted with ethyl acetate (200.0 mL x 3). The organic phases were combined, dried over anhydrous sodium sulfate, filtered, and concentrated under reduced pressure. The resulting residue was purified by silica gel column chromatography (eluent: petroleum ether/ethyl acetate = 3/1) to obtain 5,6-dichloro-8-hydroxyquinoline (115b) (3.9 g, yield 65%).

At room temperature, methyl iodide (1.35 mL, 21.7 mmol) was slowly added dropwise to a suspension of 5,6-dichloro-8-hydroxyquinoline (115b) (3.9 g, 18.2 mmol) and potassium carbonate (5.03 g, 36.4 mmol) in N,N-dimethylformamide (25.0 mL). The resulting mixture was stirred at room temperature for 18 hours, and concentrated under reduced pressure to remove the organic solvent. Water (30.0 mL) was added to the residue, and extracted with ethyl acetate (30.0 mL x 2). The organic phases were combined, dried over anhydrous sodium sulfate and filtered, and the filtrate was concentrated under reduced pressure. The resulting residue was purified by silica gel column chromatography (eluent: petroleum ether/ethyl acetate = 5/1) to obtain 5,6-dichloro-8-methoxyquinoline (115c) (3.5 g, yield 71%).

At 0°C, nitric acid (65 weight%) (13.0 mL, 197.8 mmol) was slowly added dropwise to a solution of 5,6-dichloro-8-methoxyquinoline (115c) (3.5 g, 15.4 mmol) in acetic anhydride (75.0 mL), and stirred for 10 minutes. Sulfuric acid (98 weight%) (2.5 mL, 47.0 mmol) was slowly added dropwise. The reaction mixture was stirred at 0°C for 30 minutes, then warmed up to room temperature and stirred for 72 hours, and then cooled to 0°C. The pH of the aqueous phase was adjusted to about 9 to 10 with aqueous sodium hydroxide solution (1.0 M). The resulting mixture was extracted with dichloromethane (50.0 mL x 3). The organic phases were combined, dried over anhydrous sodium sulfate, filtered, and concentrated under reduced pressure. The resulting residue was purified by silica gel column chromatography (eluent: petroleum ether/ethyl acetate = 2/1) to obtain 5,6-dichloro-8-methoxy-7-nitroquinoline (115d) (0.718 g, yield 17%).

At room temperature, 5,6-dichloro-8-methoxy-7-nitroquinoline (115d) (0.718 g, 2.64 mmol) and lithium chloride (1.38 g, 32.9 mmol) were added to N,N-dimethylformamide (15.0 mL). The reaction mixture was stirred at 120°C for 1 hour, cooled to room temperature, and concentrated under reduced pressure to remove the organic solvent. Water (25.0 mL) was added to the resulting residue, stirred for 5 minutes, and filtered under reduced pressure. The filter cake was washed with water (5.0 mL x 2), and dried under vacuum to obtain 5,6-dichloro-7-nitroquinolin-8-ol (115) (0.555 g, yield 81%).

¹H NMR (400 MHz, DMSO-*d₆*) δ: 8.69 (dd, *J* = 4.2, 1.2 Hz, 1H), 8.40 (dd, *J =* 8.4, 1.6 Hz, 1H), 7.70 (dd, *J* = 8.4, 4.4 Hz, 1H).

MS calculated: 257.96; MS found: 259.0, 261.0 [M+H]⁺.

### Example 116

### Synthesis of 7-nitroquinolin-2-d-8-ol (116)

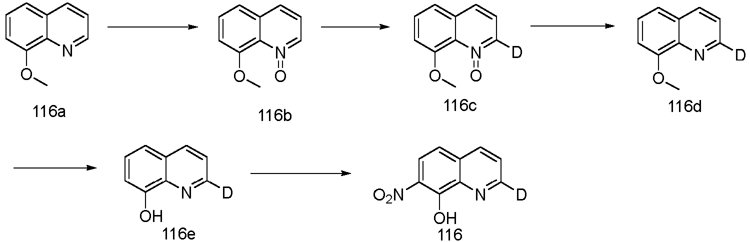

At room temperature, 3-chloroperoxybenzoic acid (85 weight%) (3.6 g, 17.9 mmol) was added in batches to a solution of 8-methoxyquinoline (116a) (1.9 g, 11.9 mmol) in dichloromethane (40.0 mL). The resulting mixture was stirred at room temperature for 16 hours, and cooled to 0°C. The pH of the aqueous phase was adjusted to about 8 to 9 with aqueous sodium hydroxide solution (1.0 M). The organic phase was separated, and the aqueous phase was extracted with dichloromethane (20.0 mL x 2). The organic phases were combined, washed with saturated brine (20.0 mL), dried over anhydrous sodium sulfate, filtered, and concentrated under reduced pressure. The resulting residue was purified by silica gel column chromatography (eluent: dichloromethane/methanol = 100%/0 to 8/1) to obtain 8-methoxyquinoline-1-oxide (116b) (0.836 g, yield 40%).

At room temperature, 8-methoxyquinoline-1-oxide (116b) (0.836 g, 4.77 mmol) was added in batches to a solution of sodium hydroxide (0.458 mg, 11.45 mmol) in heavy water (5.0 mL). The resulting mixture was stirred at 100°C for 6 hours, cooled to room temperature, diluted with water (15.0 mL), and extracted with dichloromethane (20.0 mL x 2). The organic phases were combined, washed with saturated brine (20.0 mL), dried over anhydrous sodium sulfate, filtered, and concentrated to dryness under reduced pressure to obtain 8-methoxyquinoline-1-oxide-2-d (116c) (0.80 g, yield 95%).

At 0°C, phosphorus tribromide (0.85 mL, 9 mmol) was slowly added dropwise to a solution of 8-methoxyquinoline-1-oxide-2-d (116c) (0.797 g, 4.52 mmol) in N,N-dimethylformamide (10.0 mL). The reaction solution was stirred at room temperature for 2 hours, and cooled to 0°C. The pH of the aqueous phase was adjusted to about 8 to 9 by the slow addition of saturated aqueous sodium carbonate solution. The resulting mixture was extracted with dichloromethane (20.0 mL x 2). The organic phases were combined, washed with saturated brine (20.0 mL), dried over anhydrous sodium sulfate, filtered, and concentrated under reduced pressure. The resulting residue was purified by silica gel column chromatography (gradient elution, eluent: petroleum ether/ethyl acetate = 100%/0 to 1/1.5) to obtain 8-methoxyquinoline-2-*d* (116d) (0.418 g, yield 58%).

At 0°C, a solution of boron tribromide in dichloromethane (1.0 M) (13.0 mL, 13.0 mmol) was slowly added dropwise to a solution of 8-methoxyquinoline-2-*d* (116d) (0.418 g, 2.6 mmol) in dichloromethane (3.0 ml). The reaction mixture was warmed up to room temperature, and stirred for 16 hours. The pH of the aqueous phase was adjusted to about 8 to 9 by the slow addition of saturated aqueous sodium carbonate solution. The resulting mixture was extracted with dichloromethane (10.0 mL x 3). The organic phases were combined, dried over anhydrous sodium sulfate, filtered, and concentrated under reduced pressure. The resulting residue was purified by silica gel column chromatography (eluent: dichloromethane/methanol = 30/1) to obtain 8-hydroxyquinoline-2-*d* (116e) (0.203 g, yield 53%).

At room temperature, sodium nitrite (141.0 mg, 2.0 mmol) was added to a solution of 8-hydroxyquinoline-2-*d* (116e) (100.0 mg, 0.68 mmol) in acetic acid (3.0 mL). The reaction mixture was stirred for 2 hours, and filtered under reduced pressure. The pH of the aqueous phase of the filtrate was adjusted to about 9 to 10 with ammonia (25 to 28 weight%, NH₃ content), and the mixture was filtered under reduced pressure. The resulting filter cake was dissolved in methanol (2.0 mL), and purified by reversed-phase high performance liquid chromatography (the chromatographic column was Eclipse XDB-C18 (21.2 mm×250 mm, 7µm), the mobile phase was water/acetonitrile=100%/0 - 92%/8%, gradient elution, 0.1% formic acid was added to the mobile phase by volume percentage, and the flow rate was 20.0 mL/min) to obtain 7-nitroquinolin-2-*d*-8-ol (116) (30.0 mg, yield 23%).

¹H NMR (400 MHz, DMSO-*d₆*) δ:9.20 (d, *J =* 8.8 Hz, 1H), 8.57 (d, *J =* 8.9 Hz, 1H), 7.90 (d, *J =* 8.9 Hz, 1H), 7.18 (d, *J =* 8.8 Hz, 1H).

MS calculated: 191.04; MS found: 192.2 [M+H]⁺.

### Example 117

### Synthesis of 7-nitroquinolin-6-d-8-ol (117)

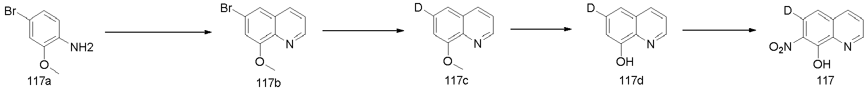

At room temperature, 4-bromo-2-methoxyaniline (117a) (1.0 g, 5.0 mmol) was added to hydrochloric acid (6.0 M) (30.0 mL). The reaction mixture was warmed up to 110°C, followed by the slowly dropwise addition of acrolein diethyl acetal (2.44 mL, 15.0 mmol), and stirred for 10 hours. The reaction mixture was cooled to room temperature, and the pH of the aqueous phase was adjusted to about 9 to 10 by the slow addition of saturated aqueous sodium carbonate solution. The resulting mixture was extracted with ethyl acetate (30.0 mL x 2). The organic phases were combined, dried over anhydrous sodium sulfate, filtered, and concentrated under reduced pressure. The resulting residue was purified by silica gel column chromatography (eluent: petroleum ether/ethyl acetate = 1/1) to obtain 6-bromo-8-methoxyquinoline (117b) (0.5 g, yield 42%).

At room temperature, 6-bromo-8-methoxyquinoline (117b) (119.0 mg, 0.5 mmol), potassium methoxide (70.0 mg, 1.0 mmol) and hexamethyldisilane (146.0 mg, 1.0 mmol) were added successively to deuterated acetonitrile (10.0 mL). The reaction mixture was stirred at room temperature for 15 hours, concentrated to dryness under reduced pressure, followed by the addition of water (30.0 mL). The resulting mixture was extracted with ethyl acetate (30.0 mL x 2). The organic phases were combined, dried over anhydrous sodium sulfate, filtered, and concentrated under reduced pressure. The resulting residue was purified by silica gel column chromatography (eluent: petroleum ether/ethyl acetate = 1/1) to obtain 8-methoxyquinoline-6-*d* (117c) (60.0 mg, yield 75%).

At 0°C, a solution of boron tribromide in dichloromethane (1.0 M) (3.0 mL, 3.0 mmol) was slowly added dropwise to a solution of 8-methoxyquinoline-6-*d* (117c) (60.0 mg, 0.38 mmol) in dichloromethane (1.0 ml). The reaction solution was warmed up to room temperature and stirred for 30 minutes. The pH of the aqueous phase was adjusted to about 8 to 9 with saturated aqueous sodium bicarbonate solution. The organic phase was separated, and the aqueous phase was extracted with dichloromethane (10.0 mL x 2). The combined organic phase was dried over anhydrous sodium sulfate, filtered, and concentrated to dryness under reduced pressure to obtain the crude product quinolin-6-d-8-ol (117d) (51.0 mg, crude yield 93%).

At 0°C, sodium nitrite (75.0 mg, 1.1 mmol) was added to a suspension of the crude quinolin-6-*d*-8-ol (117d) (51.0 mg, 0.35 mmol) in acetic acid (1.0 mL). The reaction mixture was stirred at 0°C for 1 hour, at room temperature for 18 hours, and filtered under reduced pressure. The resulting filter cake was added to methanol (1.0 mL), stirred for 10 minutes, and filtered under reduced pressure. The resulting filter cake was washed with methanol (0.5 mL x 2), and dried under vacuum to obtain 7-nitroquinolin-6-*d*-8-ol (117) (34.0 mg, yield 51%).

¹H NMR (400 MHz, DMSO-*d₆*) δ: 9.44 (dd, *J* = 8.8, 1.6 Hz, 1 H), 8.62 (dd, *J =* 4.0, 1.6 Hz, 1 H), 7.59 (dd, *J =* 8.6, 4.0 Hz, 1 H), 6.29 (s, 1 H).

MS calculated: 191.04; MS found: 192.0 [M+H]⁺.

### Example 118

### Synthesis of 5-chloro-4-(3,3-difluoropiperidin-1-yl)-7-nitroquinolin-8-ol (118)

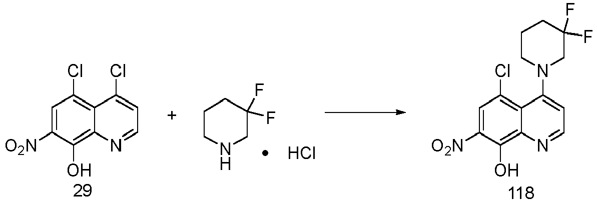

At room temperature, 4,5-dichloro-7-nitroquinolin-8-ol (29) (150.0 mg, 0.51 mmol) and 3,3-difluoroperidine hydrochloride (150.0 mg, 0.95 mmol) were added to N,N-dimethylformamide (4.0 mL). The resulting mixture was stirred at 130°C for 5 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/methanol=100%/0 - 40%/60%, gradient elution, and the flow rate was 20.0 mL/min) to obtain 5-chloro-4-(3,3-difluoropiperidin-1-yl)-7-nitroquinolin-8-ol (118) (30.0 mg, yield 17%).

¹H NMR (400 MHz, DMSO-*d₆*) δ: 8.44 (d, *J =* 8 Hz, 1 H), 7.99 (s, 1 H), 7.50 (d, *J* = 8 Hz, 1 H), 4.13-4.00 (m, 1 H), 3.94-3.83 (m, 1 H), 3.70-3.63 (m, 2 H), 2.30-2.10 (m, 2 H), 2.06-1.96 (m, 1 H), 1.88-1.78 (m, 1 H).

MS calculated: 343.05; MS found: 344.0, 346.0 [M+H]⁺.

### Example 119

### Synthesis of 5-chloro-4-(3-fluoropiperidin-1-yl)-7-nitroquinolin-8-ol (119)

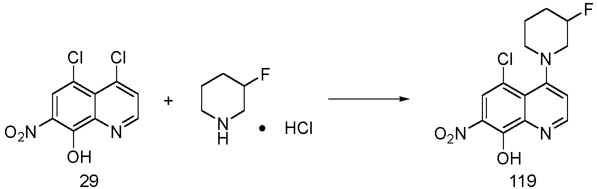

At room temperature, 4,5-dichloro-7-nitroquinolin-8-ol (29) (104.0 mg, 0.4 mmol) and 3-fluoroperidine hydrochloride (112.0 mg, 0.8 mmol) were added to N,N-dimethylformamide (4.0 mL). The resulting mixture was stirred at 130°C for 5 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/methanol=100%/0 - 40%/60%, gradient elution, and the flow rate was 20.0 mL/min) to obtain 5-chloro-4-(3-fluoropiperidin-1-yl)-7-nitroquinolin-8-ol (119) (26.4 mg, yield 20%).

¹H NMR (400 MHz, DMSO-*d₆*) δ: 8.44 (d, *J =* 8.0 Hz, 1 H), 7.99 (s, 1 H), 7.50 (d, *J =* 8.0 Hz, 1 H), 4.13-4.00 (m, 1 H), 3.94-3.83 (m, 1 H), 3.70-3.63 (m, 3 H), 2.30-2.10 (m, 2 H), 2.06-1.96 (m, 1 H), 1.88-1.78 (m, 1 H).

MS calculated: 325.06; MS found: 326.0, 328.0 [M+H]⁺.

### Example 120

### Synthesis of 5-chloro-7-nitroquinolin-4-d-8-ol (120)

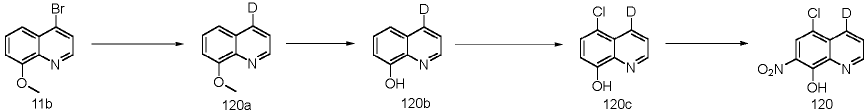

At room temperature, potassium methoxide (177.0 mg, 2.52 mmol) and hexamethyldisilane (369.0 mg, 2.52 mmol) were added successively to a solution of 4-bromo-8-methoxyquinoline (11b) (300.0 mg, 1.26 mmol) in deuterated acetonitrile (2.5 mL). The reaction mixture was stirred at room temperature for 16 hours, diluted with water (10.0 mL), and extracted with ethyl acetate (10.0 mL x 2). The organic phases were combined, washed with saturated brine (10.0 mL), dried over anhydrous sodium sulfate, filtered, and concentrated under reduced pressure. The resulting residue was purified by silica gel column chromatography (gradient elution, eluent: petroleum ether/ethyl acetate = 100%/0 to 4/1) to obtain 8-methoxyquinoline-4-*d* (120a) (125.0 mg, yield 62%).

At 0°C, a solution of boron tribromide in dichloromethane (1.0 M) (4.0 mL, 4.0 mmol) was slowly added dropwise to a solution of 8-methoxyquinoline-4-*d* (120a) (125.0 mg, 0.78 mmol) in dichloromethane (2.0 ml). The reaction solution was warmed up to room temperature, and stirred for 16 hours. The pH of the aqueous phase was adjusted to about 8 to 9 by the slow addition of saturated aqueous sodium carbonate solution. The organic phase was separated, and the aqueous phase was extracted with dichloromethane (10.0 mL x 2). The organic phases were combined, dried over anhydrous sodium sulfate, filtered, and concentrated under reduced pressure. The resulting residue was purified by silica gel column chromatography (gradient elution, eluent: dichloromethane/methanol = 100%/0 to 30/1) to obtain quinolin-4-*d*-8-ol (120b) (100.0 mg, yield 88%).

At 0°C, N-chlorosuccinimide (86.8 mg, 0.65 mmol) was added in batches to a solution of quinolin-4-*d*-8-ol (120b) (100.0 mg, 0.68 mmol) in sulfuric acid (98 weight%) (2.0 mL). The reaction mixture was stirred at 0°C for 0.5 hour, at room temperature for 5 hours, and then cooled to 0°C. The pH of the aqueous phase was adjusted to about 8 to 9 by the slow addition of saturated aqueous sodium carbonate solution. The resulting mixture was extracted with dichloromethane (10.0 mL x 3). The organic phases were combined, washed with saturated brine (10.0 mL), dried over anhydrous sodium sulfate, filtered, and concentrated under reduced pressure. The resulting residue was purified by silica gel column chromatography (gradient elution, eluent: dichloromethane/methanol = 100%/0 to 30/1) to obtain 5-chloroquinolin-4-*d*-8-ol (120c) (86.0 mg, yield 70%).

At room temperature, sodium nitrite (99.0 mg, 1.43 mmol) was added to a solution of 5-chloroquinolin-4-*d*-8-ol (120c) (86.0 mg, 0.48 mmol) in acetic acid (2.0 mL). The reaction mixture was stirred for 3 hours and filtered under reduced pressure, and the filter cake was washed with ethyl acetate (1.0 mL x 2). The resulting filtrate was left to stand at room temperature for 16 hours, and a solid precipitated. The resulting mixture was filtered under reduced pressure, the filter cake was washed with water (0.5 mL x 2), and dried under vacuum to obtain 5-chloro-7-nitroquinolin-4-*d*-8-ol (120) (24.0 mg, yield 22%).

¹H NMR (400 MHz, DMSO-*d₆*) δ: 9.13 (d, J = 4.2 Hz, 1H), 8.25 (s, 1H), 7.98 (d, J = 4.2 Hz, 1H).

MS calculated: 225.01; MS found: 226.0 [M+H]⁺.

### Example 121

### Synthesis of 5-bromo-7-nitroquinolin-8-ol (121)

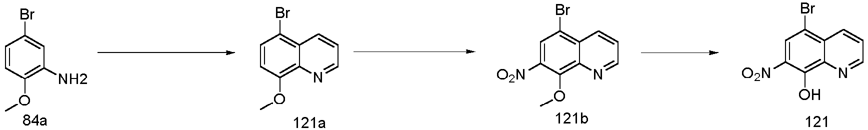

At room temperature, 5-bromo-2-methoxyaniline (84a) (2.0 g, 9.9 mmol) was added to hydrochloric acid (6.0 M) (40.0 mL). The reaction mixture was warmed up to 110°C, followed by the slowly dropwise addition of acrolein diethyl acetal (4.88 mL, 30 mmol), and stirred for 6 hours. The reaction mixture was cooled to room temperature, and the pH of the aqueous phase was adjusted to about 9 to 10 by the slow addition of saturated aqueous sodium carbonate solution. The resulting mixture was extracted with ethyl acetate (30.0 mL x 2). The organic phases were combined, dried over anhydrous sodium sulfate, filtered, and concentrated under reduced pressure. The resulting residue was purified by silica gel column chromatography (eluent: petroleum ether/ethyl acetate = 3/1) to obtain 5-bromo-8-methoxyquinoline (121a) (0.84 g, yield 36%).

At 0°C, nitric acid (65 weight%) (1.0 mL, 23.6 mmol) was slowly added dropwise to a solution of 5-bromo-8-methoxyquinoline (121a) (200.0 mg, 0.84 mmol) in acetic anhydride (2.0 mL), and stirred for 10 minutes. Sulfuric acid (98 weight%) (0.2 mL, 3.7 mmol) was slowly added dropwise. The reaction mixture was stirred at 0°C for 30 minutes, at room temperature for 18 hours, and then cooled to 0°C. The pH of the aqueous phase was adjusted to about 9 to 10 by the addition of aqueous sodium hydroxide solution (1.0 M). The resulting mixture was extracted with dichloromethane (10.0 mL x 3). The organic phases were combined, dried over anhydrous sodium sulfate, filtered, and concentrated under reduced pressure. The resulting residue was purified by silica gel column chromatography (eluent: petroleum ether/ethyl acetate = 4/1) to obtain 5-bromo-8-methoxy-7-nitroquinoline (121b) (60.0 mg, yield 25%).

At room temperature, 5-bromo-8-methoxy-7-nitroquinoline (121b) (60.0 mg, 0.21 mmol) and lithium chloride (89.0 mg, 2.1 mmol) were added to N,N-dimethylformamide (2.0 mL). The reaction mixture was stirred at 120°C for 1 hour, cooled to room temperature, and concentrated under reduced pressure to remove the organic solvent. Water (3.0 mL) was added to the resulting residue, stirred for 3 minutes, and filtered under reduced pressure. The filter cake was washed with water (0.5 mL x 2), and dried under vacuum to obtain 5-bromo-7-nitroquinolin-8-ol (121) (40.0 mg, yield 71%).

¹H NMR (400 MHz, DMSO-*d₆*) δ: 8.69 (d, *J =* 4.4 Hz, 1H), 8.23 (s, 1 H), 8.21-8.19 (m,1H), 7.68 (dd, *J =* 8.4, 4.4 Hz, 1 H).

MS calculated: 267.95; MS found: 269.0, 271.0 [M+H]⁺.

### Example 122

### Synthesis of 6-fluoro-7-nitroquinolin-8-ol (122)

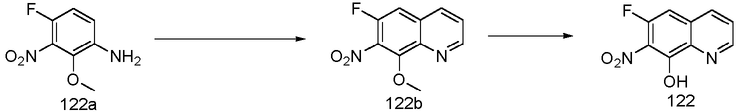

At room temperature, 2-methoxy-3-nitro-4-fluoroaniline (122a) (100.0 mg, 0.537 mmol) was added to hydrochloric acid (6.0 M) (10.0 mL). The reaction mixture was warmed up to 110°C, followed by the slowly dropwise addition of acrolein diethyl acetal (0.25 mL, 1.61 mmol), and stirred for 2 hours. The reaction mixture was cooled to room temperature, and the pH of the aqueous phase was adjusted to about 7 to 8 with aqueous sodium hydroxide solution (6.0 M). The resulting mixture was extracted with dichloromethane (15.0 mL x 3). The organic phases were combined, dried over anhydrous sodium sulfate and filtered, and the filtrate was concentrated under reduced pressure. The resulting residue was purified by silica gel column chromatography (gradient elution, eluent: petroleum ether/ethyl acetate = 100%/0 to 7/1) to obtain 6-fluoro-7-nitro-8-methoxyquinoline (122b) (53.0 mg, yield 45%).

At room temperature, 6-fluoro-7-nitro-8-methoxyquinoline (122b) (53.0 mg, 0.24 mmol) and lithium chloride (100.0 mg, 2.4 mmol) were added to N,N-dimethylformamide (2.0 mL). The reaction mixture was stirred at 130°C for 1 hour, cooled to room temperature, and concentrated under reduced pressure to remove the organic solvent. Water (2.0 mL) was added to the resulting residue, stirred for 3 minutes, and filtered under reduced pressure. The filter cake was washed with water (0.5 mL x 2), and dried under vacuum to obtain 6-fluoro-7-nitroquinolin-8-ol (122) (46.0 mg, yield 93%).

¹H NMR (400 MHz, DMSO-*d₆*) δ: 9.13 (d, *J=* 8.7 Hz, 1H), 8.58 (d, *J =* 3.6 Hz, 1H), 7.63 (dt, *J =* 17.0, 8.5 Hz, 1H), 6.14 (d, *J =* 18.0 Hz, 1H).

MS calculated: 208.03; MS found: 209.1 [M+H]⁺.

### Example 123

### Synthesis of 6-chloro-5-fluoro-7-nitroquinolin-8-ol (123)

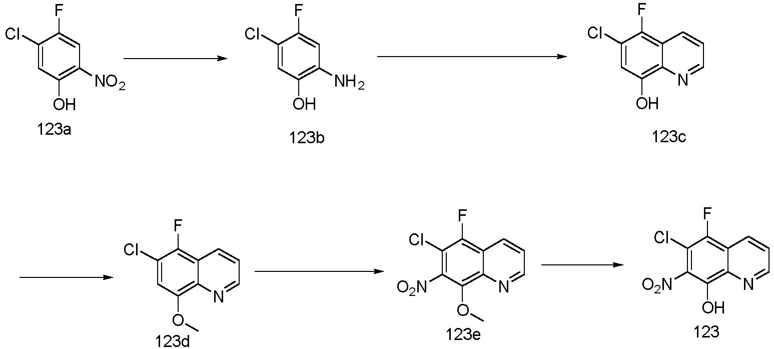

At room temperature, 2-nitro-4-fluoro-5-chlorophenol (123a) (1.92 g, 10.0 mmol) was added to a mixture of tetrahydrofuran (60.0 mL) and water (60.0 mL). Sodium dithionite (85 weight%) (12.3 g, 60.0 mmol) was added in batches. The reaction mixture was stirred at room temperature for 1 hour, and extracted with ethyl acetate (30.0 mL x 3). The organic phases were combined, washed with saturated brine (20.0 mL), dried over anhydrous sodium sulfate, filtered, and concentrated under reduced pressure. The resulting residue was purified by silica gel column chromatography (gradient elution, eluent: petroleum ether/ethyl acetate = 100%/0 to 3/1) to obtain 2-amino-4-fluoro-5-chlorophenol (123b) (0.892 g, yield 55%).

At room temperature, 2-amino-4-fluoro-5-chlorophenol (123b) (0.892 g, 5.5 mmol), sodium 3-nitrobenzene sulfonate (1.48 g, 6.57 mmol) and glycerol (1.27 g, 13.8 mmol) were added successively to sulfuric acid (70 weight%) (8.0 mL). The resulting mixture was stirred at 140°C for 4 hours, and cooled to room temperature. The pH of the aqueous phase was adjusted to about 8 to 9 with aqueous sodium hydroxide solution (6.0 M). The resulting mixture was extracted with dichloromethane (20.0 mL x 3). The organic phases were combined, dried over anhydrous sodium sulfate, filtered, and concentrated under reduced pressure. The resulting residue was purified by silica gel column chromatography (gradient elution, eluent: petroleum ether/ethyl acetate = 100%/0 to 7/1) to obtain 5-fluoro-6-chloroquinolin-8-ol (123c) (0.411 g, yield 38%).

At room temperature, methyl iodide (0.17 mL, 2.73 mmol) was slowly added dropwise to a suspension of 5-fluoro-6-chloroquinolin-8-ol (123c) (0.411 g, 2.1 mmol) and potassium carbonate (0.58 g, 4.2 mmol) in N,N-dimethylformamide (6.0 mL). The resulting mixture was stirred for 16 hours, and concentrated under reduced pressure to remove the organic solvent. Water (30.0 mL) was added to the resulting residue, and extracted with dichloromethane (10.0 mL x 3). The organic phases were combined, washed with saturated brine (10.0 mL), dried over anhydrous sodium sulfate, filtered, and concentrated under reduced pressure. The resulting residue was purified by silica gel column chromatography (gradient elution, eluent: petroleum ether/ethyl acetate = 100%/0 to 3/1) to obtain 5-fluoro-6-chloro-8-methoxyquinoline (123d) (0.412 g, yield 94%).

At 0°C, nitric acid (65 weight%) (0.8 mL, 11.7 mmol) was slowly added dropwise to a solution of 5-fluoro-6-chloro-8-methoxyquinoline (123d) (0.412 g, 1.95 mmol) in acetic anhydride (10.0 mL), and stirred for 10 minutes. Sulfuric acid (98 weight%) (0.12 mL, 2.25 mmol) was slowly added dropwise. The reaction mixture was warmed up to room temperature, stirred for 16 hours, and cooled to 0°C. The pH of the aqueous phase was adjusted to about 8 to 9 with aqueous sodium hydroxide solution (6.0 M). The resulting mixture was extracted with dichloromethane (20.0 mL x 3). The organic phases were combined, washed with saturated brine (20.0 mL), dried over anhydrous sodium sulfate, filtered, and concentrated under reduced pressure. The resulting residue was purified by silica gel column chromatography (gradient elution, eluent: petroleum ether/ethyl acetate = 100%/0 to 5/1) to obtain 5-fluoro-6-chloro-7-nitro-8-methoxyquinoline (123e) (84.0 mg, yield 17%).

At room temperature, 5-fluoro-6-chloro-7-nitro-8-methoxyquinoline (123e) (84.0 mg, 0.39 mmol) and lithium chloride (162.0 mg, 3.9 mmol) were added to N,N-dimethylformamide (2.0 mL). The reaction mixture was stirred at 130°C for 1 hour, cooled to room temperature, and concentrated under reduced pressure to remove the organic solvent. Water (2.0 mL) was added to the resulting residue, stirred for 3 minutes, and filtered under reduced pressure. The resulting filter cake was washed with water (0.5 mL x 2), and dried under vacuum to obtain 5-fluoro-6-chloro-7-nitroquinolin-8-ol (123) (77.0 mg, yield 82%).

¹H NMR (400 MHz, DMSO-*d₆*) δ: 8.69 (d, *J =* 3.2 Hz, 1H), 8.25 (d, *J =* 8.3 Hz, 1H), 7.63 (dt*, J =* 12.8, 6.4 Hz, 1H).

MS calculated: 241.99; MS found: 243.1 [M+H]⁺.

### Example 124

### Synthesis of 6-bromo-5-fluoro-7-nitroquinolin-8-ol (124)

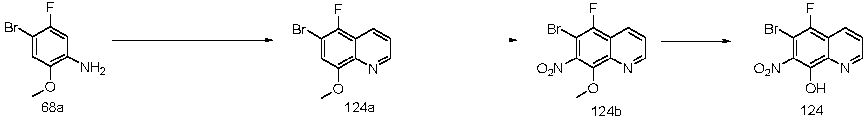

At room temperature, 4-bromo-5-fluoro-2-methoxyaniline (68a) (0.396 g, 1.8 mmol), sodium 3-nitrobenzene sulfonate (0.486 g, 2.16 mmol) and glycerol (0.33 mL, 4.5 mmol) were added successively to sulfuric acid (70 weight%) (2.7 mL). The resulting mixture was stirred at 140°C for 3 hours, and cooled to room temperature. The pH of the aqueous phase was adjusted to about 8 to 9 with aqueous sodium hydroxide solution (1.0 M). The resulting mixture was extracted with dichloromethane (10.0 mL x 3). The organic phases were combined, dried over anhydrous sodium sulfate, filtered, and concentrated under reduced pressure. The resulting residue was purified by silica gel column chromatography (gradient elution, eluent: petroleum ether/ethyl acetate = 4/1 to 2/1) to obtain 6-bromo-5-fluoro-8-methoxyquinoline (124a) (0.418 g, yield 91%).

At 0°C, nitric acid (65 weight%) (0.58 mL, 9.6 mmol) was slowly added dropwise to a solution of 6-bromo-5-fluoro-8-methoxyquinoline (124a) (0.246 g, 0.96 mmol) in acetic anhydride (4.8 mL), and stirred for 10 minutes. Sulfuric acid (98 weight%) (0.104 mL, 1.92 mmol) was slowly added dropwise. The reaction mixture was stirred at 0°C for 3 hours, at room temperature for 3 hours, and then cooled to 0°C. The pH of the aqueous phase was adjusted to about 8 to 9 with aqueous sodium hydroxide solution (1.0 M). The resulting mixture was extracted with dichloromethane (10.0 mL x 3). The organic phases were combined, washed with saturated brine (10.0 mL), dried over anhydrous sodium sulfate, filtered, and concentrated under reduced pressure. The resulting residue was purified by silica gel column chromatography (eluent: petroleum ether/ethyl acetate = 6/1) to obtain 6-bromo-5-fluoro-8-methoxy-7-nitroquinoline (124b) (78.0 mg, yield 27%).

At room temperature, 6-bromo-5-fluoro-8-methoxy-7-nitroquinoline (124b) (68.3 mg, 0.23 mmol) and lithium chloride (97.5 mg, 2.3 mmol) were added to N,N-dimethylformamide (0.46 mL). The reaction mixture was stirred at 130°C for 45 minutes, cooled to room temperature, and concentrated under reduced pressure to remove the organic solvent. Water (2.0 mL) was added to the resulting residue, stirred for 3 minutes, and filtered under reduced pressure. The resulting filter cake was washed with water (1.0 mL x 3), and dried under vacuum to obtain 6-bromo-5-fluoro-7-nitroquinolin-8-ol (124) (56.7 mg, yield 86%).

¹H NMR (400 MHz, DMSO-*d₆*) δ: 8.71 (d, *J =* 3.2 Hz, 1H), 8.26 (d, *J =* 8.4 Hz, 1H), 7.64 (dd, *J* = 8.4, 4.0 Hz, 1H).

MS calculated: 285.94; MS found: 287.0, 289.0 [M+H]⁺.

### Example 125

### Synthesis of 5,6-difluoro-7-nitroquinolin-8-ol (125)

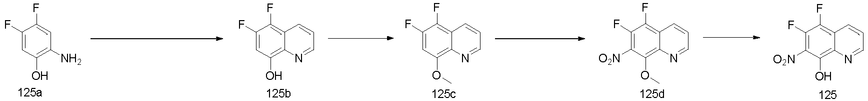

At room temperature, 2-amino-4,5-difluorophenol (125a) (0.615 g, 4.24 mmol), sodium 3-nitrobenzene sulfonate (1.432 g, 6.36 mmol) and glycerol (1.25 mL, 16.96 mmol) were added successively to sulfuric acid (70 weight%) (5.42 mL). The resulting mixture was stirred at 140°C for 8 hours, and cooled to room temperature. The pH of the aqueous phase was adjusted to about 8 to 9 with aqueous sodium hydroxide solution (1.0 M). The resulting mixture was extracted with dichloromethane (10.0 mL x 3). The organic phases were combined, dried over anhydrous sodium sulfate, filtered, and concentrated under reduced pressure. The resulting residue was purified by silica gel column chromatography (eluent: petroleum ether/ethyl acetate = 1.5/1) to obtain 5,6-difluoroquinolin-8-ol (125b) (0.603 g, yield 78%).

At room temperature, methyl iodide (0.41 mL, 6.66 mmol) was slowly added dropwise to a suspension of 5,6-difluoroquinolin-8-ol (125b) (0.603 g, 3.33 mmol) and potassium carbonate (1.381 g, 9.99 mmol) in N,N-dimethylformamide (13.3 mL). The resulting mixture was stirred for 16 hours, and concentrated under reduced pressure to remove the organic solvent. Water (20.0 mL) was added to the resulting residue, and extracted with dichloromethane (20.0 mL x 3). The organic phases were combined, washed with saturated brine (10.0 mL), dried over anhydrous sodium sulfate, filtered, and concentrated under reduced pressure. The resulting residue was purified by silica gel column chromatography (eluent: dichloromethane/ethyl acetate = 40/1) to obtain 5,6-difluoro-8-methoxyquinoline (125c) (0.533 g, yield 82%).

At 0°C, nitric acid (65 weight%) (1.79 mL, 27.3 mmol) was slowly added dropwise to a solution of 5,6-difluoro-8-methoxyquinoline (125c) (0.533 g, 2.73 mmol) in acetic anhydride (13.6 mL), and stirred for 10 minutes. Sulfuric acid (98 weight%) (0.30 mL, 5.46 mmol) was slowly added dropwise. The reaction mixture was stirred at 0°C for 5 minutes, at room temperature for 7 hours, and then cooled to 0°C. The pH of the aqueous phase was adjusted to about 8 to 9 with aqueous sodium hydroxide solution (1.0 M). The resulting mixture was extracted with dichloromethane (10.0 mL x 3). The organic phases were combined, washed with saturated brine (10.0 mL), dried over anhydrous sodium sulfate, filtered, and concentrated under reduced pressure. The resulting residue was purified by silica gel column chromatography (eluent: petroleum ether/dichloromethane = 1.2/1) to obtain 5,6-difluoro-8-methoxy-7-nitroquinoline (125d) (0.0485 g, yield 7%).

At room temperature, 5,6-difluoro-8-methoxy-7-nitroquinoline (125d) (48.5 mg, 0.20 mmol) and lithium chloride (84.8 mg, 2.0 mmol) were added to N,N-dimethylformamide (0.40 mL). The reaction mixture was stirred at 120°C for 45 minutes, cooled to room temperature, and concentrated under reduced pressure to remove the organic solvent. A mixed solvent of ethanol and water (volume ratio, ethanol/water = 1/2) (1.5 mL) was added to the resulting residue, stirred for 5 minutes, and filtered under reduced pressure. The filter cake was washed with a mixed solvent of ethanol and water (volume ratio, ethanol/water = 1/2) (0.5 mL x 3), and dried under vacuum to obtain 5,6-difluoro-7-nitroquinolin-8-ol (125) (41.5 mg, yield 92%).

¹H NMR (400 MHz, DMSO-*d₆*) δ: 8.66 (d, *J =* 2.4 Hz, 1H), 8.24 (dd, *J =* 8.4, 1.2 Hz, 1H), 7.64 (dd, *J =* 8.4, 4.4 Hz, 1H).

MS calculated: 226.02 ; MS found: 227.1 [M+H]⁺.

### Example 127

### Synthesis of 5-bromo-7-cyano-8-hydroxyquinoline (127)

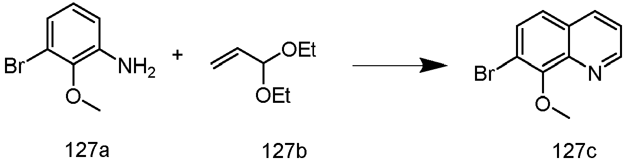

2-Methoxy-3-bromoaniline (Compound 127a) (5.7 g, 28.5 mmol, 1 eq) was dissolved in 100 mL of 6 M aqueous hydrochloric acid solution, and heated to reflux. Acrolein diethyl acetal (Compound 127b) (11.1 g, 85.5 mmol, 3 eq) was added dropwise, and the reaction was carried out for 2 hours. The reaction solution was cooled, the pH was adjusted to neutral with aqueous sodium carbonate solution, and the reaction solution was extracted with dichloromethane (30 mL×2). The organic phases were combined and concentrated to obtain a crude product, which was purified by silica gel column chromatography (eluent: PE:EA=10:1 to 1:1) to obtain 800 mg of 7-bromo-8-methoxyquinoline (Compound 127c) as a colorless oil with a purity of 90%.

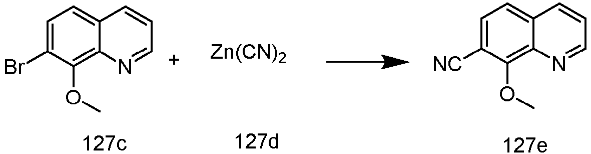

7-Bromo-8-methoxyquinoline (Compound 127c) (400 mg, 1.18 mmol, 1 eq) was dissolved in 5 mL of anhydrous dimethylformamide, and zinc cyanide (Compound 127d) (110.5 mg, 0.95 mmol, 0.8 eq) and tetrakis(triphenylphosphine)palladium (68 mg, 0.059 mmol, 0.05 eq) were added. The reaction system was purged with nitrogen, and the reaction was carried out for 8 hours at 110°C. The reaction solution was concentrated to obtain a crude product, which was purified by silica gel column chromatography (eluent: PE:EA=10:1 to 0:1) to obtain 130 mg of 7-cyano-8-methoxyquinoline (Compound 127e) with a purity of 90%, as a white solid.

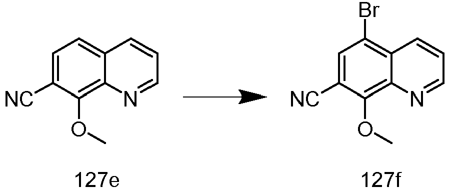

7-Cyano-8-methoxyquinoline (Compound 127e) (120 mg, 1.43 mmol, 1 eq) was dissolved in 5 mL of anhydrous dimethylformamide, and NBS (506 mg, 2.86 mmol, 2 eq) was added. The reaction was carried out for 8 hours at room temperature, and 20 mL of water was added to precipitate a solid. The solid was filtered and dried to obtain 100 mg of 5-bromo-7-cyano-8-methoxyquinoline (Compound 127f) as a white solid with a purity of 90%.

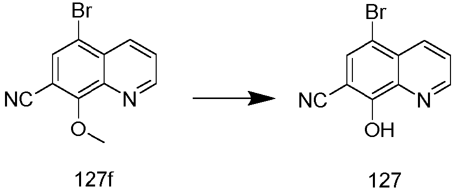

5-Bromo-7-cyano-8-methoxyquinoline (Compound 127f) (100 mg, 0.38 mmol, 1 eq) was dissolved in 5 mL of dimethylformamide, and 200 mg of lithium chloride was added. The reaction was carried out for 1 hour at 80°C, and 20 mL of water was added. The pH was adjusted to neutral, and the reaction solution was extracted with dichloromethane (5 mL×2). The organic phases were combined, and the crude product was purified by pre-HPLC to obtain 8.8 mg of 5-bromo-7-cyano-8-hydroxyquinoline (Compound 127) as a white solid with a purity of 97%.

MS calculated: 247.96, 249.96; MS found: 249.0, 250.9 [M+H]⁺.

¹H NMR (400 MHz, DMSO-d6): δ 9.10 (d, J=4Hz,1H), 8.56 (d, J=8Hz,1H), 8.13 (s, 1H), 7.93 (dd, J=4Hz, J=8Hz,1H).

### Example 128

### Synthesis of 5-trifluoromethyl-7-cyano-8-hydroxyquinoline (128)

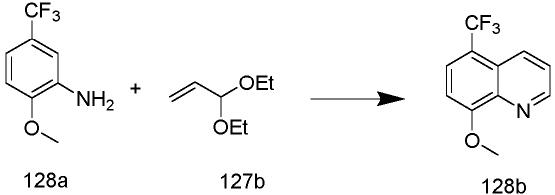

2-Methoxy-5-trifluoromethylaniline (Compound 128a) (5 g, 26.16 mmol, 1 eq) was dissolved in 50 mL of 3 M aqueous hydrochloric acid solution, and heated to reflux. Acrolein diethyl acetal (Compound 127b) (17.1 g, 130.8 mmol, 5 eq) was added dropwise, and the reaction was carried out for 3 hours. The reaction solution was cooled, the pH was adjusted to neutral with aqueous sodium carbonate solution, and the reaction solution was extracted with dichloromethane (50 mL×2). The organic phases were combined and concentrated to obtain a crude product, which was purified by silica gel column chromatography (eluent: PE:EA=10:1 to 0:1) to obtain 3.1 g of 5-trifluoromethyl-8-methoxyquinoline (Compound 128b) as a colorless oil with a purity of 90%.

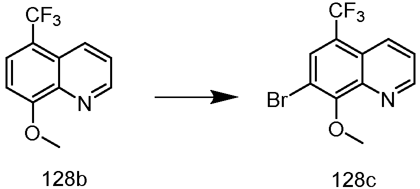

5-Trifluoromethyl-8-methoxyquinoline (Compound 128b) (3.1 g, 13.6 mmol, 1 eq) was dissolved in 30 mL of dimethylformamide, and NBS (4.84 g, 27.2 mmol, 2 eq) was added. The reaction was carried out for 3 hours at room temperature. The reaction solution was diluted with 50 mL of water, extracted with 50 mL of dichloromethane and separated into two phases. The organic phase was concentrated, and the crude product was purified by silica gel column chromatography (eluent: PE:EA=10:1 to 0:1) to obtain 1.2 g of 5-trifluoromethyl-7-bromo-8-methoxyquinoline (Compound 128c) as a light red liquid with a purity of 90%.

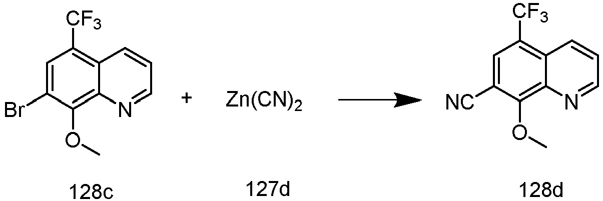

5-Trifluoromethyl-7-bromo-8-methoxyquinoline (Compound 128c) (1.2 g, 3.92 mmol, 1 eq) was dissolved in 20 mL of anhydrous dimethylformamide, and zinc cyanide (Compound 127d) (917.3 mg, 7.84 mmol, 2 eq) and tetrakis(triphenylphosphine)palladium (226 mg, 0.196 mmol, 0.05 eq) were added. The reaction system was purged with nitrogen, and the reaction was carried out for 8 hours at 110°C. The reaction solution was diluted with 50 mL of water, and extracted with dichloromethane (25 mL×2). The organic phases were combined, dried over anhydrous sodium sulfate, filtered, and concentrated to obtain 900 mg of crude 5-trifluoromethyl-7-cyano-8-methoxyquinoline (Compound 128d).

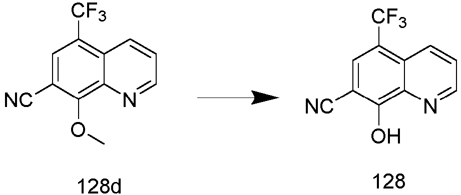

5-Trifluoromethyl-7-cyano-8-methoxyquinoline (Compound 128d) (900 mg, 3.58 mmol, 1 eq) was dissolved in 10 mL of dimethylformamide, and 767 mg of lithium chloride was added. The reaction was carried out for 2 hours at 80°C, and 50 mL of water was added. The pH was adjusted to neutral, and the reaction solution was extracted with dichloromethane (25 mL×2). The organic phases were combined, and concentrated to obtain a crude product, which was purified by silica gel column chromatography (eluent: PE:EA=10:1 to 0:1) to obtain 220 mg of a product (80% purity), which was further purified by pre-HPLC to obtain 32 mg of 5-trifluoromethyl-7-cyano-8-hydroxyquinoline (Compound 128) as a white solid with a purity of 96%.

MS calculated: 238.04; MS found: 239.0 [M+H]⁺.

¹H NMR (400 MHz, DMSO-d6): δ 9.13 (d, J=4Hz,1H), 8.50 (d, J=8Hz,1H), 8.22 (s, 1H), 7.99 (dd, J=4Hz, J=8Hz,1H).

### Example 129

### Synthesis of 4-methyl-7-cyano-8-hydroxyquinoline (129)

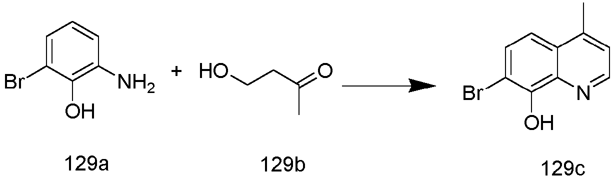

2-Amino-6-bromophenol (Compound 129a) (5 g, 26.6 mmol, 1 eq) was dissolved in 100 mL of 1 M aqueous hydrochloric acid solution, and heated to reflux. 4-Hydroxy-2-butanone (Compound 129b) (7 g, 79.8 mmol, 3 eq) was added dropwise, and the reaction was carried out for 2 hours. The reaction solution was cooled, and the pH was adjusted to neutral with aqueous sodium carbonate solution. The reaction solution was extracted with ethyl acetate (50 mL×2), and concentrated to obtain 7 g of crude 4-methyl-7-bromo-8-hydroxyquinoline (Compound 129c) as a red solid.

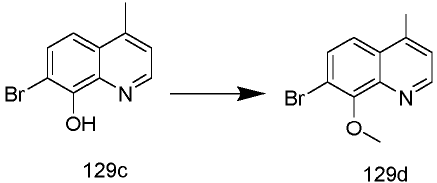

4-Methyl-7-bromo-8-hydroxyquinoline (Compound 129c) (7 g, 29.2 mmol, 1 eq) was dissolved in 70 mL of dimethylformamide solution, and potassium carbonate (6 g, 43.8 mmol, 1.5 eq) and methyl iodide (6.3 g, 43.8 mmol, 1.5 eq) were added. The reaction was carried out for 3 hours at room temperature. The reaction solution was diluted with 150 mL of water, extracted with ethyl acetate (50 mL×2) and separated into two phases. The organic phases were combined, dried over anhydrous sodium sulfate, filtered, and concentrated to obtain a crude product, which was purified by silica gel column chromatography (eluent: PE/EA=10:1 to 1:1) to obtain 2 g of a product 4-methyl-7-bromo-8-methoxyquinoline (Compound 129d) as a red liquid.

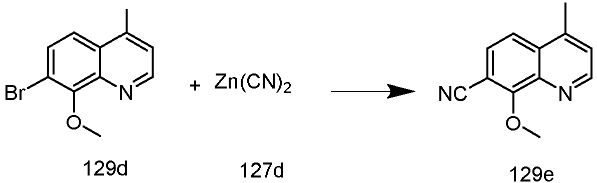

4-Methyl-7-bromo-8-methoxyquinoline (Compound 129d) (2 g, 7.88 mmol, 1 eq), zinc cyanide (Compound 127d) (900 mg, 3.94 mmol, 0.5 eq), Pd₂(dba)₃ (226 mg, 0.394 mmol, 0.05 eq) and DPPF (220 mg, 0.394 mmol, 0.05 eq) were dissolved in 20 mL of dimethylformamide. The reaction system was purged with nitrogen, and the reaction was carried out for 10 hours at 100°C. The reaction solution was diluted with 50 mL of water, extracted with ethyl acetate (20 mL×2) and separated into two phases. The organic phases were combined, dried over anhydrous sodium sulfate, filtered, and the filtrate was concentrated to obtain a crude product, which was purified by silica gel column chromatography (eluent: PE:EA=10:1 to 1:1) to obtain 800 mg of 4-methyl-7-cyano-8-methoxyquinoline (Compound 129e) as a red oil with a purity of 80%.

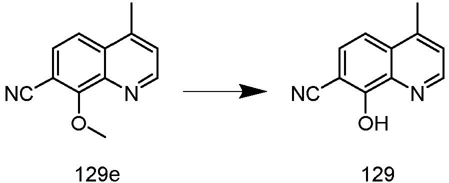

4-Methyl-7-cyano-8-methoxyquinoline (Compound 129e) (800 mg, 4 mmol, 1 eq) was dissolved in 10 mL of dimethylformamide, and 200 mg of lithium chloride was added. The reaction was carried out for 2 hours at 100°C, and 50 mL of water was added. The pH was adjusted to neutral. A solid was precipitated, filtered and dried to obtain a crude product, which was triturated twice with 20 mL of methanol/15 mL of dichloromethane to obtain 23.7 mg of 4-methyl-7-cyano-8-hydroxyquinoline (Compound 129) as a white gray solid with a purity of 98%.

MS calculated: 184.06; MS found: 185.1 [M+H]⁺.

¹H NMR (400 MHz, DMSO-d6): δ8.86 (d,J=8Hz,1H), 7.69 (d,J=12Hz,1H),7.63 (dd, J=4Hz, 1H), 7.58 (dd, J=8Hz,1H),2.71(s,3H).

### Example 130

### Synthesis of 2-fluoro-7-cyano-8-hydroxyquinoline (130)

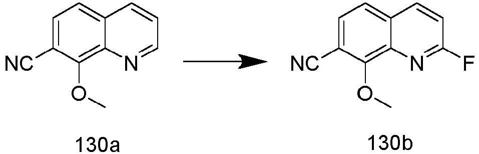

7-Cyano-8-methoxyquinoline (Compound 130a) (150 mg, 0.815 mmol, 1 eq) was dissolved in 17 mL of anhydrous acetonitrile, and silver difluoride (708 mg, 4.8 mmol, 6 eq) was added. The reaction was carried out for 1 hour at room temperature. The reaction solution was filtered, and the filtrate was concentrated to obtain 100 mg of crude 2-fluoro-7-cyano-8-methoxyquinoline (Compound 130b).

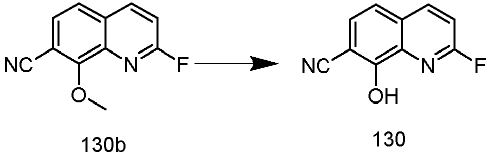

2-Fluoro-7-cyano-8-methoxyquinoline (Compound 130b) (100 mg, 0.54 mmol, 1 eq) was dissolved in 5 mL of dimethylformamide, and 228 mg of lithium chloride was added. The reaction was carried out for 2 hours at 80°C. The reaction solution was concentrated to obtain a crude product, which was purified by pre-HPLC to obtain 12 mg of 2-fluoro-7-cyano-8-hydroxyquinoline (Compound 130) as a yellow green solid with a purity of 96%.

MS calculated: 188.0; MS found: 189.1 [M+H]⁺.

¹H NMR (400 MHz, DMSO-d6): δ8.30~8.37 (m,1H), 7.70~7.75 (m,1H), 7.63 (dd, J=4Hz, J=8Hz,1H), 7.37 (dd, J=8Hz,1H).

### Example 131

### Synthesis of 4-methyl-5-chloro-7-cyano-8-hydroxyquinoline (131)

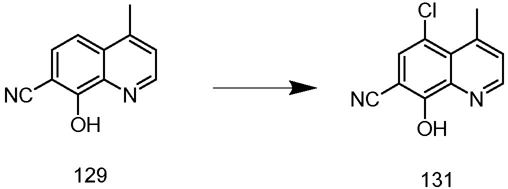

4-Methyl-7-cyano-8-hydroxyquinoline (Compound 129) (300 mg, 1.62 mmol, 1 eq) was dissolved in 10 mL of dimethylformamide, and NCS (425 mg, 3.2 mmol, 2 eq) was added. The reaction was carried out for 30 minutes at room temperature. The reaction solution was diluted with 30 mL of water, and extracted with dichloromethane (15 mL×2). The organic phases were combined, dried, filtered, and the filtrate was concentrated to obtain a crude product, which was purified by pre-HPLC to obtain 155 mg of 4-methyl-5-bromo-7-cyano-8-hydroxyquinoline (Compound 131) as a light yellow solid with a purity of 98%.

MS calculated: 218.02; MS found: 219.1 [M+H]⁺.

¹H NMR (400 MHz, DMSO-d6): δ8.85 (d, J=4Hz,1H), 7.84 (s, 1H), 7.66 (d, J=8Hz, 1H), 3.01(s,3H).

### Example 132

### Synthesis of 4-methyl-5-bromo-7-cyano-8-hydroxyquinoline (132)

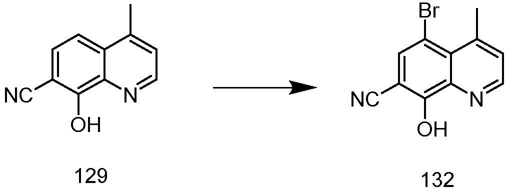

4-Methyl-7-cyano-8-hydroxyquinoline (Compound 129) (300 mg, 1.62 mmol, 1 eq) was dissolved in 10 mLof dimethylformamide, and NBS (569 mg, 3.2 mmol, 2 eq) was added. The reaction was carried out for 30 minutes at room temperature. The reaction solution was diluted with 50 mL of water, and extracted with dichloromethane (15 mL×2). The organic phases were combined, dried, filtered, and the filtrate was concentrated to obtain a crude product, which was triturated with 30 mL of methanol/5 mL of dichloromethane to obtain 105 mg of 4-methyl-5-bromo-7-cyano-8-hydroxyquinoline (Compound 132) as a light yellow solid with a purity of 97%.

MS calculated: 261.97, 263.97; MS found: 263.0, 265.0 [M+H]⁺.

¹H NMR (400 MHz, DMSO-d6): δ8.85 (d, J=4Hz,1H), 8.06 (s, 1H), 7.69 (d, J=8Hz, 1H), 3.01(s,3H).

### Example 133

### Synthesis of 5,6-dichloro-7-cyano-8-hydroxyquinoline (133)

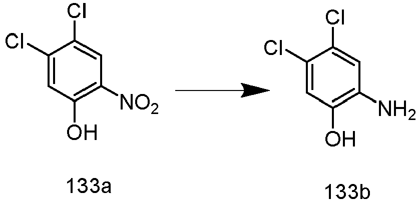

5 g of 2-nitro-4,5-dichlorophenol (Compound 133a) was dissolved in 50 mL of tetrahydrofuran, and 500 mg of wet palladium on carbon was added. The reaction system was equipped with a hydrogen balloon, and the reaction was carried out for 8 hours. The reaction solution was filtered, and the filtrate was concentrated to obtain 4.7 g of a product 2-amino-4,5-dichlorophenol (Compound 133b).

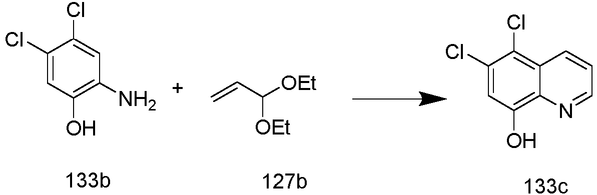

2-Amino-4,5-dichlorophenol (Compound 133b) (4.7 g, 26.5 mmol, 1 eq) was dissolved in 100 mL of 1 M aqueous hydrochloric acid solution, and heated to reflux. Acrolein diethyl acetal (Compound 127b) (10 g, 79.7 mmol, 3 eq) was added dropwise, and the reaction was carried out for 1 hour. The reaction solution was cooled, the pH was adjusted to neutral with aqueous sodium carbonate solution, and the reaction solution was extracted with dichloromethane (30 mL×2). The organic phases were combined and concentrated to obtain 3.5 g of crude 5,6-dichloro-8-hydroxyquinoline (Compound 133c) as a black solid.

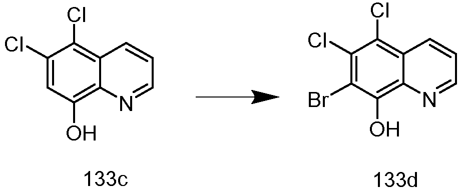

5,6-Dichloro-8-hydroxyquinoline (Compound 133c) (3.5 g, 16.3 mmol, 1 eq) was dissolved in 50 mL of dimethylformamide, and NBS (2.9 g, 16.3 mmol, 1.0 eq) was added. The reaction was carried out for 2 hours at room temperature. The reaction solution was diluted with 100 mL of water, extracted with dichloromethane (50 mL×2) and separated into two phases. The organic phases were combined, dried, filtered and concentrated to obtain 4 g of crude 5,6-dichloro-7-bromo-8-hydroxyquinoline (Compound 133d) as a yellow solid.

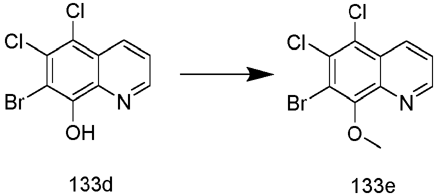

5,6-Dichloro-7-bromo-8-hydroxyquinoline (Compound 133d) (4 g, 13.7 mmol, 1 eq) was dissolved in 40 mL of dimethylformamide solution, and potassium carbonate (1.89 g, 13.6 mmol, 1 eq) and methyl iodide (1.94 g, 13.6 mmol, 1 eq) were added. The reaction was carried out for 3 hours at room temperature. The reaction solution was diluted with 150 mL of water, extracted with dichloromethane (50 mL×2) and separated into two phases. The organic phases were combined, dried over anhydrous sodium sulfate, filtered, and the filtrate was concentrated to obtain a crude product, which was purified by silica gel column chromatography (eluent: PE/EA=10:1 to 2: 1) to obtain 400 mg of a product 5,6-dichloro-7-bromo-8-methoxyquinoline (Compound 133e) as a light yellow solid with a purity of 90%.

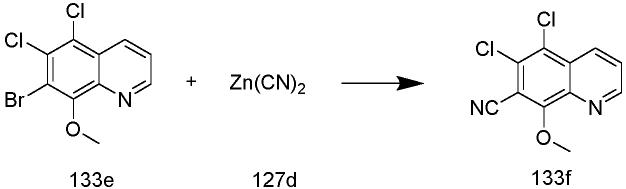

5,6-Dichloro-7-bromo-8-methoxyquinoline (Compound 133e) (400 mg, 1.31 mmol, 1 eq), zinc cyanide (Compound 127d) (75 mg, 0.65 mmol, 0.5 eq), Pd₂(dba)₃ (75 mg, 0.13 mmol, 0.1 eq) and DPPF (72 mg, 0.13 mmol, 0.1 eq) were dissolved in 10 mL of dimethylformamide. The reaction system was purged with nitrogen, and the reaction was carried out for 8 hours at 90°C. The reaction solution was diluted with 50 mL of water, extracted with ethyl acetate (30 mL×2) and separated into two phases. The organic phases were combined, dried over anhydrous sodium sulfate, filtered, and the filtrate was concentrated to obtain a crude product, which was purified by silica gel column chromatography (eluent: PE:EA=10:1 to 1:1) to obtain 200 mg of crude 5,6-dichloro-7-cyano-8-methoxyquinoline (Compound 133f) as a white solid.

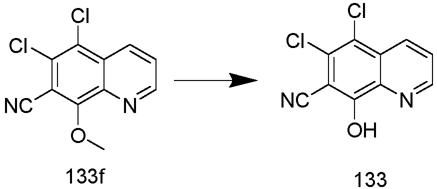

5,6-Dichloro-7-cyano-8-methoxyquinoline (Compound 133f) (200 mg, 0.8 mmol, 1eq) was dissolved in 3 mL of dimethylformamide, and 100 mg of lithium chloride was added. The reaction was carried out for 1 hour at 100°C, and the reaction solution was concentrated to obtain a crude product, which was purified by pre-HPLC to obtain 36 mg of a product 5,6-dichloro-7-cyano-8-hydroxyquinoline (Compound 133) as a white solid with a purity of 98%.

MS calculated: 237.97; MS found: 239.0 [M+H]⁺.

¹H NMR (400 MHz, DMSO-d6): δ9.10(d,J=4Hz, 1H),8.62 (d,J=8Hz,1H), 7.97 (dd,J=4Hz,J=8Hz,1H).

### Example 134

### Synthesis of 7-cyano-8-hydroxyquinoline-5-carboxylic acid (134)

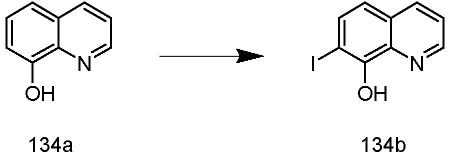

8-Hydroxyquinoline (Compound 134a) (10 g, 68.6 mmol, 1 eq) and NIS (18.6 g, 82.6 mmol, 1.2 eq) were dissolved in 200 mL of chloroform. The reaction was carried out for 20 hours at 40°C. The reaction solution was filtered, and the filtrate was washed twice with 100 mL of 5% aqueous sodium thiosulfate solution and separated into two phases. The organic phases were concentrated to obtain a crude product, which was triturated with 50 mL of methanol/50 mL of water, filtered, and the filter cake was dried to obtain 12 g of a product 7-iodo-8-hydroxyquinoline (Compound 134b).

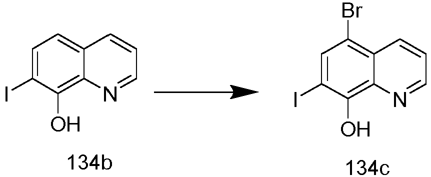

7-Iodo-8-hydroxyquinoline (Compound 134b) (12 g, 44.3 mmol, 1 eq) was dissolved in 180 mL of dimethylformamide, and NBS (9.4 g, 55.3 mmol, 1.2 eq) was added. The reaction was carried out for 1 hour at room temperature. The resulting mixture was filtered, and the filter cake was washed with water and dried to obtain 13 g of crude 5-bromo-7-iodo-8-hydroxyquinoline (Compound 134c) as a yellow solid.

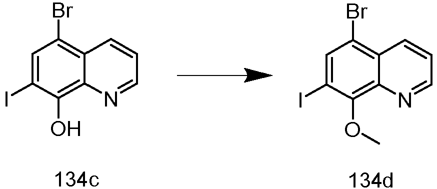

5-Bromo-7-iodo-8-hydroxyquinoline (Compound 134c) (13 g, 37.2 mmol, 1 eq) was dissolved in 150 mL of dimethylformamide solution, and potassium carbonate (7.7 g, 55.8 mmol, 1.5 eq) and methyl iodide (7.7 g, 44.7 mmol, 1.2 eq) were added. The reaction was carried out for 2 hours at room temperature. The reaction solution was diluted with 200 mL of water, extracted with dichloromethane (50 mL×2) and separated into two phases. The organic phases were combined, dried over anhydrous sodium sulfate, filtered, and the filtrate was concentrated to obtain a crude product, which was purified twice by silica gel column chromatography (eluent: PE/EA=10:1 to 1:1) to obtain 3.7 g of a product 5-bromo-7-iodo-8-methoxyquinoline (Compound 134d) as light yellow solid.

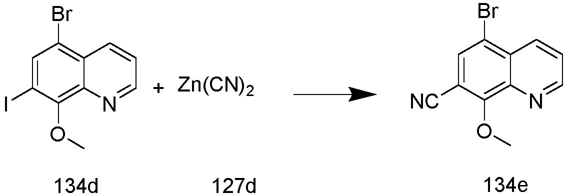

5-Bromo-7-iodo-8-methoxyquinoline (Compound 134d) (3.7 g, 10.2 mmol, 1 eq), zinc cyanide (Compound 127d) (599 mg, 5.1 mmol, 0.5 eq), Pd₂(dba)₃ (281 mg, 0.51 mmol, 0.05 eq) and DPPF (276 mg, 0.51 mmol, 0.05 eq) were dissolved in 40 mL of dimethylformamide. The reaction system was purged with nitrogen, and the reaction was carried out for 10 hours at 100°C. The reaction solution was diluted with 50 mL of water, extracted with ethyl acetate (20 mL×2) and separated into two phases. The organic phases were combined, dried over anhydrous sodium sulfate, filtered, and the filtrate was concentrated to obtain a crude product, which was purified by silica gel column chromatography (eluent: PE:EA=10:1 to 1:1) to obtain 1.4 g of a product 5-bromo-7-cyano-8-methoxyquinoline (Compound 134e) as a white solid with a purity of 90%.

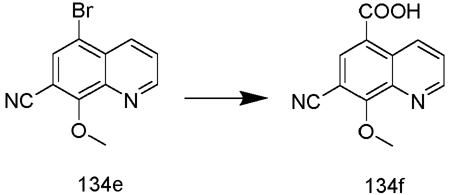

Compound 134e (200 mg, 0.763 mmol, 1 eq) was dissolved in 5 mL of anhydrous tetrahydrofuran. The reaction system was purged with nitrogen. The reaction solution was cooled to -78°C, and n-butyllithium (1 M, 1 mmol, 1.3 eq) was added and stirred for 10 minutes. Small piece of dry ice was added, and the reaction was carried out for 5 minutes, and quenched by the addition of 2 mL of water. The reaction solution was concentrated to obtain 250 mg of crude 7-cyano-8-methoxyquinoline-5-carboxylic acid (Compound 134f).

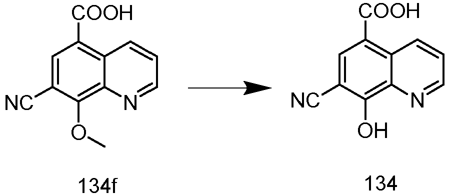

7-Cyano-8-methoxyquinoline-5-carboxylic acid (Compound 134f) (200 mg, 0.93 mmol, 1 eq) was dissolved in 3 mL of dimethylformamide, and 100 mg of lithium chloride was added. The reaction was carried out for 2 hours at 100°C, and the reaction solution was concentrated to obtain a crude product, which was purified by pre-HPLC to obtain 40 mg of a product 7-cyano-8-hydroxyquinoline-5-carboxylic acid (Compound 134) as a yellow solid with a purity of 98%.

MS calculated: 214.18; MS found: 215.1 MS [M+H]⁺.

¹H NMR (400 MHz, DMSO-d6): δ 10.1 (s, 1H), 9.58 (d,J=4Hz,1H),9.27 (d,J=4Hz,1H), 7.86(dd, J=4Hz, J=8Hz,1H), 7.71 (s,1H).

### Example 135

Synthesis of 5-chloro-7-cyano-8-hydroxyquinoline (135) 5-Chloro-7-cyano-8-hydroxyquinoline (Compound 135) was obtained by the same reaction steps as in Example 127 except that NBS in Example 127 was replaced by NCS at an equivalent molar ratio.

MS calculated: 184.06; MS found: 185.1 [M+H]⁺.

¹H NMR (400 MHz, DMSO-d6): δ 8.67 (d, J= 4.3 Hz, 1H), 8.34 (dd, J = 8.4, 1.5 Hz), 7.68 (dd, 8.4, 4.3 Hz, 1H), 7.44 (s, 1H).

### Effect Examples

In the following Effect Examples, the name and Latin name of each virus are as follows:
*Human Papillomavirus 11, HPV11*;
*Human Papillomavirus 16, HPV16*;
*Human Papillomavirus 18, HPV18*,
the "positive control drug" is 9-[2-(phosphonylmethoxy)ethyl]guanine.

### Effect Example 1: Activity of 7-nitro-8-hydroxyquinoline derivatives against human papillomavirus

The antiviral activity of 7-nitro-8-hydroxyquinoline derivatives against human papillomavirus (*HPV*) was tested and evaluated using transient transfection assays. This evaluation system included plasmids expressing viral helicase E1 and replication origin binding protein E2, plasmids containing NanoLuc gene of which the expression was driven by a TK promoter (Promega company), and replication origin sequences of human papillomavirus strains listed below. Compared with using E1 or E2 alone, the synergistic binding and unwinding activity of E1 and E2 expressed through the plasmids drove the replication of the reporter plasmid, resulting in an increase in NanoLuc activity by more than 100 folds. The positions of replication origin sequences of the three NanoLuc reporter plasmids were 7825-7993:1-99, 7662-7901:1-99, and 7740-7837:1-102 for HPV-11 (NCBI Accession No. HE611260.1), HPV-16 (NCBI Accession No. KP212151.1), and HPV-18 (NCBI Accession No. KC470230.1), respectively. The positions of E1 open reading frame (ORF) sequences corresponding to HPV-11, HPV-16 and HPV-18 were 832-2781, 864-2813 and 914-2887 in the same sequences, respectively. The positions of E2 open reading frame (ORF) sequences corresponding to HPV-11, HPV-16 and HPV-18 were 2723-3826, 2755-3852 and 2817-3908 in the same sequences, respectively. The cells used for transient transfection were C-33A cells (ATCC HTB-31), and the culture medium was DMEM supplemented with Earle's salt, L-glutamine and 2% fetal bovine serum. In the experiment, the NanoLuc expression plasmid (5 ng/well) was co-transfected with the E1 and E2 expression plasmids of the homologous virus by using Lipofectamine LTXL and Plus Reagent (Invitrogen product, Thermo Fisher Scientific company). After incubation for 1 hour, the transfected cells were seeded into wells containing 5-fold diluted test drugs and the positive control drug 9-[2-(phosphonylmethoxy)ethyl]guanine (PMEG) (Sigma-Aldrich company). Meanwhile, the transfected cells were also seeded into duplicated dishes containing the same dilution of test drugs to assess cytotoxicity. All transfected monolayer cells were incubated at 37°C for 48 hours. The NanoLuc activity expressed by the reporter plasmids was detected with Nano-Glo reagent (Promega company), the cell viability in cytotoxicity plates was measured with CellTiter-Glo reagent (Promega company), and the luminescence intensity was quantified on a microplate reader. The meanings of EC₅₀, CC₅₀, and SI₅₀ were the same as those well-known in the art (where SI₅₀=CC₅₀/EC₅₀), and were not described here.

The details for the operation method were described with reference to the following references:
Beadle, J. et al. Synthesis and Antiviral Evaluation of Octadecyloxyethyl Benzyl 9-[(2-Phosphonomethoxy)ethyl]guanine (ODE-Bn-PMEG), a Potent Inhibitor of Transient HPV DNA Amplification. J Med Chem. 2016; 59 (23): 10470-8.

Kachaeva, M. et al. In vitro activity of novel 1,3-oxazole derivatives against HPV. Ibnosina J Med Biomed Sci. 2017; 9 (4): 111-8.

The specific test results are shown in Table 1. For the three subtypes *HPV11*, *HPV16* and *HPV18*, the aforementioned positive control drug was used for comparison at the time of testing, respectively. The same applies to Tables 2 and 3 and will not be described again.

**Table 1: Data on activity of 7-nitro-8-hydroxyquinoline derivatives against HPV**

| Compound No. | Virus name | Virus strain | Cell line | Concentration range | EC₅₀ (µM) | CC₅₀ (µM) | SI₅₀ |
|---|---|---|---|---|---|---|---|
| Positive control drug | *HPV11* | HE611260.1 | C-33 A | 0.048-150 µM | 1.26 | >150.00 | >119 |
| 62 | *HPV11* | HE611260.1 | C-33 A | 0.048-150 µM | >1.20 | 5.5 | <5 |
| 64 | *HPV11* | HE611260.1 | C-33 A | 0.048-150 µM | <0.05 | 25.1 | >523 |
| 73 | *HPV11* | HE611260.1 | C-33 A | 0.048-150 µM | 0.06 | 8.5 | 137 |
| 115 | *HPV11* | HE611260.1 | C-33 A | 0.048-150 µM | >30.00 | 112.04 | <4 |
| 123 | *HPV11* | HE611260.1 | C-33 A | 0.048-150 µM | >6.00 | 29.79 | <5 |
| 7 | *HPV11* | HE611260.1 | C-33 A | 0.048-150 µM | 5.22 | 19.06 | 4 |
| 114 | *HPV11* | HE611260.1 | C-33 A | 0.048-150 µM | >6.00 | 30 | <5 |
| 74 | *HPV11* | HE611260.1 | C-33 A | 0.048-150 µM | 0.57 | 34.36 | 60 |
| 32 | *HPV11* | HE611260.1 | C-33 A | 0.048-150 µM | <0.05 | 5.37 | >112 |
| 29 | *HPV11* | HE611260.1 | C-33 A | 0.048-150 µM | 2.96 | 8.74 | 3 |
| 36 | *HPV11* | HE611260.1 | C-33 A | 0.048-150 µM | <0.05 | 12.04 | >251 |
| Positive control drug | *HPV16* | KP212151.1 | C-33 A | 0.048-150 µM | 2.06 | >150.00 | >73 |
| 64 | *HPV16* | KP212151.1 | C-33 A | 0.0006-100 µM | 0.01 | 7.62 | 762 |
| 73 | *HPV16* | KP212151.1 | C-33 A | 0.0006-100 µM | 0.07 | 8.32 | 119 |
| 32 | *HPV16* | KP212151.1 | C-33 A | 0.048-150 µM | 0.08 | 17.9 | 218 |
| 74 | *HPV16* | KP212151.1 | C-33 A | 0.048-150 µM | 0.15 | 56.47 | 369 |
| 36 | *HPV16* | KP212151.1 | C-33 A | 0.001-250 µM | 0.69 | 27.2 | 39 |
| Positive control drug | *HPV18* | KC470230.1 | C-33 A | 0.048-150 µM | 2.22 | >150.00 | >68 |
| 32 | *HPV18* | KC470230.1 | C-33 A | 0.048-150 µM | <0.05 | 11.78 | >245 |
| 74 | *HPV18* | KC470230.1 | C-33 A | 0.048-150 µM | <0.05 | 61.03 | >1271 |
| 36 | *HPV18* | KC470230.1 | C-33 A | 0.001-250 µM | 0.27 | 20.13 | 75 |

### Effect Example 2: Activity of 5-nitro-8-hydroxyquinoline derivatives against human papillomavirus

The activity of the compounds in Examples of WO2022028321A1 against human papillomavirus was tested by using the test method in Effect Example 1. The results are shown in Table 2, in which the "Example No." is the same as that in WO2022028321A1.

**Table 2: Data on activity of 5-nitro-8-hydroxyquinoline derivatives against HPV**

| Example No. | Virus name | Virus strain | Cell line | Concentration range | EC₅₀ (µM) | CC₅₀ (µM) | SI₅₀ |
|---|---|---|---|---|---|---|---|
| Positive control drug | *HPV11* | HE611260.1 | C-33 A | 0.048-150 µM | 1.26 | >150.00 | >119 |
| 19 | *HPV11* | HE611260.1 | C-33 A | 0.048-150 µM | 0.52 | 4 | 8 |
| 32 | *HPV11* | HE611260.1 | C-33 A | 0.048-150 µM | 3.99 | >150.00 | >38 |
| 25 | *HPV11* | HE611260.1 | C-33 A | 0.048-150 µM | <0.05 | 3.36 | >70 |
| 26 | *HPV11* | HE611260.1 | C-33 A | 0.048-150 µM | <0.05 | 4.78 | >100 |
| 21 | *HPV11* | HE611260.1 | C-33 A | 0.048-150 µM | >6.00 | 22.17 | <4 |
| Positive control drug | *HPV16* | KP212151.1 | C-33 A | 0.048-150 µM | 2.06 | >150.00 | >73 |
| 26 | *HPV16* | KP212151.1 | C-33 A | 0.048-150 µM | 1.07 | 69.11 | 65 |
| 25 | *HPV16* | KP212151.1 | C-33 A | 0.001-250 µM | 0.09 | 7.53 | 84 |
| Positive control drug | *HPV18* | KC470230.1 | C-33 A | 0.048-150 µM | 2.22 | >150.00 | >68 |
| 26 | *HPV18* | KC470230.1 | C-33 A | 0.048-150 µM | 0.23 | 71.76 | 319 |
| 25 | *HPV18* | KC470230.1 | C-33 A | 0.001-250 µM | 0.1 | 5.88 | 59 |

### Effect Example 3: Data on activity of 7-cyano-8-hydroxyquinoline derivatives against HPV

The 7-cyano-8-hydroxyquinoline derivatives were tested by using the test method in Effect Example 1. The results are shown in Table 3, in which Compound A1 is 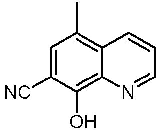 and synthesized according to the reference (Fiedler, H. (1960). Synthese von Methyl-8-hydroxy-chinolin-aldehyden. Archiv Der Pharmazie, 293(6), 609-621. Doi:10.1002/ardp.19602930606).

**Table 3: Data on activity of 7-cyano-8-hydroxyquinoline derivatives against HPV**

| Compound No. | Virus name | Virus strain | Cell line | Concentration range | EC₅₀ (µM) | CC₅₀ (µM) | SI₅₀ |
|---|---|---|---|---|---|---|---|
| Positive control drug | *HPV11* | HE611260.1 | C-33 A | 0.048-150 µM | 1.26 | >150.00 | >119 |
| 135 | *HPV11* | HE611260.1 | C-33 A | 0.048-150 µM | 9.27 | 50.73 | 5 |
| 133 | *HPV11* | HE611260.1 | C-33 A | 0.048-150 µM | >1.20 | 5.59 | <5 |
| 131 | *HPV11* | HE611260.1 | C-33 A | 0.048-150 µM | 0.53 | 5.28 | 10 |
| A1 | *HPV11* | HE611260.1 | C-33 A | 0.048-150 µM | >6.00 | 20.2 | <3 |
| Positive control drug | *HPV16* | KP212151.1 | C-33 A | 0.048-150 µM | 2.06 | >150.00 | >73 |
| 131 | *HPV16* | KP212151.1 | C-33 A | 0.048-150 µM | 0.55 | 13.33 | 24 |
| Positive control drug | *HPV18* | KC470230.1 | C-33 A | 0.048-150 µM | 2.22 | >150.00 | >68 |
| 131 | *HPV18* | KC470230.1 | C-33 A | 0.048-150 µM | 0.14 | 12.17 | 85 |

As a comparison, the antiviral activity of 5-nitro-8-hydroxyquinoline ( 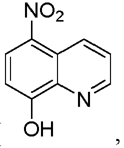 hereinafter referred to as "A2") was also tested in the present invention. The results are shown in Tables 4 and 5.

**Table 4: Data on activity of the positive control drug against HPV**

| Virus name | Virus strain | Cell line | Positive control drug | | |
|---|---|---|---|---|---|
| | | | Concentration range | EC₅₀/ µM | EC₉₀/µM |
| *HPV11* | HE611260.1 | C-33 A | 0.048-150 µM | 1.03 | 4.47 |
| *HPV16* | KP212151.1 | C-33 A | 0.001-250 µM | 0.28 | 2.3 |
| *HPV18* | KC470230.1 | C-33 A | 0.048-150 µM | 0.63 | >150.00 |

**Table 5: Data on activity of Compound A2 against HPV**

| | Virus strain | Cell line | Concentration of Compound A2 | EC₅₀/ µM | CC₅₀/µM | SI₅₀ |
|---|---|---|---|---|---|---|
| *HPV11* | HE611260.1 | C-33 A | 0.048-150 µM | 1.04 | 55.49 | 53 |
| *HPV16* | KP212151.1 | C-33 A | 0.048-150 µM | 2.82 | 66.33 | 23 |
| *HPV18* | KC470230.1 | C-33 A | 0.048-150 µM | 1.01 | 64.96 | 64 |

## Claims

1. Use of a compound of formula (I), a pharmaceutically acceptable salt thereof, a crystal form thereof, a solvate thereof, an isotope derivative thereof, or a prodrug thereof in the preparation of an antiviral drug, 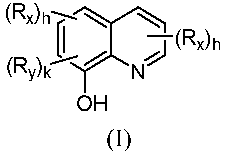 wherein:
Rₓ is each independently optional, and h is each independently 1, 2 or 3;
R_{y} is selected from the group consisting of nitro and cyano, and k is 1 or 2;
provided that not all Rₓ are H atoms simultaneously.

2. The use according to claim 1, which is a use of a compound of formula (IA), a pharmaceutically acceptable salt thereof, a crystal form thereof, a solvate thereof, an isotope derivative thereof, or a prodrug thereof in the preparation of an antiviral drug, 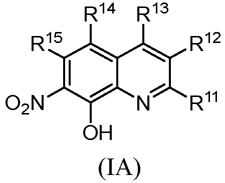 wherein:
R¹¹ is selected from the group consisting of H atom, halogen, D atom and C₁₋₆ alkyl;
R¹² is selected from the group consisting of H atom, C₁₋₆ alkyl, C₃₋₈ cycloalkyl, C₆₋₁₄ aryl, -C(O)-OR¹⁶, -CH₂-NR¹⁷R¹⁸ and -CH₂-NR¹⁹R¹¹⁰;
R¹³ is selected from the group consisting of H atom, D atom, C₁₋₆ alkyl, halogen, C₁₋₆ alkoxy, hydroxy, haloC₁₋₆ alkyl, haloC₁₋₆ alkoxy, cyano, C₃₋₈ cycloalkyl, -(CH₂)ₙ-3 to 8 membered heterocyclyl, C₆₋₁₄ aryl, 5 to 14 membered heteroaryl, -NR¹⁷R¹⁸, -O-(CH₂)ₙ-NR¹⁷R¹⁸, -O-(CH₂)ₙ-R¹¹¹, -(CH=CH)ₘC(O)-NR¹⁷R¹⁸ and -(CH=CH)ₘC(O)-NR¹⁹R¹¹⁰, wherein the 3 to 8 membered heterocyclyl is optionally further substituted by one or more substituents selected from the group consisting of C₁₋₆ alkyl, C₁₋₆ alkoxy, halogen, haloC₁₋₆ alkyl, oxo, -C(O)-NR¹⁷R¹⁸, -C(O)-OR¹⁶, -C(O)-R¹⁶, -S(O)ₓR¹⁶, -O-(CH₂)ₙ-R¹¹¹ and C₆₋₁₄ aryl;
R¹⁴ is selected from the group consisting of H atom, halogen, cyano, C₁₋₆ alkyl, C₃₋₈ cycloalkyl, haloC₁₋₆ alkyl, hydroxyC₁₋₆ alkyl, -CH₂-NR¹⁷R¹⁸ and -CH₂-NR¹⁹R¹¹⁰;
R¹⁵ is selected from the group consisting of H atom, D atom, halogen, C₁₋₆ alkyl and -NR¹⁷R¹⁸;
R¹⁶ is C₁₋₆ alkyl;
R¹⁷ and R¹⁸ are each independently selected from the group consisting of H atom, C₁₋₆ alkyl and 3 to 8 membered heterocyclyl;
R¹⁹ and R¹¹⁰ together with the nitrogen atom to which they are attached form a 3 to 8 membered nitrogen-containing heterocyclyl, the 3 to 8 membered nitrogen-containing heterocyclyl optionally comprises one or more heteroatoms selected from the group consisting of N, O and S in addition to N, and the 3 to 8 membered nitrogen-containing heterocyclyl is optionally further substituted by one or more oxo groups;
R¹¹¹ is selected from the group consisting of halogen, C₃₋₈ cycloalkyl, C₆₋₁₄ aryl, haloC₁₋₆ alkyl, C₁₋₆ alkyl and C₁₋₆ alkoxy;
m is 1;
n is 0, 1, 2 or 3; and
x is 2.

3. The use according to claim 2, wherein:
R¹¹ is selected from the group consisting of H atom, F atom, -CH₃ and D atom;
and/or, R¹² is selected from the group consisting of H atom, -CH₃, 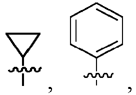 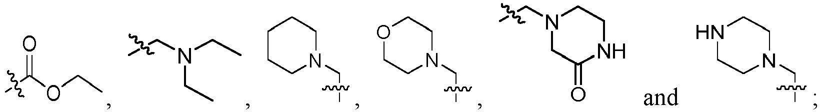
and/or, R¹³ is selected from the group consisting of H atom, -CH₃, 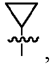 Cl atom, 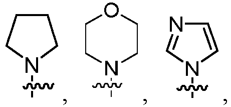 Br atom, 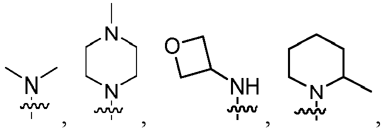 -OCH₃, 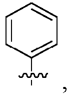 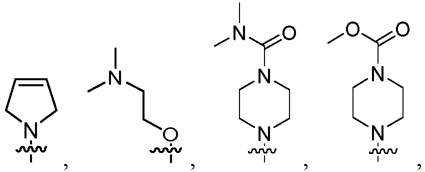 F atom, 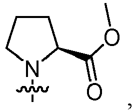 -OH, 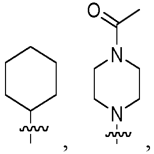 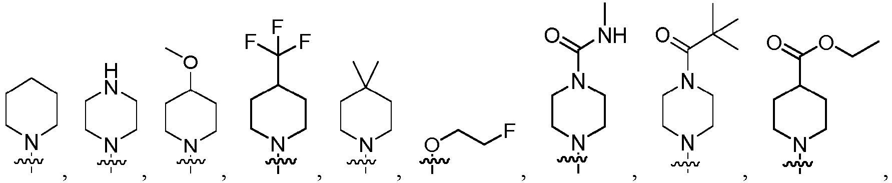 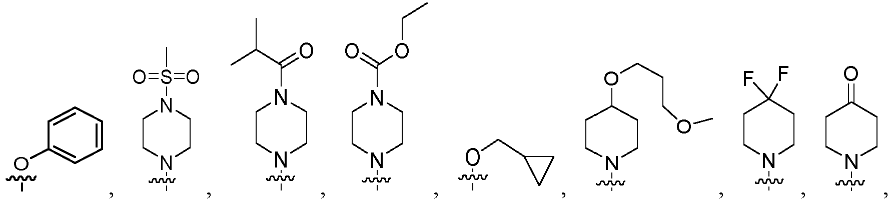 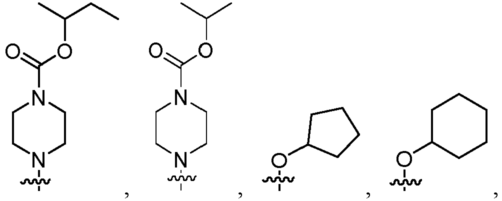 -OCH₂CH₃, -CF₃, 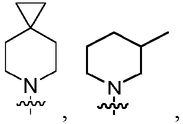 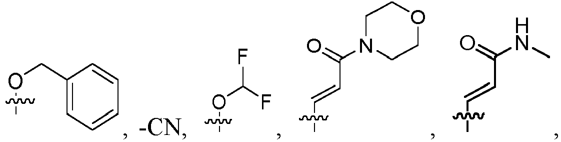 -CHF₂, 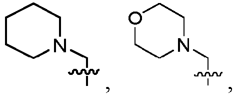 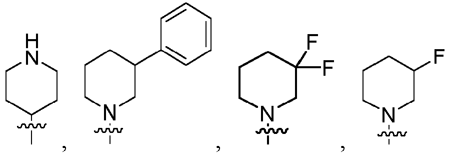 and D atom;
and/or, R¹⁴ is selected from the group consisting of H atom, I atom, -CH₃, Cl atom, -CN, F atom, 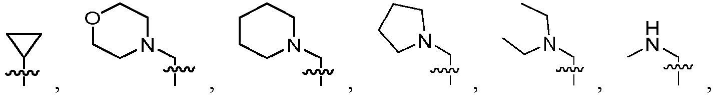 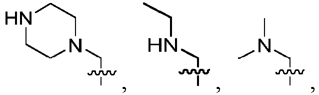 -CHF₂, -CH₂OH and Br atom;
and/or, R¹⁵ is selected from the group consisting of H atom, -CH₃, F atom, Br atom, Cl atom, D atom and -NHCH₃.

4. The use according to claim 2 or 3, wherein the compound of formula (IA) is selected from the group consisting of:

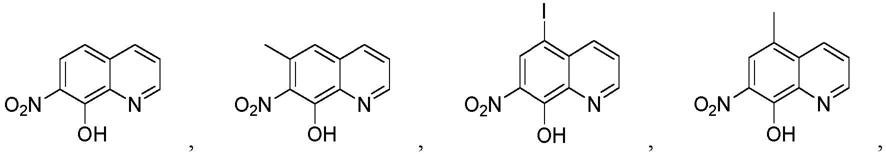

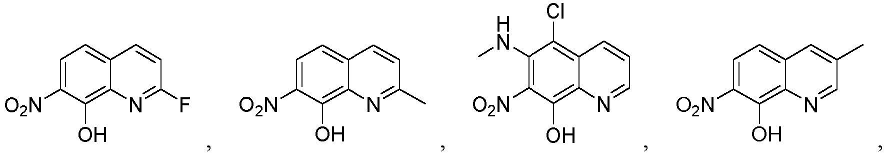

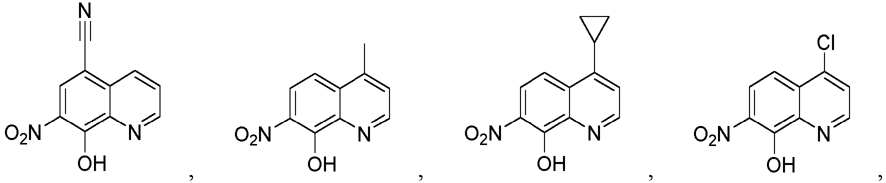

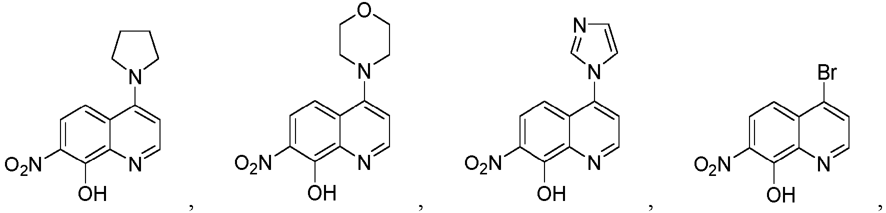

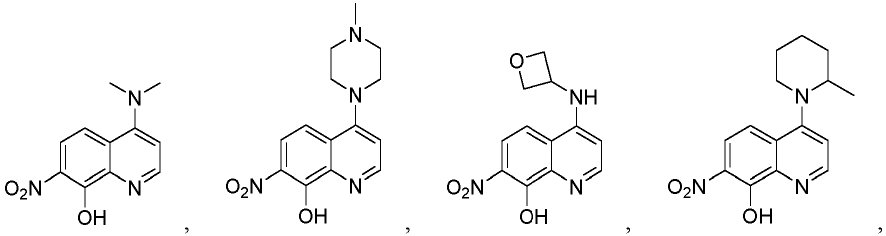

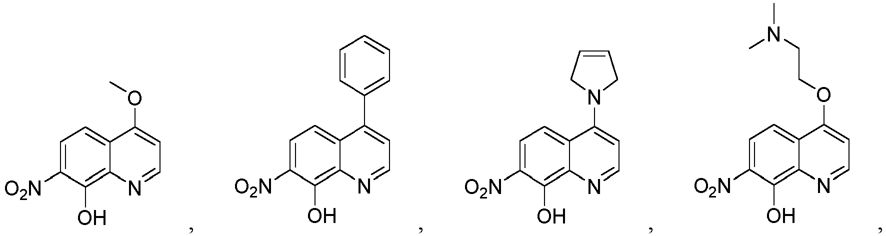

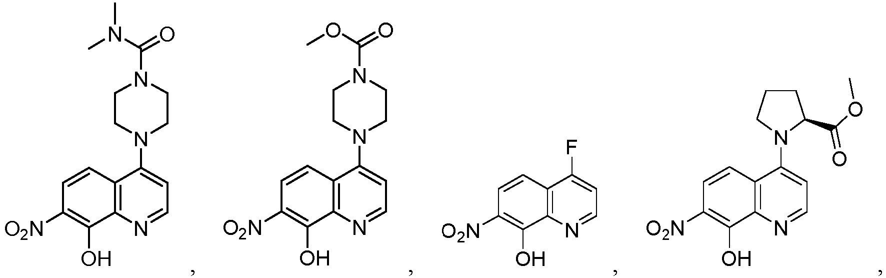

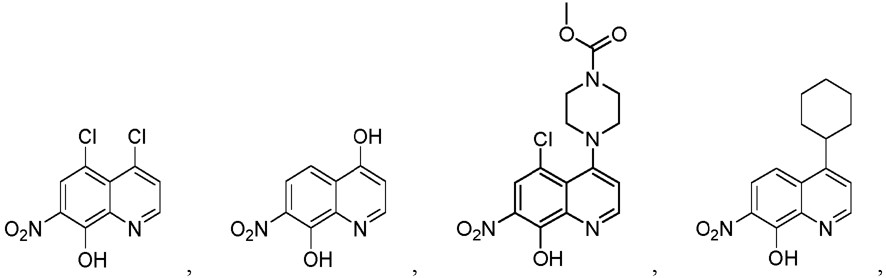

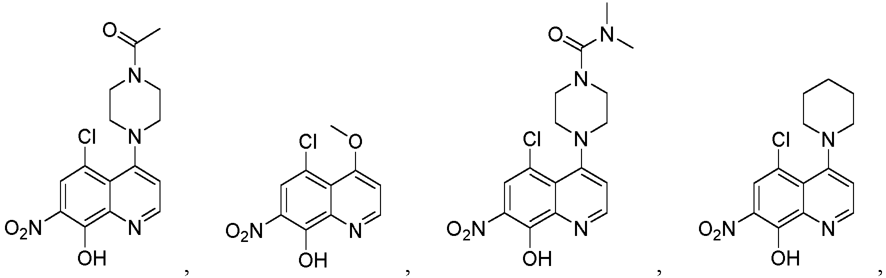

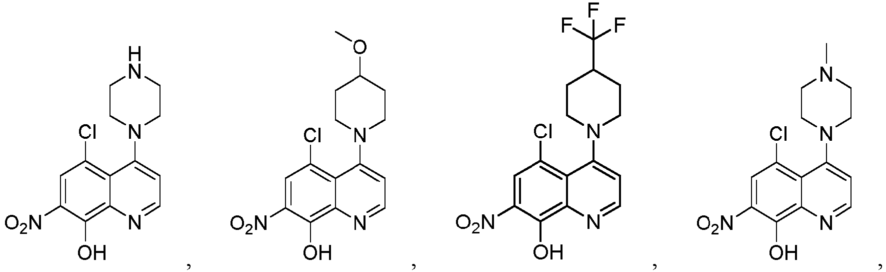

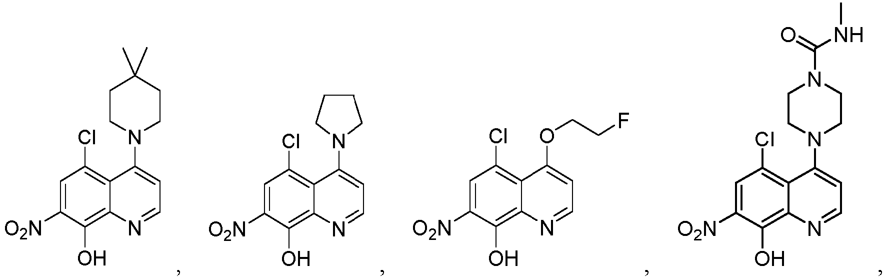

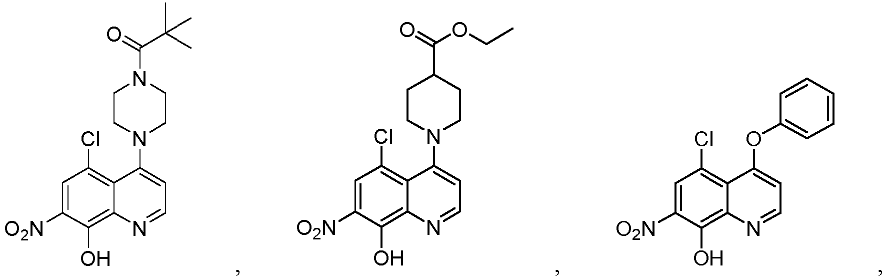

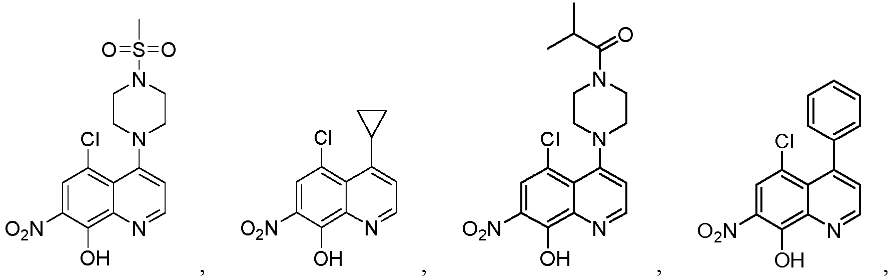

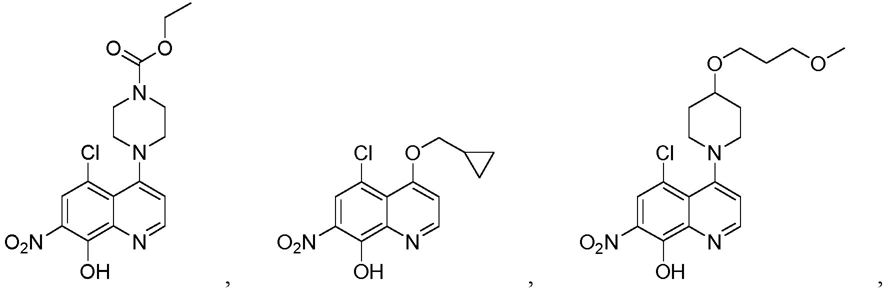

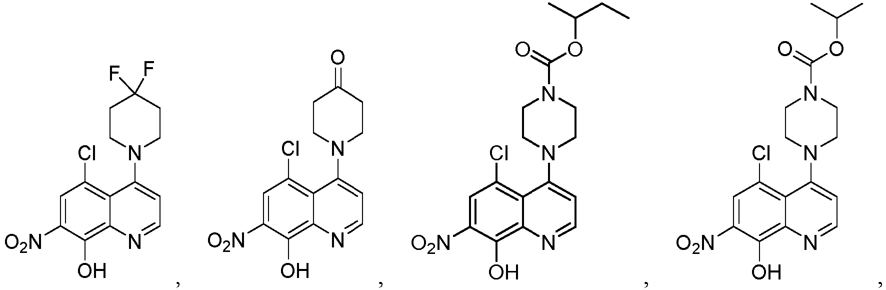

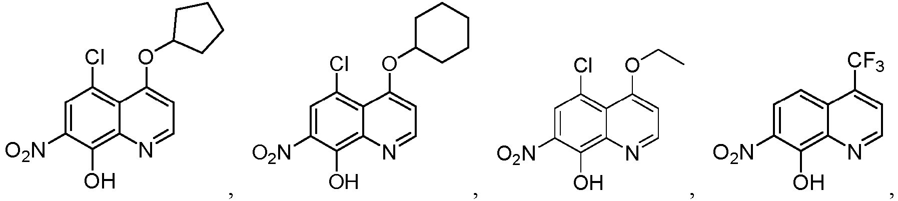

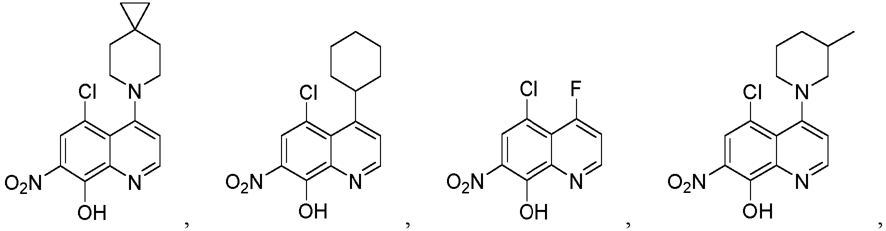

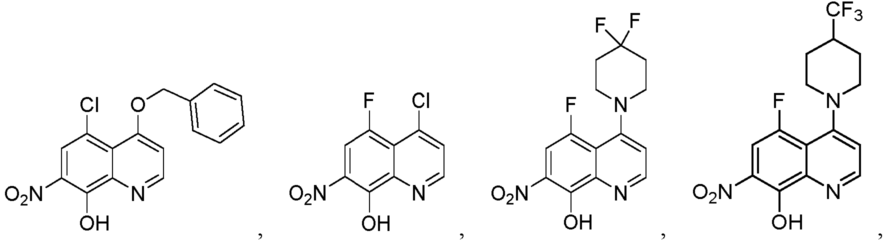

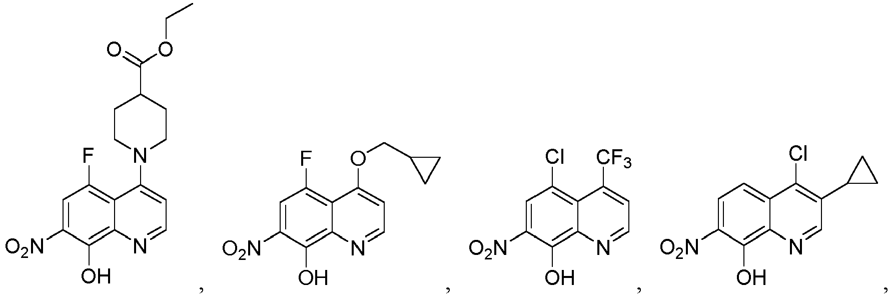

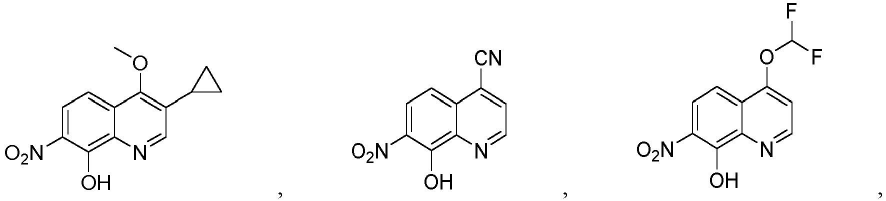

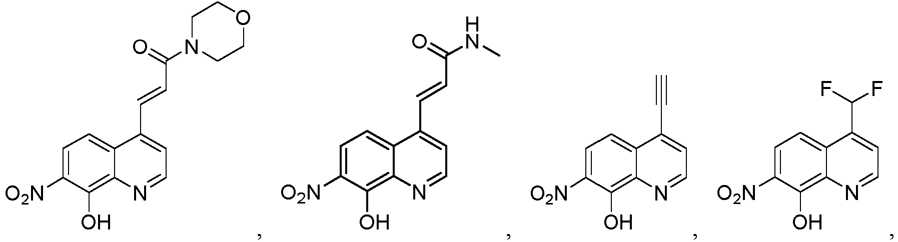

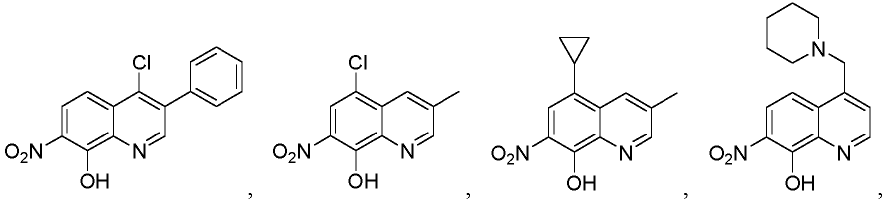

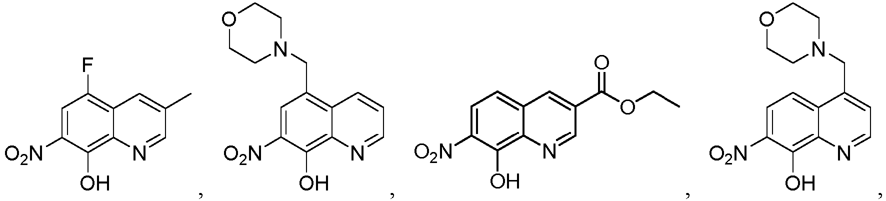

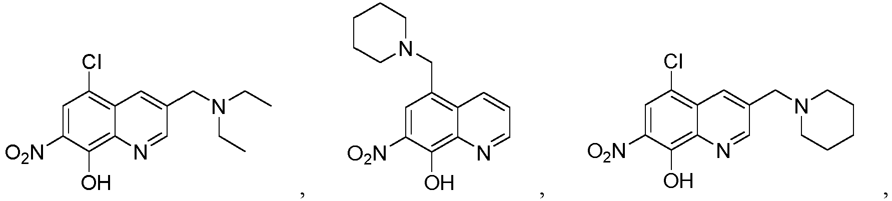

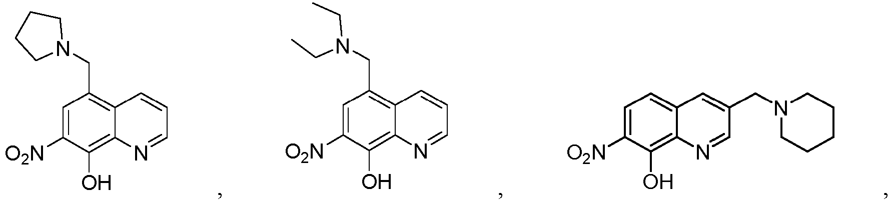

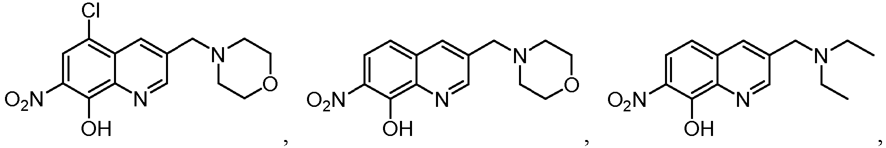

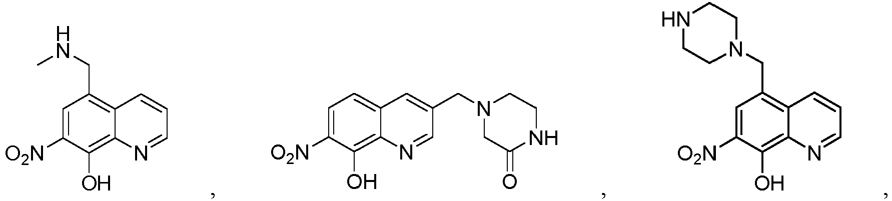

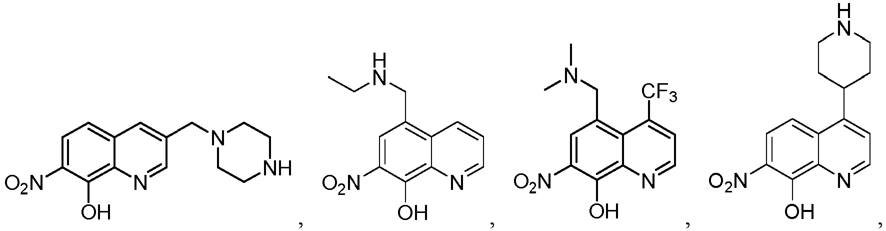

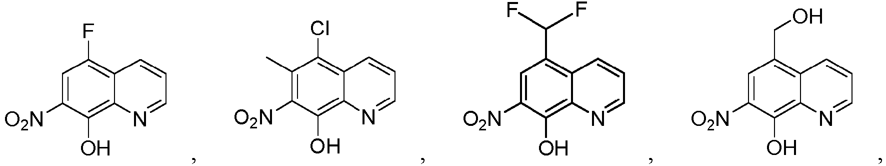

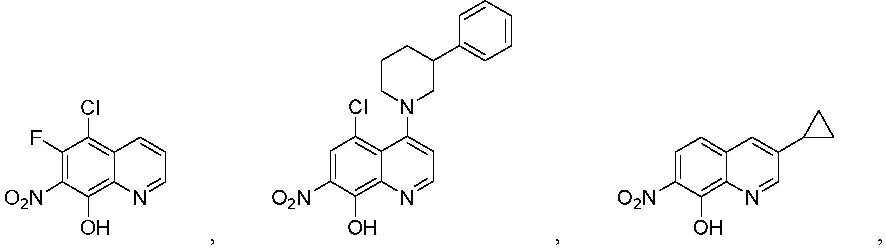

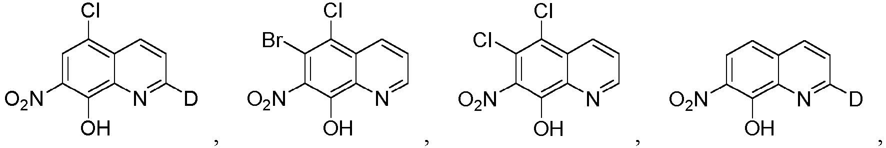

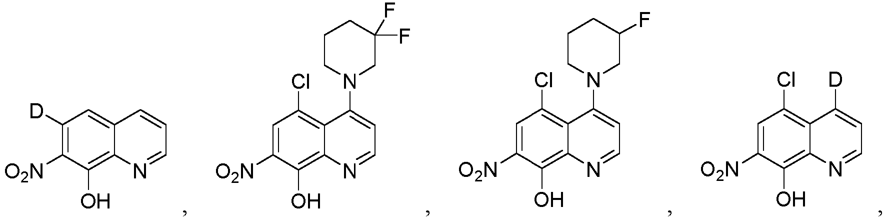

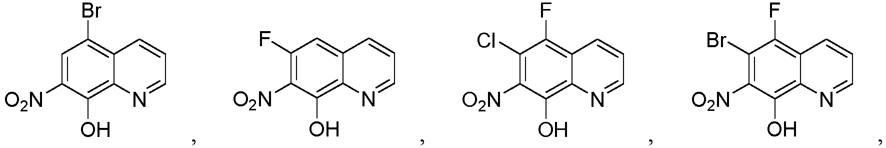

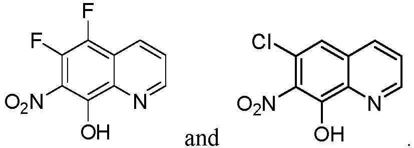

5. The use according to claim 1, wherein:
R¹¹ is selected from the group consisting of H atom, halogen, D atom and C₁-C₆ alkyl;
R¹² is H atom, C₁-C₆ alkyl or C₃₋₈ cycloalkyl;
R¹³ is selected from the group consisting of H atom, D atom, C₁-C₆ alkyl, halogen, C₁₋₆ alkoxy, hydroxy, cyano, haloC₁₋₆ alkyl and C₃₋₈ cycloalkyl;
R¹⁴ is selected from the group consisting of H atom, halogen, cyano, haloC₁₋₆ alkyl and C₁-C₆ alkyl;
R¹⁵ is selected from the group consisting of H atom, D atom, halogen, C₁-C₆ alkyl and -NR¹⁷R¹⁸;
R¹⁷ and R¹⁸ are each independently H atom or C₁₋₆ alkyl.

6. The use according to claim 5, wherein the compound of formula (IA) is selected from the group consisting of: 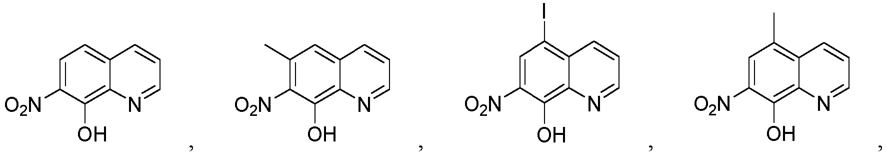 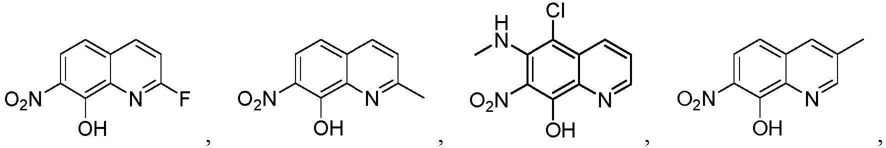 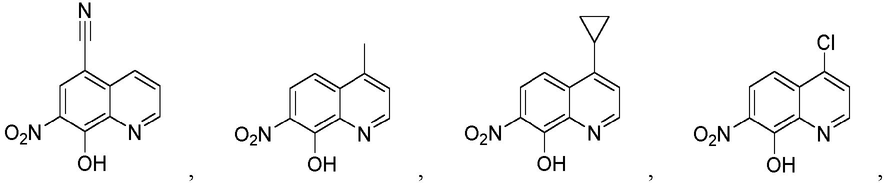 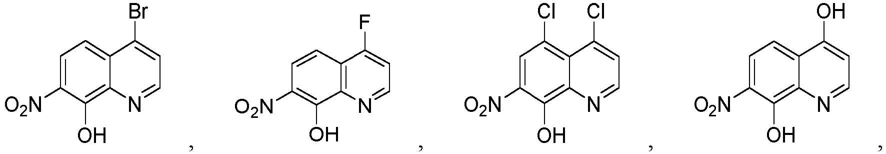 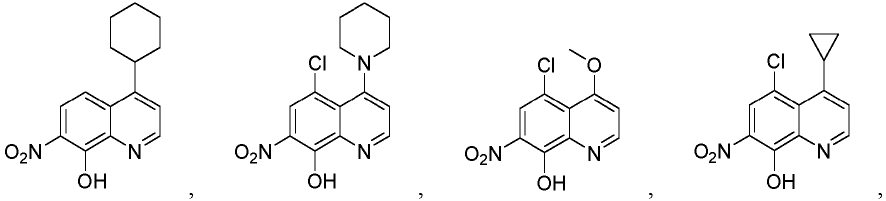 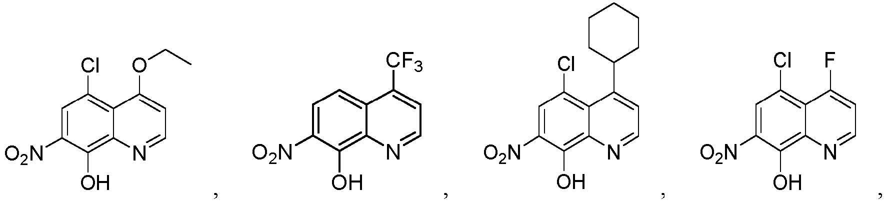 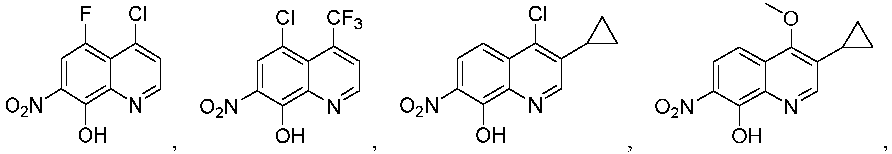 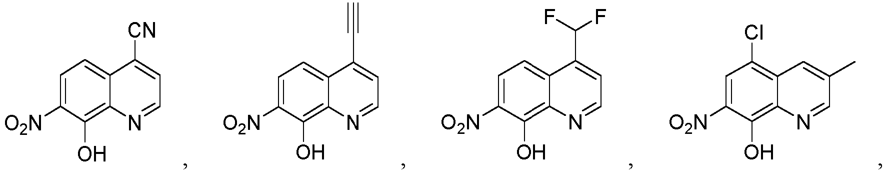 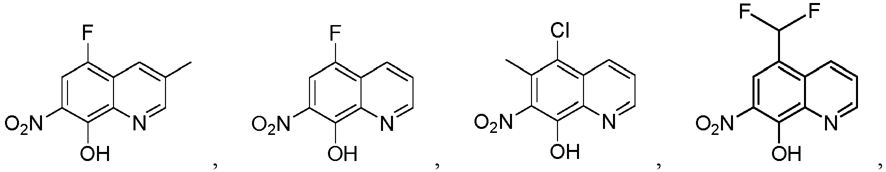 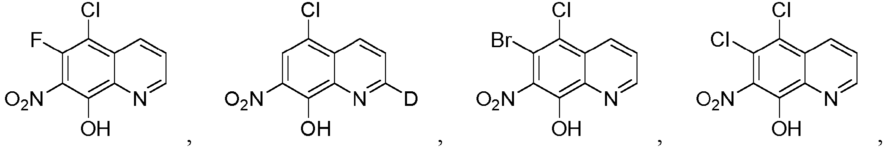 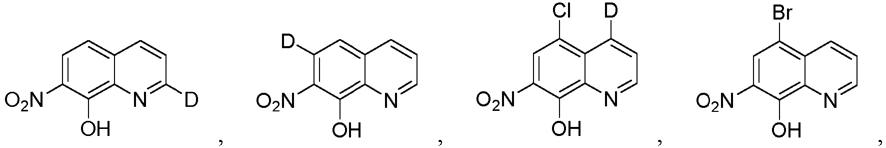 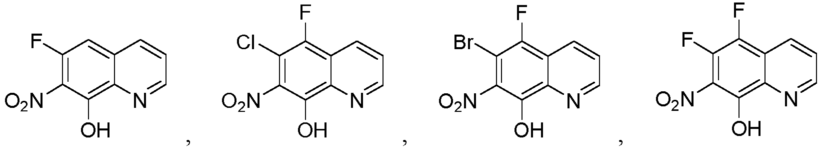 and

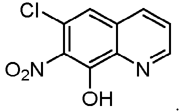

7. The use according to claim 1 or 5, wherein:
R¹¹ is H atom;
R¹² is H atom or C₃₋₈ cycloalkyl;
R¹³ is selected from the group consisting of H atom, haloC₁₋₆ alkyl and C₃₋₈ cycloalkyl;
R¹⁴ is H atom or halogen;
R¹⁵ is selected from the group consisting of H atom, halogen and -NR¹⁷R¹⁸;
R¹⁷ and R¹⁸ are each independently H atom or C₁₋₆ alkyl.

8. The use according to any one of claims 2 to 7, wherein the compound of formula (IA) is selected from the group consisting of: 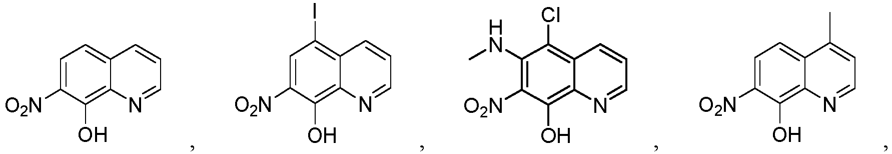 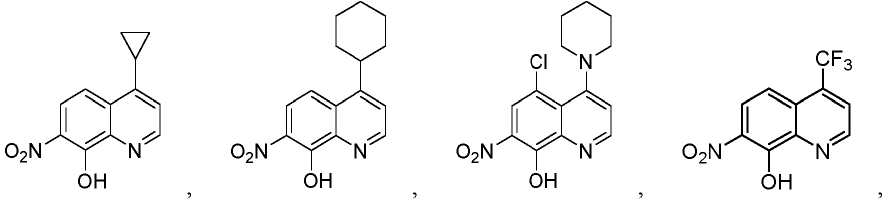 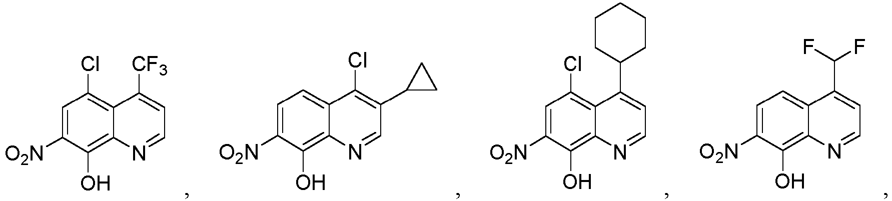 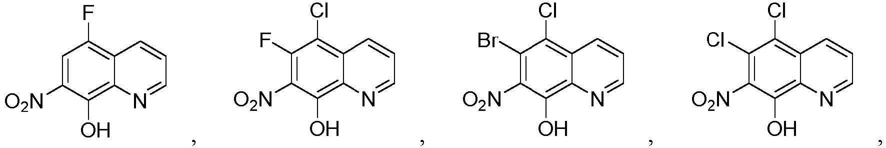 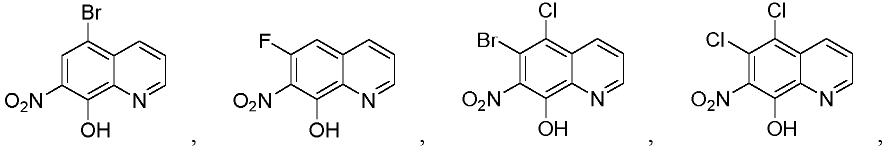 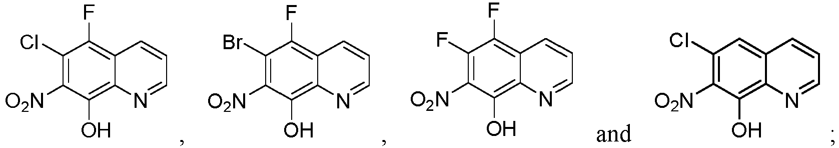 and preferably selected from the group consisting of 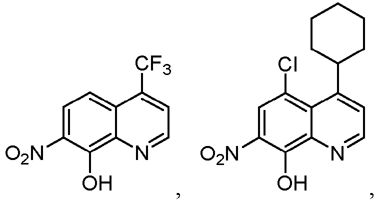 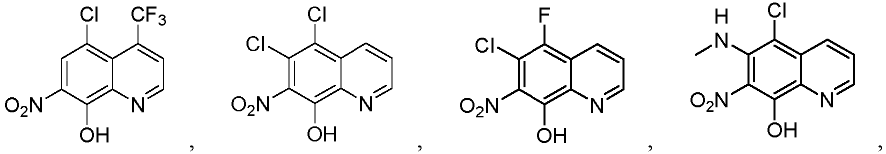 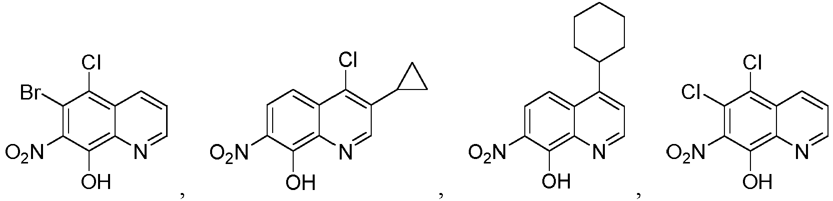 and

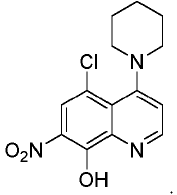

9. The use according to claim 1, which is a use of a compound of formula (IB), a pharmaceutically acceptable salt thereof, a crystal form thereof, a solvate thereof, an isotope derivative thereof, or a prodrug thereof in the preparation of an antiviral drug, 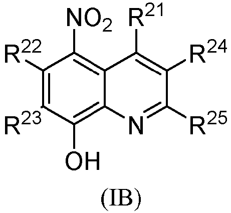 wherein:
R²¹ is selected from the group consisting of hydrogen atom, halogen, cyano, alkyl, alkenyl, cycloalkyl, heterocyclyl, aryl, heteroaryl, -C(O)OR^{a}, -NR^{a}R^{b}, -OR^{a} and -S(O)ₚR^{a}; wherein the alkyl, alkenyl, cycloalkyl, heterocyclyl, aryl and heteroaryl are each optionally further substituted by one or more groups selected from Q;
R²² is selected from the group consisting of hydrogen atom, halogen, alkyl and cycloalkyl;
R²³ is selected from the group consisting of hydrogen atom, halogen and alkyl;
R²⁴ is selected from the group consisting of hydrogen atom, halogen, alkyl, - C(O)OR^{a} and -C(O)NR^{a}R^{b}, wherein R^{a} and R^{b} are each independently selected from the group consisting of hydrogen atom, C₁-C₆ alkyl, C₃-C₆ cycloalkyl, C₃-C₆ cycloalkyl-C₁-C₆ alkyl;
R²⁵ is selected from the group consisting of hydrogen atom, halogen and alkyl;
R^{a} and R^{b} are each independently selected from the group consisting of hydrogen atom, alkyl and cycloalkyl;
Q is halogen, alkyl, oxo, -C(O)R^{c}, -C(O)OR^{c}, -C(O)NR^{c}R^{d} or -C(O)N(R^{c})(CH₂)_{q}R^{d};
R^{c} and R^{d} are each independently selected from the group consisting of hydrogen atom, alkyl, aryl and heteroaryl, wherein the alkyl, aryl and heteroaryl are each optionally further substituted by alkoxy;
or, R^{c} and R^{d} together with the nitrogen atom to which they are attached form a nitrogen-containing heterocyclyl, and the nitrogen-containing heterocyclyl optionally comprises one or more heteroatoms selected from the group consisting of N and O in addition to N;
p is 1 or 2;
q is an integer from 0 to 6;
provided that R²¹, R²², R²³, R²⁴ and R²⁵ are not hydrogen atoms simultaneously.

10. The use according to claim 9, wherein:
R²¹ is as defined in claim 9, and the halogen is chlorine or fluorine;
and/or, R²¹ is as defined in claim 9, and the alkyl is C₁-C₆ alkyl; the C₁-C₆ alkyl is preferably C₁-C₆ alkyl substituted by halogen, more preferably -CF₃, -CHF₂ or -CH₂F, and further more preferably -CF₃;
and/or, R²¹ is as defined in claim 9, and the alkenyl is C₂-C₆ alkenyl; preferably, the C₂-C₆ alkenyl is alkenyl substituted by one or more groups selected from Q, Q is -C(O)R^{c}, -C(O)OR^{c}, -C(O)NR^{c}R^{d} and -C(O)N(R^{c})(CH₂)_{q}R^{d}, R^{c} and R^{d} are as defined in claim 9; and more preferably, the C₂-C₆ alkenyl is -CH=CH-COOH, 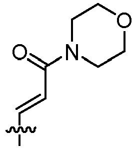 or 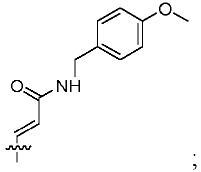
and/or, R²¹ is as defined in claim 9, and the cycloalkyl is C₃-C₁₀ cycloalkyl, preferably C₃-C₆ cycloalkyl, more preferably unsubstituted C₃-C₆ cycloalkyl, and further more preferably unsubstituted cyclopropyl, unsubstituted cyclopentyl or unsubstituted cyclohexyl;
and/or, R²¹ is as defined in claim 9, and the heterocyclyl is C₄-C₇ heterocyclyl; preferably, the C₄-C₇ heterocyclyl is nitrogen-containing C₄-C₇ heterocyclyl; more preferably, the C₄-C₇ heterocyclyl is -NR^{e}R^{f}, R^{e} and R^{f} together with the nitrogen atom to which they are attached form a nitrogen-containing heterocyclyl, the nitrogen-containing heterocyclyl optionally comprises one or more heteroatoms selected from the group consisting of N and O in addition to N, the nitrogen-containing heterocyclyl is optionally further substituted by one or more groups selected from Q, and Q is C₁-C₆ alkyl, oxo or C₁-C₆ alkoxycarbonyl, wherein the C₁-C₆ alkyl is preferably methyl, the C₁-C₆ alkoxycarbonyl is preferably -C(O)OCH₂CH₃; and further more preferably, the C₄-C₇ heterocyclyl is 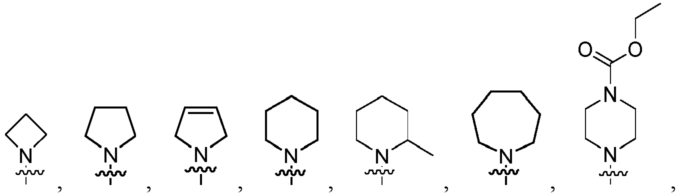 or 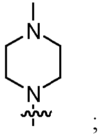
and/or, R²¹ is as defined in claim 9, and the aryl is C₆-C₁₀ aryl, and preferably unsubstituted phenyl;
and/or, R²¹ is as defined in claim 9, and the heteroaryl is 5 to 10 membered heteroaryl, preferably pyridyl, pyrazolyl or imidazolyl, unsubstituted or substituted by C₁-C₆ alkyl, and more preferably 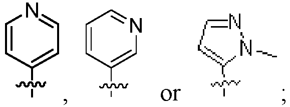
and/or, R²¹ is as defined in claim 9, and in the -C(O)OR^{c}, R^{g} is C₁-C₆ alkyl; preferably -C(O)OR^{c} is -C(O)OCH₂CH₃;
and/or, R²¹ is as defined in claim 9, and the -NR^{a}R^{b} is -NH₂ or -N(CH₃)₂;
and/or, R²¹ is as defined in claim 9, and the -OR^{a} is -OCH₃;
and/or, R²¹ is as defined in claim 9, and the -S(O)ₚR^{a} is -S(O)₂CH₃;
and/or, R²² is as defined in claim 9, and the halogen is fluorine or chlorine;
and/or, R²² is as defined in claim 9, and the alkyl is C₁-C₆ alkyl, and preferably methyl;
and/or, R²² is as defined in claim 9, and the cycloalkyl is C₁-C₆ cycloalkyl, and preferably cyclopropyl;
and/or, R²³ is as defined in claim 9, and the halogen is fluorine or chlorine;
and/or, R²³ is as defined in claim 9, and the alkyl is C₁-C₆ alkyl, and preferably methyl;
and/or, R²⁴ is as defined in claim 9, and the halogen is fluorine or chlorine;
and/or, R²⁴ is as defined in claim 9, and the alkyl is C₁-C₆ alkyl, and preferably methyl;
and/or, R²⁴ is as defined in claim 9, and R^{a} in the -C(O)OR^{a} is alkyl or cycloalkyl, the alkyl is C₁-C₆ alkyl, and preferably ethyl; and the cycloalkyl is C₁-C₆ cycloalkyl, and preferably methylcyclopropyl or cyclohexyl;
and/or, R²⁴ is as defined in claim 9, and R^{a} and R^{b} in the -C(O)NR^{a}R^{b} are each independently alkyl, and the alkyl is C₁-C₆ alkyl, and preferably ethyl, methyl or isopropyl;
and/or, R²⁵ is as defined in claim 9, and the halogen is fluorine or chlorine;
and/or, R²⁵ is as defined in claim 9, and the alkyl is C₁-C₆ alkyl, and preferably methyl;
provided that R²¹, R²², R²³, R²⁴ and R²⁵ are not hydrogen atoms simultaneously.

11. The use according to claim 9 or 10, wherein the compound of formula (IB) is selected from the group consisting of:

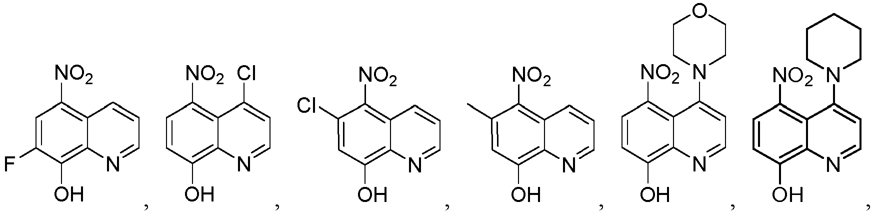

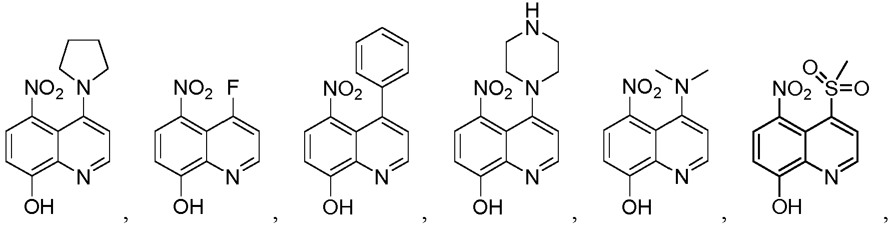

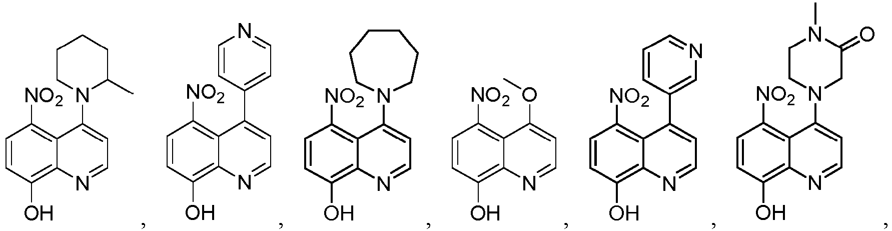

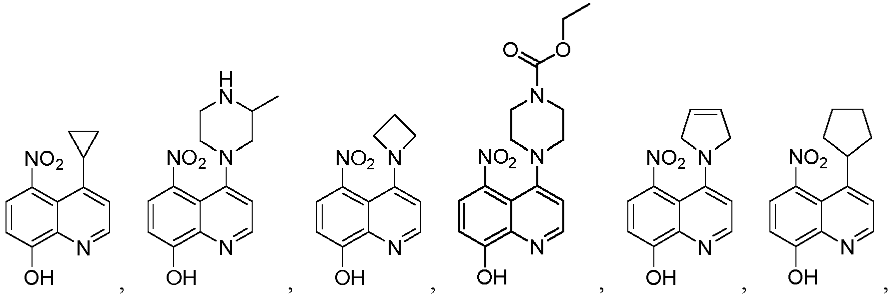

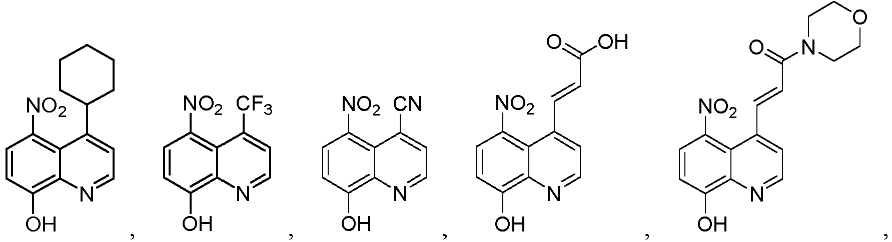

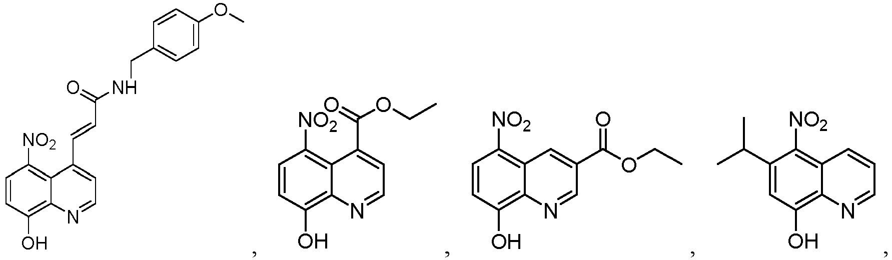

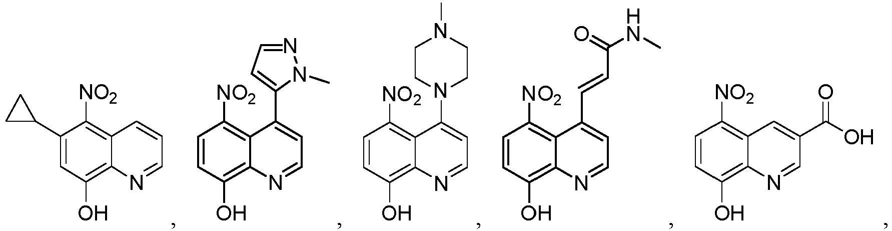

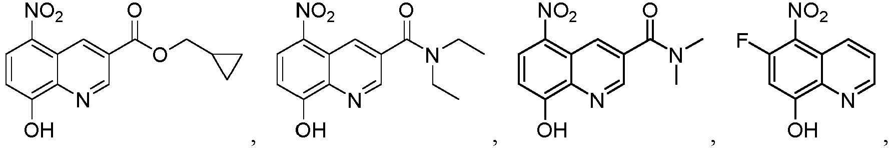

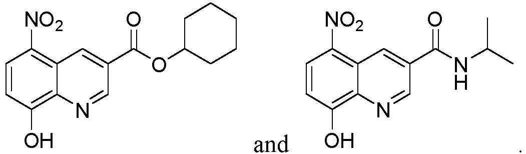

12. The use according to claim 9, wherein:
R²¹ is selected from the group consisting of hydrogen atom, halogen, cyano, C₁-C₆ alkyl, 4 to 8 membered heterocyclyl, haloC₁-C₆ alkyl, -C(O)OR^{a} and C₃-C₆ cycloalkyl;
R²² is selected from the group consisting of hydrogen atom, halogen and C₁-C₆ alkyl;
R²³ is selected from the group consisting of hydrogen atom, halogen and C₁-C₆ alkyl;
R²⁴ is selected from the group consisting of hydrogen atom, halogen, C₁-C₆ alkyl and -C(O)OR^{a}, wherein R^{a} is selected from the group consisting of hydrogen atom, C₁-C₆ alkyl and C₃-C₆ cycloalkyl; and
R²⁵ is selected from the group consisting of hydrogen atom, halogen and C₁-C₆ alkyl;
provided that R²¹, R²², R²³, R²⁴ and R²⁵ are not hydrogen atoms simultaneously.

13. The use according to claim 12, wherein the compound of formula (IB) is selected from the group consisting of:

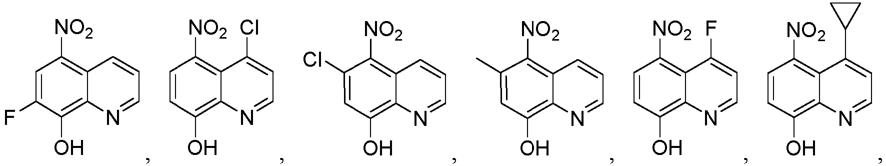

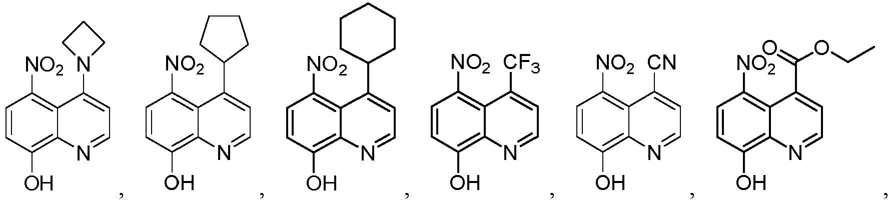

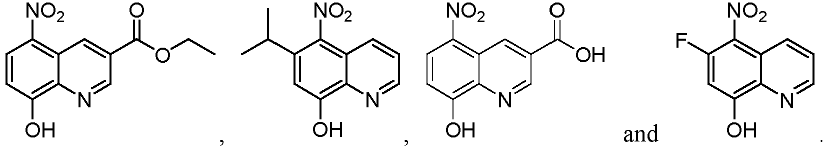

14. The use according to claim 9 or 12, wherein:
R²¹ is selected from the group consisting of hydrogen atom, 4 to 8 membered heterocyclyl, haloC₁-C₆ alkyl, -C(O)OR^{a} and C₃-C₆ cycloalkyl;
R²² is hydrogen atom;
R²³ is hydrogen atom;
R²⁴ is hydrogen atom or -C(O)OR^{a};
R²⁵ is hydrogen atom;
wherein R^{a} is hydrogen atom or C₁-C₆ alkyl.

15. The use according to any one of claims 9 to 14, wherein the compound of formula (IB) is selected from the group consisting of

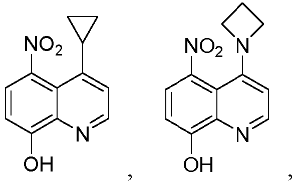

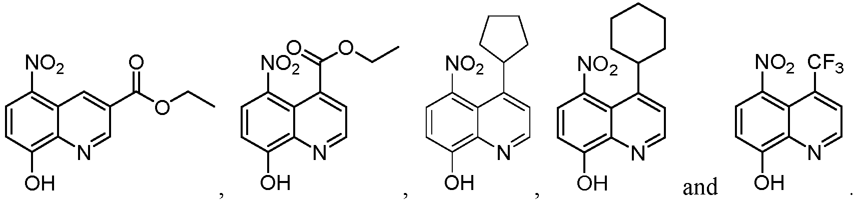

16. The use according to claim 1, wherein the compound is a compound of formula (IC), a stereoisomer thereof or a pharmaceutically acceptable salt thereof: 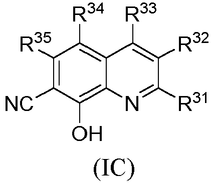 wherein:
R³¹ is selected from the group consisting of hydrogen atom, halogen, alkyl, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
R³² is selected from the group consisting of hydrogen atom, halogen, alkyl, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
R³³ is selected from the group consisting of hydrogen atom, alkyl, halogen, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
R³⁴ is selected from the group consisting of hydrogen atom, halogen, alkyl, haloalkyl, carboxy, hydroxy, hydroxyalkyl, alkoxy, haloalkoxy, amino, thiol, alkenyl, alkynyl, cycloalkyl, heterocyclyl, aryl and heteroaryl;
R³⁵ is selected from the group consisting of hydrogen atom, halogen, alkyl, hydroxy, haloalkyl, hydroxyalkyl, alkoxy, haloalkoxy, cyano, amino, thiol, nitro, carboxy, alkenyl, alkynyl, cycloalkyl, heterocyclyl, aryl and heteroaryl;
provided that R³¹, R³², R³³, R³⁴ and R³⁵ are not hydrogen atoms simultaneously.

17. The use according to claim 16, wherein R³⁴ is selected from the group consisting of hydrogen atom, halogen, C₁₋₆ alkyl, haloC₁₋₆ alkyl, carboxy and hydroxy, preferably selected from the group consisting of hydrogen atom, halogen, C₁₋₆ alkyl, haloC₁₋₆ alkyl and carboxy, and more preferably selected from the group consisting of hydrogen atom, bromine atom, chlorine atom, methyl, trifluoromethyl and carboxy.

18. The use according to claim 16 or 17, wherein R³³ is selected from the group consisting of hydrogen atom, C₁₋₆ alkyl, halogen and hydroxy, preferably hydrogen atom or C₁₋₆ alkyl, and more preferably hydrogen atom or methyl.

19. The use according to any one of claims 16 to 18, wherein the compound of formula (IC) is selected from the group consisting of: 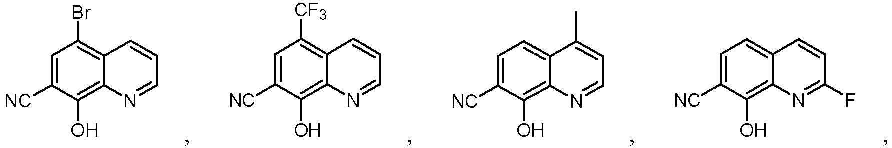 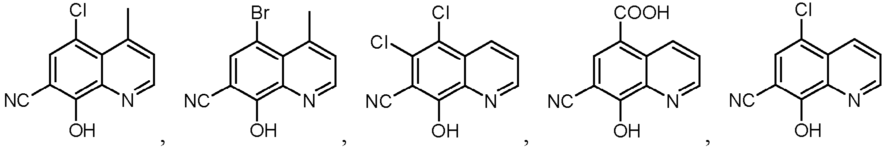 and 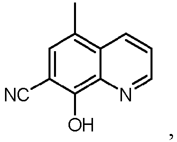 and preferably selected from the group consisting of

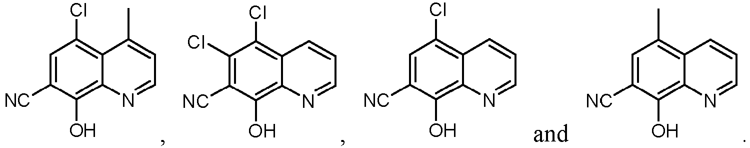

20. The use according to any one of claims 1 to 19, wherein the antiviral drug is used in a mammal, preferably in a human.

21. The use according to any one of claims 1 to 20, wherein the virus is human papillomavirus; preferably one or more of *Human Papillomavirus 6 (HPV6), Human Papillomavirus 11* (*HPV11*), *Human Papillomavirus 16* (*HPV16*) and *Human Papillomavirus 18 (HPV18);* and more preferably *Human Papillomavirus 6, Human Papillomavirus 11, Human Papillomavirus 16* or *Human Papillomavirus 18.*

22. The use according to any one of claims 1 to 21, wherein the daily dose of the compound of formula (I) is 0.01 mg to 1000 mg per kilogram of body weight, and preferably 0.1 mg to 500 mg per kilogram of body weight.

23. The use according to any one of claims 1 to 22, wherein the drug further comprises another antiviral agent, and preferably, the antiviral agent is selected from the group consisting of acyclovir, valacyclovir, zidovudine, ganciclovir, penciclovir, famciclovir, foscarnet, ribavirin, lamivudine, amantadine, IFNα, cidofovir and rimantadine.

24. The use according to any one of claims 1 to 23, wherein the drug is in a dosage form as follows: oral liquid, suspension, powder, granule, tablet, capsule, pill, emulsion, syrup, aerosol or injection.

25. The use according to any one of claims 1 to 24, wherein the drug is administrated through an oral, intramuscular, intraperitoneal, intravenous, subcutaneous, intranasal, epidural, sublingual, intranasal, intracerebral, inraventricular, intrathecal, intravaginal, rectal, inhalable or topical route.

26. A method for preventing or treating viral infection, comprising a step of administering a therapeutically effective amount of the 8-hydroxyquinoline derivative, the pharmaceutically acceptable salt thereof, the crystal form thereof, the solvate thereof, the isotope derivative thereof, or the prodrug thereof as defined in the use according to any one of claims 1 to 19, to a subject.

27. A method for preventing or treating viral infection in a mammal, comprising a step of administering a therapeutically effective amount of the 8-hydroxyquinoline derivative, the pharmaceutically acceptable salt thereof, the crystal form thereof, the solvate thereof, the isotope derivative thereof, or the prodrug thereof as defined in the use according to any one of claims 1 to 19, to a subject, wherein the mammal is preferably a human.

28. A method for *in vitro* disinfection, comprising the following step of:
contacting an environment or object to be treated with an effective amount of the 8-hydroxyquinoline derivative, the pharmaceutically acceptable salt thereof, the crystal form thereof, the solvate thereof, or the isotope derivative thereof as defined in the use according to any one of claims 1 to 19.

29. The method according to any one of claims 26 to 28, wherein the virus is human papillomavirus; preferably one or more of *Human Papillomavirus 6* (*HPV6*), *Human Papillomavirus 11* (*HPV11*), *Human Papillomavirus 16* (*HPV16*) and *Human Papillomavirus 18* (*HPV18*); and more preferably *Human Papillomavirus 6* (*HPV6*), *Human Papillomavirus 11* (*HPV11*), *Human Papillomavirus 16* (*HPV16*) or *Human Papillomavirus 18* (*HPV18*).

30. A pharmaceutical composition for treating viral infection, comprising the 8-hydroxyquinoline derivative, the pharmaceutically acceptable salt thereof, the crystal form thereof, the solvate thereof, the isotope derivative thereof, or the prodrug thereof as defined in the use according to any one of claims 1 to 19, and pharmaceutically acceptable excipients.

31. A pharmaceutical composition for treating viral infection in a mammal, comprising the 8-hydroxyquinoline derivative, the pharmaceutically acceptable salt thereof, the crystal form thereof, the solvate thereof, the isotope derivative thereof, or the prodrug thereof as defined in the use according to any one of claims 1 to 19, and pharmaceutically acceptable excipients.

32. The pharmaceutical composition according to claim 30 or 31, wherein the virus is human papillomavirus; preferably one or more of *Human Papillomavirus 6* (*HPV6*), *Human Papillomavirus 11* (*HPV11*), *Human Papillomavirus 16* (*HPV16*) and *Human Papillomavirus 18 (HPV18);* and more preferably *Human Papillomavirus 6* (*HPV6*), *Human Papillomavirus 11* (*HPV11*), *Human Papillomavirus 16* (*HPV16*) or *Human Papillomavirus 18 (HPV18).*
